(19) Europäisches Patentamt
European Patent Office
Office européen des brevets

(11) Publication number: **0 451 700 A1**

(12) **EUROPEAN PATENT APPLICATION**

(21) Application number: **91105332.0**

(51) Int. Cl.[5]: **C12N 15/00**, C12N 15/12

(22) Date of filing: **04.04.91**

(30) Priority: **10.04.90 US 507705**
**20.02.91 US 656248**

(43) Date of publication of application:
**16.10.91 Bulletin 91/42**

(84) Designated Contracting States:
**AT BE CH DE DK ES FR GB GR IT LI LU NL SE**

(71) Applicant: **MILES INC.**
**1127 Myrtle Street**
**Elkhart,IN 46515(US)**

(72) Inventor: **Wirak, Dana O.**
**168 Haverford Street**
**Hamden, CT 06517(US)**
Inventor: **Bayney, Richard**
**96 Strathmore Avenue**
**Milford, CT 06460(US)**
Inventor: **Ramabhadran, Triprayer V.**
**112 Murray Lane**
**Guilford, CT 06437(US)**
Inventor: **Unterbeck, Axel**
**Rommerscheider Höhe 3**
**W-5060 Bergisch-Gladbach 2(DE)**
Inventor: **Rae, Peter**
**Am Oberen Werth 27**
**W-4000 Düsseldorf(DE)**
Inventor: **Scangos, George**
**Waldsee Strasse**
**W-4030 Ratingen(DE)**

(74) Representative: **Dänner, Klaus, Dr. et al**
**c/o Bayer AG Konzernverwaltung RP**
**Patentabteilung**
**W-5090 Leverkusen 1 Bayerwerk(DE)**

(54) **Recombinant APP minigenes for expression in transgenic mice as models for Alzheimers's disease.**

(57) Functional recombinant APP minigene constructs and their introduction into the germline of transgenic mice. Such transgenic mice are useful to generate models of Alzheimer's disease and pathogenesis and are also useful to identify molecular mechanisms of the pathogenesis of Alzheimer's disease.

EP 0 451 700 A1

## BACKGROUNDOF THE INVENTION

This invention relates to recombinant gene constructs, minigene constructs, and transgenic mice for phenotypic expression of Alzheimer-like pathology. The invention further relates to transgenic animal models for Alzheimer's disease. In particular, the present invention provides a variety of minigene constructs which include all or portions of the coding sequences of the amyloid precursor proteins, and which can be expressed in a cell and tissue in a specific manner in transgenic mice carrying the minigene constructs.

Alzheimer's disease (AD) is the most common single cause of dementia in late life. Individuals with AD are characterized by progressive memory impairments, loss of language and visuospatial skills and behavior deficits (McKhann et al., 1986, Neurology 34: 939-944). The cognitive impairment of individuals with AD is the result of degeneration of neuronal cells located in the cerebral cortex, hippocampus, basal forebrain and other brain regions (for reviews, see Kemper, in Clin. Neurol. Aging, M.L. Albert, ed., pp. 9-52, Oxford University Press, New York, 1984; Price, 1986, Annu. Rev. Neurosci. 9: 489-512). Histologic analyses of AD brains obtained at autopsy demonstrated the presence of neurofibrillary tangles (NFT) in perikarya and axons of degenerating neurons, extracellular neuritic (senile) plaques, and amyloid plaques inside and around some blood vessels of affected brain regions (Alzheimer, 1907, Allg. Z. Psychiat. u. Psych. Gerichtl. Med. 64: 146-148). Neurofibrillary tangles are abnormal filamentous structures containing fibers (about 10 nm in diameter) that are paired in a helical fashion, therefore also called paired helical filaments (Kidd, 1963, Nature 197: 192-193; Wisniewski et al., 1976, J. Neurol. Sci. 27: 173-181; Selkoe et al., 1982, Science 215: 1243-1245; Brion et al., 1985, J. Submicrosc. Cytol. 17: 89-96; Grundke-Iqbal et al., 1986, J. Biol. Chem. 261: 6084-6089; Wood et al., 1986, Proc. Natl. Acad. Sci. USA 83: 4040-4043; Kosik et al., 1986, Proc. Natl. Acad. Sci. USA 83: 4044-4048; Goedert et al., 1988, Proc. Natl. Acad. Sci. USA 85: 4051-4055; Wischik et al., 1988a, Proc. Natl. Acad. Sci. USA 85: 4884-4888; Wischik et al., 1988b, Proc. Natl. Acad. Sci. USA 85: 4506-4510). Neuritic plaques are located at degenerating nerve terminals (both axonal and dendritic), and contain a core composed of amyloid protein fibers (Masters et al., 1985a, EMBO J. 4: 2757-2763; Masters et al., 1985b, Proc. Natl. Acad. Sci. USA 82: 4245-4249). Cerebrovascular amyloid protein material is found in blood vessels in the meninges and the cerebral cortex (Glenner and Wong, 1984a, Biochem. Biophys. Res. Commun. 120: 885-890; Glenner and Wong, 1984b, Biochem. Biophys. Res. Commun. 122: 1131-1135; Wong et al., 1985, Proc. Natl. Acad. Sci. USA 82: 8729-8732).

During the past several years, primary pathological markers associated with AD have been characterized. The biochemical analyses of three forms of Alzheimer brain lesions (for reviews, see Kemper, supra; Wurtman, 1985, Sci. Amer. 252: 62-74; Katzman, 1986, N. Engl. J. Med. 314: 964-973; Price, 1986, supra; Selkoe, 1989, Ann. Rev. Neurosci. 12: 463-490; Muller-Hill and Beyreuther, 1989, Ann. Rev. Biochem. 58: 287-307), tangles, neuritic plaques, and cerebrovascular plaques, has revealed protein sequence information, and has facilitated subsequent cDNA cloning and chromosomal mapping of some of the corresponding genes. Immunological studies have identified several candidates for protein constituents of the paired helical filaments (PHF), including microtubule-associated protein 2 (MAP-2), tau, ubiquitin and the amyloid protein (A4). Degenerating nerve cells express specific antigens such as A68, a 68 kDa protein. This abnormal antigen is detectable with the monoclonal antibody ALZ-50 (Wolozin et al., 1986, Science 232: 648-650; Wolozin et al., 1987, Ann. Neurol. 22: 521-526; Wolozin et al., 1988, Proc. Natl. Acad. Sci. USA 85: 6202-6206).

A central feature of the pathology of AD is the deposition of amyloid protein within plaques. The 4 kDa amyloid protein (also referred to as A4 (APC, $\beta$-amyloid or BAP) is a truncated form of the larger amyloid precursor protein (APP) which is encoded by a gene localized on chromosome 21 (Goldgaber et al., 1987, Science 235: 877-880; Kang et al., 1987, Nature 325: 733-736; Jenkins et al., 1988, Biochem. Biophys. Res. Commun. 151: 1-8; Tanzi et al., 1987, Science 235: 880-885). Genetic linkage analysis, using DNA probes that detect restriction fragment-length polymorphisms (RFLPs, Botstein et al., 1980, Am. J. Hum. Genet. 32: 314-331), has resulted in the localization of a candidate gene (FAD, familial AD) on human chromosome 21 in families with high frequencies of AD (St. George-Hyslop et al., 1987a, Science 235: 885-890). However, the FAD locus has not been localized precisely, and very little is known about its function. Initial studies of individuals with Down syndrome (DS), caused by trisomy of part or all of chromosome 21, indicate that these individuals develop Alzheimer-like pathology beyond the second decade of life. However, analysis of multiple Alzheimer pedigrees revealed that the APP gene does not segregate with familial AD (Van Broeckhoven et al., 1987, Nature 328: 153-155; Tanzi et al., 1987, Nature 329: 156-157). Furthermore, two recent studies with new families demonstrated the absence of a linkage of chromosome 21 markers to familial AD (Schellenberg et al., 1988, Science 241: 1507-1510; Roses et al., 1988, Neurology 38: 173).

Age, genetic elements, and possibly environmental factors appear to contribute to cellular pathology of AD. A fundamental but unanswered question in the pathogenesis of AD is the relationship between abnormalities of neurons and the deposition of amyloid. Specifically, the cellular origin of pathological events leading to the deposition of amyloid fibrils adjacent to some areas of the blood-brain barrier (cerebrovascular amyloid) and in the proximity of nerve terminals (neuritic plaques) in specific brain regions as well as extracellular amyloid in plaque cores is not known. Glenner and Wong have described the purification and characterization of meningeal amyloid from both brains of individuals with AD (Glenner and Wong, 1984a, supra) or DS (Glenner and Wong, 1984b, supra) and determined the N-terminal peptide sequences. Among 24 residues analyzed, the two amyloid peptides showed only one difference, namely, at amino acid position 11 (glutamine in AD amyloid versus glutamic acid in DS amyloid) among 24 residues analyzed. Subsequent studies of amyloid from Alzheimer brain plaque cores revealed amino acid sequences identical to the reported DS cerebrovascular amyloid data (Masters et al. 1985b, Proc. Natl. Acad. Sci. USA 82: 4245-4249). Copy-DNA analysis of APP transcripts from both normal tissue and Alzheimer brain material demonstrated the presence of the codon for glutamic acid at this position (Kang et al., 1987, supra; Goldgaber et al., 1987, supra; Robakis et al., 1987, Lancet: 384-385; Tanzi et al., 1987, Science 235: 880-884; Zain et al., 1988, Proc. Natl. Acad. Sci. USA 85: 929-933; Vitek et al., 1988, Mol. Brain Res. 4: 121-131).

The availability of protein sequence information from the amyloid protein in Alzheimer brains enabled the design of synthetic oligonucleotides complementary to the putative messenger RNA transcripts. Four groups independently reported successful cloning of cDNAs including the region of the amyloid protein sequence (Goldgaber et al., 1987, supra; Kang et al., 1987, supra; Robakis et al., supra; Tanzi et al., 1987, supra). One group (Kang et al.) cloned the apparent full-length transcript (approximately 3.4 kb) for APP from a human fetal brain cDNA library. The 695-residue amyloid precursor protein (APP-695) shows typical features of a glycosylated cell-surface transmembrane protein. The C-terminal 12 to 14 residues of the A4 protein reside in the putative transmembrane domain of the precursor and 28 N-terminal residues are in the "extracellular domain" (Dyrks et al., 1988, EMBO J. 7: 949-957). Genomic mapping localized the APP gene on human chromosome 21 using human/rodent somatic cell hybrids (Goldgaber et al., 1987, supra; Kang et al., 1987, supra; Tanzi et al., 1987, supra). Applying in situ hybridization techniques, this gene was sublocalized to chromosome 21q21 (Robakis et al., supra) and more recently at the border of 21q21-22 (Blanquet et al., 1987, Ann. Genet. 30: 68-69; Patterson et al., 1988, Proc. Natl. Acad. Sci. USA 85: 8266-8270).

Chromosome 21 has been the subject of intensive studies because of its involvement in DS (trisomy 21). While 95% of individuals with DS are trisomic for the entire chromosome 21, 2-3% are mosaics, i.e., trisomic in only some cells, and 3-4% are caused by triplication (translocation) of the distal part of the long arm (21q22) of chromosome 21 (Crome and Stern, 1972, Pathology of Mental Retardation, Churchill Livingstone, Edinburgh). The occurrence of such translocations has led to the conclusion that DS can be attributed to trisomy of the distal part (the "pathological region") of chromosome 21 (Summitt, 1981, in Trisomy 21 (Down Syndrome): Research Prospectives, de la Cruz and Gerald, eds., pp. 225-235, University Park Press, Baltimore). To date, it is not known precisely where the breakpoint on the q arm of chromosome 21 is located, and it is not known whether individuals with DS, who have partial trisomy, develop Alzheimer pathology. In this context, it will be of particular interest to determine if the APP gene maps within the "pathological region" of chromosome 21. The localization of the APP gene on the long arm of chromosome 21, together with the apparent development of AD pathology in individuals with DS, provides a potential mechanism for the formation of amyloid on the basis of over-expression of a number of genes on chromosome 21, including the APP gene and the FAD gene locus. Initial studies of genomic DNA from sporadic (non-familial) AD cases and "karyotypically normal" individuals with DS have implicated the presence of microduplication of a segment of chromosome 21 including the APP gene (Delabar et al., 1987, Science 235: 1390-1392; Schweber et al., 1987, Neurology 37: 222). However, subsequent analyses of large numbers of individuals with AD by several laboratories has not confirmed these findings (Tanzi et al., 1987c, Science 238: 666-669; St. George-Hyslop et al., 1987b, Science 238: 664-666; Podlisny et al., 1987, Science 238: 669-671; Warren et al., 1987, Genomics 1: 307-312).

Chromosomal mapping experiments, using human APP probes in human/rodent cell hybrids, have shown cross-hybridization with mouse and hamster genomic DNA (Kang et al., 1987, supra; Tanzi et al., 1987a, supra; Goldgaber et al., 1987, supra). Southern-blot analysis of DNA from various species has indicated that the APP gene is highly conserved during evolution. Comparison of the mouse APP sequence (Yamada et al., 1987, Biochem. Biophys. Res. Commun. 158: 906-912) with the sequence from rat (Shivers et al., 1988, EMBO J. 7: 1365-1370) shows 99% homology on the protein level; furthermore, the human sequence is 96.8% homologous to the mouse sequence and 97.3% homologous to the rat sequence. Based on the striking conservation of APP proteins, Yamada et al., supra, have calculated the evolutionary rate of changes at the amino acid level to be 0.1 x $10^{-9}$/site/year, which is comparable to that of cytochrome C, and suggests an essential biological function for APP proteins. Recently, K. White and colleagues have cloned a Drosophila gene (vnd locus) which is highly homologous to large regions of the APP sequence. Northern-blot experiments have confirmed these data at the level of mRNA and have demonstrated for various mammalian species the ubiquitous expression of APP transcripts in a number of different tissues (Manning et al., 1988, Brain Res. 427: 293-297).

Kang et al., supra, reported the presence of two distinct bands (~3.2 kb and ~3.4 kb) by Northern-blot analysis of human fetal brain mRNA using APP cDNA as a probe. This finding suggests either differential splicing of mRNA or alternative usage of polyadenylation sites. Both post-transcriptional events were found to be operative following detailed investigation by several groups. First, Kang et al., supra, indicated a potential polyadenylation signal (AATAAA tandem repeat) 259 bp upstream of the 3'-end of the reported APP full-length cDNA. The analysis of eight other full-length APP cDNA clones obtained from a human fetal brain cDNA library (Unterbeck, 1986, Dissertation, University Cologne, FRG) demonstrated in a 1:1 ratio between shorter cDNAs (~3.2 kb) using the first polyadenylation signal versus the original cDNA forms (~3.4 kb) using the second polyadenylation signal. Interestingly, all eight clones encoded for 695 residues of APP. The alternative use of different polyadenylation signals in APP transcripts was confirmed by other laboratories (Goldgaber, 1988, in The Molecular Biology of Alzheimer's Disease, Finch and Davis, eds., pp. 66-70, Cold Spring Harbor Laboratory, Cold Spring Harbor, New York; Johnson et al., 1988, Exp. Neurol. 102: 264-268). A number of groups have screened several tumour cell-line derived cDNA libraries for the presence of APP transcripts and identified clones encoding new APP molecules containing an additional domain. This domain possesses striking homology to the Kunitz family of serine protease inhibitors (Tanzi et al., 1988, Nature 351: 528-530; Ponte et al., 1988, Nature 331: 525-527; Kitaguchi et al., 1988, Nature 331: 530-532). In particular these cDNA sequences contain an additional 167 bp insert at residue 289 of the APP-695 precursor (SEQ ID NO:42/43) (Figure 1) which encodes a 56 amino acid sequence of high sequence of homology to aprotinin (Laskowski and Kato, 1980, Ann. Rev. Biochem. 49: 593-626), a well-characterized inhibitor of "trypsin-like" serine proteases. The peptide sequences flanking this region of insert are identical to the original APP-695 clone, resulting in an open reading frame of 751 residues (APP-751). Kitaguchi et al., supra, isolated a third APP form with another addition of a 19 amino acid domain at the C-terminal end of the 56 amino acid "aprotinin-like" region of APP-751, thus resulting in a larger protein of 770 residues (APP-770). Transient expression of APP-770 in COS-1 cells conferred a marked inhibition of trypsin activity in cell lysates (Kitaguchi et al., supra). Both additional domains have been found to be encoded by discrete exons (Kitaguchi et al., supra) and all three transcripts (APP-695, APP-751, APP-770) are generated by differential splicing of a single gene on chromosome 21. These protease inhibitor domains have also recently been found to be present in mouse (Yamada et al., 1989, Biochem. Biophys. Res. Commun. 158: 906-912) and rat (Kang and Muller-Hill, 1989, Nucleic Acids Res. 17: 2130) species.

The relationship between the three different amyloid precursor forms and the formation of amyloid in AD is not known. In particular, it is not known whether a specific form of APP contributes to A4 deposition. It is possible that either an imbalance in the relative expression levels of the three APP forms or their over-expression might be involved in AD pathology. Initial in situ hybridization analyses using APP cDNA probes in human CNS sections indicated that many neuronal cell types express these mRNAs (Bahmanyar et al., 1987, Science 237: 77-79; Goedert, 1987, EMBO J. 6: 3627-3632; Cohen et al., 1988, Proc. Natl. Acad. Sci. USA 85: 1227-1231; Higgins et al., 1988, Proc. Natl. Acad. Sci. USA 85: 1297-1301; Lewis et al., 1988, Proc. Natl. Acad. Sci. USA 85: 1691-1695; Schmechel et al., 1988, Alzheimer Dis. Assoc. Disord. (US) 2: 96-111), but because of the nature of the probes used, these studies did not allow a differential analysis of the various APP transcripts. Furthermore, there is little documented correlation between APP mRNA levels, amyloid deposition and neuronal degeneration in AD. However, it appears that high levels of APP mRNAs alone do not form a sufficient prerequisite for cellular pathology in either the aging or AD brain (Higgins et al., supra). Specific probes which discriminate between the APP transcripts have been used for Northern analysis and the results suggest a developmental and tissue-specific pattern of expression of these mRNAs (Tanzi et al., 1988, supra; Kitaguchi et al., 1988, supra; Neve et al., 1988, Neuron 1: 669-677).

Recently, 5'-end cDNA probes from full-length APP cDNA clones (Kang et al., 1987, supra), have been used to isolate genomic clones containing the 5'-end of the APP gene, also referred to as precursor of Alzheimer's Disease A4 amyloid protein (PAD) gene (Salbaum et al., 1988, EMBO J. 7: 2807-2813; La Fauci et al., 1989, Biochem. Biophys. Res. Commun. 159: 297-304). Approximately 3.7 kb of sequences upstream of the strongest RNA start site have been analyzed by Salbaum et al., 1988, supra. By a combination of primer extension and S1 protection analyses, five putative transcription initiation sites have been determined within a 10 bp region. This ~3.7 kb region lacks a typical TATA box and displays a 72% GC-rich content in a region (-1 to -400) that confers promoter activity to a reporter gene in an in vivo assay system (Salbaum et al., 1988, supra). The absence of a typical TATA and CAAT box and the presence of multiple RNA start sites is suggestive of its function as a housekeeping gene but does not imply constitutive gene expression (Salbaum et al., 1988, supra). The regulatory region contained within 400 bp upstream of the strongest RNA start site shows a variety of typical promoter-binding elements, including: two AP-1 consensus sites (Lee et al., 1987, Nature 325: 368-372), a single heat shock recognition consensus element (Wu et al., 1987, Science 238: 1247-1253), and several copies of a 9 bp-long GC-rich consensus sequence where sequence-specific binding has been shown to occur by gel-retardation studies (Salbaum et al., 1988, supra). In addition, the CpG:GpC ratio in this promoter region has been found to be 1:1 in contrast to a 1:5 ratio found in many eucaryotic DNAs (Razin and Riggs, 1980, Science 210: 604-610); CpG dinucleotides are known to control gene expression via DNA methylation (Doerfler, 1983, Annu. Rev. Biochem. 52: 93-124). In addition, palindromic sequences capable of forming hairpin-like structures are found around the RNA start sites (La Fauci et al., 1989, supra).

Recently, several groups of investigators have determined the consensus binding sequence (AT rich decamer) for a number of different homeobox proteins (Desplan et al., 1988, Cell 54: 1081-1090; Hoey and Levine, 1988, Nature 322: 858-861; Ko et al., 1988, Cell 55: 135-144; Odenwald et al., 1989, Genes Dev. 1: 482-496), which act most likely as transcription factors in specific regions during embryogenesis (for review, see Gehring, 1987, Science 236: 1245-1252; Holland and Hogan, 1988, Genes Dev. 2: 773-782). As yet, target genes, which might be developmentally regulated by the homeobox proteins have not been identified. Such genes, however, will have an important role during embryogenesis and potentially throughout the entire life span. The APP gene promoter contains at least five homeobox binding sites upstream of the RNA start sites. Preliminary experiments have shown that the homeobox protein Hox-1.3 (Odenwald et al., 1987, Genes Dev. 1: 482-496; Odenwald et al., 1989, Genes Dev. 3: 158-172) can bind at two of these sites. Thus, the APP gene, whose expression is developmentally regulated, appears to be a candidate gene for homeobox protein regulation. It is not known whether any of these putative recognition consensus elements modulate the expression of the APP gene promoter.

Despite all that is known about the APP gene, the primary defect leading to AD is not yet known, and specific mutations in the APP gene or other genes which cause AD in humans have not been defined. With the exception of aged primates (Price et al., 1989, BioEssays 10: 69-74), no laboratory animal model for AD exists. The introduction of genes into the germline of animals is an extremely powerful technique for the generation of disease models which will lead to a better understanding of disease mechanisms (Cuthbertson and Klintworth, 1988, Laboratory Investigation 58: 484-501; Jaenisch, 1988, Science 240: 1468-1474; Rosenfeld et al., 1988, Ann. Rev. Neurosci 11: 353-372), including the mechanisms of AD. Cell culture and in vitro systems cannot duplicate the complex physiological interactions inherent in animal systems. Transgenic animals have been successfully generated from a number of species including mice, sheep, and pigs (Church, 1987, Trends in Biotech. 5: 13-19; Clark et al., 1987, Trends in Biotech. 5: 20-24). The gene or genes of interest are microinjected directly into the pronuclei of a one-cell embryo. A high percentage of reimplanted embryos develop normally and, in a significant proportion of progeny, the transgene becomes integrated into the chromosomal DNA. Usually, multiple copies of the transgene integrate as a head-to-tail array. Although mosaic animals can be generated, germline transmission of the transgene usually occurs (Hogan et al., 1986, in Manipulating the Mouse Embryo; A Laboratory Manual, Cold Spring Harbor Laboratory, Cold Spring Harbor, New York; DePamphilis et al., 1988, BioTechniques 6: 662-680). The generation of a transgenic mouse would be useful in defining the role of APP in the pathology of AD. For example, mice carrying APP transgenes which have been altered in either their protein-coding sequences or in their expression levels, might display dominant mutant phenotypes resembling those displayed in AD pathology. The construction of recombinant genes and minigenes for expression in transgenic mice is a critical step in the development of transgenic mouse models. Particularly critical is the choice of an appropriate gene promoter for the minigene and other regulatory elements for the cell and tissue specific expression of the minigene. A gene promoter must be utilized which will facilitate the expression of recombinant genes with a cell and tissue specificity consistent with the formation of amyloid plaque and perhaps with the expression pattern of the endogenous mouse APP gene.

To date, the identification of essential regulatory elements for many genes has not been straightforward and is, at best, unpredictable. A number of critical factors contribute to the complexity of this problem. Firstly, gene promoters that exert cell specific regulation in DNA transfection experiments do not necessarily confer cell and tissue specificity in transgenic animals. For example, transfection experiments have revealed that important cell specific regulatory elements reside within 400 bp upstream of the cap site of the rat albumin gene (Ott et al., 1984, EMBO J. 3: 2505-2510; Friedman et al., 1986, Mol. Cell Biol. 6: 3791-3797). However, an additional enhancer, located 10 kb upstream from the albumin promoter, was found to be necessary to obtain liver-specific expression in transgenic mice (Pinkert et al., 1987, Genes Dev. 1: 268-276). While promoter sequences of the α-fetoprotein gene confer cell specificity in cell culture (Godbout et al., 1986, Mol. Cell Biol. 6: 477-487; Muglia and Rothman-Denes, 1986, Proc. Natl. Acad. Sci. USA 83: 7653-7657; Widen and Papaconstantinou, 1986, Proc. Natl. Acad. Sci. USA 83: 8196-8200), additional enhancer elements located between -1 kb and -7 kb, were found to be necessary for liver specific expression in transgenic animals (Hammer et al., 1987, Science 235: 53-58). Secondly, the organization of various genes differs considerably and essential regulatory elements have been found in numerous positions. In some cases, the necessary regulatory elements are located within a compact region proximal to the cap site. For example, sequences residing within nucleotide -205 to nucleotide +8 of the rat elastase I gene are sufficient to confer an appropriate expression pattern in transgenic mice (MacDonald et al., 1987, Progress in Brain Research 71: 3-12). A tightly defined regulatory region has also been identified in the human γ-crystallin gene (Goring et al., 1987, Science 235: 456-458). The human β-globin gene, however, has at least four separate regulatory elements: a positive globin specific promoter element, a negative regulatory element, and two gene enhancers, one located within the second intron and the other located 3' of the structural gene (Behringer et al., 1987, Proc. Natl. Acad. Sci. USA 84: 7056-7060; Grosveld et al., 1987, Cell 51: 975-985). Thirdly, in many cases, the site of integration exerts a strong influence on the level and pattern of expression of transgenes. Regions of several genes have been identified which overcome, at least in part, these position effects. DNase I hypersensitive sites located approximately 50 kb 5' to and 20 kb 3' of the β-globin gene facilitate position-independent, high-level expression of a β-globin minigene in transgenic mice (Grosveld et al., 1987, supra). Furthermore, introns of the rat growth hormone and mouse metallothionein genes increase transcriptional efficiency of transgenes on average 10- to 100-fold (Brinster et al., 1988, Proc. Natl. Acad. Sci. USA 85: 836-840). Rat growth hormone intronic sequences exerted a positive effect even on heterologous gene constructions utilizing either the metallothionein or elastase promoters. The effect of these introns is not related to an increased efficiency of RNA processing but is due to an actual increase in the rate of transcription (Brinster et al., 1988, supra). It is also possible that introns and other genomic regions contain sequence elements which are recognized at particular stages of development or may contain elements which influence chromatin structure. In many cases, the inclusion of genomic elements which diminish position effects may be essential for a transgene to maintain an expression level sufficient to generate a phenotype. The identification of these elements may in some cases be a formidable task; for example, the APP gene locus encompasses at least 50 kb (Lemaire et al., 1989, Nucleic Acids Res. 17: 517-522). The identification of such elements would be extremely useful in the construction of recombinant APP minigenes. These minigenes can then be introduced into the germline of transgenic mice, thus providing animal models for AD.

## SUMMARYOF THE INVENTION

The present invention provides recombinant minigenes for the expression of alternative forms of APP, including APP-695, APP-751, APP-770, and a variety of mutant forms of APP. The present invention also provides for the introduction of such functional APP minigene constructs into the germline of mice thereby generating transgenic animal models of AD useful in the identification of the molecular mechanisms of AD pathogenesis. The recombinant minigenes according to the present invention contain essentially five different elements: (1) gene promoter (DNA elements responsible for gene regulation), (2) and (3) APP protein coding region (cDNA or mutated cDNA), (4) mRNA polyadenylation signals, (5) RNA splicing signals and genomic elements required for developmentally appropriate and cell/tissue-specific expression of the APP-encoding DNA. The identification of such genomic elements is highly unpredictable. The location of these sequence elements varies from gene to gene and may be found in the 5' regions, within introns, in 3' regions, or in other locations.

It has now been unexpectedly found that an ~4.6 kb EcoRI human genomic fragment (or portions thereof), comprising ~2.8 kb 5' to the APP in RNA start site, the first exon of the APP gene and ~1.6 kb of the first intron, is sufficient to direct cell and tissue-specific expression of a reporter gene in transgenic mice, and in a manner consistent with the expression pattern of the endogenous mouse APP gene. This genomic fragment contains a promoter and perhaps other regulatory elements that facilitate the expression of recombinant APP minigenes with a cell and tissue specificity consistent with the formation of amyloid plaque and the expression patterns of the APP gene. Since the primary defect leading to AD has not yet been determined, and specific mutations which cause AD in humans have not been identified, transgenic mice with recombinant APP minigenes according to the present invention provide animal models for the disease. For example, the generation of transgenic animal models for A4 amyloidosis is essential for defining the role of A4 in the pathogenesis of AD. Transgenic mice, according to the present invention, carrying APP genes altered in their protein-coding sequences or in their expression levels, provide models for exhibition of dominant mutant phenotypes resembling some aspects of AD pathology. As Alzheimer's pathology is restricted to specific regions of the brain, only those minigene constructs with the appropriate cell and tissue-specific genomic regulatory elements, such as those provided by the present invention, will enable for the development of transgenic mouse models of AD.

## BRIEFDESCRIPTION OF THE DRAWINGS

Figure 1 is the cDNA sequence (SEQ ID NO:42) of the amyloid precursor protein (APP) (SEQ ID NO:43) cloned in pFC4.

Figure 2 is a circular map of pFC4.

Figure 3 is an illustration of the 5'-end of the APP gene.

Figure 4a is an illustration of gene products of nonmutated forms of APP encoded in APP minigenes.

Figure 4b is an illustration of gene products of mutated forms of APP encoded in APP minigenes.

Figure 5 is an illustration of the construction intermediates and products: pMTI-2302, pMTI-2303, pMTI-2305 and pMTI-2304.

Figure 6a is an illustration of polylinkers in cloning vectors: pWB16, pMTI-2110, pMTI-2300 and pMTI-2301.

Figure 6b is a circular map of pMTI-2301.

Figure 7a is an illustration of construction intermediates pMTI-2306, pMTI-2307, pMTI-2311 and pMTI-2312 and minigene pMTI-2314.

Figure 7b is a circular map of pMTI-2307.

Figure 7c is a circular map of pMTI-2312.

Figure 8a is an illustration of minigene constructs pMTI-2310, pMTI-2314, pMTI-2319, pMTI-21320, pMTI-2321, pMTI-2322, and pMTI-2325, encoding alternate forms of APP.

Figure 8b is a circular map of pMTI-2314.

Figure 9 is an illustration of construction intermediates pMTI-2307, pMTI-2316, and pMTI-2317 and minigene pMTI-2318.

Figure 10a is an illustration of construction intermediates pMTI-2312 and mouse metallothionein-I genomic sequences and minigenes pMTI-2323, pMTI-2331, pMTI-2332, pMTI-2324, and pMTI-2326.

Figure 10b is a circular map of pMTI-2323.

Figure 11a is an illustration of construction intermediate pMTI-2323 and minigenes pMTI-2327 and pMTI-2337.

Figure 11b is a circular map of pMTI-2337.

Figure 12 shows the DNA (SEQ ID NO:44)/amino acid (SEQ ID NO:45) sequence of sp-spacer A4 and MC-100.

Figure 13 shows the DNA (SEQ ID NO:46)/amino acid (SEQ ID NO:47) sequence of sp-spacer A4 and SP-A4.

Figure 14 is an illustration of construction intermediate pMTI-2328, a pFC4 fragment, and minigenes pMTI-2329, pMTI-2333, pMTI-2334, pMTI-2335 and pMTI-2336.

Figure 15a is an illustration of APP 3'-end genomic clone pSV1 and minigene pMTI-2339.

Figure 15b is a circular map of pMTI-2339.

Figure 16 is the DNA (SEQ ID NO:48) sequence of the 3'-end of the APP gene.

Figure 17 is a circular map of pNotSV2neo.

Figure 18a is an illustration of pNotSV2neo subclones for minigenes pMTI-2360, pMTI-2361, pMTI-2362, pMTI-2363, pMTI-2364, pMTI-2365, pMTI-2366, pMTI-2367, pMTI-2368, and pMTI-2369.

Figure 18b is a circular map of pMTI-2360.

Figure 19 is an illustration of pNotSV2neo and minigenes pMTI-2339, pMTI-2369, pMTI-2342, pMTI-2343 and pMTI-2344.

Figure 20 is an illustration of the APP-lacZ reporter gene pMTI-2402.

Figure 21(a-d) illustrates the cellular distribution of APP mRNA in normal mouse detected by in situ hybridization with labeled single-stranded human APP DNA probe. (a) Section of mouse cerebral cortex. (b) Section of mouse cerebellar cortex. (c) Section of mouse trigeminal ganglia. (d) Section of mouse liver.

Figure 22 illustrates the histochemical staining pattern of E. coli $\beta$-galactosidase activity in brain section of a BE803 transgenic mouse.

Figures 23(a-d) illustrates the histochemical staining pattern of E. coli $\beta$-galactosidase activity in serial brain sections of a BE803 transgenic mouse (a, b and c) and in a section of a normal mouse brain (d).

Figure 24(a-d) illustrates the histochemical staining pattern of E. coli $\beta$-galactosidase activity in sections of cerebellar cortex (a and b), trigeminal ganglion (c), and liver (d) from transgenic BE803 mouse.

Figures 25(a-d) illustrates the cellular and subcellular distribution of E. coli β-galactosidase in transgenic BE803 mouse brain. Light microscopic image of histochemical staining pattern of E. coli β-galactosidase activity in cerebral cortex (a). Normarksi optic image of histochemical staining pattern of E. coli β-galactosidase activity in cerebral cortex (b and c). Immunogold localization of E. coli β-galactosidase in cerebral cortex section from a BE803 transgenic mouse.

Figure 26 shows an S1 analysis of human APP RNA expression in the brain of a series of transgenic mice.

Figure 27 shows an S1 analysis of human APP RNA expression in the brain of a second series of transgenic mice.

Figure 28a is a Western-blot of APP-695, APP-751 and APP-770 protein expression in the brain of a normal mouse and transgenic mice carrying human APP minigenes using monoclonal antibody (mAb) 22C-II.

Figure 28b is a Western-blot of human APP-751 protein expression in the brain of a normal mouse and transgenic mice carrying human APP minigenes using mAb 56-1.

Figure 29 is a Western-blot (using mAb 22C-11) of APP-695, APP-751 and APP-770 protein expression in COS cells transfected with human APP minigenes.

Figure 30a illustrates circular maps of pMTI-4 and pMTI-38.

Figure 30b illustrates circular maps of KS Bluescript, pMTI-41, pMTI-43 & 44 and pMTI-42.

Figure 31 is an illustration of construction intermediates and products pMTI-52 to pMTI-53, pMTI-57 and pMTI-58.

Figure 32 illustrates reactivities of Kunitz monoclonal antibodies (56-1, 56-2, 56-3) with APP proteins.

Figure 33 illustrates the primate specificity of monoclonal antibody 56-1.

Figure 34 shows the immunocytochemical staining of a brain tissue section from transgenic mouse AE301 + 207(F1) using rabbit polyclonal antibody pAb 90-29.

Figure 35 shows the immunocytochemical staining of a brain tissue section from transgenic mouse AE301 + 207(F1) using rabbit polyclonal antibody pAb 90-29 (higher magnification of similar field described in Figure 34).

Figure 36 shows the immunocytochemical staining of a brain tissue section from transgenic mouse AE301 + 207(F1) using rabbit polyclonal antibody pAb 90-28 (magnification similar to that described in Figure 35).

Figure 37 shows the immunocytochemical staining of a brain tissue section from transgenic mouse FE803 + 105(F1) using rabbit polyclonal antibody pAb 90-29 (magnification similar to that described in Figure 35).

Figure 38a is an electron micrograph of a thin section of brain tissue from the hippocampal region of transgenic mouse AE301 + 201(F2).

Figure 38b is an electron micrograph of a thin section of brain tissue from the hippocampal region of transgenic mouse AE301 + 201(F2) (different field than described in Figure 38a).

Figure 39 is an electron micrograph of immunogold staining of ultrathin cryosections of brain tissue from the hippocampal region of transgenic mouse AE301 + 201(F2) using rabbit polyclonal antibody (pAb) 90-29.

Figure 40 is a circular map of pMTI-70.

Figure 41 is a circular map of pMTI-2371.

Figure 42 is a Western-blot, using pAb SG369, of MC-100 protein expression in pMTI-70 transfected cell lines cMTI70-B1, cMTI70-B2, and cMTI70-B3, and cell lines cMTI52-A4, cMTI66-B6, cMTI66-C5, cMTI69-C6, cMTI69-A4, and cMTI69-A5 which are included as negative controls.

Figure 43 is a Western-blot, using pAb SG369, of MC-100 protein expression induced with cadmium in transfected cell lines cMTI70-A2, cMTI70-A3, cMTI70-A6, cMTI70-B1, cMTI70-B2, and cMTI70-B3 (transfected with pMTI-70). Cell lines cMTI63-B1, cMTI63-C2, and cMTI53-A1 are included as negative controls.

Figure 44a shows the immunofluorescence, using pAb SG369, of MC-100 protein expression in transfected cell line cMTI70-A6.

Figure 44b shows the immunofluorescence, using pAb SG369, of MC-100 protein expression in transfected cell line cMTI70-A6 (higher magnification, different field than described in Figure 44a).

Figure 44c shows the immunofluorescence, using pAb SG369, of control transfected cell line pMTI53-A1 (same magnification as described in Figure 44a).

Figure 45 is a Western-blot of immunoprecipitated (using pAb SC369) MC-100 protein from cadmium-induced, pMTI-70 transfected cell line cMTI70-A6.

Figure 46 demonstrates human APP RNA expression, using riboprobe analysis, in the brain of a series of transgenic mice.

## DESCRIPTIONOF THE PREFERRED EMBODIMENT

AD (Alzheimer, 1907, supra) is characterized by a widespread functional disturbance of the human brain. Fibrillar amyloid proteins are deposited inside neurons as neurofibrillary tangles (Katzman, 1983, supra) and extracellularly both as amyloid plaque cores (Katzman, 1983, supra) and as cerebrovascular amyloid, (Katzman, 1983, supra). The major protein subunit (A4) of the amyloid fibril of plaques, blood vessel deposits, and potentially of tangles is an insoluble, highly aggregating 40-42 residue peptide of relative molecular mass 4,500 (Masters et al., 1985, supra and 1985, supra; and Glenner and Wong, 1984, supra). The A4 peptide which derives from a larger amyloid precursor protein is encoded by a gene on chromosome 21 (Kang et al., 1987, supra; Goldgaber et al., 1987, supra; Tanzi et al., 1987, supra). APP mRNAs are detected in neurons and in other tissues both within and outside the brain (Goedert, 1987, supra; Cohen et al., 1988, supra; Higgins et al., 1988, supra).

Age, genetic elements, and, potentially, environmental factors appear to contribute to cellular pathology in AD, but mechanisms that lead to these brain lesions are not yet understood. A fundamental question in the pathogenesis of AD is the relationship between the observed neuronal abnormalities and the deposition of amyloid.

Because the primary defect leading to AD is not yet known, and specific mutations which cause AD in humans have not been defined, animal models for the study of AD would be especially useful. With the exception of aged primates, no laboratory animal model for AD exists. Due to these limitations, the generation of transgenic mouse models for AD may be the best approach in defining the role APP plays in the etiology of AD. Transgenic mice carrying APP genes which have been altered either in their protein-coding sequences or in their expression levels may lead to dominant mutant phenotypes resembling those displayed by the AD pathology. The introduction of functional minigene constructs described herein into the germline of mice has been used to generate models of AD and to identify the molecular mechanisms of pathogenesis.

A critical step for the development of a transgenic mouse model for AD was the design of a minigene that allows high-level expression of a foreign gene in a predictable tissue-specific fashion. Recombinant minigenes according to the present invention contain essentially five different elements: gene promoter (DNA elements responsible for gene regulation), protein coding region (cDNA), mRNA polyadenylation signals, RNA splicing signals, and genomic elements required for correct developmental expression of DNA that has participated in a developmental program (the location of these sequence elements can vary from one gene to another and can be found within introns, 3' regions, and in other locations).

The following paragraphs and examples describe essential steps leading to the design and construction of such minigenes for the generation of animal models for AD. A gene promoter has been isolated and characterized which in transgenic animals confers an expression pattern of foreign genes that is comparable with the pattern of expression of the endogenous mouse APP gene. A series of minigenes comprising the APP gene promoter and a variety of different APP gene products including mutant forms have been generated. Transgenic animals, expressing these minigenes, are useful in the investigation of the in vivo function of various APP gene products, the regulation and expression patterns of the APP gene, and the relationships of these processes to the formation of amyloid. The use of various RNA splicing and polyadenylation signals in the minigenes allows for the optimization of post-transcription processing and stability of human APP transcripts in transgenic animals.

Appropriate recombinant minigenes were generated and tested. The minigenes were microinjected directly into the male pronucleus of mouse 1-cell embryos. The manipulated embryos were subsequently transferred to the oviducts of pseudopregnant females. Litters from recipients were screened for the presence of the transferred minigene (transgene) in their genome by polymerase chain reaction (PCR) analysis and Southern-blot analysis of DNA derived from tail biopsies.

Because Alzheimer's pathology is restricted to specific regions of the brain (Price, 1986, supra), the choice of an appropriate gene promoter for minigene constructions was critical for the development of the transgenic mouse model. A regulatory element comprising a gene promoter and perhaps other regulatory sequences must be utilized which will facilitate the expression of recombinant genes with a cell and tissue specificity consistent with the formation of amyloid plaque and the expression patterns of the APP gene. The present invention provides such a regulatory element.

An ~4.5 kb genomic fragment described herein encompassing the 5'-end of the human APP gene (Figure 3) had sufficient sequence information to direct cell and tissue-specific expression of the protein product of a reporter gene, E. coli lacZ, in transgenic mice (Figures 20 to 25). The expression pattern of the reporter gene product β-galactosidase in the central nervous system (CNS) was strikingly consistent with the expression pattern of the endogenous mouse APP gene and is consistent with the pattern of senile plaque deposition characteristic of AD patients. In situ hybridizations, using human APP cDNA as probe, revealed APP mRNA expression in specific brain regions, including: hippocampus, dentate gyrus, cerebral cortex, cerebellar cortex, pons, and spinal cord. β-galactosidase staining, in transgenic brain tissue, was restricted to areas containing neuronal perikarya. In most cases, the β-galactosidase staining in the CNS of BE803 transgenic mice was consistent with in situ hybridization patterns of mouse APP mRNA. One exception was the CA3 region of the hippocampus where the β-galactosidase staining was not as intense as would be expected from the observed levels of mouse APP mRNA. This difference may have been due to a lowered expression level of the reporter gene in this region or due to altered stability of the β-galactosidase fusion protein. The majority of β-galactosidase fusion protein was localized in secondary lysosomes within neuronal perikarya, therefore, E. coli β-galactosidase fusion protein may be relatively unstable in neurons.

A variety of cDNAs encoding various forms of APP and mutants of APP were constructed. Three alternate forms of APP exist, designated APP-695, APP-770 and APP-751, all of which are encoded by a single common gene on human chromosome 21. The mRNA of the APP gene is differentially spliced to yield three gene products of 695 amino acids (aa), 751 aa, and 770 aa in length. The 751 aa and 770 aa forms contain an additional domain which has striking homology to a Kunitz type serine protease inhibitor (Kitaguchi et al., 1988, Nature 331: 530-532; Ponte et al., 1988, Nature 331: 525-527; Tanzi et al., 1988, Nature 331: 528-530). Expression of one or more forms of the human APP gene products in transgenic mice provides a model with which to test the hypothesis that over-expression or anomalous expression of one or more forms of the gene results in Alzheimer's pathology. This hypothesis is not inconsistent with the observation that Alzheimer's pathology (i.e., A4 plaques in brain tissue) has been found in individuals with DS past the age of 30-40 (Glenner and Wong, 1984, Biochem. Biophys. Res. Commun. 122: 1131-1135). Such individuals are trisomic for chromosome 21 which contains the APP gene, and the levels of APP mRNA in these individuals appears to be elevated (Tanzi et al., 1988, Science 235: 880-885).

Human APP cDNA clones corresponding to the three alternative APP forms were isolated. Plasmid pFC4 contains the full-length cDNA (SEQ ID NO:42) for the 695 aa (SEQ ID NO:43) form of APP (Kang et al., supra). Using pFC4 as a probe, a human neuroblastoma cDNA library was screened for the presence of additional transcripts corresponding to additional forms of human APP. An ~1.8 kb cDNA was identified which contained both additional exons found in APP-770 (167 bp plus 58 bp), and represented a partial cDNA of the mRNA. Unique restriction sites (AccI and BglII) were used to subclone this 1775 bp fragment into the original pFC4 full-length clone, thus generating a full-length cDNA clone (pFC4-770) for the 770 aa form of APP. The 751 residue APP encoding cDNA clone (pFC4-751) was engineered by in vitro mutagenesis of pFC4-770. The deletion of 58 bp (M13-looping-out) was confirmed by DNA sequence analysis.

Using the various APP cDNAs, minigenes expressing each APP form were constructed: pMTI-2314 for APP-695 expression, pMTI-2319 for APP-770 expression and pMTI-2320 for APP-751 expression. As an initial step in the construction of an APP-695 minigene, the EcoRI promoter fragment of APP was inserted into the HindIII site of pMTI-2301 by blunt-end ligation to produce pMTI-2307. The construction of the APP-695 minigene was completed in a stepwise fashion. pMTI-2311 was generated by ligating the BamHI fragment from pFC4 into the BamHI site of pMTI-2307. Next, the XhoI fragment from pFC4 was inserted into the XhoI site of pMTI-2311 to generate pMTI-2312. Finally, a SphI fragment of pMTI-2304 containing the SV40 RNA splicing and polyadenylation signals was inserted at the SphI site of pMTI-2312 to yield pMTI-2314. The APP-751 and APP-770 minigenes were constructed by subcloning the AccI-BglII fragments from pFC4-751 and pFC4-770 into the AccI/BglII sites of pMTI-2314 to generate pMTI-2320 and pMTI-2319, respectively.

A second minigene series for expression of APP-695, APP-770 and APP-751 can be ccnstructed using a truncated APP promoter. For minigene, pMTI-2310 (APP-695), the ~2.6 kb HindIII fragment of the 5'-end of the APP gene [Figure 3] was inserted into the HindIII site of the pMTI-2301 to generate pMTI-2306 (Figure 7a). The minigene expressing the 695 form of APP, pMTI-2310, was constructed in the same manner as pMTI-2314 described above (Figure 7a). The corresponding 751 and 770 minigenes can be generated as described above using the ~1.8 kb AccI-BglII restriction fragment.

The accumulation of A4 peptide in amyloid plaques may be the result of anomalous proteolytic degradation of one or more APP forms (695, 751, 770). Minigenes have been constructed which can directly express either the A4 peptide or other fragments of APP that may exist as proteolytic intermediates during in vivo generation of A4. Such APP fragments may, if they contain the A4 region, self-aggregate, and be further processed by the cell to alternately generate A4. The types of minigene which were constructed and which express such mutants are summarized in Figure 4b. Gene product IV is devoid of a portion of the transmembrane domain and the entire cytoplasmic domain, leaving the A4 domain intact. This mutant gene product is expected to be secreted from the cell and perhaps further degraded to produce the A4 peptide. The secreted protein may also have other biological effects because at least some portion of APP has been shown to be shed from cell surfaces. Gene product V (designated as MC-100) is translated into the membrane and, therefore, a precursor protein was constructed which contains the 17-residue signal peptide of APP at the N-terminus. If the signal peptide is omitted, the C-100 protein (gene product VI) would be translated into the cytoplasm and perhaps have significantly different properties than if inserted into a membrane. Gene product VIII in which the signal peptide is also omitted should produce intracellular A4 directly, and which will not be inserted into a membrane. Another construct also expressing the A4 peptide including the APP signal peptide (gene product VII) was prepared. After the signal peptide cleavage point, gene product VII includes 40 amino acids encompassing the A4 peptide as well as 12 additional amino acids N-terminal of the A4 peptide region. This protein is expected to translocate through the cellular membrane and aggregate following proteolytic cleavage by the cell to generate A4.

To construct mutant APP minigenes for expression of truncated APP product, gene product IV and VII mutants, C-terminal frameshift mutations were generated. Frameshift mutations (-1, +2) of the cDNA sequences immediately following the A4 coding region brought a translation stop codon into the reading frame following the A4 peptide coding region. The resulting sequence encodes a truncated APP species (Figure 4b, gene product IV). A frameshift mutation (deleting nucleotide C) at the nucleotide position 2045 generated a stop codon after 40 amino acids from the N-terminus of the A4 sequence (amino acids 38, 39 and 40 are different than the native A4 sequence), and a +2 mutation (TG) after nucleotide position 2050 generated a stop codon after 41 amino acids from N-terminus of the A4 sequence (the last amino acid is different than the native A4 sequence). The +2 mutation was utilized in construct pMTI-2321 (Figure 8a). The generation of these frameshift mutations is described in co-assigned patent application U.S. Serial No. 194,053. A third frameshift mutation, "mutant 40-1," deleted an adenosine nucleotide at nucleotide 2055 (APP-695 cDNA sequence; Figure 15) and brought a translation stop codon into the reading frame directly following the 40th codon of the A4 peptide coding region (used in plasmids pMTI-2322, pMTI-2326, pMTI-2341, pMTI-2343, pMTI-2361). The frameshift mutations were inserted into pMTI-2314 (APP-695), pMTI-2319 (APP-770), or pMTI-2320 (APP-751) by swapping sequence domains between the unique BglII and ClaI restriction sites (Figure 8a). The deletion mutation was also generated by site-directed mutagenesis which placed the stop codon directly past the A4 sequences (pMTI-26). The mutation in pMTI-26 was inserted into the minigenes in a similar manner as described above.

To construct mutant APP minigenes for expression of gene product VIII mutant, the following steps were taken. Minigene pMTI-2318 (gene product VIII; Figure 4b) was generated in stepwise fashion (Figure 9). A BglII-BamHI fragment from pMTI-26 containing the 42 aa A4 peptide sequence was inserted into the BamHI site of pMTI-2307. Next, the BamHI to BamHI fragment of pFC4 was inserted into the BamHI site of pMTI-2316. Finally, the SphI fragment containing the SV40 RNA processing signal was inserted into the SphI site of pMTI-2317.

Because of substantial sequence homology between mouse and human APP gene products, it has been difficult to generate adequate antibodies that will allow unequivocal identification of APP using immunohistochemical analysis of tissue sections. To circumvent this problem, a highly antigenic epitope of Chlamydia was inserted into the APP-695 sequences at either the site of the Kunitz inhibitor domain insertion or the extreme C-terminus of the protein. The sequences were transferred into the minigenes using either the AccI and BglII restriction sites to generate pMTI-2325 (Figure 8a) or pMTI-2324 (Figure 10a).

In another series of minigene constructs, alternate RNA processing signals were used. Because minigenes utilizing RNA processing signals derived from the human APP gene or from an exogenous mouse gene might be expressed more efficiently in transgenic mice than those derived from SV40, constructs were generated which utilize RNA splicing and polyadenylation sequences of the mouse metallothionein gene. Alternatively, a genomic fragment from the 3'-end of the human APP gene which encompassed the APP polyadenylation signals was utilized. Minigenes expressing all of the gene products described above and additional forms were generated using the alternate RNA processing signals as follows.

Using the metallothionein gene body (Figure 10a) as a source of RNA processing signals, minigenes expressing the three alternate forms of APP (695, 770, 751) and mutant APP forms described above were constructed. To generate minigene pMTI-2323 for expression APP-695, the ~2.2 kb BglII to EcoRI fragment from the EcoRI genomic clone of the mouse metallothionein-I gene, pJYMMT(L), was inserted into the ClaI site of pMTI-2312 by blunt-end ligation to generate pMTI-2323. Minigenes expressing alternates APP forms, APP-770 and APP-751, were generated by switching sequence domains (AccI to BglII fragment) from

minigenes (expressing APP-751 or APP-770) utilizing the SV40 polyadenylation sequence (Figure 8a) to pMTI-2323 (Figure 10) to generated pMTI-2331 and pMTI-2332. To generate a minigene pMTI-2326 expressing an APP C-terminal frameshift mutant, the BglII to ClaI fragment from pMTI-2322 (Figure 8a), containing mutation 40-I, was switched with sequences between BglII to ClaI site of pMTI-2312 (Figure 7a) to generated pMTI-2326a. Then, the ~2.2 kb metallothionein fragment (BglII to EcoRI fragment, see Figure 10a) was inserted into the ClaI site of pMTI-2326a to generate pMTI-2326. Alternatively, the BglII to SpeI fragment of pMTI-2322 was swapped directly into pMTI-2323 to produce pMTI-2326 (Figure 10a).

To generate minigene pMTI-2327 coding for C-100 (gene product VI, Figure 4b), the sequences between the NruI and BglII restriction sites of pMTI-2323 were deleted (Figure 11a). A translation initiation codon, ATG, directly precedes the first codon of the A4 sequences. To generate minigene pMTI-2337 coding for MC-100, the sequences between the KpnI and BglII sites of pMTI-2323 (Figure 11a) were deleted and the clone was ligated using synthetic oligonucleotide adaptor, sp-spacer-A4 (SEQ ID NO:44) (Figure 12). MC-100 (gene product V, Figure 4b) required the 17 residue signal peptide of APP to direct insertion of the translated mutant protein into the membrane. The signal peptide should be cleaved and eliminated during the process. The nucleotide (SEQ ID NO:44) and amino acid (SEQ ID NO:45) sequence of MC-100 is presented in Figure 12.

To generate minigene pMTI-2341, a process analogous to that used to generate pMTI-2337 (Figure 11a) was used. This involved deleting the sequences between the KpnI and BglII sites of pMTI-2326 (Figure 10a) and ligating the clone using synthetic oligonucleotide adaptor sp-spacer-A4 (SEQ ID NO:46) (Figure 13). Minigene pMTI-2341 (gene product VII or sp-A4; Figure 4b) thereby generated should express an A4 peptide linked to the APP signal peptide. The nucleotide (SEQ ID NO:46) and amino acid (SEQ ID NO:47) sequence of sp-A4 is shown in Figure 13.

For the alternate series of the minigenes incorporating the human APP gene-derived RNA processing signals, the 3'-end of the APP gene was isolated in clone pVS-1. Clone pVS-1 contained an ~1.5 kb EcoRI fragment of human genomic DNA which encompasses the 3'-end of the terminal exon of human APP and the APP polyadenylation signal inserted into the EcoRI site of pUC19, Figure 15a. The ~1.5 kb APP genomic fragment was isolated from a charon 21A lambda library of human chromosome 21 DNA (A.T.C.C. No. LA21NS01). The SmaI-SphI (nucleotides 3102 to 3269) fragment from the APP cDNA clone, pFC4, was labeled as probe and used to screen the lambda library for the APP 3'-end genomic. The nucleotide sequence of the ~1.5 kb APP genomic fragment is shown in (SEQ ID NO:48) and Figure 16.

The minigene construct, pMTI-2339, designed to express APP-695 was generated by inserting the ~1.3 kb SphI fragment from pVS-1 into the SphI site of pMTI-2312 (Figure 7a) yielding pMTI-2339 (Figure 15a). Minigenes expressing APP-751 and APP-770 and the mutants indicated below were generated by switching sequence domains of pNotSV2neo subclones of the APP constructs (Figure 18a). The subclones were utilized for switching sequence domains because of the presence of convenient restriction enzyme sites. NotI fragments of many APP minigenes were subcloned into pNotSV2neo (see Figure 18a) so that APP expression could be determined in transient transfections of COS cells. The construct encoding APP-770 (pMTI-2342, Figure 19) was generated by swapping the PvuI to SpeI fragment from pMTI-2363 (Figure 18a) to pMTI-2369 (Figure 19). A construct which encodes APP-751, pMTI-2345, was generated in an analogous fashion.

To generate minigene pMTI-2343 expressing mutant 40-1, the sequence domain encompassing the frameshift mutant, 40-1, was inserted into pMTI-2369 by swapping sequences between the PvuI and SpeI restriction sites from pMTI-2361 (Figure 18a) to pMTI-2369 (Figure 19).

To generate minigene pMTI-2340 encoding mutant, MC-100, the sequences between the KpnI and BglII sites of pMTI-2339 (Figure 15a) were deleted and the clone ligated using synthetic oligonucleotide linkers (sp-spacer-A4, Figure 12).

To generate minigene pMTI-2344 encoding mutant sp-A4, the sp-A4 mutant was inserted into pMTI-2369 by swapping sequences between the PvuI and SpeI restriction sites from pMTI-2365 (Figure 18a) to pMTI-2369 (Figure 19).

A number of the APP minigenes prepared as described in the Examples which follow were used to prepare transgenic mice expressing an APP transgene. Such transgenic mice are useful as models for the study of AD.

## EXAMPLE1

**Elements of APP Minigenes:**
**Gene Promoter and Regulator Elements for Cell- and Tissue-Specific Expression**

A critical step in the development of a transgenic mouse model for the pathology of AD is the isolation of an appropriate gene promoter for minigenes to be used as transgenes. A gene promoter and perhaps other regulatory elements must be identified that facilitate expression of recombinant APP minigenes with a cell and tissue specificity consistent with the formation of amyloid plaque. As a first step, fragments containing various portions of the 5'-end of the human APP gene from human genomic libraries were isolated. The 5'-end of the APP gene has been shown to contain DNA sequence elements which function as gene promoters in cell culture (Salbaum et al., supra). The starting material for the isolation of an ~2.5 kb HindIII fragment was a human genomic library available from the A.T.C.C. under accession number LL21NS02. This library was prepared by using a fluorescence-activated cell sorter to obtain a fraction enriched for human chromosome 21. This fraction was digested with HindIII and cloned into the lambda vector Charon 21A. This HindIII human chromosome 21 library was plated on 6 plates at an approximate density of 5 X $10^4$ pfu/plate. Screening of duplicate filters of the library representative of 3 X $10^5$ total pfu was done by conventional methods (Benton and Davis, 1977, Science 196: 180) using an ~1.0 kb cDNA probe derived from plasmid pFC4. Plasmid pFC4 (Figure 2) is described in Example 3. It contains an ~3.3 kb cDNA insert having the sequence shown in (SEQ ID NO:42) and Figure 1. The ~1.0 kb probe was obtained from the ApaI site at nucleotide position 52 to the XhoI site at nucleotide position 1056. A 91 bp probe was also used to confirm the initial screen with the ~1.0 kb probe. This small confirming probe was derived from pFC4 as an ApaI (nucleotide 52) to NruI (nucleotide 144) fragment. Clones which hybridized were plaque-purified through three subsequent cycles of screening and purification until a 100% positive hybridization response was obtained. One such plaque-purified clone was designated $\phi$MTI 3509 ($\lambda$12A). $\phi$MTI 3509 contained a genomic insert of ~2495 bp. This HindIII insert was subcloned into the HindIII site of plasmid pUC18 (Yanisch-Peron et al., 1985, Gene 33: 103) and designated pMTI-3501 (pUC18/pAL12A-12). Plasmid pMTI-3501 was found to contain ~487 bp of sequence 5' to the first nucleotide of the cDNA insert of plasmid pFC4 (shown in SEQ ID NO:42 and Figure 1).

Using an ~181 bp genomic probe derived from pMTI-3501 (from the ApaI site at -128 to ApaI at 52), an EcoRI human genomic chromosome 21 cell sorter-enriched library available from the A.T.C.C. under accession number LA21NS01 was screened in a manner similar to that described above for the HindIII library. One plaque-purified clone, designated $\phi$MTI 3522 ($\lambda$pE-1) contained an insert of ~4638 bp. This ~4.6 kb insert was subcloned into the EcoRI site of plasmid pUC19 (Yanisch-Peron et al., supra) and designated pMTI-3515 (pUC19/pE-12). Plasmid pMTI-3515 was found to contain ~2831 bp 5' to the first nucleotide of the cDNA insert of plasmid pFC4 (Figure 1).

The genomic inserts of both pMTI-3501 and pMTI-3515 containing sequences 5' to the cDNA sequence of pFC4 interrupt the KpnI site of the cDNA insert of the pFC4 at position 207. This KpnI site was not present in the genomic DNA at the junction of the boundary between exon 1 and intron 1, but was created at the splice site of the mRNA. Plasmid pMTI-3501 and plasmid pMTI-3515 encode ~1.6 kb and ~1.4 kb of intron 1, respectively. Plasmid pMTI-3515 was shown to contain ~2.8 kb of sequences 5' to the APP start site of transcription, along with exon 1 (encoding amino acids 1-19 of APP) and part of the first intron (~1.6 kb) as shown in Figure 3.

## EXAMPLE2

### Elements of APP Minigenes: SV40-derived Polyadenylation and RNA Splicing Signals

SV40 virion DNA (Hay and DePamphilis, 1982, Cell 28: 767-779; also commercially available from International Biotechnologies, Inc. (IBI) as catalog no. 33200) was digested with BamHI and BclI. The small ~0.2 kb BamHI-BclI fragment (2533 bp to 2770 bp) containing two RNA polyadenylation signals (one in each strand) (see, DePamphilis and Bradley, 1986, in The Papoyaviridae, Volume 1, Salzman (ed.), Plenum Publishing Corp., NY) was "shotgun" cloned into plasmid pUC19 as follows. The BamHI- and BclI-digested SV40 DNA was mixed with BamHI-digested pUC19 DNA. The restriction enzymes were removed via phenol-chloroform extractions and the DNA was ligated overnight at 12° C using T4 ligase (commercially available from New England Biolabs (NEB) as catalog no. 202). Impurities, including any residual phenol or chloroform were removed from the ligated DNA by GENECLEAN (available from BIO 101 Inc., P.O. Box 2284, La Jolla, CA. 92038). This DNA was used to transform competent DH5$\alpha$ E. coli cells (commercially available from Bethesda Research Laboratories (BRL), Gaithersburg, MD 20877). The transformants were screened by miniprep analysis using BamHI digestion and HpaI/HindIII digestions to determine the orientation of the inserted DNA. The desired ~2.9 kb plasmid was designated pMTI-2302 (Figure 5).

SV40-derived RNA splicing signals from plasmid pFC4 were inserted into pMTI-2302 as follows. First,

the NruI restriction endonuclease site at nucleotide position 144 of the APP cDNA sequence (shown in Figure 1) was converted to a BglII restriction endonuclease site using blunt-end linkers to yield plasmid pMTI-2303 (Figure 5). For this conversion, pFC4 was digested with NruI and the linear ~6.4 kb DNA fragment was purified with GENECLEAN and then ligated with 0.5 $OD_{260}$ units of BglII linkers (NEB catalog no. 1036) using T4 ligase [incubation for 5 hours at 16° C]. Linkers were removed by gel-filtration using "Quick Spin Columns" (Boehringer Mannheim, catalog no. 100408). The linear DNA was recovered using GENECLEAN and was circularized using T4 ligase to generate pMTI-2303 [diagnostic minipreps with BglII and XhoI digestion revealed fragments of ~0.35 kb, ~1.4 kb, and ~2.95 kb]. This procedure deleted APP encoding sequences from nucleotide position 145 to the BglII site downstream at nucleotide position 1915. An ~0.35 kb fragment containing the SV40 splicing signals could be excised from plasmid pMTI-2303 DNA by digestion with XhoI and BglII. This XhoI-BglII fragment was gel-purified on a 5% polyacrylamide gel, eluted from the gel, then used for ligation with SalI-BamHI digested pMTI-2303 DNA to generate plasmid pMTI-2305 (Figure 5). In the next step, an SphI site was inserted into the EcoRI site of pMTI-2305 to generate plasmid pMTI-2304 (Figure 5). Plasmid pMTI-2305 was digested with EcoRI to yield an ~3.2 kb fragment and then dephosphorylated using CIAP (calf intestine alkaline phosphatase, reaction conditions suggested by manufacturer, Boehringer Mannheim, catalog no. 1097075). The DNA was extracted with phenol/chloroform/isoamylalcohol (24/24/1) and precipitated in 2.5 M ammonium acetate and 70% ethanol. The DNA fragment was ligated, using T4 ligase, to an EcoRI-SphI adaptor. The adaptor is a self-annealing oligonucleotide (Sequence 5'-AATTCCCGCATGCGGG-3' (SEQ ID NO:49); synthesized using an Applied Biosystems instrument and manufacturer's recommended methods, model no. 380A DNA Synthesizer) and was annealed by heating in solution (1 mM EDTA, 10 mM Tris pH 7.6) to 65° C and allowing sample to slowly cool to room temperature. Diagnostic minipreps of pMTI-2304 with SphI revealed fragments of ~0.6 and ~2.6.

An ~0.6 kb SphI cassette containing SV40-derived splicing signals and polyadenylation signals could be excised from pMTI-2304 DNA by digestion with SphI. This cassette was useful in the construction of APP minigenes described in the Examples below.

## EXAMPLE3

### Elements of APP Minigenes:
### APP-695, -770, -751 Protein Coding Regions

Plasmid pFC4 is a cDNA clone similar to clone 9-110 described by Kang et al., 1987, Nature 325: 733-736 and contains a full-length cDNA encoding APP-695 (Figure 2). The cDNA library described by Kang et al., supra, was constructed by the method of Okayama and Berg, 1983, Mol. Cell. Biol. 3: 280-289, using polyA$^+$ RNA isolated from brain cortex of a 5-month-old aborted human fetus. This cDNA library (~$10^5$ E. coli HB101 transformants) was originally screened using a mixture of 64 20-mers as probes. The 20-mers had the sequence (SEQ ID NO:1):

```
5'-T T T T G A T G A T G C A C T T C A T A-3'
       C   G   G           C   G G
```

This sequence was deduced from the amino acid sequences of residues 10-16 of the A4 peptide. Nine positive clones were obtained, and one (clone 9-110) was selected for sequence analysis. The complete sequence of the clone 9-110 insert encoding a full-length APP-695 sequence is shown in Figure 1 of Kang et al., supra. This cDNA library was replated and screened with two different synthetic oligonucleotide probe mixtures of 17 and 20 nucleotides. The 17-mers had the sequence (SEQ ID NO:2):

```
5'-A C G T C T T C N G C G A A G A A-3'
       A   C         A   A
```

where N is A, G, C or T. The 20-mers had the sequence (SEQ ID NO:3):

```
5'-T T T T G G T G G T G N A C T T C G T A-3'
       C   A   A           C   A
```

where N is A, G, C or T. Two positive clones, designated pFC4 and pFC7, were obtained which contained identical inserts as determined by restriction endonuclease mapping and partial DNA sequence analysis. Clone pFC4 was selected for further analysis and contained an ~3.4 kb insert encoding the full-length APP-695 sequence shown in Figure 1. The nucleotide sequence is identical to the sequence of 9-110 shown as Figure 1 Kang et al., supra.

A human neuroblastoma cDNA library was purchased from Clontech, Palo Alto, CA, catalog no. HL1007a, and screened for the presence of APP transcripts of the 751 aa and 770 aa forms of human APP. This λ library was probed with an ~1.4 kb BamHI fragment (nucleotide 99-1475) of pFC4. Two positive clones (λ$E_1$-$b_1$-1 and λ$E_1$-$b_1$-3) with identical inserts were obtained. These two clones each contained an ~2.0 kb cDNA insert comprising both of the additional exons (167 bp and 58 bp) found in APP-770 but represented only a partial cDNA of the full-length mRNA for APP-770. The ~2.0 kb insert was subcloned into the EcoRI site of plasmid pUC19 to generate plasmid pMTI-3525. A full-length cDNA for APP-770 was obtained by replacing the ~1.7 kb KpnI-BglII fragment of pFC4 (nucleotide 207 - nucleotide 1915 of pFC4 sequence shown in Figure 1) with the ~.96 kb KpnI-BglII fragment of pMTI-3525, generating plasmid pMTI-3521 (pFC4-770). Specifically, pMTI-3525 was digested with KpnI and BglII, and the ~.96 kb KpnI-BglII fragment was gel-purified. Plasmid pFC4 was similarly digested with KpnI and BglII, and the ~4.7 kb KpnI-BglII fragment was gel-purified. The two gel-purified fragments were mixed, ligated and used to transform E. coli DH5α cells. The resulting ~6.6 kb plasmid pMTI-3521 was the source of the APP gene fragment for the construction of minigenes for the expression of the APP-770.

In order to obtain a full-length cDNA encoding the 751 aa form of APP, in vitro mutagenesis of plasmid pMTI-3521 was performed to delete the 58 bp sequence encoding the C-terminal 19 amino acids of the 75 aa protease inhibitor domain of APP-770. This was achieved by the M13-looping out procedure as described by Geisselsoder et al., 1987, Biotechniques 5: 786-791. DNA sequence analysis confirmed the successful deletion of the 58 bp segment of pMTI-3521 to generate plasmid pMTI-3524. Plasmid pMTI-3524 was the source of the APP gene fragment for the construction of minigenes for the expression of APP-751.

Plasmid pMTI-3524 was prepared according to the following series of steps. First, plasmid pMTI-3522 was constructed as follows. Plasmid pMTI-4 was partially digested with AccI, then filled-in with the Klenow fragment of DNA polymerase (Klenow) to remove one of the two AccI sites, ligated and used to transform E. coli DH5α cells to yield plasmid pMTI-3526. Plasmid pMTI-3526 was digested with AccI and BglII; the ~3.8 kb large fragment was gel-purified and ligated with the ~1.7 kb AccI-BglII gel-purified fragment from pMTI-3525, then used to transform E. coli DH5α cells. The desired transformant plasmid was designated pMTI-3522. Plasmid pMTI-3522 was then used to transform competent E. coli CJ236 cells which are uracil N-glycosylase-deficient. Several transformants were propagated and single-stranded uracil containing pMTI-3522 (phage) DNA was generated with the use of R408 helper phage (available from Stratagene as catalog no. 200252). MUTA-1, a synthetic 60-mer which spans the junction of APP-751 and APP-695 was used to "loop out" the 57 nucleotides of pMTI-3522 to generate pMTI-3523. MUTA-1 has the sequence (SEQ ID NO:4):

5'agtactgcatggccgtgtgtggcagcgccattcctacaacagcagccagtacccctgatg 3'

and was 5' phosphorylated and annealed to the single-stranded pMTI-3522 DNA in the presence of Gene 32 protein which assists the enzyme T4 DNA polymerase in copying the complementary strand. This mixture was used to transform competent E. coli XL-1 Blue Cells (available from Stratagene as catalog no. 200268) which are uracil N-glycosylase positive. Colorless plaques were picked for miniprep and sequence analysis. This procedure (Geisselsoder et al., 1987, Biotechniques 5: 786-791) greatly reduced the propagation of parental phage, thus enriching for the mutant strand. One of these positive clones was designated pMTI-3523.

## EXAMPLE4

### Construction of Minigenes pMTI-2314 and pMTI-2310 for Expression of APP-695

A. Minigene pMTI-2314

For the first step of the construction of minigene pMTI-2314 for the expression of APP-695, an ~4.6 kb EcoRI fragment derived from plasmid pMTI-3515 (Example 1; Figure 3) was inserted into the HindIII site of

plasmid pMTI-2301 (Figures 6a and 6b) by blunt-end ligation to yield plasmid pMTI-2307 (Figures 7a and 7b). Plasmid pMTI-2301 contains a unique HindIII cloning site, flanked by NotI restriction endonuclease sites, and was prepared by first replacing the EcoRI-HindIII polylinker of pUC19 (obtained from Bethesda Research Laboratories, Life Technologies, Inc., Gaithersburg, MD, catalog no. 95357) with the polylinker of pWE16 (available from Stratagene as catalog no. 251202), and then converting the HindIII site to an EcoRI site using adaptors. For the first step in the construction of pMTI-2301, two oligonucleotides purchased from NEB (catalog nos. 1107 and 1105) were annealed to yield the following double-stranded adaptor:

```
5'-AATTCGAACCCCTTCG-3'          (#1105) (SEQ ID NO:5)
        3'-GCTTGGGGAAGCTCGA-5'  (#1107) (SEQ ID NO:6)
```

Then, plasmid pUC19 DNA was digested with EcoRI and HindIII and the ~2.7 kb fragment was gel-purified (using low melt agarose), ligated with the adaptor, and used to transform E. coli DH5α cells. The desired transformant was designated pMTI-2110 (Figure 6a). For the second step in the construction of pMTI-2301, plasmid pMTI-2110 DNA was digested with EcoRI, then treated with calf intestine alkaline phosphatase (CIAP), then gel-purified. CIAP was obtained from Boehringer Mannheim Biochemicals, Biochemicals Division, P.O. Box 50816, Indianapolis, IN 46250 (catalog no. 713 023). Plasmid pWE16 DNA was also digested with EcoRI. The EcoRI-digested pWE16 and gel-purified EcoRI-digested pMTI-2110 DNAs were mixed, ligated, treated with GENECLEAN and used to transform E. coli DH5α cells. The desired transformant plasmid was designated pMTI-2300. Miniprep analysis showed that NotI linearizes the ~2.7 kb plasmid. For the third step in the construction of pMTI-2301, plasmid pMTI-2300 DNA was digested with BamHI and ligated to self-annealing synthetic oligonucleotide adaptor (sequence 5'-GATCGGGAAGCTTCCC-3' (SEQ ID NO: 7); synthesized using an Applied Biosystems instrument and manufacturers recommended methods, model no. 380A DNA Synthesizer) in order to convert the BamHI site to HindIII. The oligonucleotide was annealed to yield the following double-stranded adaptor:

```
5'-GATCGGGAAGCTTCCC-3' (SEQ ID NO:7)
    5'-CCCTTCGAAGGGCTAG-5' (SEQ ID NO:8)
```

The ligated DNA was treated with GENECLEAN and used to transform E. coli DH5α cells. Miniprep analysis of transformant DNA was performed using BamHI (plasmid remains uncut) and HindIII (linearizes plasmid). The desired transformant was designated pMTI-2301.

Plasmid pMTI-2301 DNA, thus obtained, was digested with HindIII, gel-purified, then treated with Klenow and CIAP. Then, plasmid pMTI-3515 DNA was digested with EcoRI and an ~4.6 kb fragment was gel-purified, treated with Klenow, and blunt-end ligated with the pMTI-2301 DNA prepared as described above. The ligated DNA was treated with GENECLEAN and used to transform E. coli DH5α cells. Transformants were screened by miniprep analysis using EcoRI. The desired transformant plasmid was designated pMTI-2307 and contained EcoRI fragments of ~4.7 kb and ~2.6 kb by miniprep analysis.

For the second step of the construction of pMTI-2314, an ~1.4 kb BamHI fragment of pFC4 comprising nucleotides 100-1475 (Example 3, Figure 1) was ligated into the BamHI site at nucleotide position 99 of the APC cDNA sequence in plasmid pMTI-2307 to yield plasmid pMTI-2311 (Figure 7a). Diagnostic miniprep analysis of pMTI-2311 DNA digested with XhoI and NotI revealed fragments of ~3.9 kb, ~2.7 kb and ~2.2 kb.

For the third step of the construction of pMTI-2314, an ~2.4 kb XhoI fragment of pFC4 comprising nucleotides 1056-3353 and including 3' sequences, poly A track and SV40 sequences found in the Okayama and Berg vector (Okayama and Berg, 1983, supra) was ligated into the XhoI site at nucleotide position 1056 to yield plasmid pMTI-2312 (Figures 7a and 7c).

For the final step of the construction of pMTI-2314, an ~0.6 kb Sph I fragment of pMTI-2304 containing SV40-derived RNA splicing and polyadenylation signals was ligated into the SphI site of pMTI-2312 to yield plasmid pMTI-2314 (Figures 7a and 8b). Plasmid pMTI-2314 DNA was used as a minigene for the construction of APP-695 expressing transgenic mice as described in Example 11 below.

B. Minigene pMTI-2310

A second minigene for the expression of APP-695, pMTI-2310 (Figure 8a), was constructed according to the same four steps as outlined above for the construction of pMTI-2314, except that in the first step, an

~2.4 kb HindIII fragment derived from plasmid pMTI-3501 (Example 11 was inserted into the HindIII site of pMTI-2301 (part A above) to yield plasmid pMTI-2306. Diagnostic miniprep analysis of pMTI-2306 DNA digested with NotI and BamHI revealed fragments of ~2.7, ~0.9 and ~0.6 kb. The subsequent three steps yielded plasmids pMTI-2308 (diagnostic minipreps with NotI and XhoI revealed fragments of ~2.6, ~2.3 and ~1.6 kb), pMTI-2309 (diagnostic minipreps with HindIII to determine orientation revealed fragments of ~3.4, ~2.9 and ~2.6 kb) and pMTI-2310 (diagnostic minipreps with EcoRI revealed fragments of ~2.7, ~2.4, ~2.3, ~1.1 and ~0.9 kb), respectively. Plasmid pMTI-2310, containing the same APP-695 gene as pMTI-2314 but with a truncated regulatory region, was also used as a minigene for the construction of APP-695 expressing transgenic mice as described in Example 11 below.

## EXAMPLE5

### Construction of Minigenes pMTI-2319 and pMTI-2320 for Expression of APP-770 and APP-751

Minigenes pMTI-2319 and pMTI-2320 (Figure 8a) for the expression of APP-770 and APP-751, respectively, were each constructed in a single step digestion and ligation procedure via a simple interchange of AccI-BglII fragments. Plasmid pMTI-3521 DNA (Example 3) encoding a full-length cDNA for APP-770 was digested with AccI and BglII. An ~1.8 kb AccI-BglII fragment of pFC4-770 was ligated into the AccI and BglII sites of pMTI-2314 to yield pMTI-2319. Diagnostic miniprep analysis of pMTI-2319 DNA digested with ScaI revealed fragments of ~7.3 and ~4.8 kb. A ScaI site exists in the inhibitor encoding domains of APP-770 and APP-751. Similarly, plasmid pMTI-3524 DNA (Example 3) encoding a full-length cDNA for APP-751 was digested with AccI and BglII. An ~1.75 kb AccI -BglII fragment of pMTI-3524 was ligated into the AccI and BglII sites of pMTI-2314 to yield pMTI-2320. Diagnostic miniprep analysis of pMTI-2320 DNA digested with ScaI revealed fragments of ~7.2 and ~4.8 kb. Plasmids pMTI-2319 and pMTI-2320, containing a full-length cDNA for APP-770 and APP-751, respectively, were used as minigenes for the construction of APP-770 and APP-751 expressing transgenic mice as described in Example 12 below. Thus, the inhibitor encoding domains found in APP-770 and APP-751 may be inserted in the APP-695 sequence of pMTI-2314 by swapping sequence domains between the unique AccI and BglII sites.

## EXAMPLE6

### Construction of pMTI-2321 and pMTI-2322 Minigenes for Expression of APP C-Terminal Frameshift Mutants

Minigenes pMTI-2321 and pMTI-2322 (Figure 8a) for the expression of a truncated APP protein were constructed by making frameshift mutations in the C-terminal region of the APP coding sequence. These frameshift mutations were made in the APP cDNA sequences immediately following the A4 coding region so as to bring a translation stop codon into the reading frame (i.e., in-frame termination) following the A4 peptide coding region. The resulting mutated sequence codes for a truncated APP species as exemplified by gene product IV shown in Figure 4b.

In vitro mutagenesis procedures described by Kunkel et al., 1987, Methods In Enzymol. 154: 367-382, were used to generate the frameshift mutants briefly summarized as follows. The starting material for the mutagenesis was plasmid pMTI4 DNA. Plasmid pMTI4 is the mutagenesis vector KS Bluescript (+) available from Stratagene into which the ~2.3 kb NruI-SpeI fragment of pFC4 containing a segment of the APP-695 cDNA has been inserted. For this construction, pFC4 DNA was digested with NruI and SpeI, treated with Klenow, then blunt-end ligated into the SmaI site of SmaI-digested KS Bluescript(+) DNA to yield pMTI-4. Single-stranded pMTI4 DNA was prepared from E. coli CJ236 host cells, in which cells uracil replaces thymine in DNA. The DNA was then made double-stranded by in vitro DNA synthesis using one of three mutagenizing synthetic oligonucleotides described below as primer for a particular preparation. The hetercduplex DNA (one uracil-containing normal pMTI4 strand and one newly synthesized thymine-containing mutated strand) was used to transform E. coli MV1190 cells, in which cells the sequence of the thymine-containing mutated strand is selectively propagated because the uracil-containing wildtype strand is degraded. Miniprep plasmid preparations from such transformed E. coli colonies were screened for incorporation of the mutation by direct DNA sequence analysis. For sequence analysis, the primer was an oligonucleotide having the sequence from nucleotide position 1881-1897 of the APP cDNA. The sequence ~200 nucleotides downstream of the primer was analyzed to confirm the mutated sequence. Those clones having the desired mutation of the APP coding sequence were used for preparative purification of mutant plasmid DNA by conventional methods, for use in the construction of truncated APP minigenes.

Three types of mutants (a, b, c) were generated which introduced premature translation termination signals in APP mRNA to yield truncated APP proteins. The wildtype (wt) and mutant sequences beginning at nucleotide position 2040 are shown with the termination codons underlined as follows:

wt (nucleotide SEQ ID NO:9/amino acid SEQ ID NO:10)

| | | | | | | | | | | |
|---|---|---|---|---|---|---|---|---|---|---|
| | | a | | b | | c | | | | |
| wt | GTG | GGC | GGT | GTT | GTC | ATA | GCG | ACA | GTG | ATC... |
| | Val | Gly | Gly | Val | Val | Ile | Ala | | | |

a (nucleotide SEQ ID NO:11/amino acid SEQ ID NO:12)

| | | | | | | | | | | |
|---|---|---|---|---|---|---|---|---|---|---|
| a | GTG | GGG | GTG | TTG | TCA | TAG | CGA | CAG | TGA | TCG... |
| | Val | Gly | Val | Leu | Ser | * | | | | |

b (nucleotide SEQ ID NO:13/amino acid SEQ ID NO:14)

| | | | | | | | | | | |
|---|---|---|---|---|---|---|---|---|---|---|
| b | GTG | GGC | GGT | GTT | GTG | TCA | TAG | CGA | CAG | TGA |
| TGC... | | | | | | | | | | |
| | Val | Gly | Gly | Val | Val | Ser | * | | | |

c (nucleotide SEQ ID NO:15/amino acid SEQ ID NO:16)

| | | | | | | | | | | |
|---|---|---|---|---|---|---|---|---|---|---|
| c | GTG | GGC | GGT | GTT | GTC | TAG | CGA | CAG | TGA | TCG... |
| | Val | Gly | Gly | Val | Val | * | | | | |

The synthetic oligonucleotides used for mutagenesis were:

(SEQ ID NO:17)

a 5'-CATGGTGGGGGTGTTGTCATAGC-3' [23-mer, 2036-2059]

(SEQ ID NO:18)

b 5'-GGGCGGTGTTGTGTCATAGCGACAG-3' [25-mer, 2042-2064]

(SEQ ID NO:19)

c 5'-GGCGGTGTTGTCTAGCGACAGTGA-3' [24-mer, 2043-2067]

For mutant-a, one nucleotide (C) that is marked above the wildtype sequence with the letter "a", is deleted, generating two in-frame termination codons. The first in-frame termination codon in mutant-a is the codon for aa 40 of the A4 sequence. In mutant-a, amino acids 38, 39 and 40 are different than those of the wildtype sequence. For mutant-b, two nucleotides (TG) were inserted at the position marked above the wildtype sequence with the letter "b", generating two in-frame termination codons. The first in-frame termination codon in mutant-b is after the codon for aa 41 of the A4 sequence. In mutant-b, aa 41 is different than that of the wildtype sequence. Mutant-b (also known as the +2 mutant) was utilized in the construction of plasmid pMTI-2321 described below. For mutant-c, one nucleotide (A) that is marked in the wildtype sequence shown above with the letter "c" is deleted, generating an in-frame termination codon in the reading frame directly following the codon for aa 40 of the A4 sequence. Mutant-c has been designated mutant 40-1, and was utilized in the construction of plasmid pMTI-2322 described below.

The portion of APP-695 cDNA that contains the A4 coding sequence lies within an ~0.7 kb BglII-ClaI fragment (corresponding to nucleotide position 1915-2620) that can conveniently be moved from one APP gene construct to another since BglII and ClaI each cleave APP-695 cDNA only once. The following steps were used to insert the mutated part of pMTI4 into another APP construct to generate minigenes for expression of truncated APP proteins. In the first step, mutated pMTI4 DNA was digested with BglII and ClaI. The ~0.7kb BglII-ClaI fragment was then isolated from a preparative agarose gel. In the second step, DNA from the other construct was digested with BglII and ClaI and then treated with CIAP. For the preparation of plasmids pMTI-2321 and pMTI-2322, this other construct was pMTI-2314 (Example 4) encoding a full-length (wildtype) cDNA for APP-695. The small ~0.7 kb BglII-ClaI fragment of pMTI-2314 was removed from the digest by preparative agarose gel electrophoresis. In the third step, the large ~11.1 kb BglII-ClaI fragment of pMTI-2314 remaining after removal of the ~0.7 kb fragment to be replaced (step 2) was mixed with the ~0.7 kb mutated BglII-ClaI fragment (step 1), then ligated and used to transform E. coli DH5α cells. Transformant plasmids were initially screened for appropriate inserts by miniprep analysis. Diagnostic miniprep analysis of the plasmids using EcoRI revealed fragments of ~4.7, ~2.7, ~2.6, ~1.1 and ~0.9 kb. Then, the integrity of each selected plasmid preparation was confirmed by DNA sequence analysis of the mutated sequence and sequences surrounding the mutation. The resulting selected plasmids designated pMTI-2321 (mutant-b) and pMTI-2322 (mutant 40-1), were used as minigenes for the construction of transgenic mice expressing truncated APP proteins. A plasmid for the expression of mutant-a may be constructed and selected by fragment swapping as described above for mutant-b (pMTI-2321) and mutant 40-1 (pMTI-2322).

## EXAMPLE7

### Construction of Minigene pMTI-2318 for Expression of A4 Amyloid Peptide

In order to construct plasmid pMTI-2318 (Figure 9) containing a minigene for the expression of A4 peptide, first a portable gene encoding the 42 aa A4 peptide sequence was prepared. This gene was obtained by in vitro mutagenesis of a fragment of the cDNA for APP-695 as described in Example 6 above. The starting material was the same as that described in Example 6, plasmid pMTI-4. A 38-base oligonucleotide mutagenesis primer corresponding to the A4 sequence, but with desired changes for the creation of an in-frame termination (TAG) and a convenient BamHI restriction site immediately following the in-frame termination codon, was synthesized chemically. The sequence (SEQ ID NO: 20) of this synthetic primer was:

```
                       BamHI
5'-GGTGTTGTCATAGCGTAGGATCCGTCATCACCTTGGTG-3'
                  Ter
```

This primer was used to mutate the APP-695 sequence in pMTI4 in substantial accordance with the procedure in Example 6 above to create plasmid pMTI-26. The wildtype (native) and mutated sequences are shown as follows:

(nucleotide SEQ ID NO:21/amino acid SEQ ID NO:22)

```
        BglII
wt      AGATCTCTGAAGTGAAGATG GAT---GGT GTT GTC ATA GCG ACA GTG ATC-
                         MET asp    gly val val ile ala thr val ile
```

(nucleotide SEQ ID NO:23/amino acid SEQ ID NO:24 )

```
        BglII                                                BamHI
mutant  AGATCTCTGAAGTGAAGATG GAT---GGT GTT GTC ATA GCG TAGGATCCGT
                         MET asp    gly val val ile ala ter
```

The newly created BamHI site and the BglII site preceding the ATG codon provide convenient restriction sites for cloning the A4 gene into other APP constructs to generate minigenes for expression of 42 aa A4 peptide. One such minigene construct was pMTI-2318, prepared according to the following steps (Figure 9). In the first step pMTI-26 DNA was digested with BglII and BamHI. The ~150 bp fragment was then isolated from a 5% polyacrylamide gel by electroelution. In the second step, DNA from another construct, for example, pMTI-2307 (Example 4) was digested with BamHI, gel-purified and treated with CIAP. In the third step, the ~150 bp mutated BglII-BamHI fragment (step 1) was mixed with the BamHI-cut pMTI-2307 DNA (step 2), then ligated and used to transform E. coli DH5α cells. Transformant plasmids were screened for appropriate inserts by miniprep and DNA sequence analysis. For each miniprep analysis restriction endonucleases were chosen that would allow starting and ending materials to be distinguished and also allow determination of desired orientation. Then, other restriction endonucleases were chosen to confirm the integrity of the construction (e.g., no anomalous rearrangements). The resulting ~7.6 kb plasmid was designated pMTI-2316. Miniprep analysis with BamHI and EcoRI revealed fragments of ~6.8 and ~0.8 kb. In the next steps, pMTI-2316 DNA was digested with BamHI, then mixed with and ligated to the ~2.0 kb BamHI fragment of pFC4 to yield pMTI-2317 by transformation and selection as described above. Miniprep analysis of the ~9.6 kb pMTI-2317 DNA with HindIII revealed fragments of ~7.3 and ~2.2 kb. Insertion of this BamHI fragment provided a convenient SphI site along with a portion of 3' untranslated sequences so as to be a template for a messenger RNA transcript of stable size. In the final steps, an ~0.6 kb SphI fragment of pMTI-2304 containing SV40 splicing and polyadenylation signals was inserted at the SphI site of pMTI-2317 by appropriate restriction endonuclease digestion, ligation, transformation and selection as described above, to yield desired plasmid pMTI-2318. Miniprep analysis of pMTI-2318 DNA with EcoRI revealed fragments of ~2.9, ~2.7, ~2.0, ~1.1, ~0.9 and ~0.6 kb. Plasmid pMTI-2318 was utilized as a minigene for the construction of transgenic mice expressing a 42 aa A4 peptide.

## EXAMPLE8

### Epitope Tagging of Recombinantly Expressed APP

Immunochemical studies of the subcellular localization and processing of alternative forms of APP, including APP-695, APP-770 and APP-751, and mutated forms of APP, using antibodies elicited to synthetic peptides and/or recombinant precursor proteins are difficult for the following reasons. Firstly, the APPs are highly conserved among species (mouse and rat 99%, human and rat 97.3%) and are ubiquitously expressed. In order to easily obtain adequate antibody production against a variety of APP peptides and recombinant proteins, a highly antigenic epitope of Chlamydia (Huguenel et al., 1989, Intl. Soc. Sex. Trans. Dis. Res., 8th Meeting, Copenhagen, Denmark, Abstract no. 22) was inserted into the APP sequence at either the Kunitz inhibitor domain or the extreme C-terminus of the protein to produce "tagged" APP cDNAs. Minigenes containing such tagged APP cDNA can be used to prepare transgenic mice, and the APP translation products in such mice can be detected using antibodies against this epitope.

The peptide sequence of the Chlamydia epitope is TVFDVTTLNPTI. This epitope has been shown to be very antigenic as an isolated peptide and as part of a larger protein (Huguenel et al., supra; Baehr et al., 1988, Proc. Natl. Acad. Sci. USA 85: 4000-4004; Stephens et al., 1988, J. Exp. Med. 167: 817-831). Synthetic oligonucleotides were generated for the site-directed mutagenesis in the APP coding region to insert the sequences for the Chlamydia epitope by M13 "looping-in" experiments. The synthetic oligonucleotide:

```
            (nucleotide SEQ ID NO:25/amino acid SEQ ID NO:26)

5'                                                Aat II
GGCTGCTGTG GCGGGGGTCTA AAT AGT TGG GTT CAG AGT GGT GAC GTC AAA
                        *   I   T   P   N   L   T   T   V   D   F

GAC AGT GTT CTG CAT CTG CTC AAA GA     77-mer (CC-TAG)
 V   T   N   Q   M   Q   B   F   F
```

was used to engineer pMTI-63 which carries a C-terminal addition of sequences encoding the Chlamydia epitope to APP695. The synthetic oligonucleotide:

```
         (nucleotide SEQ ID NO:27/amino acid SEQ ID NO:28)
5'                                                    Aat II
G GGT ACT GGC TGC TGT TGT AGG AAT AGT TGG GTT CAG AGT GGT GAC GTC
   T   S   A   A   T   T   P   I   T   P   N   L   T   T   V   D

AAA GAC AGT AAC TCG AAC CAC CTC TTC C     77-mer (IC-TAG)
 F   V   T   V   R   V   V   E   E
```

was used to engineer pMTI-35 which carries an addition of the amino acid sequences encoding the Chlamydia epitope into the APP sequence of amino acid residue 289, where the (Kunitz) protease inhibitor-like domain is spliced into the APP-695 molecule.

Antibodies prepared against this Chlamydia epitope are useful to investigate the tissue, cellular and subcellular localization of tagged APP proteins in vivo, to study the biochemical properties and processing of APP in transfected animal cells including cell-sorting of transfectants, to study APP in vitro translation products and APP transformed E. coli products on Western-blots, and to follow processing of APP and its subcellular localization in transgenic animals. Such studies should permit the identification of the functional role of APP in normal individuals and in individuals with AD or DS. Minigenes pMTI-2324 and pMTI-2325 (Figure 8a) for the expression of APP-695 (IC-TAG) were each constructed in a single step digestion and ligation procedure via a simple interchange of AccI and BglII fragments. Plasmid pMTI-35 DNA was digested with AccI and BglII. An ~1.6 kb AccI a-BglII fragment of pMTI-35 was gel-purified and ligated either into the AccI and BglII sites of the gel-purified large DNA fragment of pMTI-2314 to generate pMTI-2325 (Figure 8a) or into the AccI and BglII sites of the gel-purified large fragment of pMTI-2323 to generate pMTI-2324 (Example 9, Figure 10a). Diagnostic miniprep analysis of pMTI-2324 DNA digested with HindIII revealed fragments of ~1.3, ~4.4 and ~7.7 kb. Diagnostic miniprep analysis of pMTI-2325 DNA digested with AatII revealed fragments of ~4.2, ~3.3, ~3.1, ~0.6 and ~.055 kb. In an analogous manner, the ~1.6 kb AccI-BglII fragment of pMTI-35 can be ligated into the AccI and BglII sites of the gel-purified large DNA fragment of pMTI-2329 (Example 9, Figure 14) to generate pMTI-2335.

## EXAMPLE9

### APP Minigenes with Metallothionein-Derived Regulator or Processing Sequences

A. APP Minigenes with Metallothionein-Derived RNA Processing Signals

Minigenes utilizing RNA processing signals derived from an exogenous mouse gene might be more efficiently expressed in transgenic mice as compared with minigenes utilizing SV40-derived RNA processing signals as described in Examples 4-8 above. Therefore, alternate minigene constructs were generated in which RNA splicing and polyadenylation sequences of the mouse metallothionein gene were utilized. One source of the mouse metallothionein gene is plasmid pJYMMT(L) (alternatively designated pCL-28 or T25). Plasmid pJYMMT(L) is an ~12.4 kb genomic clone of the metallothionein gene described by Hamer and Walling, 1982, J. Mol. App. Gen. 1: 274-288. This alternate series of minigenes utilizing metallothionein RNA signals was constructed using pJYMMT(L). Many of these alternate minigenes were generated via fragment swaps using the minigenes containing SV40 RNA signals described in Examples 4-8.

1. Alternate Minigenes Expressing APP-695, APP-770 and APP-751

A minigene utilizing mouse metallothionein-I gene RNA processing signals (splicing and polyadenylation) and expressing APP-695 was constructed in a single step as follows. A Klenow- treated ~2.2 kb BglII-EcoRI fragment of pJYMMT(L) containing all of the mouse metallothionein-I genomic gene sequence except the promoter was inserted by blunt-end ligation into the ClaI site of plasmid pMTI-2312 DNA (Example 4) that had been digested with ClaI and treated with Klenow and CIAP to generate plasmid pMTI-2323 (Figure 10a and 10b). Plasmid pMTI-2323 was selected in a two-step screening procedure. First, transformant plasmids from the blunt-end ligation were screened by colony hybridization using the insert fragment (~2.2 kb BglII-EcoRI) of pJYYMT(L) labelled with $^{32}P$ as probe. Colony hybridization was used as a first step in screening a variety of constructs disclosed herein when the background of transformant plasmids that were vector alone (i.e., no insert) was high. In the second screening step, the desired plasmid was selected from those positively hybridizing colonies by miniprep analysis of restriction endonuclease digested DNA. For pMTI-2323 (~13.3 kb), miniprep analysis using HindIII revealed fragments of ~7.7, ~4.4 and ~1.3 kb.

Minigenes utilizing metallothionein RNA signals and expressing APP-770 or APP-751 can be constructed via AccI-BglII fragment exchanges (Figure 10a) with either pMTI-3521 or pMTI-3524 (Example 3) respectively. Specifically, the ~1.8 kb AccI-BglII fragment of pMTI-3521 was inserted into the AccI-BglII sites of pMTI-2323, replacing the ~1.5 kb AccI-BglII fragment of pMTI-2323, to generate plasmid pMTI-2331 (Figure 10a). For example, for pMTI-2331, pMTI-2323 DNA was digested with AccI and BglII and the ~11.8 kb fragment was gel-purified, treated with CIAP and ligated with the ~1.8 kb AccI-BglII fragment that was gel-purified from pMTI-3521 DNA. The ligation mixture was used to transform E. coli DH5α cells. The desired plasmid, pMTI-2331 (~13.3 kb), was identified by miniprep analysis. Using ScaI, miniprep analysis of pMTI-2331 revealed fragments of ~9.0 and ~5.0 kb.

2. Alternate Minigenes Expressing APP C-Terminal Frameshift Mutants

A minigene utilizing metallothionein RNA signals and expressing a truncated APP protein was constructed via a fragment swap using minigene pMTI-2322 (Example 6, Figure 8a) containing SV40 RNA signals. Specifically, the ~0.7 kb BglII-ClaI fragment of pMTI-2322 containing mutation 40-1 (Example 6), was inserted into pMTI-2312 (Example 4, Figure 7a), via ligation of the ~0.7 kb fragment of pMTI-2322 with the ~10.5 kb BglII-ClaI fragment of pMTI-2312 (that had been gel-purified and treated with CIAP prior to ligation), to generate pMTI-2326a. Miniprep analysis of the ~11.2 kb pMTI-2326a DNA using HindIII revealed fragments of ~7.8 and ~3.4 kb. Then, the ~2.2 kb BglII-EcoRI metallothionein fragment of pJYMMT(L) was inserted into the ClaI site of pMTI-2326a by blunt-end ligation to generate pMTI-2326. Miniprep analysis of the ~13.4 kb pMTI-2326 DNA using HindIII revealed fragments of ~7.7, ~4.4 and ~1.3 kb. Alternatively, plasmid pMTI-2326 can be constructed in a one-step fragment swap. Specifically, the ~0.6 kb BglII-SpeI fragment of pMTI-2322 (Example 6, Figure 8a) can be inserted directly into pMTI-2323, replacing the ~0.6 kb BglII-SpeI fragment of pMTI-2323 to generate pMTI-2326 (Figure 10a). Alternative minigenes can be generated by analogous BglII-SpeI fragment exchanges between pMTI-2323 and constructs encoding alternate truncated forms of APP-695 (Examples 6 and 7).

3. Alternate Minigenes Expressing C-100 (Plasmid pMTI-2327) and MC-100 (Plasmid pMTI-2337) APP Mutants

A minigene utilizing metallothionein RNA signals and coding for the mutation designated C-100 (gene product VI, Figure 4b) was generated by deleting the ~1.8 kb NruI-BglII fragment of pMTI-2323 (this example, part A) with blunt-end ligation to generate plasmid pMTI-2327 (Figure 11a). Plasmid pMTI-2323 DNA was digested with NruI and BglII; the ~11.5 kb fragment was gel-purified, treated with Klenow, ligated, and used to transform *E. coli* DH5$\alpha$ cells. Transformant plasmids were screened by miniprep analysis and the desired plasmid pMTI-2327 was selected. Miniprep analysis of pMTI-2327 DNA using HindIII revealed fragments of ~7.7, ~2.6 and ~1.3 kb. A translation initiation codon, ATG, directly precedes the first codon of the A4 sequences in C-100.

A minigene for expressing the mutation designated MC-100 (gene product V, Figure 4b) was also prepared from plasmid pMTI-2323 to generate pMTI-2337 (Figures 11a and 11b). Specifically, pMTI-2337 was generated by deleting the ~1.7 kb KpnI-BglII fragment of pMTI-2323 via gel purification of the ~11.6 kb fragment and ligating using a synthetic oligonucleotide linker, sp-spacer-A4. The linker sp-spacer-A4 was inserted between the KpnI site at position 207 and the BglII site at position 1915 in APP-695, and had the following sequence:

| | |
|---|---|
| 5'-GGACGGAGGA-3' | 10-mer (SEQ ID NO:29) |
| 3'-CATGCCTGCCTCCTCTAG-5' | 18-mer (SEQ ID NO:30) |

The two oligonucleotide sequences that comprise sp-spacer-A4 were synthesized (using an Applied Biosystems instrument and manufacturer's recommended methcds, model no. 380A DNA synthesizer), kinased and annealed according to conventional methods before ligation with the gel-purified ~11.6 kb KpnI-BglII fragment of pMTI-2323 to generate pMTI-2337. Miniprep analysis of pMTI-2337 DNA with HindIII revealed fragments of ~7.7, ~2.6 and ~1.3 kb.

MC-100 requires the 17 residue signal peptide of APP to direct translation and insertion of the mutant protein into the membrane. The signal peptide will be cleaved and eliminated during the membrane insertion. The nucleotide and amino acid sequence of MC-100 is shown in Figure 12.

4. Alternate Minigene Expressing A4 Peptide (Plasmid pMTI-2341)

A minigene utilizing metallothionein RNA signals for expressing the A4 peptide (gene product VII or sp-A4, Figure 4b) was prepared by deleting the ~11.6 kb KpnI-BglII fragment of plasmid pMTI-2326 (this example, part A,2) and ligating using the sp-spacer-A4 linker (described in part A,3 above) to generate plasmid pMTI-2341. Minigene pMTI-2341 generates sp-A4, which an A4 peptide linked to the APP signal sequence. The nucleotide and amino acid sequence of sp-A4 is shown in Figure 13.

B. APP Minigenes With A Metallothionein-Derived Promoter

The generation of transgenic mice which express APP (or derivatives of APP) in cells and tissues not normally expressing the gene may lead to dominant phenotypes. The new phenotypes may facilitate a better understanding of the function of APP. To this end, a series of minigenes was constructed which minigenes are under the regulation of the mouse metallothionein gene promoter (Figure 14).

1. Alternate minigenes expressing APP-695, APP-770 and APP-751

A minigene utilizing a metallothionein promoter and expressing APP-695 was constructed as follows. Plasmid pMTI-2301 DNA (Example 4) was digested with HindIII, treated with Klenow and CIAP. Plasmid pJYMMT(L) DNA (part A above) was digested with EcoRI. An ~4.0 kb EcoRI fragment was gel-purified, treated with Klenow and blunt-end ligated to the pMTI-2301 DNA treated as described above. The desired transformant plasmid was designated pMTI-2328 (~6.7 kb). In the next step, the pMTI-2328 DNA thus obtained was digested with BglII, treated with Klenow and CIAP, gel-purified and then blunt-end ligated to an ~2.8 kb gel-purified SmaI-HindIII fragment of pMTI-2314 (~11.8 kb, Example 4). Transformant plasmids were screened by miniprep analysis and the desired plasmid pMTI-2329 (Figure 14) was selected. Miniprep analysis of pMTI-2329 DNA using EcoRI revealed fragments of ~3.7, ~3.1 and ~2.7 kb.

Minigenes utilizing a metallothionein promoter from pMTI-2329 and expressing APP-770 or APP-751 are constructed via fragment swaps with pMTI-2319 (alternatively, pMTI-2331 or pMTI-2342) or pMTI-2320 (alternatively, pMTI-2345), respectively. Specifically, an ~7.4 kb AccI-SpeI fragment is gel-purified and ligated with an ~2.4 kb AccI-SpeI fragment of pMTI-2319 or pMTI-2330 to yield pMTI-2333 for APP-770 expression and pMTI-2334 for APP-751 expression, respectively.

2. Alternate Minigenes Expressing APP C-Terminal Frameshift Mutants

By a similar fragment swap, a minigene utilizing a metallothionein promoter and expressing a truncated APP protein is constructed. Specifically, an ~2.1 kb AccI-SpeI gel-purified fragment of pMTI-2322 (alternatively, pMTI-2343 or pMTI-2326) containing mutation 40-1 (Example 6) was ligated with the ~7.4 kb AccI-SpeI gel-purified fragment of pMTI-2329 described above.

3. Alternate Minigene Expressing MC-100 APP Mutants

An alternate minigene for the expression of the MC-100 mutation (part A above) using a metallothionein promoter may also be prepared. For example, the ~1.7 kb KpnI-BglII fragment of pMTI-2329 may be deleted via digestion with KpnI and BglII, then gel purification of the ~7.8 kb KpnI-BglII fragment and finally ligation with the sp-spacer-A4 (part A above). The desired plasmid is confirmed by sequence analysis.

## EXAMPLE10

### APP Minigenes with Genomic APP-Derived RNA Processing Signals

APP minigenes utilizing RNA processing signals derived from the human APP gene might be more efficiently expressed in transgenic mice as compared with minigenes described in Examples 4-8 above utilizing SV40 derived RNA processing signals or minigenes described in Example 9 above utilizing metallothionein gene-derived signals. Therefore, minigene constructs were generated in which RNA polyadenylation signals of the human APP gene were utilized. The source of the human APP genomic sequences for these constructs was plasmid pVS-1. Plasmid pVS-1 is an ~4.3 kb genomic clone of the human APP gene which comprises an ~1.5 kb EcoRI genomic fragment inserted into the EcoRI site of pUC19 in the orientation shown in Figure 15a, so that the APP polyadenylation signal can be recovered as an ~1.3 kb SphI fragment. The ~1.5 kb EcoRI fragment encompasses the 3'-end of the terminal exon of human APP and the APP polyadenylation signal and was isolated as follows. A Charon 21A lambda library of human chromosome 21 DNA (available from the A.T.C.C. as accession no. LA21NS01) was screened for clones containing 3'-end genomic sequences with a small SmaI-SphI fragment (nucleotides 3102-3269) from plasmid pFC4 labelled as a probe. The nucleotide sequence of the ~1.5 kb APP genomic fragment is shown in Figure 16. An alternate series of minigenes utilizing APP RNA signals derived from pVS-1 were constructed. Many of these alternate minigenes were generated via fragment swaps using pNotSV2neo subclones of the APP constructs. These pNotSV2neo subclones were utilized for switching sequence domains via fragment swaps because of the presence of convenient PvuI and SpeI restriction enzyme sites. NotI fragments of many of the APP minigenes described in Examples 4-8 were subcloned into pNotSV2neo (see Figures 17 and 18a) so that APP expression could be determined in transient transfections of COS cells (Gluzman, 1981, Cell 23: 175-182). Plasmid pNotSV2neo (Figure 17) was prepared by converting the unique BamHI site of pSV2-neo (available from the A.T.C.C. as accession no. 37149) to a NotI site using linkers (NEB catalog no. 1045). Plasmid pSV2-neo was digested with BamHI, treated with Klenow and CIAP, and ligated to NotI linkers as recommended by the supplier. The pNotSV2neo subclones of the APP constructs used in the preparation of alternate minigenes were prepared as summarized in Table I below and Figure 18a. The construction of each of the APP minigenes utilizing APP genomic RNA processing signals from pVS-1 listed in Table I is described below.

**TABLE I**

**pNotSV2neo APP Subclones**

| Subclone | APP Sequence | Insert in pNotSV2neo* |
|---|---|---|
| pMTI-2360 | APP-695 | ~10.6 kb NotI fragment of pMTI-2323 |
| pMTI-2362 | APP-695 | ~6.8 kb NotI fragment of pMTI-2329 |
| pMTI-2369 | APP-695 | ~9.8 kb NotI fragment of pMTI-2339 |
| pMTI-2363 | APP-770 | ~10.8 kb NotI fragment of pMTI-2331 |
| pMTI-2368 | APP-751 | ~9.2 kb NotI fragment of pMTI-2320 |
| pMTI-2361 | Mutation 40-1 | ~10.8 kb NotI fragment of pMTI-2326 |
| pMTI-2364 | MC-100 | ~8.0 kb NotI fragment of pMTI-2340 |
| pMTI-2366 | MC-100 | ~8.8 kb NotI fragment of pMTI-2337 |
| pMTI-2365 | Sp-A4 | ~8.8 kb NotI fragment of pMTI-2341 |
| pMTI-2367 | β-gal | ~8.6 kb NotI fragment of pMTI-2402 |

* For preparation of the subclones, each insert was gel-purified and ligated into pNotSV2neo vector DNA that had been digested with NotI, gel-purified and treated with CIAP.

A. Alternate Minigenes Expressing APP-695, APP-770 and APP-751 (Plasmids pMTI-2339, pMTI-2342 and pMTI-2345)

The minigene construct designed to express APP-695 was generated by inserting an ~1.3 kb SphI fragment from pVS-1 into the SphI site of pMTI-2312 (Example 4) to generate pMTI-2339 (Figures 15a and 15b). Minigene pMTI-2342 expressing the APP-770 alternate form of APP was generated by inserting the ~6.9 kb PvuI-SpeI fragment of pMTI-2363 (Table I, Figure 18a) into the PvuI-SpeI fragment of pMTI-2369 (Figure 19). Plasmid pMTI-2369 was itself generated by inserting the ~9.8 kb NotI fragment of pMTI-2339 into pNotSV2neo (Table I and Figure 19). Minigene pMTI-2345 expressing the APP-751 alternate form of APP was generated analogously by inserting the ~6.9 kb PvuI-SpeI fragment of pMTI-2368 (Table I) into the ~8.8 kb PvuI-SpeI fragment of pMTI-2369 (Table I).

B. Alternate Minigenes for Expressing C-Terminal Frame shift Mutants

1. Mutant 40-1 (Plasmid pMTI-2343)

Minigene pMTI-2343 expressing the 40-1 frameshift mutant was generated by a fragment swap. The ~6.7 kb PvuI-SpeI fragment of pMTI-2361 (Table I, Figure 18a) was inserted into pMTI-2369 (Figure 19).

C. Alternate Minigene For MC-100 Mutant

Minigene pMTI-2340 expressing the MC-100 deletion mutant was generated by deleting an ~1.7 kb KpnI-BglII fragment of pMTI-2339 (Figures 15a and 15b) and ligating using the sp-spacer-A4 synthetic linker described in Example 9 above.

D. Alternate Minigene for A4 Peptide

Minigene pMTI-2344 expressing the A4 peptide was generated by a fragment swap (Figure 19). The ~5.0 kb PvuI-SpeI fragment of pMTI-2365 (Table I, Figure 18a) was inserted into the ~8.8 kb PvuI-SpeI fragment of pMTI-2369 (Figure 19).

**EXAMPLE11**

**Preparation and Analysis of Transgenic Mice Expressing APP Minigenes**

Transgenic mice are mice that contain exogenous DNA integrated into their genomes (Gordon and Ruddle, 1981, Science 214: 1244-1246). The DNA thereby integrated is called a transgene. APP minigenes prepared as described in Examples 4-10 may be used to prepare corresponding transgenic mice expressing these transgenes. The technical aspects of the procedure for preparing transgenic mice have been the subject of extensive review by Gordon and Ruddle, 1983, Methods Enzymol. 101C: 411-433, and Hogan et al., 1986, Manipulation of the Mouse Embryo; A Laboratory Manual, Cold Spring Harbor Lab., Cold Spring Harbor, NY, and are hereby incorporated by reference.

The general procedure involves gene transfer by microinjection. Fertilized 1-cell mouse embryos are dissected from superovulated female mice [strain: Hsd:(ICR)BR] mated with male mice (strains: Hsd: (ICR)-BR or B6D2F$_1$/HsdBR). Transgenic mice generated from a homozygous, or inbred, strain of mice are created using embryos from C57BL/6NHsdBR mating partners. Embryos are cultured in vitro as described in Hogan et al. (supra). Microinjections were performed as described in DePamphilis et al., (supra). Approximately 100 to 500 copies of a linear NotI fragment (~6-11 kb in size) of an APP minigene (listed in Table II) are loaded into a microinjection pipet and expelled into one of the pronuclei of a 1-cell mouse embryo. Approximately 1 to 3 pl of DNA injection fragment solution (approximately 5-10 µg/ml linear DNA, 0.3 mM EDTA, and 10mM Tris pH 7.5) is injected into a pronucleus of each 1-cell embryo. During injection, mouse embryos are held in-place by a microscopic cell holder. Surviving embryos were then surgically reimplanted into pseudo-pregnant foster mice (strain: Hsd:(ICR)BR) as described in DePamphilis et al. (supra) and Hogan et al. (supra). Progeny mice are born approximately 19 days post-implantation and approximately 10-30% of the progeny are transgenic (i.e., their chromosomes carry one or more copies of the injected APP minigene) and are designated as transgenic founders. Positive transgenic mice are designated by either Southern-blot or PCR analysis of tail-biopsy DNA (See below). Transgenic founder mice are bred with appropriate partners, strain: Hsd:(ICR)BR for outbred strain background, or C57BL/6NHsdBR) for inbred strain background, to generate heterozygote F1 progeny. Transgenic siblings (F1) are then inbred to generate a homozygous (for transgene) line of mice. Glass cell-holders are constructed using borosilicate glass capillaries (1mm od. and 0.58mm id.; from Sutter Instruments Co., San Rafael, CA, part #B100-58-15) on a microfuge (de Fonbrune-type; Technical Products International Inc., St. Louis, MO). The tips of the cell-holders are fire-polished and have a diameter of approximately 50 microns. Microinjection pipets are beveled and have a diameter of approximately 2 microns at their terminus, To make microinjection pipets, glass capillaries (1mm od. and 0.78mm id.; from Sutter Instruments Co., part #B100-78-15) were pulled on a Sutter Instruments Co. micropipet puller (model #P-80/PC) and then the tips were beveled on a Sutter micropipet beveler (model #BV-10; bevel angle approximately 25° to 30°). Pulled pipets are siliconized by incubation in a glass chamber saturated with hexamethyldisilazane (HMDS; Pierce #84769) for approximately 8 hours at room temperature. Microinjections are performed on a Zeiss IM-35 inverted microscope using Nomarski optics. Microinjection pipets and cell holders are controlled using Narishigi (Japan) micromanipulators (model #MO-102M and #MN-2). The flow of the injection solution in microinjection pipets is controlled using an Eppindorf Microinjector (model #5242). Surgical reimplantations are performed using a Zeiss SV8 dissection microscope.

DNA injection fragments were isolated from vector sequences by NotI digestion and agarose gel electrophoresis. Linear DNA fragments were recovered from the agarose gels by electrophoresis onto a NA45 membrane (Schleicher and Schuell, catalog no. 23410). The NotI linear DNA fragment was recovered from the membranes according to the manufacturer's instructions. Ethidium bromide was extracted from the DNA solution using isopropanol (buffer saturated, 1 mM EDTA and 10 mM Tris pH 7.6). DNA was precipitated by addition of a half volume of 7.5 M ammonium acetate and then by 2.4 volumes of absolute ethanol. The DNA pellet was resuspended in TE buffer (1 mM EDTA and 10 mM Tris pH 7.6) and then reprecipitated in ammonium acetate and ethanol as described above. DNA was reprecipitated a total of three to four times. DNA injection fragments were finally resuspended in injection buffer (0.3 mM EDTA, and 10 mM Tris pH 7.5). DNA concentration of the fragment was estimated by ethidium bromide staining on diagnostic agarose gels against known concentrations of DNA as standards. Fragments obtained in this manner were diluted to a concentration of 5 μg/ml.

Positive transgenic mice are identified by either Southern-blot or PCR analysis of tail-biopsy DNA. Southern-blot analysis is performed as described in Wirak et al., 1985, Mol. Cell Biol. 5: 2924-2935 and Maniatis et al. (supra). PCR analysis of tail-biopsy DNA is described below.

Tail biopsies are performed by dissecting approximately 1 cm of mouse tail from each mouse. Tail segments are cut into small fragments and incubated in 1.0 ml of tail extraction buffer (0.5 mg/ml proteinase K, 0.5% SDS, 100 mM EDTA, and 50 mM Tris pH 8.0) at 55°C for 12 to 16 hours. Samples are then extracted with 1.0 ml phenol (equilibrated with 1 mM EDTA and 10 mM Tris pH 7.6). The samples were further extracted with addition of 1.0 ml of CIA (chloroform: isoamylalcohol; 24:1). Samples are centrifuged at 10,000 x g for 10 minutes at room temperature and 0.7 ml of the aqueous phase is transferred to an Eppendorf tube. DNA is precipitated at room temperature by addition of 0.07 ml sodium acetate, pH 6.0, and 0.7 ml 100% ethanol. DNA is pelleted by centrifugation at 12,000 x g for 2 minutes at room temperature. Ethanol is decanted and DNA pellets are washed with 1.0 ml 70% ethanol and samples are centrifuged at 12,000 x g for 1 minute at room temperature. DNA pellets are dried in vacuum and resuspended in 0.05 ml TE (1 mM EDTA and 10 mM Tris pH 7.6). Samples are incubated at 55°C for 5 minutes and then refrigerated overnight to rehydrate DNA. DNA concentrations were determined by reading absorbance at 260 nm in a spectrophotometer.

PCR analysis of tail-biopsy DNA was performed using two sets of oligonucleotides; one set (either oligonucleotides #11 and #12 or #40 and #41) which generates a 322 bp or 320 bp DNA fragment, respectively. These oligonucleotides amplify DNA sequences specifically from human APP minigenes. A second set of oligonucleotides (oligonucleotides #6 and #7) is included with each reaction which serves as an internal control for the PCR reaction and which amplifies a 154 bp DNA fragment from the mouse ribosomal protein L32 gene (Dudov and Perry, 1984, Cell 37: 457-468). The sequences of the oligonucleotides are as follows:

**oligonucleotide #6 (SEQ ID NO:31):**

5'-CCTCGGCCTTTGGTGTGTGTTTTATATGACATGACCCCCTTGA-3'

**oligonucleotide #7 (SEQ ID NO:32):**

5'-CACCCCTGTTGTCAATGCCTCTGGGTTTCCGCCAGTTTCG-3'

**oligonucleotide #11 (SEQ ID NO:33):**

5'-ATGAACTTCATATCCTGAGTCCATGTCGGAATTCT-3'

oligonucleotide #12 (SEQ ID NO:34) :

5'-GGCAACATGATTAGTGAACCAAGG-3'

oligonucleotide #40 (SEQ ID NO:35):

5'-GGAGGGTGCTCTGCTGGTCTTCAATTACC-3'

oligonucleotide #41 (SEQ ID NO:36):

5'-AAGGGTTTGTCCAGGCATGCCTTCCTCATCC-3'

The PCR reaction conditions are: 50 µg/ml DNA, 5.0 µg/ml of each oligonucleotide, 25 units/ml Taq polymerase (Cetus), 0.2 mM dATP, 0.2 mM dGTP, 0.2 mM dCTP, 0.2 mM TTP, 50 mM KCl, 1.5 mM $MgCl_2$, 0.01% gelatin, and 10 mM Tris pH 8.3. In many cases the oligonucleotides are end-labelled with $^{32}P$ using polynucleotide kinase as described in Example 13. The specific activity of each oligonucleotide is approximately 2 x $10^5$ cpm/µg. The PCR reactions are performed in a Perkin Elmer DNA thermal cycler using the following reaction cycles (files): twenty-one cycles of 1 minute at 94° C, then 2 minutes at 55° C, then 3 minutes at 72° C with an auto extension for sequence 3 of 10 seconds/cycle, followed by a cycle of 1 minute at 94° C, then 2 minutes at 55° C, then 12 minutes at 72° C with an auto extension for sequence 3 of 10 seconds/cycle. The samples are then maintained at 18° C until removal from thermal cycler. DNA fragments are separated by electrophoresis on a 5% polyacrylamide gel and visualized by either staining with ethidium bromide or by radioautography.

Table II shows a number of APP minigene constructs useful for the preparation of transgenic mice. Table III shows a listing of representative APP transgenic founder mice generated according to the above-described methods. The transgenic founder mice are bred to establish permanent strains as described above. Table III also summarizes RNA and protein expression of APP minigenes in various transgenic mice as described in Examples 12, 13, 14 and 15.

TABLE II

APP MINIGENE CONSTRUCTS

| Construct | Promoter and Genomic Regulatory Elements | APP cDNA Sequences | Splicing and/ or Poly-Adenylation Signals |
|---|---|---|---|
| pMTI-2310 | ~2.4kb HindIII (APP) | APP-695 (pFC4) | SV40 |
| pMTI-2314 | ~4.6kb EcoRI (APP) | APP-695 (pFC4) | |
| pMTI-2319 | | APP-770 (pFC4-770) | |
| pMTI-2320 | | APP-751 (pFC4-751) | |
| pMTI-2321 | | APP-695 +2 frame shift | |
| pMTI-2322 | | APP-695 - mutant 40-1 | |
| pMTI-2325 | | APP-695 + Chlamydia antigen | |
| pMTI-2318 | | A4 | |
| pMTI-2323 | ~4.6kb EcoRI (APP) | APP-695 (pFC4) | Mouse metallo-thionein |
| pMTI-2331 | | APP-770 | |
| pMTI-2332 | | APP-751 | |
| pMTI-2324 | | APP-695 + Chlamydia antigen | |
| pMTI-2326 | | APP-695 - mutant 40-1 | |
| pMTI-2327 | | C-100 | |
| pMTI-2337 | | MC-100 | |
| pMTI-2341 | | A4 | |
| pMTI-2329 | ~2.2 kb EcoRI/BglII mouse metallothionein | APP-695 (pFC4) | Mouse metallo-thionein |
| pMTI-2333 | | APP-770 | |
| pMTI-2334 | | APP-751 | |
| pMTI-2335 | | APP-695 + Chlamydia antigen | |
| pMTI-2336 | | APP-695 - mutant 40-1 | |
| pMTI-2330 | | APP mutant 40 - alternative con-struct | |

| Construct | Promoter and Genomic Regulatory Elements | APP cDNA Sequences | Splicing and Poly-Adenylation Signals |
|---|---|---|---|
| pMTI-2339 | ~4.6kb EcoRI (APP) | APP-695 | APP 3'-end |
| pMTI-2342 | | APP-770 | |
| pMTI-2345 | | APP-751 | |
| pMTI-2343 | | Mutant 40-1 | |
| pMTI-2340 | | MC-100 | |
| pMTI-2344 | | sp-A4 | |

**TABLE III**
**Transgenic Mouse Strains with Human APP Minigenes**

| Constructs | Strain Designation | Transgenic Founders | Gene Expression (brain) RNA | Protein |
|---|---|---|---|---|
| pMTI-2401 | HB | HB805 | . | - |
| | | HB909 | . | . |
| | | HB1002 | . | . |
| pMTI-2402 | BE | BE803 | . | + |
| | | BE1805 | . | + |
| | | BE3002 | . | + |
| pMTI-2310 | DH | DH106 | + | . |
| | | DH108 | . | . |
| | | DH110 | . | . |
| | | DH409 | . | . |
| pMTI-2314 | ED | ED106 | - | . |
| | | ED801 | + | . |
| | | ED803 | . | . |
| | | ED1001 | + | . |
| pMTI-2318 | AE | AE101 | ++ | . |
| | | AE201 | + | . |
| | | AE601 | + | . |
| | | AE301 | ++ | . |
| | | AE302 | - | . |
| pMTI-2319 | JE | JE711 | + | - |
| | | JE1005 | - | - |
| | | JE1308 | . | . |
| pMTI-2320 | IE | IE205 | - | - |
| | | IE206 | + | - |
| | | IE301 | + | - |
| | | IE302 | . | . |
| | | IE504 | + | - |
| | | IE505 | - | - |
| | | IE508 | . | . |
| | | IE602 | - | - |
| | | IE606 | + | - |
| | | IE608 | . | . |
| | | IE801 | ++ | + |
| | | IE803 | - | - |
| pMTI-2321 | FE | FE403 | ++ | . |
| | | FE803 | + | . |
| | | FE805 | . | . |
| | | FE1001 | + | . |
| pMTI-2322 | GE | GE106 | . | . |
| | | GE107 | - | . |

| Constructs | Strain Designation | Transgenic Founders | Gene Expression (brain) RNA | Protein |
|---|---|---|---|---|
| pMTI-2323 | DM | DM101 | ++ | . |
| | | DM301 | . | . |
| | | DM309 | - | . |
| | | DM405 | + | . |
| | | DM407 | . | . |
| | | DM406 | + | . |
| | | DM606 | + | . |
| | | DM706 | . | . |
| | | DM1007 | . | . |
| | | DM1102 | . | . |
| | | DM1107 | . | . |
| | | DM1110 | . | . |
| pMTI-2329 | DL | DL110 | . | . |
| | | DL413 | . | . |
| pMTI-2331 | JM | JM201 | . | . |
| | | JM316 | . | . |
| pMTI-2344 | SA | SA110 | . | . |
| | SA | SA602 | . | . |
| | SA | SA706 | . | . |
| pMTI-2343 | FA | FA105 | . | . |
| | | FA201 | + | . |
| | | FA501 | . | . |
| | | FA510 | . | . |
| | | FA1001 | . | . |
| pMTI-2340 | CA | CA507 | . | . |
| | | CA408 | . | . |
| | | CA507 | + | . |
| | | CA1102 | . | . |
| | | CA3603 | . | . |
| | | CA3701 | . | . |
| | | CA3704 | . | . |
| | | CA4402 | . | . |
| | | CA4404 | . | . |
| pMTI-2342 | JA | JA407 | ++ | + |
| | | JA1301 | ++ | + |
| | | JA1302 | . | . |

\+ EXPRESSION
\- EXPRESSION NOT WITH LIMITS OF DETECTION
. NOT DETERMINED

## EXAMPLE12

### Tissue-Specific Expression of APP Minigene in Transgenic Mice Using the lacZ Reporter Gene

The design of recombinant minigenes is a critical step in the generation of a transgenic mouse model for A4-amyloidosis. An essential element is a gene-regulatory region required for tissue-specific gene expression. Minigene constructs should exhibit expression patterns in transgenic animals which are consistent with the occurrence of amyloid in AD brains and preferentially resemble expression patterns of the endogenous mouse APP gene. For this purpose, the human APP gene regulatory region was isolated as described in Example 1, and utilized for the construction of APP minigenes. To monitor the tissue specificity of this human regulatory element in transgenic mice, a reporter gene, E. coli lacZ, encoding $\beta$-galactosidase was utilized. This reporter gene allows for the convenient histochemical localization of protein expression regulated by the human APP gene regulatory region. Using this reporter gene, the 5'-end sequences of the human APP gene were demonstrated to contain sufficient information to target expression in the CNS of transgenic animals with patterns consistent with endogenous mouse APP gene expression as follows.

The minigene pMTI-2402 (Figure 20) was constructed by fusing the ~4.6 kb EcoRI fragment described in Example 1 comprising the human APP gene regulatory region including the APP promoter, with the lacZ reporter gene in the following steps. First, the cloning vector pMTI-2301 was prepared. Plasmid pMTI-2301 contains a unique HindIII cloning site, flanked by NotI restriction sites, and was constructed as described in Example 4. Second, the ~4.6 kb EcoRI fragment isolated from the chromosome 21 library as described in Example 1 encompassing the 5'-end of the human APP gene was inserted by blunt-end ligation into the HindIII site of pMTI-2301 to generate pMTI-2307. Finally, minigene pMTI-2402 containing the lacZ gene was constructed by inserting an ~3.9 kb HindIII-BamHI fragment from plasmid pCH126, containing the lacZ fusion protein and SV40 polyadenylation signal, into the NruI site of pMTI-2307 by blunt-end ligation. Plasmid pCH126 is identical to plasmid pCH110 described by Hall et al., 1983, J. Mol. Appl. Gen. 2: 101-109, except that the SV40 promoter (the PvuII-HindIII fragment. See Figure 1 in Hall et al., 1983, J. Mol. Appl. Gen. 2: 101-109.) has been deleted but the HindIII site remains. (Goring et al., 1987, Science 235: 456-458).

Plasmid pMTI-2402 DNA was double-purified in CsCl/ethidium bromide equilibrium density gradients. The ~8.6 kb linear DNA fragment, encompassing the APP/lacZ reporter gene, was excised from vector sequences using NotI and isolated from an agarose gel using NA45 paper (Schleicher and Schuell, Keene, NH). The DNA was precipitated in ethanol-ammonium acetate three times and resuspended, at a concentration of 6 $\mu$g/ml, in filtered (0.2 $\mu$ membrane) injection buffer (10 mM Tris, pH 7.5, and 0.3 mM EDTA; Brinster et al., 1985, Proc. Natl. Acad. Sci. USA 82: 4438-4442). Purified DNA fragments were microinjected into 1-cell embryos of Hsd:(ICR)BR female mice and B6D2F$_1$/Hsd BR male mice and reimplanted into Hsd:-(ICR)BR female mice as described (DePamphilis et al, 1988, BioTechniques 6: 662-680). Transgenic founder mice were identified by PCR analysis of tail biopsy DNA using 30 bp oligonucleotides #15 and #16, complementary to the E. coli lacZ gene and internal control oligonucleotides #6 and #7 (described in Example 11). The sequence of oligonucleotides #15 (SEQ ID NO:37) and #16 (SEQ ID NO:38) are as follows:

#15 5'-CCTGGCGTTACCCAACTTAATCGCCTTGCAGCACAT-3'

#16 5'-AATAAATGTGAGCGAGTAACAACCCGTCGGATTCT-3'

DNA from transgenic mice was further analyzed by restriction enzyme digestion and Southern-blot analysis (Wirak et al., 1985, Mol. Cell. Bio. 5: 2924-2935).

A. In situ hybridization

In situ hybridization techniques were used to establish the cellular distribution of APP mRNA in normal mice. The distribution of APP mRNA within the central nervous system (CNS) of other species (humans, primates, rats) has been previously determined with the majority of the CNS APP mRNA localized to neuronal cytoplasm. For these experiments, four-to-five-week-old mice were anesthetized and perfused with 4% paraformaldehyde in 0.08 M phosphate buffer, pH 7.6. The following tissues were removed and processed to paraffin by standard procedures: cerebral hemispheres plus diencephalon, pons, medulla, cervical and lumbar spinal cord, trigeminal nerve, and liver. All tissues from individual mice were embedded in a single block. Sections were cut at a thickness of 6 $\mu$m and hybridized according to published procedures (Trapp et al., 1987, Proc. Natl. Acad. Sci. USA 84:7773-7777; Trapp et al., 1988, J. Neuro Sci. 8: 3515-3521). Briefly, pre-hybridization treatment consisted of 0.2 N HCl for 20 minutes and 25 $\mu$g/ml protease K for 15 minutes at 37° C. Slides were hybridized at room temperature for 16 hours in a standard buffer containing 0.2 ng/$\mu$l of single-stranded, full-length human APP cDNA, labeled with $^{35}$S by the Klenow procedure (specific activity, 2.3 x $10^9$ cpm/$\mu$g). Stringency washes included 50% formamide containing 0.3 H NaCl, 1 mM EDTA, and 5 mM Tris (pH 8.0) for 30 minutes at room temperature and 2 X SSC (1 X SSC = 0.3 M NaCl, 0.03 M sodium citrate, pH 7.4) in 1 mM EDTA for one hour at 55° C. Slides were then dehydrated, air dried, dipped in emulsion (Kodak, NTB-3), exposed for 7 days, developed for autoradiography and counter-stained with hematoxylin. Sections were photographed with a Zeiss Axiophot using dark-field and bright-field optics. Specific brain regions and neuronal subpopulations were identified according to published criteria (Sidman et al., 1971, Atlas of the Mouse Brain and Spinal Cord, Harvard University Press, Cambridge, MA). Silver grains, representing APP mRNA, occurred in clusters that reflected the general distribution of neurons in all brain regions studied (Figure 21). For example, neuronal perikarya present in the pyramidal layer of the hippocampus and granular layer of the dentate gyrus were labeled intensely (Figure 21a). Significantly less hybridization signal was detected in other layers of the hippocampal formation in subcortical white matter. The cerebral cortex contained significant levels of APP mRNA (Figure 21a); and the labeling pattern in various cortical areas (i.e., occipital, temporal, and frontal) reflected the distribution of neuronal perikarya in the various layers. Layer I, which contains few neurons, had the lowest hybridization signal in all cortical regions. In sections of cerebellar cortex, Purkinje and granular cells were labeled by APP cDNA (Figure 21b). Sections of trigeminal ganglia (from peripheral nervous system) were hybridized with APP cDNA and the neuronal perikarya, which occur in clusters, were labeled intensely but little hybridization signal was found in myelinated fiber tracts in the PNS (Figure 21c). APP mRNA was not detected in sections of liver (Figure 21d), a finding consistent with Northern-blot analysis (Yamada et al., 1989, Biochem. Biophys. Res. Commun. 158: 906-912). The distribution of silver grains was concentrated within perinuclear cytoplasm of neurons (Figure 21e). Few silver grains were present over neuronal nuclei or scattered throughout the neurophil.

B. Histochemical Detection of β-Galactosidase

For the light microscopic histochemical detection of β-galactosidase in the transgenic mice carrying the APP/lacZ reporter gene, transgenic mice and normal mice as controls, four-to-five-weeks of age, were anesthetized and perfused with 4% paraformaldehyde and 0.08 M phosphate buffer, pH 7.6. The CNS, trigeminal nerve, and liver were removed and placed in the fixative overnight at 4°C. These tissues were cut into 0.5 cm thick slices that were either stained histochemically for β-galactosidase or sectioned at a thickness of 20 µm on a vibrating microtome prior to staining.

β-galactosidase activity was detected histochemically by incubating the tissue in a reaction buffer [2.7 mM $KH_2PO_4$, 8.0 mM $Na_2HPO_4 \cdot 7H_2O$, pH 7.6, 2.7 mM KCl, 140 mM NaCl, 2 mM $MgCl_2$, 22.5 mM $K_4Fe(CN)_6$, 25.0 mM $K_3Fe(CN)_6$, 0.27 mg/ml sodium spermidine, 0.5 mg/ml X-gal (20 mg/ml stock in diethylformamide), 0.02% NP-40, and 0.01% sodium deoxycholate] that was maintained at 30°C for 18 to 24 hours. Vibratome sections were infiltrated with 100% glycerol, mounted on glass slides, and then photographed with a Zeiss Axiophot microscope using bright-field or Nomarski optics.

The tissue and cellular expression pattern of an APP promoter-lacZ reporter gene in adult transgenic mice as determined by the above-described histochemical method was strikingly similar to the distribution of endogenous mouse and endogenous human APP mRNA. Minigene pMTI-2402, for the expression of the reporter gene in transgenic mice, was constructed as described above by inserting sequences encoding a lacZ fusion protein and SV40 polyadenylation signals into an ~4.5 kb genomic fragment encompassing the 5'-end of the human APP gene. The genomic fragment contains 2831 bp of sequences 5' to the primary transcriptional start site, exon I, and approximately 1.6 kb of the first intron. Three lines of transgenic mice were identified which carried multiple head- to-tail integrations of the intact reporter gene (Table III). Tissue distribution analysis of the three lines showed that one line, BE803, exhibited intense β-galactosidase expression throughout the CNS, while two lines (BE1805 and BE3002) exhibited lower levels of expression.

In adult BE803 mouse brain, staining was concentrated in regions having high concentrations of neuronal perikarya (Figures 22 and 23a-c). Thus, cerebral cortex, dentate gyrus, basal ganglion, thalamus, and regions of the hippocampus were stained intensely. Prominent white matter tracts such as the corpus callosum and internal and external capsule were not stained. Staining of brain stem and spinal cord tissue was observed in a pattern similar to endogenous mouse APP mRNA. β-galactosidase was not detected in slices of normal mouse brain, used as a control (Figure 23d).

Regions of cerebellar cortex that contain high concentrations of neuronal perikarya were positive for β-galactosidase (Figures 24a and 24b) as were neuronal perikarya in the trigeminal ganglion. White matter tracts in the cerebellum and trigeminal nerve (Figure 24c), and slices of liver (Figure 24d) from BE803 mice were negative for β-galactosidase. Identical β-galactosidase staining patterns were observed in tissue slices from several BE803 transgenic mice. The cellular and subcellular distribution of β-galactosidase was determined in several brain regions by light microscopic procedures. β-galactosidase was localized histochemically in 20-µm-thick vibratome sections. In these sections, β-galactosidase reaction product occurred as small dots that were restricted to regions of the CNS that contained neuronal perikarya. Reaction product was detected in all layers of the cerebral cortex (Figure 25a), including occasional deposits in Layer I. When examined at higher magnification with Nomarski optics, β-galactosidase reaction product was restricted to perinuclear regions of neurons (Figures 25b and 25c). β-galactosidase was not detectable in endothelial cells and cellular perikarya within white matter tracts. In the cerebellar cortex, β-galactosidase was localized in perinuclear regions of Purkinje cells (Figure 24b). Analysis of vibratome sections also detected the presence of β-galactosidase in regions of the CNS that were not labeled intensely in the brain slices. For example, consistent but weak staining of some but not all neurons in CA-3 region of the hippocampus was found.

C. Immunocytochemical Detection of β-Galactosidase

For the electron microscope (EM) immunocytochemical detection of β-galactosidase, transgenic mice, between four-to-five-weeks of age, were perfused with 2.5% glutaraldehyde and 4% paraformaldehyde in 0.08 M phosphate buffer. The brains were removed and placed in the fixative overnight at 4° C. Segments of the cerebral cortex (2 $mm^2$) were infiltrated with 2.3 M sucrose and 30% polyvinyl pyrrolidon, placed on specimen stubs, and frozen in liquid nitrogen. Ultrathin frozen sections (~120 nm-thick) were cut in a Reichert Ultracut-FC4 ultracryomicrotome maintained at approximately -110° C. The sections were transferred to formvar and carbon-coated hexagonal mesh grids and stained by immunogold procedures using a modification of standard procedures. Following immunostaining, the grids were placed in PBS containing 2.5% glutaraldehyde for 15 minutes and rinsed. The sections were stained with neutral uranyl acetate followed by embedding in 1.3% methylcellulose containing 0.3% uranyl acetate. Grids were examined in a Hitachi H600 electron microscope.

The cellular and subcellular distribution of β-galactosidase in the cerebral cortex and other brain regions as determined by the immunogold procedure revealed that the majority of gold particles in electron micrographs was localized to the perinuclear cytoplasm of neurons (Figure 25d). Glial cells and endothelial cells were not labeled.

The striking conclusion from the in situ hybridization, light microscopic and electron microscopic detection of mouse APP and β-galactosidase was that the ~4.5 kb genomic fragment encompassing the 5'-end of the human APP gene isolated as described in Example 1 had sufficient sequence information to direct cell- and tissue-specific expression of a reporter gene, E. coli lacZ, in transgenic mice. The expression pattern of the reporter gene in the CNS was strikingly consistent with the expression pattern of the endogenous mouse APP gene. This ~4.5 kb genomic fragment which includes the APP promoter and perhaps other regulatory elements was incorporated in nearly all APP minigene constructs described in Examples 4-10 above. Such constructs are particularly useful in the preparation of transgenic mice as described in Example 11. The identification of such an appropriate gene promoter and other regulatory elements for minigene constructs is a critical step for the development of transgenic mouse models for AD, since AD pathology is restricted to specific regions of the brain [Price, 1986, Annu. Rev. Neurosci. 9: 489-512). The ~4.5 kb genomic fragment described and characterized herein is the type of regulatory element that must be utilized to facilitate the expression of recombinant APP genes with a cell and tissue specificity that is consistent with the formation of amyloid plaque and the expression patterns of the APP gene.

## EXAMPLE13

### Expression of Human APP mRNA in Transgenic Mice

Several transgenic mouse lines express human APP mRNA in brain tissue (Figures 26, 27, and 46). Expression of human APP mRNA in transgenic animals was determined by nuclease S1 protection analysis (Figures 26 and 27) and by riboprobe analysis (Figure 46).

A. Nuclease S1 Protection Analysis

S1 nuclease digests single-stranded DNA and RNA but not double-stranded species. Therefore, specific $^{32}P$-labeled oligonucleotides that hybridize with complementary mRNA sequences, are protected from digestions by S1 nuclease and can be identified by denaturing polyacrylamide gel electrophoresis (PAGE). A human specific oligonucleotide (designated oligonucleotide #29 and described below) will produce an approximately 70 bp-protected fragment in an S1 digestion when annealed to human APP mRNA. A mouse-specific oligonucleotide (designated oligonucleotide #30 and described below) will produce an approximately 50 bp-protected fragment when annealed to mouse APP mRNA in an S1 assay. RNA from the human cell line, Hela (A.T.C.C. No. CCL2) was used for a positive control for human APP RNA (Hela cells express APP; Weidemann et al., 1989, Cell 57: 115-126) and RNA from a control non-transgenic mouse was used for a negative control in the assay.

Oligonucleotides complementary to either human (oligonucleotide #29) or mouse (oligonucleotide #30) APP mRNA sequences were synthesized using an automated Applied Biosystems oligonucleotide synthesizer (model 380A). Oligonucleotides were generated using reagents and protocols provided by the manufacturer. The sequence of oligonucleotide #29 (SEQ ID NO:39) is:

5'-GAGATAGAATACATTACTGATGTGTGGATTAATTCAAGTTCAGGCATCTACTTGTGTTACA
GCACAGCTGGGCGTCCATA-3'

This 80 bp oligonucleotide contains a 10 bp non-homologous sequence domain at the 3'-end so that, after S1 digestion, the protected oligonucleotide fragment (approximately 70 bp) can be distinguished from non-hybridized oligonucleotide probe. The actual size of the protected fragment(s) can only be determined by experimentation because specific single- and double-stranded nucleotide sequences exhibit variability in their sensitivity to S1. The sequence of oligonucleotide #30 (SEQ ID NO:40) is:

5'-CGCGGGTGGGGCTTAGTTCTGCATTTGCTCAAAGAACTTGTAAGTTGGATAGGTTCCAAG-3'

This 60 bp oligonucleotide contains a 10 bp non-homologous sequence domain at the 3'-end so that, after S1 digestion, the protected oligonucleotide fragment (approximately 50 bp) can be distinguished from non-hybridized oligonucleotide probe.

The 5'-end of each oligonucleotide was labeled with $^{32}P$ using T4 polynucleotide kinase and [gamma-$^{32}P$]dATP. The reaction conditions were as follows: 200 ng oligonucleotide, 1 µl (10,000 units/ml) polynucleotide kinase (NEB), 1.0 mCi[gamma-$^{32}P$]dATP (3000 Ci/nmole; Amersham PB15068), 1X kinase buffer (Maniatis et al., supra), and incubation at 37° C for 45 minutes. Unincorporated nucleotide was removed by gel-filtration (Sephadex G-50). The specific activity of each probe was: oligonucleotide #29, 6.04 x $10^8$ cpm/µg; oligonucleotide #30, 5.72 x $10^8$ cpm/µg.

RNA was extracted from mouse brain and Hela cell pellets using a procedure described in Basic Methods in Molecular Biology (Davis et al., 1986, Elsevier, New York, Amsterdam, and London; pp. 130-135).

Total RNA, 50 µg/sample, was mixed with 1 x $10^6$ cpm of each $^{32}P$-labelled oligonucleotide (oligonucleotide #29 and oligonucleotide #30) and then dried in vacuum. The RNA/oligonucleotide pellet was resuspended in 20 µl of Hybridization buffer (1 mM EDTA, 0.4M NaCl, 50% formamide, and 40 mM Pipes pH 6.4). Hybridization was performed in a Perkin Elmer Cetus DNA Temperature Cycler (model #PCR-10000). Samples were incubated at 90° C for 10 minutes and then at 70° C for 20 minutes. The temperature was then lowered 1° C every 18 minutes until the temperature reached 30° C. The reaction was terminated by placing samples on ice. S1 nuclease digestion was initiated by addition of 300 µl of S1 reaction buffer (0.2 M NaCl, 5 mM $ZnCl_2$, 30 mM sodium acetate pH 4.5, and 400 units S1) and samples were incubated at 20° C for 2 hours. S1 reaction was terminated by adding EDTA to a final concentration of 25 mM. Samples were extracted with equal volumes of phenol and then phenol/chloroform/isoamylalcohol (24/24/1). The oligonucleotides in each sample were precipitated at -70° C for 1 hour by addition of 10 µg tRNA, 175 µl of 7.5M $NH_4$-acetate, and 875 µl of absolute ethanol. The oligonucleotides were resuspended in 10 µl of 10 mM Tris and 1 mM EDTA, pH 7.6. Samples were denatured by addition of 10 µl of 2X Sequencing loading Buffer (from USB) and incubation at 90° C for 3 minutes. Samples were then transferred to ice and then loaded onto a 10% denaturing polyacrylamide gel (1X TBE and 7M urea) that had been prerun for 20 minutes at 1600V, constant voltage. The samples were electrophoresed at 1600 V for approximately one hour. The gel was dried and the migration of the oligonucleotides was detected by autoradiography using Kodak X-ray film.

Figure 26 demonstrates that transgenic lines AE301, AE101, FE801, FE403, ED1001, ED801, and DH106 express human APP RNA in brain (i.e., have an approximately 70 bp-protected fragment after S1 digestion). The intensity of the approximately 70 bp band of the protected fragment in these samples was greater than the background observed in control mouse brain RNA (lane 3). The level of human-specific expression, however, is low compared to the endogenous mouse APP expression level. For size markers: gel lane 1 contains oligonucleotides 29 and 30 (9.7 x $10^2$ and 6.1 x $10^2$ cpm respectively) and lane 2 contains a 1 bp DNA sequencing ladder. Both oligonucleotides 29 and 30 were annealed to 50 µg of brain RNAs and samples were digested with S1 nuclease as described below. The gel contains the following RNA samples: lane 3, mouse normal brain; lane 4, Hela cell; lane 5, AE301 brain; lane 6, AE302 brain; lane 7, AE601 brain; lane 8, AE101 brain; lane 9, FE801 brain; lane 10, FE403 brain; lane 11, ED1001 brain; lane 12, ED106 brain; lane 13, ED801 brain; lane 14, JE711 brain; lane 15, JE1005 brain; lane 16, DH106 brain; lane 17, GE107 brain.

Figure 27 demonstrates that transgenic lines IE504, IE801, IE301, IE606, IE206, DM101, DM405, DM406, and DM606 express human APP RNA in brain (i.e., have an approximately 70 bp-protected fragment after S1 digestion). The intensity of the approximately 70 bp band of the protected fragment in these samples was significantly greater than the background observed in control mouse brain RNA (lane 3). The level of human-specific expression, however, is low compared to the endogenous mouse APP expression level. For size markers: gel lane 1 contains oligonucleotides 29 and 30 ($9.7 \times 10^2$ and $6.1 \times 10^2$ cpm respectively) and lane 2 contains a 1 bp DNA sequencing ladder. Both oligonucleotides 29 and 30 were annealed to 50 µg of brain RNAs and samples were digested with S1 nuclease as described below. The gel contains the following RNA samples: lane 3, normal mouse brain; lane 4, Hela cell; lane 5, IE602 brain; lane 6, IE504 brain; lane 7, IE801 brain; lane 8, IE301 brain; lane 9, IE205 brain; lane 10, IE606 brain; lane 11, IE206 brain; lane 12, IE505 brain; lane 13, IE803 brain; lane 14, DM101 brain; lane 15, DM309 brain; lane 16, DM405 brain; lane 17, DM406 brain; lane 18, DM606 brain.

B. Riboprobe Analysis

RNase A and RNase T1 digest single-stranded RNA but not double-stranded RNA species. Therefore, specific riboprobes ($^{32}P$-labelled anti-sense RNA) that hybridize with complementary mRNA sequences, are protected from digestion by a cocktail of RNase A and RNase T1 and can be identified by denaturing polyacrylamide gel electrophoresis (PAGE). The Bluescript M13 phagemid (Stratagene, San Diego, CA) contains a multiple restriction enzyme polylinker flanked by promoters for T7 and T3 RNA polymerase. The promoters are positioned in opposite orientations and can be utilized to transcribe $^{32}P$-labelled anti-sense RNA probes specific to any sequence inserted into the polylinker region. Clone pMTI-2371 (see Example 16, part B, and Figure 41) contains the human APP sequences encoding the MC-100 gene product (gene product V, Figure 4b; see also Figure 12 and Example 9, part A, section 3) inserted into Bluescript KS+. A riboprobe which specifically hybridizes to human APP mRNA was generated using T7 RNA polymerase and linearized pMTI-2371 (phagemid digested with HincII) as template. The riboprobe was ~408 bp in length and the portion complementary to human APP was ~373 bp. Therefore, RNase A/RNase T1 digestion of the riboprobe, which has been hybridized with human APP mRNA, would generate an ~373 bp-protected fragment. RNase A/RNase T1 digestion of riboprobe, which has been hybridized with mouse APP mRNA, would result in numerous fragments which are considerably smaller than 373 bp. The template was prepared and the $^{32}P$-labelled riboprobe was generated (using 60 µCi of $^{32}P$-rUTP [sp. act.: 800 mCi/mmol] obtained from Amersham (Arlington Heights, IL). RNA was prepared from the Hela cell line, the brain of a normal mouse, and the brains of individuals from the following lines of transgenic mice: AE101, AE301, CA507, FA201, FE1001, FE403, IE801, JA407, JA1301, SA110, SA602, and SA706 using methods described in Example 13, part A. RNA samples (20 µg) were precipitated with 1/10 volume, 3 M sodium acetate pH 5.2, and 2.5 volumes ethanol. Each RNA sample was resuspended in 20 µl of 1X hybridization buffer (80% formamide, 40 mM PIPES pH 6.4, 0.4 M NaCl, and 1 mM EDTA) and 10 µl of riboprobe ($2 \times 10^5$ cpm in 1X hybridization buffer). Samples were incubated at 85° C for 10 minutes and then incubated at 45° C overnight. The RNA samples were digested by addition of 350 µl of ribonuclease buffer (10 mM Tris pH 7.5, 300 mM NaCl, and 5 mM EDTA) with 40 µg/ml RNase A and 2 µg/ml RNase T1 and incubation at 30° C for 60 minutes. To each sample was added 20 µl of 10% SDS and 2.5 µl of 20 mg/ml proteinase K. Samples were incubated at 37° C for 15 minutes and then extracted with phenol/isoamylalcohol/chloroform. The samples were precipitated by addition of 10 µg of tRNA and 1 ml of ethanol. Samples were resuspended and electrophoresed on a denaturing polyacrylamide/urea gel as described in Example 13, part A. The gel represented in Figure 46 contains the following RNA samples: lane 1, Hela cell RNA; lane 2, normal mouse; lane 3, AE301; lane 4, AE301; lane 5, AE101; lane 6, CA507; lane 7, FA201; lane 8, FE1001; lane 9, FE403; lane 10, IE801; lane 11, JA407; lane 12, JA1301; lane 13, SA110; lane 14, SA602; lane 15, SA706; lane 16, blank; lane 17, riboprobe (undigested); and lane 18, riboprobe (undigested). The protected riboprobe fragments were detected by autoradiography as shown in Figure 46. The experiment demonstrated that the following transgenic mouse lines express human APP RNA: AE101, AE301, CA507, FE1001, IE801, JA407, JA1301, SA602, and SA706 (see Table III).

## EXAMPLE14

### Expression of Human APP and APP Derivatives in Transgenic Mice

A. Expression of APP-751

Transgenic mouse line IE801 (see Table III) expresses human APP-751 protein in the brain (Figure 28a and 28b). Human APP-751 expression (Figure 28b) was detected in protein extracts of transgenic mouse brain by Western-blot analysis using the human-specific monoclonal antibody (mAb), mAb 56-1 (see Example 17). Western-blots of protein extracts from transgenic mouse brains were also stained using mAb 22C-11 which reacts with APP-695, APP-751 and APP-770 from both human and mouse (Figure 28a). The monoclonal antibody, mAb 22C-11, was a gift from Dr. Beyruether (Weidemann et al., 1989, Cell 57: 115-126).

Figure 28a contains the following samples: lane 1, low molecular weight protein markers; lane 2, DH106 brain lysate; lane 3, DM606 brain lysate; lane 4, JE711 brain lysate; lane 5, IE508 brain lysate; lane 6, IE801 brain lysate; lane 7, IE301 brain lysate; lane 8, normal mouse (ICR strain) brain lysate; lane 9, media from cell line, cMTI-53; and lane 10, high molecular weight protein markers.

Figure 28b contains the following samples: lane 1, high molecular weight protein markers; lane, 2, cell line cMTI-53; lane 3, normal mouse (ICR) brain lysate; lane 4, IE301 brain lysate; lane 5, IE801 brain lysate; lane 6, IE508 brain lysate; lane 7, JE711 brain lysate; lane 8, DM606 brain lysate; lane 9, DH106 brain lysate; and lane 10, low molecular weight protein markers.

Figure 28a demonstrates that each brain extract contains approximately equal amounts of APP protein and that APP-695 is the predominant form of APP in mouse brain extracts. The extracellular forms of APP-695 and APP-751 (or 770) have apparent molecular weights of ~93-105 kDa and ~112-125 kDa respectively (Weidemann et al., 1989, supra and Palmert et al., 1989, Proc. Natl. Acad. Sci. USA 86: 6338-6342) Protein from the culture media of a mouse cell line (line cMTI-53; see Example 16) which secretes human APP-751 was included as control (Figure 28b, lane 2). We could not determine whether transgenic mouse lines DH101 or DH106 expressed human APP-695 because of the cross-reactivity of mAb 22C-11 for mouse and human APP-695.

Figure 28b demonstrates that transgenic mouse line IE801 (lane 5) expresses a protein which reacts with mAb 56-1 and has a gel migration mobility equal to that of APP-751 secreted by the cell line cMTI-53 (lane 2). A non-transgenic mouse (lane 3) or transgenic mice carrying minigenes encoding human APP-695 (DH101 and DH106) do not exhibit immunostaining of this protein. Transgenic mouse line IE508 also expressed cross-reactive proteins species. However, the migration of the proteins does not correspond to human APP-751. It is possible that human APP-751 is anomalously expressed or metabolized in the IE301 line and no APP-770 expression was observed in the JE711 line.

Protein was extracted from the brain of a non-transgenic control mouse (ICR strain) and the brains of transgenic animals from the following lines: DH106, DM606, JE711, IE508, and IE301. Whole brains were dissected from the animals and weighed to estimate tissue volume. Two volumes of lysis buffer (0.2M NaCl, 1% Triton X-100, 2 mM PMSF (Sigma #P-7626), 1 mM DFP, 1X protease inhibitor solution, 10 mM Tris pH 8.0) was added to each brain. Protease inhibitor solution, 100X, consisted of: 1 mg/ml leupeptin (Sigman #L-2884), 1 mg/ml pepstatin-A (Sigman #P-4265), 10 TIU/ml aprotinin (Sigma 3A-6012), 0.1 mM EDTA, and 0.2H Tris pH 8.0. Brain tissue was then homogenized for ~1 minute with a Polytron homogenizer (model CH6010). Each sample was centrifuged at 10,000 x g at 4°C for 30 minutes and the supernatant (lipid layer removed), or "brain lysate," was stored at -70°C. Protein in culture media for cell line cMTI-53 was concentrated by acid precipitation. Approximately 1.5 ml of culture media, ice cold, was harvested and a 1.5 ml aliquot of 25% trichloroacetic acid (TCA), ice cold, was added. Samples were centrifuged at 15,000 x g for 10 minutes at room temperature. The protein pellets were washed three times with 100% acetone and then centrifuged after each wash at 15,000 x g for 10 minutes at room temperature. The pellets were dried in a vacuum for ~20 seconds, resuspended in 100 µl of NRSB buffer (2% SDS, 5% betamercaptoethanol, 5% loading dye, 10% glycerol, 0.125 M Tris pH 6.8) and boiled for 5 minutes.

The "brain lysate" proteins and cMTI-53 cell supernatants were fractionated by polyacrylamide gel electrophoresis (10% running gel and 4% stacking gel) and transferred to Immobilon-P membrane by the technique of electroblotting using a Biorad Mini-Protean II apparatus and using procedures recommended by the manufacturer. Prior to electrophoresis, 10 µl human APP-751 control cell supernatant (cell line cMTI-53), 1 µl of control mouse brain lysate, and 2 µl aliquots of transgenic mouse brain lysates were denatured by addition of 1X NRSB and boiling for 5 minutes. Each gel also included pre-stained high and low molecular weight standards (BRL catalog #6041LA and #6040SA, respectively).

Human and mouse APP proteins, transferred from the polyacrylamide gels onto Immobilon-P membrane, were detected by Western-blot staining. Mouse and human APP-695, APP-751 and APP-770 proteins were detected using mAb 22C-11. Human APP-751 was detected using mAb 56-1; this antibody does not recognize mouse APP-751. After protein transfer, the Immobilon membranes (12 x 12 cm) were incubated with 50 ml 1X blocking buffer (0.15M NaCl, 5% non-fat dry milk, and 10 mM Tris pH 8.0) for one hour at room temperature. Membranes were then stained with 10 ml of "first" antibody solution (22C-11: 1:10,000 dilution of mAb stock into blocking; or mAb 56-1: 1:100 dilution of mAb stock into blocking buffer) for 2 hours at room temperature. Membranes are next washed with blocking buffer and then stained with 15 ml of "second" antibody solution [goat anti-mouse IgG conjugated with alkaline phosphatase (Promega): 1:7500 dilution of antibody into blocking buffer] for 30 minutes at room temperature. Membranes are then washed with blocking buffer and then with AP buffer (0.1M NaCl, 5 mM $MgCl_2$, 0.1M Tris pH 9.5). Membranes are next stained with "AP substrate" solution (15 ml AP buffer, 99 µl NBT stock solution, and 49 µl BCIP stock solution) for one hour at room temperature. NBT stock solution consists of 50 mg/ml nitro blue tetrazolium (Sigma #N-6876) in 70% dimethylformamide and BCIP stock solution consists of 50 mg/ml 5-bromo-4-chloro-3-indolyl phosphate in 100% dimethylformamide. The AP staining reaction was determined by washing membranes in deionized water.

B. Expression of A4 APP Peptide

Transgenic mouse line AE301 (see Table III) carries minigene pMTI-2318 (gene product VIII, Figure 4b), which encodes the 42 amino acid A4 peptide of APP (see Example 7 above). This line of transgenic mice has been shown to express APP RNA in brain (see Example 13 above). In further studies, it was shown that AE301 transgenic mice exhibit A4 aggregates in the hippocampus region of the brain. This transgenic line can be used to examine the neurotoxicity of the A4 peptide in brain tissue. In addition, the A4 aggregates present in the transgenic mice may represent an early stage of senile plaque formation. These transgenic mice can serve, therefore, as a model for early pathological events occurring in patients affected with AD. Aggregation of A4 peptide was demonstrated by several methods, including immunocytochemical analysis and electron microscopic (EM) analysis.

1. Immunocytochemical Analysis of A4 Aggregates

Rabbit polyclonal antibodies (pAb) 90-25, 90-28 and 90-29, used for the immunocytochemical analysis, were generated by standard methods. Subcutaneous injections of the A4 peptide (amino acid residues 1 to 28 for pAb 90-25, and amino acids 1 to 42 for pAbs 90-28 and 90-29) were administered to rabbits using Freund's adjuvant. Rabbit sera were screened for immunoreactivity to the A4 peptide and several, including pAb 90-25, 90-28 and 90-29, tested positive. These positive antibodies were further characterized by reaction with pathological human brain tissue from a patient with AD. The pAb 90-25, 90-28 and 90-29 immunostain A4 amyloid plaques (senile plaques) found in the pathological tissues.

Once the specificity of pAb 90-25, 90-28 and 90-29 with A4 peptide had been established, these antibodies were used to immunostain cross-sections of brain tissue from mice. Light microscopic immunochemistry was performed using paraffin tissue sections, according to the method of Trapp et al., 1983, J. Neurochem. 40: 47-54. The results showed immunostaining of specific areas of the hippocampus region of the brain from an AE301 transgenic mouse as shown in Figures 34, 35, and 36. The transgenic mouse, designated AE301 + 207 (F1), used for this immunocytochemical analysis was a transgenic progeny of a mating between AE301(F0), the founder mouse, and a non-transgenic female (ICR200). Transgenic progeny of this mating were identified by PCR analysis as described in Example 11 above.

Figure 34 illustrates a cross-section of brain from mouse AE301 + 207(F1) immunostained with pAb 90-29. A4 immunoreactive regions can be observed as dark-brown areas, are punctate in nature, and appear in clusters in the hippocampus (Figure 34, representative immunostained clusters are highlighted with arrows). Figure 35 is a higher magnification of the hippocampal region of mouse AE301 + 207(F1) brain tissue stained with pAb 90-29 (representative immunoreactive regions are highlighted with arrows). Similar immunostaining in the hippocampus of AE301 + 207(F1) brain tissue can be observed with a second A4 immunoreactive antibody, pAb 90-28 (Figure 36, representative immunoreactive regions are highlighted with arrows). A third A4 immunoreactive antibody, pAb 90-25, also showed similar immunostaining.

This immunostaining was specific to the AE301 transgenic line because an age-matched mouse, designated FE803 + 105(F1), from transgenic line FE803 which carries pMTI-2321 (see Example 6 and Table II) does not exhibit immunostaining with pAb 90-29 in the hippocampus or in other regions of the cross-section of brain (Figure 37).

2. Electron Microscopic Analysis

Transmission electron microscopic analysis of thin sections of fixed and stained brain tissue was performed according to the method of Trapp et al., 1982, J. Neurosci. 2: 986-993. The transgenic mouse used for this electron microscopic analysis was designated AE301 + 201(F2) and is the progeny of a mating between AE301 + 210(F1) and AE301 + 207(F1); AE301 + 210(F1) and AE301 + 207(F1) are progeny of a

mating between AE301+(F0) and a non-transgenic female (ICR200). These transgenic progeny were identified by PCR analysis as described in Example 11 above.

The results showed electron-dense aggregates in specific areas of the hippocampal region of the brain from this transgenic mouse. The electron-dense aggregates were found in the same brain regions which exhibited immunochemical staining with pAbs 90-25, 90-28 and 90-29. The aggregates appear to be located within the intracellular space of neuron dendrites. Figures 38a and 38b illustrate electron-dense aggregates in thin sections of hippocampal brain tissue isolated from transgenic mouse AE301+201(F2). The borders of the electron-dense aggregates are highlighted with arrows.

That electron-dense regions are aggregates of the A4 peptide was demonstrated since immunoreactivity with pAb 90-29 co-localized with the electron-dense aggregates (Figure 39). This co-localization was shown using EM immunocytochemistry of ultrathin cryosections as performed according to the method of Trapp et al., 1989, J. Cell Biol. 109: 2417-2426. The immunoreactivity of pAb 90-29 was detected in the electron micrographs using immunogold particles. Immunogold particles appear as discrete dots of uniform size in the electron micrographs. Representative regions, where gold particles co-localize with the electron-dense aggregates, are indicated by arrows.

**EXAMPLE15**

**Expression of Human APP Gene Products in COS Cell Transfections**

DNA transfections of COS cells (Gluzman, 1981, Cell 23: 175-182) demonstrate that pMTI-2360, pMTI-2362, pMTI-2369, and pMTI-46 express and secrete human APP-695 as described below and shown in Figure 29.

For DNA transfections, 60 mm culture dishes were seeded with approximately 2-5 x $10^5$ COS cells/dish (~50% confluency) in 3 ml DMEM and 10% fetal calf serum. Cells were cultured overnight at 37° C in a 6% $CO_2$ atmosphere. Cells were washed with PBS (no $Ca^{++}$ or $Mg^{++}$) and then 2 ml of DMEM plus 10% NuSerum (catalog #50000) was added to each plate. Then 2 µl of 1000X chloroquine stock solution (0.1 M chloroquine, Sigma no. C-6628), 32 µl of DEAE dextran sulfate stock solution (25 mg/ml DEAE dextran sulfate, Sigma no. D-9885), and 4 µg of DNA was added to each plate. Cells were incubated for 3.5 hours at 37° C in a 6% $CO_2$ atmosphere. Cells washed with PBS and then "shocked" with 2 ml of 10% DMSO in PBS and incubated at 37° C in a 6% $CO_2$ atmosphere with DMEN plus 10% fetal calf serum for 48 hours then washed 3 times with PBS and cells were further incubated at 37° C in a 6% $CO_2$ atmosphere with "Cutter" media for an additional 24 hours.

Protein in culture media from COS cells, transfected COS cells, and human neuroglioma cell line H4 (A.T.T.C. no. HTB148) was concentrated by acid precipitation. Approximately 3 ml of each culture media, ice cold, was harvested and a 3 ml aliquot of 25% trichloroacetic acid (TCA), ice cold, was added. Samples were centrifuged at 20,000 x g for 30 minutes at 4° C. The protein pellets were washed three times with 100% acetone and then centrifuged after each wash at 10,000 x g for 15 minutes at 4° C. The pellets were dried in a vacuum for ~20 seconds, resuspended in 10 µl of NRBS buffer (2% SDS, 10% betamercaptoethanol, 5% loading dye, 10% glycerol, 0.125 M Tris pH 6.8), and boiled for 5 minutes.

Cell supernatant protein was fractionated by polyacrylamide gel electrophoresis (8% running gel and 4% stacking gel) and transferred to Immobilon-P membrane by the technique of electroblotting using a Biorad Mini-Protean II apparatus and using procedures recommended by the manufacturer. Human and mouse APP proteins, transferred from the polyacrylamide gels into Immobilon-P membranes, were detected by Western-blot staining as described in Example 14. Mouse and human APP-695, APP-751 and APP-770 proteins were detected using monoclonal antibody (mAb) 22C-11.

APP protein secreted into media from various transfected cell cultures was detected in Western-blots using mAb 22C-11. COS cells express predominately APP-751 (and/or 770) and a smaller amount of APP-695 (Figure 29, lane 8). The secreted forms of APP-695 and APP-751 (or 770) have apparent molecular weights of ~93-105 kDa and ~112-125 kDa, respectively (Weidemann et al., 1989, supra, and Palmert et al., 1989, supra). Human cerebral spinal fluid (CSF) contains predominately APP-695 (Palmert et al., 1989, supra.) and is included on the Western-blot as a control for APP-695 expression (lane 9, Figure 29). Several DNA transfections (pMTI-2360, lane 7; pMTI-2362, lane 6; pMTI-2369, lane 5; and pMTI-46, lane 4) exhibit significant increases in APP-695 immunostaining relative to APP-751(770) immunostaining (Figure 29). Therefore, these constructs express human APP-695 in COS cells. These APP-695 encoding minigenes are used as a "template" for construction of minigenes encoding alternate or mutant forms of APP. Because the parent APP-695 constructs express protein, it is highly likely that the other constructs also will express their proteins.

## EXAMPLE16

### Expression of APPs in Mammalian Cell Lines

Stable cell lines expressing the 695, 751 and 770 forms of APP, as well as a mutated form of APP MC-100, were constructed as follows using bovine papilloma virus- (BPV) based vectors.

A. Cell Lines for APP-695, APP-751 and APP-770

Plasmid pMTI-4 described in Example 6 was mutagenized at the 5'-end of the APP-695 cDNA to create a new SalI restriction site. In addition, during the mutagenesis procedure, the bases flanking the initiation codon, AUG, were altered to conform to the optimum sequence for translation initiation described by Kozak (Kozak, 1989, Cell Biol. 108: 229-241). The oligonucleotide primer used in the mutagenesis and map of the resulting vector, pMTI-38, are shown in Figure 30a.

Plasmid pMTI-41 was constructed by deleting the unique KpnI site in Bluescript KS (Stratagene; parent vector for pMTI-4). Bluescript KS was digested with KpnI and the overhangs were digested with mung bean nuclease by standard methods. The digested DNA was treated with ligase to circularize the vector and pMTI-41, lacking the KpnI site was isolated.

The XbaI - HindIII fragment from pMTI-38, containing the APP-695 cDNA, was introduced into XbaI - HindIII digested pMTI-41 as shown in Figure 30 to obtain pMTI-42 which has only one Kpn site within the APP cDNA. pMTI-43 and pMTI-44 containing respectively the 751 and 770 forms of APP were constructed by replacing the KpnI - BglII fragment in pMTI-42 with the corresponding fragments from pFC4-751 and pFC4-770 described in Example 3.

SalI fragments containing the APP regions in pMTI-42, pMTI-43 and pMTI-44 were introduced into the XhoI site of the BPV vector pMTI-52, placing them under the control of the mouse metallothionine promoter illustrated in Figure 31. As shown in Figure 31, pMTI-52 contains the colE1 replicon, the ampicillin resistance gene, the mouse metallothionine promoter a unique cloning site for cDNAs followed directly by the polyadenylation signal of SV40. Specifically, pMTI-52 contains BamHI and XhoI cloning sites for introduction of cDNAs of interest. In addition, the pMTI-52 vector contains the entire 8 kb genome of BPV. The presence of BPV sequences allows the vector to replicate as a multicopy episome in mouse C127 and NIH3T3 cells resulting in stably transformed cell lines. The plasmid pMTI-52 was constructed by ligating the -237 bp BamHI-BclI fragment (containing the viral polyadenylation signals) from SV40 viral DNA into the unique BamHI site of pMTI-32. Diagnostic restriction digestion of pMTI-52 with BamHI and PvuII gave the following DNA restriction fragments: ~11.5 kb, ~0.55 kb, and ~0.25 kb. pMTI-32 was generated by ligating an ~1.8 kb BamHI-BglII restriction fragment from pMTI-29 (this DNA fragment contains the mouse metallothionein gene promoter, which can be obtained from alternative sources, for example, the ~1.9 kb EcoRI-BglII fragment from plasmid pJYMMT(L) described in Example 9 also contains an analogous promoter fragment) into the unique BamHI restriction site of plasmid BPV-240.7. Plasmid BPV-240.7 was used as a source of the entire BPV genome and is a variant of the BPV vectors described and prepared by Howley et al., 1983, in Methods of Enzymology, Volume 101, Wu et al., eds., Academic Press, NY, pp. 387-402. Alternative sources of the -8 kb BPV genome may be used, in particular, any number of the BPV vectors described by Howley et al., supra, with minor changes in restriction enzyme cleavage sites, can serve as a source of the BPV genome in place of BPV 240.7 in the construction of pMTI-52. Diagnostic restriction digestion of pMTI-32 with BamHI and HindIII gave the following DNA restriction fragments: 8.0 kb and 4.1 kb. pMTI-29 was generated by inserting BglII, XbaI, and SalI restriction sites (using a synthetic DNA linker) into the unique EcoRi restriction site of plasmid pMVBneo. Plasmid pMVBneo has been described by Pavlakis et al., 1987, in Gene Transfer Vectors, Miller and Calos, eds., Cold Spring Harbor Laboratory Press, Cold Spring Harbor, NY, pp. 29-58, and was used as a source of the mouse metallothionine gene promoter. Alternative sources of this promoter may be used, for example, plasmid pJYMMT(L) (see Example 9). Diagnostic restriction digestions of pMTI-29 with either SalI or XbaI yielded a single 6.7 kb DNA restriction fragment. The BPV vectors pMTI-53, pMTI-57 and pMTI-58 contain the 695, 751 and 770 forms of APP, respectively.

Each BPV vector, pMTI-53, pMTI-57 or pMTI-58 was transfected into mouse C127I cells (a variant of C127 obtained from Dr. D. DiMaio, Yale University) which are permissive for the high-copy-number, episomal replication of BPV vectors (Howley et al., 1983, Methods in Enzymol. 101 387-403). Vectors were introduced into cells by calcium phosphate precipitation and the transformed foci were isolated as described (Howley et al., 1983, supra). Alternatively, in some cases, the vectors were co-transfected (Howley et al., 1983, supra) with pSV2neo (Southern and Berg, 1982, J. Mol. Appl. Gen. 1: 327-341) which is capable of conferring resistance to the antibiotic G418. BPV vector DNAs were mixed with pSV2neo DNA at 5- to 10-fold molar excess (BPV vectors in excess) and transfected into C127I cells by calcium phosphate precipitation. Colonies resistant to G418 were isolated. The molar excess of BPV DNA over pSV2neo DNA ensured that almost every G418 resistant colony contained the cotransfected BPV vector.

Transfection of APP cDNAs into various cell types has shown that the amino-terminal region of APP, including the Kunitz domain, is released into the medium (Weidemann et al., 1989, Cell 57: 115-126 and Palmert et al., 1989, supra. Therefore, serum-free, 24-hour supernatants from transformed foci and the G418 resistant colonies were screened for the appropriate form of APP by Western-blot analysis. Proteins in 1.5 ml of supernatants from semi-confluent to confluent 25 square cm flasks were concentrated approximately 15-fold by precipitation with trichloroacetic acid (TCA) prior to loading onto polyacrylamide gels. APP bands were visualized using the mouse monoclonal antibody 22C11 (see Example 14). Clones producing high levels of the appropriate APP form were expanded and propagated in culture. Supernatants from these cell lines, cMTI-53, cMTI-57 and cMTI-58 provided standards for the three forms of human APP.

B. Cell Lines for MC-100

Further transfections of mouse C127I cells were performed using the plasmid vector pMTI-70 (~12.9 kb, Figure 40). This plasmid was constructed by cloning an ~615 bp XhoI-PvuII fragment from the vector pMTI-2371 (Figure 41) into the BPV vector pMTI-52. pMTI-52 was first digested with BamHI and then a blunt-end was generated using Klenow. The vector was then digested with XhoI and the large restriction fragment was gel-purified and ligated with the ~615 bp XhoI-PvuII fragment from the vector pMTI-2371 to generate pMTI-70. Diagnostic digestion of pMTI-70 with HindIII revealed an ~3.6 kb and an ~9.3 kb restriction fragment. The pMTI-2371 plasmid was derived by cloning the ~707 bp BamHI-SpeI fragment from pMTI-2337 between the BamHI and the XbaI sites of the Bluescript KS+ vector (see Example 6). Construction of plasmid pMTI-2337 is described in Example 9 (part A, section 3).

Plasmid pMTI-70 contains the sequences derived from pMTI-2337 which encode the mutation designated MC-100 (gene product V, Figure 4b, see also Figure 12). The fragment obtained from pMTI-2337 (with pMTI-2371 as an intermediate) used for the construction of pMTI-70 encodes the C-terminal segment common to the three forms of APP (695, 751, 770), including the A4 region, preceded by the secretion signal (i.e., signal peptide) of the APPs. Thus, translation of this APP minigene is expected to result in the incorporation of the APP C-terminus into the membrane of the cell transfected with this minigene.

Plasmid pMTI-70 was transfected into mouse C127 cells as described above and colonies resistant to G418 were isolated to generate stable transfectant cell lines which included lines: cMTI70-A2, cMTI70-A3, cMTI70-A6, cMTI70-B1, cMTI70-B2, and cMTI70-B3. Cell lysates of such resistant clones were analyzed by Western-blotting using a rabbit polyclonal antibody (pAb) SG369. The pAb SG369 (described in Buxbaum et al., 1990, Proc. Natl. Acad. Sci. USA 87: 6003-6006 was raised by immunization of a rabbit with a synthetic peptide corresponding to the C-terminus of human APP-695 using standard immunization procedures and techniques (as described in Buxbaum et al., 1990, supra)` The synthetic peptide consisted of APP amino acid residues 645-694, wherein the numbering of amino acids corresponds to those of human APP-695 as described in Kang et al., 1987, supra) and was prepared by the Yale Protein and Nucleic Acid Chemistry Facility (New Haven, CT). Rabbit polyclonal antibodies with similar characteristics as those of pAb SG369 have also been generated by other laboratories using various human APP-695 C-terminal peptides (Ishii et al., 1989, Neuropath. Appl. Neurobiol. 15: 135-147; Palmert et al., 1989, supra; and Bush et al., 1990, J. Biol. Chem. 265: 15977-15983). Figure 42 shows the results of the Western-blot analysis of cell lysates of pMTI-70 transfected BPV cell lines cMTI70-B1, cMTI70-B2, and cMTI70-B3. The Western-blot shown in Figure 42 also shows the results of using cell extracts from control BPV cell transfectants which do not express MC-100. Cell cultures were grown to 100% confluency, washed with 1 mM EDTA in PBS, and extracted by boiling for 10 minutes in 1X SSB (2% SDS, 63 mM Tris pH 6.8, and 10% glycerol), 5% β-mercaptoethanol, and 5% bromphenol blue. The Western-blot analysis was performed as described above. The Western-blot illustrated in Figure 42 contains the following samples: lane 1, molecular weight markers; lane 2, cMTI52-A4 cell extract; lane 3, cMTI66-B6 cell extract; lane 4, cMTI66-C5 cell extract; lane 5, cMTI69-C6 cell extract; lane 6, cMTI69-A4 cell extract; lane 7, cMTI69-A5 cell extract; lane 8, cMTI70-B1 cell extract; lane 9, cMTI70-B2 cell extract; and lane 10, cMTI70-B3 cell extract. Polyclonal antibody SG369 was used in this Western-blot analysis. BPV cell line cMTI52-A4 was transfected with pMTI-52 (BPV cloning vector); BPV cell transfectant lines cMTI66-B6 and cMTI66-C5 carries BPV vector pMTI-66 which encodes the A4 peptide of human APP (gene product VIII, Figure 4b; see Example 7 above); and BPV cell transfectant lines cMTI69-C6, cMTI69-A4, and cMTI69-A5 carry the BPV vector pMTI-69 which encodes the Sp-A4 peptide of human APP (gene product VII, Figure 4b; see Example 9A, section 4). A major immunoreactive band between 14 kD and 21 kD representing the product of the APP minigene is seen. Also present are immunoreactive bands of higher molecular weights consistent with their being aggregation products of the primary translation product (indicated by arrows).

The transcription of the APP minigene in pMTI-70 is under the control of the mouse metallothionine promoter which is inducible by heavy metals such as cadmium (Hamer, D.H. and Welling, M.J., 1982, J. Mol. Appl. Genet. 1: 273-288). Induction of cell lines cMTI70-A2, cMTI70-A3, cMTI70-A6, cMTI70-B1, cMTI70-B2, and cMTI70-B3 with cadmium would be expected to result in increases in mRNA levels and resultant increases in MC-100 protein levels as shown in the Western-blot illustrated in Figure 43. Cell cultures were grown to 100% confluency, washed with PBS, incubated with DMEM with 5 µg/ml cadmium chloride at 37° C in 5% $CO_2$ for 16 hours, and then extracted by boiling for 10 minutes in 1X SSB (2% SDS, 63 mM Tris pH 6.8, and 10% glycerol), 5% β-mercaptoethanol, and 5% bromophenol blue. The Western-blot analysis was performed as described above. The Western-blot illustrated in Figure 43 contains the following samples: lane 1, cMTI63-B1 cell extract; lane 2, cMTI63-C2 cell extract; lane 3, molecular weight markers; lane 4, cMTI53-A1 cell extract; lane 5, cMTI70-A2 cell extract; lane 6, cMTI70-A3 cell extract; lane 7, cMTI70-A6 cell extract; lane 8, cMTI70-B1 cell extract; lane 9, cMTI70-B2 cell extract; and lane 10, cMTI70-B3 cell extract. BPV cell transfectant lines cMTI63-B1 and cMTI63-C2 carry BPV vector pMTI-63 which encodes the human APP-695 with a C-terminal addition of the Chlamydia epitope (see Example 8) and BPV cell transfectant line cMTI53-A1 which carries BPV vector pMTI-53 which encodes human APP-695. The higher molecular weight bands corresponding to the aggregated molecules increase in intensity upon cadmium induction (as indicated by arrows). This observation is consistent with the expectation that aggregation is a concentration dependent phenomenon.

The pMTI-70 transfected and G418 selected cells were also analyzed by immunofluorescence of stained cells and immunoprecipitation of cell lysates using the SG369 antibody. The results demonstrated the accumulation of the MC-100 fragment in the transfected cells. Figures 44a and 44b show immunofluorescence results of two representative fields where a limited number of cells in the population of cMTI70-A6 cells show intense fluorescence. Transfected cell line cMTI-53 (which expresses human APP 695) does not exhibit these immunofluorescent cells (Figure 44c). Cultures of cell lines cMTI70-A6 (transfected with pMTI-70, see above) and cMTI53-A1 (express human APP 695) were grown to 70% confluency using standard culture conditions, the cells were washed with PBS, and incubated for 16 hours with DMEM supplemented with 5 µg/ml cadmium chloride. The induced cMTI53-A1 and cMTI70-A6 cells were then washed twice with PBS, and fixed using 4% paraformaldehyde in PBS at room temperature for 10 minutes. The cells were permeabilized with 0.2% Triton X-100, 10 mM Tris pH 8.0, 0.2 mM EDTA at room temperature for 5 minutes. The fixed and permeabilized cells were then incubated with affinity purified pAb SG369 (1:200 dilution of stock) in PBS and 3% bovine serum albumin (BSA) at room temperature for 60 minutes. The cells were washed 5 times with 3% BSA in PBS and then incubated with goat anti-rabbit IgG conjugated with rhodamine (obtained from Boehringer Mannheim) in PBS and 3% BSA at room temperature for 30 minutes. The cells were then washed 5 times with 3% BSA in PBS. The fluorescence of the cells was observed on mounted slides using a Zeiss IM fluorescent microscope. As shown in Figure 44, the staining is punctate in nature and localized at the cell periphery, away from the site of synthesis in the endoplasmic reticulum (ER) and Golgi. This staining pattern suggests highly localized concentrations of MC-100 protein. Upon continued passage of the C127I/pMTI-70 transfected cells, it has been observed that fluorescent cells are lost from the population with continued passage. This suggests that production of MC-100 may confer a selective disadvantage to these cells.

Figure 45 shows a Western-blot of immunoprecipitated MC-100 from extracts from the cell line cMTI70-A6 (transfected with pMTI-70, see above). The results indicate that the MC-100 aggregates, observed in Figures 42 and 43, can be immunoprecipitated from cell lysates (Figure 45, lane 6). Cultures of cell line cMTI70-A6 (transfected with pMTI-70, see above) were grown to 100% confluency using standard culture conditions, the cells were washed with PBS, and incubated for 16 hours with DMEM supplemented with 5 µg/ml cadmium chloride. The induced cMTI70-A6 cells were resuspended twice, washed with 1 mM EDTA in PBS, and the cells were pelleted by centrifugation (1000 x g) and resuspended in IP (lysis) buffer (100 mM Tris pH 7.4, 150 mM NaCl, 2 mM $NaN_3$, 1% Nonidet P-40, 0.5% sodium deoxycholate, 0.1% SDS, and 40 units/ml aprotinin). An aliquot of this cell lysate appears in Figure 45, lane 1. The cell lysate was incubated with a 1:50 dilution of affinity purified pAb SG369 for 2 hours at 4° C. The extract was then incubated with a 1:10 dilution of protein-G Sepharose (stock, 2 mg/ml in PBS; obtained from Sigma) at 4° C overnight with gentle agitation. The protein-G Sepharose beads were then collected by centrifugation (12,000 x g for 15 seconds at 4° C) and an aliquot of the supernatant appears in Figure 45, lane 2. The pellet was washed 3 times with IP (lysis) buffer. An aliquot of each wash appears in Figure 45, lanes 3, 4, and 5. The proteins were solubilized, boiling for 10 minutes in 1X SSB (2% SDS, 63 mM Tris pH 6.8, and 10% glycerol), 5% β-mercaptoethanol, and 5% bromophenol blue. An aliquot of the solubilized immunoprecipitant appears in Figure 45, lane 6. The Western-blot analysis was performed as described above using the SG369 antibody.

The C127I/pMTI-70 clones thus provide a mammalian cell host/vector system in which the aggregation of a segment of APP containing the A4 region was observed. Since this reaction is a critical step in amyloid formation, this host/vector system is valuable for studying: (i) the steps in amyloid formation by studying the aggregation process in vitro and (ii) methods for intervention into this process by characterizing chemical and physical agents that accelerate or interfere with amyloid aggregation. In addition, the MC-100 minigene in pMTI-70 may be expressed in other cell lines including neurons to study amyloid formation in different cell lines.

## EXAMPLE17

### Generation of Human APP-specific Mouse Monoclonal Antibodies, 56-1 and 56-2

Monoclonal antibodies reactive to the 56 and 75 amino acid Kunitz domain inserts of APP were generated as follows:

Immunogen: The immunogen used in the immunization of mice was the enriched pellet fraction of bacterium E. coli expressing the 75 amino acids of the Kunitz domain as a fusion to the first 36 amino acids of E. coli recA protein (Sancar et al., 1980, Proc. Natl. Acad. Sci. USA 77: 2611-2615). The fusion protein, which segregated into the pellet fraction of the expressing strain, was enriched by detergent and water washes. The resulting insoluble pellet was solubilized in 8 M urea and the urea was removed by dialysis against phosphate buffered saline (PBS). Dialysis caused the precipitation of a part of the solubilized material. The emulsion resulting from the dialysis was used to immunize mice. The recA-75 fusion represented over 30% of the protein in the emulsion.

Immunizations: Three 8-week-old Ba1b/c mice were immunized intraperitoneally with 100 μg of immunogen emulsified with an equal volume of complete Freunds adjuvant. Then, 21 and 28 days later, mice were given additional intraperitoneal injections of 100 μg of immunogen emulsified in an equal volume of incomplete Freunds adjuvant. After an additional seven days, the mice were boosted intravenously with 20 μg of immunogen. Three days later the spleens were removed and somatic cell hybrids were prepared by the method of Herzenberg (Herzenberg et al., 1978, Handbook of Experimental Immunology (D. M. Weir, ed.) Blackwell Scientific Publications, Oxford, pp. 25.1-25.7) with some modifications (Lerner et al., 1980, J. Exp. Med. 152: 1085-1101).

ELISA assay: The enriched pellet fraction containing the recA-75 fusion was dissolved in PBS and used in an ELISA assay. As a negative control, similar pellet fraction prepared from an E. coli strain expressing a fusion of the same 36 amino acids of recA (as in recA-75) with a segment of APP-695 (which does not have the Kunitz insert) was used.

The ELISA assay was conducted as follows. Culture fluids from growing hybridomas were tested for the presence of specific antibody using ELISA. 1 μg of extracts containing recA fusion proteins was allowed to adsorb to each well of Immunolon II EIA plates (Dynatech, Chantilly, VA) by overnight incubation at 4° C in 50 μl 0.01M sodium carbonate pH 9.5. Non-specific protein binding sites in each well were blocked by incubation with 200 μl PBS containing 0.05% Tween-20 and 1% BSA followed by washing with PBS/0.05% Tween-20. Wells were then sequentially incubated with 100 μl of hybridoma tissue culture supernatant, washed, and 100 μl of a 1:1,000 dilution (in PBS/Tween-20) of peroxidase labelled affinity purified goat anti-mouse IgG (Kirkegaard and Perry, Gaithersburg, MD). All incubation steps, lasting one hour each, were done at room temperature. Bound peroxidase labelled "second antibody" was detected using the peroxidase substrate tetramethylabenzidine (TMB) according to the manufacturer's instructions (Kirkegaard and Perry, Gaithersburg, MD); optical density at 450 nanometers was then determined for each well. Isotypes of positive hybrid culture fluids was determined using an ELISA assay in which 1 μg of anti-mouse Fab was adsorbed to each well of Immunolon II EIA plates followed by sequential incubations with culture supernatants and peroxidase labelled antiserum specific for mouse $IgG_1$, $IgG_{2a}$, $Ig_{2b}$, $IgG_3$, and IgM.

Characterization of the monoclonal antibodies: The antibodies were characterized on Western-blots by comparing their reactivities against the whole recA protein and with fusions of the first 36 amino acids of recA protein with the 56 and 75 amino acids of Kunitz domain. These comparisons were used to eliminate antibodies directed against the recA portion of the immunogen and to localize the reacting epitopes to the 56 or the 19 amino acid regions comprising the 75 amino acids of the Kunitz antigen. Three antibodies, 56-1, 56-2, 56-3 reacting with the 56 amino acid Kunitz domain were isolated by this procedure. They were then tested similarly for reactivity against the 695, 751 and 770 APP forms secreted from mammalian cells described in Example 16. All three were found to react with human APP-751 and APP-770 from transfectants but not with the APP-695 form.

It has been observed (Weidemann et al., 1989, supra and Palmert et al., 1989, supra) that many cell lines in culture secrete all three forms of APP to various extents, with the APP-751 and APP-770 forms predominating in most cases. The 56-1 and 56-2 mAbs showed no cross-reactivity with the endogenous mouse versions of APP (Figure 32). All forms of mouse and human APPs were found to react with the 22C11 antibody raised against human 695 precursor. The 56-1 mAb was further tested against supernatants of 751 and 770 transfectants described in Example 16 and also against supernatants of mouse L-cells and COS monkey cells. As shown in Figure 33, the 56-1 mAb reacted strongly with supernatants of the 751 transfectant and with the monkey APP but not against mouse APPs either endogenous in the C127 mouse cell host or in mouse L-cells. The 22C11 mAb detected all forms of APP from all animal species tested here. Thus, the results in Figures 32 and 33 establish that the 56-1 and 56-2 mAbs are being specific for primate (human and monkey) APPs.

## SEQUENCE LISTING

(1) TITLE OF INVENTION:
Recombinant APP Minigenes for Expression in Transgenic Mice as Models for Alzheimer's Disease

NUMBER OF SEQUENCES: 49

(2) INFORMATION FOR SEQ ID NO: 1

(i) SEQUENCE CHARACTERISTICS:
- (A) LENGTH: 20 base pairs
- (B) TYPE: nucleic acid
- (C) STRANDEDNESS: single
- (D) TOPOLOGY: linear

(ii) MOLECULE TYPE: cDNA

(xi) SEQUENCE DESCRIPTION: SEQ ID NO:1:

TTYTGRTGRT GCACYTSRTA 20

(2) INFORMATION FOR SEQ ID NO:2:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:2:

ACRTCYTCNG CRAARAA 17

(2) INFORMATION FOR SEQ ID NO:3:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:3:

TTYTGRTGRT GNACYTCRTA 20

(2) INFORMATION FOR SEQ ID NO:4:

(i) SEQUENCE CHARACTERISTICS:
(A) LENGTH: 60 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:4:

AGTACTGCAT GGCCGTGTGT GGCAGCGCCA TTCCTACAAC AGCAGCCAGT ACCCCTGATG 60

(2) INFORMATION FOR SEQ ID NO:5:

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:5:

AATTCGAACC CCTTCG 16

(2) INFORMATION FOR SEQ ID NO:6:

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:6:

GCTTGGGGAA GCTCGA 16

(2) INFORMATION FOR SEQ ID NO:7:

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:7:

GATCGGGAAG CTTCCC 16

(2) INFORMATION FOR SEQ ID NO:8:

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:8:

CCCTTCGAAG GGCTAG 16

(2) INFORMATION FOR SEQ ID NO:9:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION: 1..21

(ix) FEATURE:
(A) NAME/KEY: mat_peptide
(B) LOCATION: 1..21

(xi) SEQUENCE DESCRIPTION: SEQ ID NO:9:

```
GTG GGC GGT GTT GTC ATA GCG ACAGTGATC                              30
Val Gly Gly Val Val Ile Ala
  1               5
```

(2) INFORMATION FOR SEQ ID NO:10:

(i) SEQUENCE CHARACTERISTICS:
(A) LENGTH: 7 amino acids
(B) TYPE: amino acid
(D) TOPOLOGY: linear

(ii) MOLECULE TYPE: protein

(xi) SEQUENCE DESCRIPTION: SEQ ID NO:10:

```
Val Gly Gly Val Val Ile Ala
  1               5
```

(2) INFORMATION FOR SEQ ID NO:11:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION: 1..18

(ix) FEATURE:
(A) NAME/KEY: mat_peptide
(B) LOCATION: 1..15

(xi) SEQUENCE DESCRIPTION: SEQ ID NO:11:

```
GTG GGG GTG TTG TCA TAGCGACAGT GATCG                                  30
Val Gly Val Leu Ser
  1             5
```

(2) INFORMATION FOR SEQ ID NO:12:

(i) SEQUENCE CHARACTERISTICS:
(A) LENGTH: 5 amino acids
(B) TYPE: amino acid
(D) TOPOLOGY: linear

(ii) MOLECULE TYPE: protein

(xi) SEQUENCE DESCRIPTION: SEQ ID NO:12:

```
Val Gly Val Leu Ser
  1             5
```

(2) INFORMATION FOR SEQ ID NO:13:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION: 1..21

(ix) FEATURE:
(A) NAME/KEY: mat_peptide
(B) LOCATION: 1..18

(xi) SEQUENCE DESCRIPTION: SEQ ID NO:13:

```
GTG GGC GGT GTT GTG TCA TAGCGACAGT GATGC                          33
Val Gly Gly Val Val Ser
  1               5
```

(2) INFORMATION FOR SEQ ID NO:14:

(i) SEQUENCE CHARACTERISTICS:
(A) LENGTH: 6 amino acids
(B) TYPE: amino acid
(D) TOPOLOGY: linear

(ii) MOLECULE TYPE: protein

(xi) SEQUENCE DESCRIPTION: SEQ ID NO:14:

```
Val Gly Gly Val Val Ser
  1               5
```

(2) INFORMATION FOR SEQ ID NO:15:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION: 1..18

(ix) FEATURE:
(A) NAME/KEY: mat_peptide
(B) LOCATION: 1..15

(xi) SEQUENCE DESCRIPTION: SEQ ID NO:15:

```
GTG GGC GGT GTT GTC TAGCGACAGT GATCG                    30
Val Gly Gly Val Val
  1               5
```

(2) INFORMATION FOR SEQ ID NO:16:

(i) SEQUENCE CHARACTERISTICS:
(A) LENGTH: 5 amino acids
(B) TYPE: amino acid
(D) TOPOLOGY: linear

(ii) MOLECULE TYPE: protein

(xi) SEQUENCE DESCRIPTION: SEQ ID NO:16:

```
Val Gly Gly Val Val
  1               5
```

(2) INFORMATION FOR SEQ ID NO:17:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:17:

```
CATGGTGGGG GTGTTGTCAT AGC                    23
```

(2) INFORMATION FOR SEQ ID NO:18:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:18:

```
GGGCGGTGTT GTGTCATAGC GACAG                    25
```

(2) INFORMATION FOR SEQ ID NO:19:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:19:

GGCGGTGTTG TCTAGCGACA GTGA 24

(2) INFORMATION FOR SEQ ID NO:20:

(i) SEQUENCE CHARACTERISTICS:
(A) LENGTH: 38 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:20:

GGTGTTGTCA TAGCGTAGGA TCCGTCATCA CCTTGGTG 38

(2) INFORMATION FOR SEQ ID NO:21:

(i) SEQUENCE CHARACTERISTICS:
(A) LENGTH: 47 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION: 18..47

(ix) FEATURE:
(A) NAME/KEY: mat_peptide
(B) LOCATION: 18..47

(xi) SEQUENCE DESCRIPTION: SEQ ID NO:21:

```
AGATCTCTGA AGTGAAG ATG GAT GGT GTT GTC ATA GCG ACA GTG ATC          47
                   Met Asp Gly Val Val Ile Ala Thr Val Ile
                    1               5                  10
```

(2) INFORMATION FOR SEQ ID NO:22:

(i) SEQUENCE CHARACTERISTICS:
(A) LENGTH: 10 amino acids
(B) TYPE: amino acid
(D) TOPOLOGY: linear

(ii) MOLECULE TYPE: protein

(xi) SEQUENCE DESCRIPTION: SEQ ID NO:22:

```
Met Asp Gly Val Val Ile Ala Thr Val Ile
 1               5                  10
```

(2) INFORMATION FOR SEQ ID NO:23:

(i) SEQUENCE CHARACTERISTICS:
(A) LENGTH: 48 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION: 18..41

(ix) FEATURE:
(A) NAME/KEY: mat_peptide
(B) LOCATION: 18..38

(xi) SEQUENCE DESCRIPTION: SEQ ID NO:23:

```
AGATCTCTGA AGTGAAG ATG GAT GGT GTT GTC ATA GCG TAGGATCCGT          48
                   Met Asp Gly Val Val Ile Ala
                    1               5
```

(2) INFORMATION FOR SEQ ID NO:24:

(i) SEQUENCE CHARACTERISTICS:
(A) LENGTH: 7 amino acids
(B) TYPE: amino acid
(D) TOPOLOGY: linear

(ii) MOLECULE TYPE: protein

(xi) SEQUENCE DESCRIPTION: SEQ ID NO:24:

```
Met Asp Gly Val Val Ile Ala
  1               5
```

(2) INFORMATION FOR SEQ ID NO:25:

(i) SEQUENCE CHARACTERISTICS:
(A) LENGTH: 77 base pairs
(B) TYPE: nucleic acid
(C) STRANDEDNESS: single
(D) TOPOLOGY: linear

(ii) MOLECULE TYPE: cDNA

(iv) ANTI-SENSE: YES

(ix) FEATURE:
(A) NAME/KEY: CDS
(B) LOCATION: 22..77

(ix) FEATURE:
(A) NAME/KEY: mat_peptide
(B) LOCATION: 22..75

(xi) SEQUENCE DESCRIPTION: SEQ ID NO:25:

```
GGCTGCTGTG GCGGGGGTCT A AAT AGT TGG GTT CAG AGT GGT GAC GTC AAA        51

GAC AGT GTT CTG CAT CTG CTC AAA GA                                     77
```

(2) INFORMATION FOR SEQ ID NO:26:

(i) SEQUENCE CHARACTERISTICS:
(A) LENGTH: 18 amino acids
(B) TYPE: amino acid
(D) TOPOLOGY: linear

(ii) MOLECULE TYPE: protein

(xi) SEQUENCE DESCRIPTION: SEQ ID NO:26:

```
Ile Thr Pro Asn Leu Thr Thr Val Asp Phe Val Thr Asn Gln Met Gln
  1               5                  10                  15

Asx Phe
```

(2) INFORMATION FOR SEQ ID NO:27:

(i) SEQUENCE CHARACTERISTICS:
(A) LENGTH: 77 base pairs
(B) TYPE: nucleic acid
(C) STRANDEDNESS: single
(D) TOPOLOGY: linear

(ii) MOLECULE TYPE: cDNA

(iv) ANTI-SENSE: YES

(ix) FEATURE:
(A) NAME/KEY: CDS
(B) LOCATION: 2..76

(ix) FEATURE:
(A) NAME/KEY: mat_peptide
(B) LOCATION: 2..76

(xi) SEQUENCE DESCRIPTION: SEQ ID NO:27:

G GGT ACT GGC TGC TGT TGT AGG AAT AGT TGG GTT CAG AGT GGT GAC 46

GTC AAA GAC AGT AAC TCG AAC CAC CTC TTC C 77

(2) INFORMATION FOR SEQ ID NO:28:

(i) SEQUENCE CHARACTERISTICS:
(A) LENGTH: 25 amino acids
(B) TYPE: amino acid
(D) TOPOLOGY: linear

(ii) MOLECULE TYPE: protein

(xi) SEQUENCE DESCRIPTION: SEQ ID NO:28:

Thr Ser Ala Ala Thr Thr Pro Ile Thr Pro Asn Leu Thr Thr Val Asp
1 5 10 15

Phe Val Thr Val Arg Val Val Glu Glu
20 25

(2) INFORMATION FOR SEQ ID NO:29:

(i) SEQUENCE CHARACTERISTICS:
(A) LENGTH: 10 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:29:

GGACGGAGGA 10

(2) INFORMATION FOR SEQ ID NO:30:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:30:

CATGCCTGCC TCCTCTAG 18

(2) INFORMATION FOR SEQ ID NO:31:

(i) SEQUENCE CHARACTERISTICS:
(A) LENGTH: 43 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:31:

CCTCGGCCTT TGGTGTGTGT TTTATATGAC ATGACCCCCT TGA 43

(2) INFORMATION FOR SEQ ID NO:32:

(i) SEQUENCE CHARACTERISTICS:
(A) LENGTH: 40 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:32:

CACCCCTGTT GTCAATGCCT CTGGGTTTCC GCCAGTTTCG 40

(2) INFORMATION FOR SEQ ID NO:33:

(i) SEQUENCE CHARACTERISTICS:
(A) LENGTH: 35 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:33:

ATGAACTTCA TATCCTGAGT CCATGTCGGA ATTCT 35

(2) INFORMATION FOR SEQ ID NO:34:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:34:

GGCAACATGA TTAGTGAACC AAGG 24

(2) INFORMATION FOR SEQ ID NO:35:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:35:

GGAGGGTGCT CTGCTGGTCT TCAATTACC 29

(2) INFORMATION FOR SEQ ID NO:36:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:36:

AAGGGTTTGT CCAGGCATGC CTTCCTCATC C 31

(2) INFORMATION FOR SEQ ID NO:37:

(i) SEQUENCE CHARACTERISTICS:
(A) LENGTH: 36 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:37:

CCTGGCGTTA CCCAACTTAA TCGCCTTGCA GCACAT 36

(2) INFORMATION FOR SEQ ID NO:38:

(i) SEQUENCE CHARACTERISTICS:
(A) LENGTH: 35 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:38:

AATAAATGTG AGCGAGTAAC AACCCGTCGG ATTCT 35

(2) INFORMATION FOR SEQ ID NO:39:

(i) SEQUENCE CHARACTERISTICS:
(A) LENGTH: 80 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:39:

GAGATAGAAT ACATTACTGA TGTGTGGATT AATTCAAGTT CAGGCATCTA CTTGTGTTAC 60

AGCACAGCTG GGCGTCCATA 80

(2) INFORMATION FOR SEQ ID NO:40:

(i) SEQUENCE CHARACTERISTICS:
(A) LENGTH: 60 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:40:

CGCGGGTGGG GCTTAGTTCT GCATTTGCTC AAAGAACTTG TAAGTTGGAT AGGTTCCAAG 60

(2) INFORMATION FOR SEQ ID NO:41:

(i) SEQUENCE CHARACTERISTICS:
(A) LENGTH: 12 amino acids
(B) TYPE: amino acid
(D) TOPOLOGY: linear

(ii) MOLECULE TYPE: peptide

(v) FRAGMENT TYPE: internal

(xi) SEQUENCE DESCRIPTION: SEQ ID NO:41:

Thr Val Phe Asp Val Thr Thr Leu Asn Pro Thr Ile
1 5 10

(2) INFORMATION FOR SEQ ID NO:42:

(i) SEQUENCE CHARACTERISTICS:
(A) LENGTH: 3353 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION: 147..2234

(ix) FEATURE:
(A) NAME/KEY: mat_peptide
(B) LOCATION: 147..2231

(xi) SEQUENCE DESCRIPTION: SEQ ID NO:42:

```
AGTTTCCTCG GCAGCGGTAG GCGAGAGCAC GCGGAGGAGC GTGCGCGGGG CCCCGGGAGA      60

CGGCGGCGGT GGCGGCGCGG GCAGAGCAAG GACGCGGCGG ATCCCACTCG CACAGCAGCG     120

CACTCGGTGC CCCGCGCAGG GTCGCG ATG CTG CCC GGT TTG GCA CTG CTC CTG      173
                             Met Leu Pro Gly Leu Ala Leu Leu Leu
                               1               5

CTG GCC GCC TGG ACG GCT CGG GCG CTG GAG GTA CCC ACT GAT GGT AAT       221
Leu Ala Ala Trp Thr Ala Arg Ala Leu Glu Val Pro Thr Asp Gly Asn
 10                  15                  20                  25

GCT GGC CTG CTG GCT GAA CCC CAG ATT GCC ATG TTC TGT GGC AGA CTG       269
Ala Gly Leu Leu Ala Glu Pro Gln Ile Ala Met Phe Cys Gly Arg Leu
                 30                  35                  40

AAC ATG CAC ATG AAT GTC CAG AAT GGG AAG TGG GAT TCA GAT CCA TCA       317
Asn Met His Met Asn Val Gln Asn Gly Lys Trp Asp Ser Asp Pro Ser
             45                  50                  55

GGG ACC AAA ACC TGC ATT GAT ACC AAG GAA GGC ATC CTG CAG TAT TGC       365
Gly Thr Lys Thr Cys Ile Asp Thr Lys Glu Gly Ile Leu Gln Tyr Cys
         60                  65                  70

CAA GAA GTC TAC CCT GAA CTG CAG ATC ACC AAT GTG GTA GAA GCC AAC       413
Gln Glu Val Tyr Pro Glu Leu Gln Ile Thr Asn Val Val Glu Ala Asn
     75                  80                  85

CAA CCA GTG ACC ATC CAG AAC TGG TGC AAG CGG GGC CGC AAG CAG TGC       461
Gln Pro Val Thr Ile Gln Asn Trp Cys Lys Arg Gly Arg Lys Gln Cys
 90                  95                 100                 105

AAG ACC CAT CCC CAC TTT GTG ATT CCC TAC CGC TGC TTA GTT GGT GAG       509
Lys Thr His Pro His Phe Val Ile Pro Tyr Arg Cys Leu Val Gly Glu
                110                 115                 120

TTT GTA AGT GAT GCC CTT CTC GTT CCT GAC AAG TGC AAA TTC TTA CAC       557
Phe Val Ser Asp Ala Leu Leu Val Pro Asp Lys Cys Lys Phe Leu His
            125                 130                 135

CAG GAG AGG ATG GAT GTT TGC GAA ACT CAT CTT CAC TGG CAC ACC GTC       605
Gln Glu Arg Met Asp Val Cys Glu Thr His Leu His Trp His Thr Val
        140                 145                 150
```

```
GCC AAA GAG ACA TGC AGT GAG AAG AGT ACC AAC TTG CAT GAC TAC GGC      653
Ala Lys Glu Thr Cys Ser Glu Lys Ser Thr Asn Leu His Asp Tyr Gly
    155                 160                 165

ATG TTG CTG CCC TGC GGA ATT GAC AAG TTC CGA GGG GTA GAG TTT GTG      701
Met Leu Leu Pro Cys Gly Ile Asp Lys Phe Arg Gly Val Glu Phe Val
170                 175                 180                 185

TGT TGC CCA CTG GCT GAA GAA AGT GAC AAT GTG GAT TCT GCT GAT GCG      749
Cys Cys Pro Leu Ala Glu Glu Ser Asp Asn Val Asp Ser Ala Asp Ala
                190                 195                 200

GAG GAG GAT GAC TCG GAT GTC TGG TGG GGC GGA GCA GAC ACA GAC TAT      797
Glu Glu Asp Asp Ser Asp Val Trp Trp Gly Gly Ala Asp Thr Asp Tyr
            205                 210                 215

GCA GAT GGG AGT GAA GAC AAA GTA GTA GAA GTA GCA GAG GAG GAA GAA      845
Ala Asp Gly Ser Glu Asp Lys Val Val Glu Val Ala Glu Glu Glu Glu
        220                 225                 230

GTG GCT GAG GTG GAA GAA GAA GAA GCC GAT GAT GAC GAG GAC GAT GAG      893
Val Ala Glu Val Glu Glu Glu Glu Ala Asp Asp Asp Glu Asp Asp Glu
    235                 240                 245

GAT GGT GAT GAG GTA GAG GAA GAG GCT GAG GAA CCC TAC GAA GAA GCC      941
Asp Gly Asp Glu Val Glu Glu Glu Ala Glu Glu Pro Tyr Glu Glu Ala
250                 255                 260                 265

ACA GAG AGA ACC ACC AGC ATT GCC ACC ACC ACC ACC ACC ACC ACA GAG      989
Thr Glu Arg Thr Thr Ser Ile Ala Thr Thr Thr Thr Thr Thr Thr Glu
                270                 275                 280

TCT GTG GAA GAG GTG GTT CGA GTT CCT ACA ACA GCA GCC AGT ACC CCT     1037
Ser Val Glu Glu Val Val Arg Val Pro Thr Thr Ala Ala Ser Thr Pro
            285                 290                 295

GAT GCC GTT GAC AAG TAT CTC GAG ACA CCT GGG GAT GAG AAT GAA CAT     1085
Asp Ala Val Asp Lys Tyr Leu Glu Thr Pro Gly Asp Glu Asn Glu His
        300                 305                 310

GCC CAT TTC CAG AAA GCC AAA GAG AGG CTT GAG GCC AAG CAC CGA GAG     1133
Ala His Phe Gln Lys Ala Lys Glu Arg Leu Glu Ala Lys His Arg Glu
    315                 320                 325

AGA ATG TCC CAG GTC ATG AGA GAA TGG GAA GAG GCA GAA CGT CAA GCA     1181
Arg Met Ser Gln Val Met Arg Glu Trp Glu Glu Ala Glu Arg Gln Ala
330                 335                 340                 345

AAG AAC TTG CCT AAA GCT GAT AAG AAG GCA GTT ATC CAG CAT TTC CAG     1229
Lys Asn Leu Pro Lys Ala Asp Lys Lys Ala Val Ile Gln His Phe Gln
                350                 355                 360

GAG AAA GTG GAA TCT TTG GAA CAG GAA GCA GCC AAC GAG AGA CAG CAG     1277
Glu Lys Val Glu Ser Leu Glu Gln Glu Ala Ala Asn Glu Arg Gln Gln
            365                 370                 375
```

```
CTG GTG GAG ACA CAC ATG GCC AGA GTG GAA GCC ATG CTC AAT GAC CGC      1325
Leu Val Glu Thr His Met Ala Arg Val Glu Ala Met Leu Asn Asp Arg
        380                 385                 390

CGC CGC CTG GCC CTG GAG AAC TAC ATC ACC GCT CTG CAG GCT GTT CCT      1373
Arg Arg Leu Ala Leu Glu Asn Tyr Ile Thr Ala Leu Gln Ala Val Pro
    395                 400                 405

CCT CGG CCT CGT CAC GTG TTC AAT ATG CTA AAG AAG TAT GTC CGC GCA      1421
Pro Arg Pro Arg His Val Phe Asn Met Leu Lys Lys Tyr Val Arg Ala
410                 415                 420                 425

GAA CAG AAG GAC AGA CAG CAC ACC CTA AAG CAT TTC GAG CAT GTG CGC      1469
Glu Gln Lys Asp Arg Gln His Thr Leu Lys His Phe Glu His Val Arg
                430                 435                 440

ATG GTG GAT CCC AAG AAA GCC GCT CAG ATC CGG TCC CAG GTT ATG ACA      1517
Met Val Asp Pro Lys Lys Ala Ala Gln Ile Arg Ser Gln Val Met Thr
            445                 450                 455

CAC CTC CGT GTG ATT TAT GAG CGC ATG AAT CAG TCT CTC TCC CTG CTC      1565
His Leu Arg Val Ile Tyr Glu Arg Met Asn Gln Ser Leu Ser Leu Leu
        460                 465                 470

TAC AAC GTG CCT GCA GTG GCC GAG GAG ATT CAG GAT GAA GTT GAT GAG      1613
Tyr Asn Val Pro Ala Val Ala Glu Glu Ile Gln Asp Glu Val Asp Glu
    475                 480                 485

CTG CTT CAG AAA GAG CAA AAC TAT TCA GAT GAC GTC TTG GCC AAC ATG      1661
Leu Leu Gln Lys Glu Gln Asn Tyr Ser Asp Asp Val Leu Ala Asn Met
490                 495                 500                 505

ATT AGT GAA CCA AGG ATC AGT TAC GGA AAC GAT GCT CTC ATG CCA TCT      1709
Ile Ser Glu Pro Arg Ile Ser Tyr Gly Asn Asp Ala Leu Met Pro Ser
                510                 515                 520

TTG ACC GAA ACG AAA ACC ACC GTG GAG CTC CTT CCC GTG AAT GGA GAG      1757
Leu Thr Glu Thr Lys Thr Thr Val Glu Leu Leu Pro Val Asn Gly Glu
            525                 530                 535

TTC AGC CTG GAC GAT CTC CAG CCG TGG CAT TCT TTT GGG GCT GAC TCT      1805
Phe Ser Leu Asp Asp Leu Gln Pro Trp His Ser Phe Gly Ala Asp Ser
        540                 545                 550

GTG CCA GCC AAC ACA GAA AAC GAA GTT GAG CCT GTT GAT GCC CGC CCT      1853
Val Pro Ala Asn Thr Glu Asn Glu Val Glu Pro Val Asp Ala Arg Pro
    555                 560                 565

GCT GCC GAC CGA GGA CTG ACC ACT CGA CCA GGT TCT GGG TTG ACA AAT      1901
Ala Ala Asp Arg Gly Leu Thr Thr Arg Pro Gly Ser Gly Leu Thr Asn
570                 575                 580                 585

ATC AAG ACG GAG GAG ATC TCT GAA GTG AAG ATG GAT GCA GAA TTC CGA      1949
Ile Lys Thr Glu Glu Ile Ser Glu Val Lys Met Asp Ala Glu Phe Arg
                590                 595                 600
```

```
CAT GAC TCA GGA TAT GAA GTT CAT CAT CAA AAA TTG GTG TTC TTT GCA      1997
His Asp Ser Gly Tyr Glu Val His His Gln Lys Leu Val Phe Phe Ala
            605                 610                 615

GAA GAT GTG GGT TCA AAC AAA GGT GCA ATC ATT GGA CTC ATG GTG GGC      2045
Glu Asp Val Gly Ser Asn Lys Gly Ala Ile Ile Gly Leu Met Val Gly
        620                 625                 630

GGT GTT GTC ATA GCG ACA GTG ATC GTC ATC ACC TTG GTG ATG CTG AAG      2093
Gly Val Val Ile Ala Thr Val Ile Val Ile Thr Leu Val Met Leu Lys
    635                 640                 645

AAG AAA CAG TAC ACA TCC ATT CAT CAT GGT GTG GTG GAG GTT GAC GCC      2141
Lys Lys Gln Tyr Thr Ser Ile His His Gly Val Val Glu Val Asp Ala
650                 655                 660                 665

GCT GTC ACC CCA GAG GAG CGC CAC CTG TCC AAG ATG CAG CAG AAC GGC      2189
Ala Val Thr Pro Glu Glu Arg His Leu Ser Lys Met Gln Gln Asn Gly
                670                 675                 680

TAC GAA AAT CCA ACC TAC AAG TTC TTT GAG CAG ATG CAG AAC TAGACCCCCG   2241
Tyr Glu Asn Pro Thr Tyr Lys Phe Phe Glu Gln Met Gln Asn
            685                 690                 695

CCACAGCAGC CTCTGAAGTT GGACAGCAAA ACCATTGCTT CACTACCCAT CGGTGTCCAT    2301

TTATAGAATA ATGTGGGAAG AAACAAACCC GTTTTATGAT TTACTCATTA TCGCCTTTTG    2361

ACAGCTGTGC TGTAACACAA GTAGATGCCT GAACTTGAAT TAATCCACAC ATCAGTAATG    2421

TATTCTATCT CTCTTTACAT TTTGGTCTCT ATACTACATT ATTAATGGGT TTTGTGTACT    2481

GTAAAGAATT TAGCTGTATC AAACTAGTGC ATGAATAGAT TCTCTCCTGA TTATTTATCA    2541

CATAGCCCCT TAGCCAGTTG TATATTATTC TTGTGGTTTG TGACCCAATT AAGTCCTACT    2601

TTACATATGC TTTAAGAATC GATGGGGGAT GCTTCATGTG AACGTGGGAG TTCAGCTGCT    2661

TCTCTTGCCT AAGTATTCCT TTCCTGATCA CTATGCATTT TAAAGTTAAA CATTTTTAAG    2721

TATTTCAGAT GCTTTAGAGA GATTTTTTTT CCATGACTGC ATTTTACTGT ACAGATTGCT    2781

GCTTCTGCTA TATTTGTGAT ATAGGAATTA AGAGGATACA CACGTTTGTT TCTTCGTGCC    2841

TGTTTTATGT GCACACATTA GGCATTGAGA CTTCAAGCTT TTCTTTTTTT GTCCACGTAT    2901

CTTTGGGTCT TTGATAAAGA AAAGAATCCC TGTTCATTGT AAGCACTTTT ACGGGGCGGG    2961

TGGGGAGGGG TGCTCTGCTG GTCTTCAATT ACCAAGAATT CTCCAAAACA ATTTTCTGCA    3021

GGATGATTGT ACAGAATCAT TGCTTATGAC ATGATCGCTT TCTACACTGT ATTACATAAA    3081

TAAATTAAAT AAAATAACCC CGGGCAAGAC TTTTCTTTGA AGGATGACTA CAGACATTAA    3141

ATAATCGAAG TAATTTTGGG TGGGGAGAAG AGGCAGATTC AATTTTCTTT AACCAGTCTG    3201
```

AAGTTTCATT TATGATACAA AAGAAGATGA AAATGGAAGT GGCAATATAA GGGGATGAGG 3261

AAGGCATGCC TGGACAAACC CTTCTTTTAA GATGTGTCTT CAATTTGTAT AAAATGGTGT 3321

TTTCATGTAA ATAAATACAT TCTTGGAGGA GC 3353

(2) INFORMATION FOR SEQ ID NO:43:

(i) SEQUENCE CHARACTERISTICS:
(A) LENGTH: 695 amino acids
(B) TYPE: amino acid
(D) TOPOLOGY: linear

(ii) MOLECULE TYPE: protein

(xi) SEQUENCE DESCRIPTION: SEQ ID NO:43:

```
Met Leu Pro Gly Leu Ala Leu Leu Leu Leu Ala Ala Trp Thr Ala Arg
  1               5                  10                  15

Ala Leu Glu Val Pro Thr Asp Gly Asn Ala Gly Leu Leu Ala Glu Pro
             20                  25                  30

Gln Ile Ala Met Phe Cys Gly Arg Leu Asn Met His Met Asn Val Gln
         35                  40                  45

Asn Gly Lys Trp Asp Ser Asp Pro Ser Gly Thr Lys Thr Cys Ile Asp
     50                  55                  60

Thr Lys Glu Gly Ile Leu Gln Tyr Cys Gln Glu Val Tyr Pro Glu Leu
 65                  70                  75                  80

Gln Ile Thr Asn Val Val Glu Ala Asn Gln Pro Val Thr Ile Gln Asn
                 85                  90                  95

Trp Cys Lys Arg Gly Arg Lys Gln Cys Lys Thr His Pro His Phe Val
            100                 105                 110

Ile Pro Tyr Arg Cys Leu Val Gly Glu Phe Val Ser Asp Ala Leu Leu
        115                 120                 125

Val Pro Asp Lys Cys Lys Phe Leu His Gln Glu Arg Met Asp Val Cys
    130                 135                 140

Glu Thr His Leu His Trp His Thr Val Ala Lys Glu Thr Cys Ser Glu
145                 150                 155                 160

Lys Ser Thr Asn Leu His Asp Tyr Gly Met Leu Leu Pro Cys Gly Ile
                165                 170                 175

Asp Lys Phe Arg Gly Val Glu Phe Val Cys Cys Pro Leu Ala Glu Glu
            180                 185                 190

Ser Asp Asn Val Asp Ser Ala Asp Ala Glu Glu Asp Asp Ser Asp Val
        195                 200                 205
```

Trp Trp Gly Gly Ala Asp Thr Asp Tyr Ala Asp Gly Ser Glu Asp Lys
210 215 220

Val Val Glu Val Ala Glu Glu Glu Glu Val Ala Glu Val Glu Glu Glu
225 230 235 240

Glu Ala Asp Asp Asp Glu Asp Asp Glu Asp Gly Asp Glu Val Glu Glu
245 250 255

Glu Ala Glu Glu Pro Tyr Glu Glu Ala Thr Glu Arg Thr Thr Ser Ile
260 265 270

Ala Thr Thr Thr Thr Thr Thr Thr Glu Ser Val Glu Glu Val Val Arg
275 280 285

Val Pro Thr Thr Ala Ala Ser Thr Pro Asp Ala Val Asp Lys Tyr Leu
290 295 300

Glu Thr Pro Gly Asp Glu Asn Glu His Ala His Phe Gln Lys Ala Lys
305 310 315 320

Glu Arg Leu Glu Ala Lys His Arg Glu Arg Met Ser Gln Val Met Arg
325 330 335

Glu Trp Glu Glu Ala Glu Arg Gln Ala Lys Asn Leu Pro Lys Ala Asp
340 345 350

Lys Lys Ala Val Ile Gln His Phe Gln Glu Lys Val Glu Ser Leu Glu
355 360 365

Gln Glu Ala Ala Asn Glu Arg Gln Gln Leu Val Glu Thr His Met Ala
370 375 380

Arg Val Glu Ala Met Leu Asn Asp Arg Arg Arg Leu Ala Leu Glu Asn
385 390 395 400

Tyr Ile Thr Ala Leu Gln Ala Val Pro Pro Arg Pro Arg His Val Phe
405 410 415

Asn Met Leu Lys Lys Tyr Val Arg Ala Glu Gln Lys Asp Arg Gln His
420 425 430

Thr Leu Lys His Phe Glu His Val Arg Met Val Asp Pro Lys Lys Ala
435 440 445

Ala Gln Ile Arg Ser Gln Val Met Thr His Leu Arg Val Ile Tyr Glu
450 455 460

Arg Met Asn Gln Ser Leu Ser Leu Leu Tyr Asn Val Pro Ala Val Ala
465 470 475 480

Glu Glu Ile Gln Asp Glu Val Asp Glu Leu Leu Gln Lys Glu Gln Asn
485 490 495

```
Tyr Ser Asp Asp Val Leu Ala Asn Met Ile Ser Glu Pro Arg Ile Ser
            500                 505                 510

Tyr Gly Asn Asp Ala Leu Met Pro Ser Leu Thr Glu Thr Lys Thr Thr
        515                 520                 525

Val Glu Leu Leu Pro Val Asn Gly Glu Phe Ser Leu Asp Asp Leu Gln
    530                 535                 540

Pro Trp His Ser Phe Gly Ala Asp Ser Val Pro Ala Asn Thr Glu Asn
545                 550                 555                 560

Glu Val Glu Pro Val Asp Ala Arg Pro Ala Ala Asp Arg Gly Leu Thr
                565                 570                 575

Thr Arg Pro Gly Ser Gly Leu Thr Asn Ile Lys Thr Glu Glu Ile Ser
            580                 585                 590

Glu Val Lys Met Asp Ala Glu Phe Arg His Asp Ser Gly Tyr Glu Val
        595                 600                 605

His His Gln Lys Leu Val Phe Phe Ala Glu Asp Val Gly Ser Asn Lys
    610                 615                 620

Gly Ala Ile Ile Gly Leu Met Val Gly Gly Val Val Ile Ala Thr Val
625                 630                 635                 640

Ile Val Ile Thr Leu Val Met Leu Lys Lys Lys Gln Tyr Thr Ser Ile
                645                 650                 655

His His Gly Val Val Glu Val Asp Ala Ala Val Thr Pro Glu Glu Arg
            660                 665                 670

His Leu Ser Lys Met Gln Gln Asn Gly Tyr Glu Asn Pro Thr Tyr Lys
        675                 680                 685

Phe Phe Glu Gln Met Gln Asn
    690                 695
```

(2) INFORMATION FOR SEQ ID NO:44:

(i) SEQUENCE CHARACTERISTICS:
(A) LENGTH: 1575 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION: 148..537

(ix) FEATURE:
(A) NAME/KEY: mat_peptide
(B) LOCATION: 148..534

(xi) SEQUENCE DESCRIPTION: SEQ ID NO:44:

```
AGTTTCCTCG GCAGCGGTAG GCGAGAGCAC GCGGAGGAGC GTGCGCGGGG GCCCCGGGAG      60

ACGGCGGCGG TGGCGGCGCG GGCAGAGCAA GGACGCGGCG GATCCCACTC GCACAGCAGC     120

GCACTCGGTG CCCCGCGCAG GGTCGCG ATG CTG CCC GGT TTG GCA CTG CTC         171
                              Met Leu Pro Gly Leu Ala Leu Leu
                                1               5

CTG CTG GCC GCC TGG ACG GCT CGG GCG CTG GAG GTA CGG ACG GAG GAG       219
Leu Leu Ala Ala Trp Thr Ala Arg Ala Leu Glu Val Arg Thr Glu Glu
     10                  15                  20

ATC TCT GAA GTG AAG ATG GAT GCA GAA TTC CGA CAT GAC TCA GGA TAT       267
Ile Ser Glu Val Lys Met Asp Ala Glu Phe Arg His Asp Ser Gly Tyr
 25                  30                  35                  40

GAA GTT CAT CAT CAA AAA TTG GTG TTC TTT GCA GAA GAT GTG GGT TCA       315
Glu Val His His Gln Lys Leu Val Phe Phe Ala Glu Asp Val Gly Ser
                 45                  50                  55

AAC AAA GGT GCA ATC ATT GGA CTC ATG GTG GGC GGT GTT GTC ATA GCG       363
Asn Lys Gly Ala Ile Ile Gly Leu Met Val Gly Gly Val Val Ile Ala
             60                  65                  70

ACA GTG ATC GTC ATC ACC TTG GTG ATG CTG AAG AAG AAA CAG TAC ACA       411
Thr Val Ile Val Ile Thr Leu Val Met Leu Lys Lys Lys Gln Tyr Thr
         75                  80                  85

TCC ATT CAT CAT GGT GTG GTG GAG GTT GAC GCC GCT GTC ACC CCA GAG       459
Ser Ile His His Gly Val Val Glu Val Asp Ala Ala Val Thr Pro Glu
     90                  95                 100

GAG CGC CAC CTG TCC AAG ATG CAG CAG AAC GGC TAC GAA AAT CCA ACC       507
Glu Arg His Leu Ser Lys Met Gln Gln Asn Gly Tyr Glu Asn Pro Thr
105                 110                 115                 120

TAC AAG TTC TTT GAG CAG ATG CAG AAC TAGACCCCCG CCACAGCAGC             554
Tyr Lys Phe Phe Glu Gln Met Gln Asn
                125                 130

CTCTGAAGTT GGACAGCAAA ACCATTGCTT CACTACCCAT CGGTGTCCAT TTATAGAATA     614

ATGTGGGAAG AAACAAACCC GTTTTATGAT TTACTCATTA TCGCCTTTTG ACAGCTGTGC     674

TGTAACACAA GTAGATGCCT GAACTTGAAT TAATCCACAC ATCAGTAATG TATTCTATCT     734

CTCTTTACAT TTTGGTCTCT ATACTACATT ATTAATGGGT TTTGTGTACT GTAAAGAATT     794

TAGCTGTATC AAACTAGTGC ATGAATAGAT TCTCTCCTGA TTATTTATCA CATAGCCCCT     854
```

```
TAGCCAGTTG TATATTATTC TTGTGGTTTG TGACCCAATT AAGTCCTACT TTACATATGC     914
TTTAAGAATC GATGGGGGAT GCTTCATGTG AACGTGGGAG TTCAGCTGCT TCTCTTGCCT     974
AAGTATTCCT TTCCTGATCA CTATGCATTT TAAAGTTAAA CATTTTTAAG TATTTCAGAT    1034
GCTTTAGAGA GATTTTTTTT CCATGACTGC ATTTTACTGT ACAGATTGCT GCTTCTGCTA    1094
TATTTGTGAT ATAGGAATTA AGAGGATACA CACGTTTGTT TCTTCGTGCC TGTTTTATGT    1154
GCACACATTA GGCATTGAGA CTTCAAGCTT TTCTTTTTTT GTCCACGTAT CTTTGGGTCT    1214
TTGATAAAGA AAAGAATCCC TGTTCATTGT AAGCACTTTT ACGGGGCGGG TGGGGAGGGG    1274
TGCTCTGCTG GTCTTCAATT ACCAAGAATT CTCCAAAACA ATTTTCTGCA GGATGATTGT    1334
ACAGAATCAT TGCTTATGAC ATGATCGCTT TCTACACTGT ATTACATAAA TAAATTAAAT    1394
AAAATAACCC CGGGCAAGAC TTTTCTTTGA AGGATGACTA CAGACATTAA ATAATCGAAG    1454
TAATTTTGGG TGGGGAGAAG AGGCAGATTC AATTTTCTTT AACCAGTCTG AAGTTTCATT    1514
TATGATACAA AAGAAGATGA AAATGGAAGT GGCAATATAA GGGGATGAGG AAGGCAGCAT    1574
G                                                                    1575
```

(2) INFORMATION FOR SEQ ID NO:45:

(i) SEQUENCE CHARACTERISTICS:
(A) LENGTH: 129 amino acids
(B) TYPE: amino acid
(D) TOPOLOGY: linear

(ii) MOLECULE TYPE: protein

(xi) SEQUENCE DESCRIPTION: SEQ ID NO:45:

```
Met Leu Pro Gly Leu Ala Leu Leu Leu Leu Ala Ala Trp Thr Ala Arg
  1               5                  10                  15

Ala Leu Glu Val Arg Thr Glu Glu Ile Ser Glu Val Lys Met Asp Ala
             20                  25                  30

Glu Phe Arg His Asp Ser Gly Tyr Glu Val His His Gln Lys Leu Val
         35                  40                  45

Phe Phe Ala Glu Asp Val Gly Ser Asn Lys Gly Ala Ile Ile Gly Leu
     50                  55                  60

Met Val Gly Gly Val Val Ile Ala Thr Val Ile Val Ile Thr Leu Val
 65                  70                  75                  80

Met Leu Lys Lys Lys Gln Tyr Thr Ser Ile His His Gly Val Val Glu
                 85                  90                  95
```

```
Val Asp Ala Ala Val Thr Pro Glu Glu Arg His Leu Ser Lys Met Gln
            100                 105                 110

Gln Asn Gly Tyr Glu Asn Pro Thr Tyr Lys Phe Phe Glu Gln Met Gln
        115                 120                 125

Asn
```

(2) INFORMATION FOR SEQ ID NO:46:

(i) SEQUENCE CHARACTERISTICS:
(A) LENGTH: 1574 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION: 148..360

(ix) FEATURE:
(A) NAME/KEY: mat_peptide
(B) LOCATION: 148..357

(xi) SEQUENCE DESCRIPTION: SEQ ID NO:46:

```
AGTTTCCTCG GCAGCGGTAG GCGAGAGCAC GCGGAGGAGC GTGCGCGGGG GCCCCGGGAG      60

ACGGCGGCGG TGGCGGCGCG GGCAGAGCAA GGACGCGGCG GATCCCACTC GCACAGCAGC     120

GCACTCGGTG CCCCGCGCAG GGTCGCG ATG CTG CCC GGT TTG GCA CTG CTC         171
                              Met Leu Pro Gly Leu Ala Leu Leu
                                1               5

CTG CTG GCC GCC TGG ACG GCT CGG GCG CTG GAG GTA CGG ACG GAG GAG       219
Leu Leu Ala Ala Trp Thr Ala Arg Ala Leu Glu Val Arg Thr Glu Glu
    10                  15                  20

ATC TCT GAA GTG AAG ATG GAT GCA GAA TTC CGA CAT GAC TCA GGA TAT       267
Ile Ser Glu Val Lys Met Asp Ala Glu Phe Arg His Asp Ser Gly Tyr
 25                  30                  35                  40

GAA GTT CAT CAT CAA AAA TTG GTG TTC TTT GCA GAA GAT GTG GGT TCA       315
Glu Val His His Gln Lys Leu Val Phe Phe Ala Glu Asp Val Gly Ser
                 45                  50                  55

AAC AAA GGT GCA ATC ATT GGA CTC ATG GTG GGC GGT GTT GTC TAGCGACAGT   367
Asn Lys Gly Ala Ile Ile Gly Leu Met Val Gly Gly Val Val
             60                  65                  70
```

GATCGTCATC ACCTTGGTGA TGCTGAAGAA GAAACAGTAC ACATCCATTC ATCATGGTGT 427

GGTGGAGGTT GACGCCGCTG TCACCCCAGA GGAGCGCCAC CTGTCCAAGA TGCAGCAGAA 487

CGGCTACGAA AATCCAACCT ACAAGTTCTT TGAGCAGATG CAGAACTAGA CCCCCGCCAC 547

AGCAGCCTCT GAAGTTGGAC AGCAAAACCA TTGCTTCACT ACCCATCGGT GTCCATTTAT 607

AGAATAATGT GGGAAGAAAC AAACCCGTTT TATGATTTAC TCATTATCGC CTTTTGACAG 667

CTGTGCTGTA ACACAAGTAG ATGCCTGAAC TTGAATTAAT CCACACATCA GTAATGTATT 727

CTATCTCTCT TTACATTTTG GTCTCTATAC TACATTATTA ATGGGTTTTG TGTACTGTAA 787

AGAATTTAGC TGTATCAAAC TAGTGCATGA ATAGATTCTC TCCTGATTAT TTATCACATA 847

GCCCCTTAGC CAGTTGTATA TTATTCTTGT GGTTTGTGAC CCAATTAAGT CCTACTTTAC 907

ATATGCTTTA AGAATCGATG GGGGATGCTT CATGTGAACG TGGGAGTTCA GCTGCTTCTC 967

TTGCCTAAGT ATTCCTTTCC TGATCACTAT GCATTTTAAA GTTAAACATT TTTAAGTATT 1027

TCAGATGCTT TAGAGAGATT TTTTTTCCAT GACTGCATTT TACTGTACAG ATTGCTGCTT 1087

CTGCTATATT TGTGATATAG GAATTAAGAG GATACACACG TTTGTTTCTT CGTGCCTGTT 1147

TTATGTGCAC ACATTAGGCA TTGAGACTTC AAGCTTTTCT TTTTTTGTCC ACGTATCTTT 1207

GGGTCTTTGA TAAAGAAAAG AATCCCTGTT CATTGTAAGC ACTTTTACGG GGCGGGTGGG 1267

GAGGGGTGCT CTGCTGGTCT TCAATTACCA AGAATTCTCC AAAACAATTT TCTGCAGGAT 1327

GATTGTACAG AATCATTGCT TATGACATGA TCGCTTTCTA CACTGTATTA CATAAATAAA 1387

TTAAATAAAA TAACCCCGGG CAAGACTTTT CTTTGAAGGA TGACTACAGA CATTAAATAA 1447

TCGAAGTAAT TTTGGGTGGG GAGAAGAGGC AGATTCAATT TTCTTTAACC AGTCTGAAGT 1507

TTCATTTATG ATACAAAAGA AGATGAAAAT GGAAGTGGCA ATATAAGGGG ATGAGGAAGG 1567

CAGCATG 1574

(2) INFORMATION FOR SEQ ID NO:47:

(i) SEQUENCE CHARACTERISTICS:
(A) LENGTH: 70 amino acids
(B) TYPE: amino acid
(D) TOPOLOGY: linear

(ii) MOLECULE TYPE: protein

(xi) SEQUENCE DESCRIPTION: SEQ ID NO:47:

```
Met Leu Pro Gly Leu Ala Leu Leu Leu Leu Ala Ala Trp Thr Ala Arg
  1               5                  10                  15

Ala Leu Glu Val Arg Thr Glu Glu Ile Ser Glu Val Lys Met Asp Ala
            20                  25                  30

Glu Phe Arg His Asp Ser Gly Tyr Glu Val His His Gln Lys Leu Val
        35                  40                  45

Phe Phe Ala Glu Asp Val Gly Ser Asn Lys Gly Ala Ile Ile Gly Leu
    50                  55                  60

Met Val Gly Gly Val Val
 65                  70
```

(2) INFORMATION FOR SEQ ID NO:48:

(i) SEQUENCE CHARACTERISTICS:
(A) LENGTH: 1297 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:48:

```
GCATGCCTGG ACAAACCCTT CTTTTAAGAT GTGTCTTCAA TTTGTATAAA ATGGTGTTTT     60
CATGTAAATA AATACATTCT TGGAGGAGCC ACATTGTGCT GGTGTGAATG ATTCCATAGT    120
AACAATCTTG ACCATTTACT GACGTACAGA CCAGTGAGAA GTCTTCGCAT GTTGGGTACC    180
CACACCTGTT GTGTCTTAAT TGCAAGTCTG AGTAGGAAGT TGGGGCCAAC ATGTGTCTCC    240
CAGTGCTGGG AAAATATTTC ATAGACCTAA TTTACAGTCT TTACTTGATC TAAAACATTT    300
TGCTGCCATA TTTTGGCCCT CAAGTTTGTC CCAAATGAGA GACAAAGGGA AAAGTTCCAG    360
GGAAATAAAA ATTAAGACAG CTGATTATCT GTAAAGCATG GTTTCTCATC CTGAACGCTA    420
CTAACATTTT GCAGGGAATA ATTCCTTGTT GAAGGGAGTT GTCCTGACCA GTGTAGGATA    480
TTTATTTATT TTATTTATGT TTTTTGAGAC GGAGTCTCGC TCTGTCACCC AGGCTGGAGT    540
GCAGTGGCAC AATCTCGGCT CACTGCAAGC TCCGCCTCCC GGGTTCACGC CATTCTCCTG    600
CCTCAGCCTC CTGAATAGCT GGGACTCTAG GTGCCCGCCA CCACGCCCGG CTAATTTTTT    660
GTATTTTTAG TAGAGACGGG GTTTCACCGT GTTAGCCAGG ACAGTCTTGG TCTCCTGACC    720
```

```
TCGTGATCTG CCTGCCTCGG CCTCCCAAAG TGCTGAGATT ACAGGCGTGC AAGCCGCGCC     780
CAGCCAGTGC TCTCCTTTTA AAAGTAGCCC ATTGGCTGGG CGCAGTGGCT CACGCCTGTA     840
ATCCCAGCAC TTTGGGAGGC TGAGGCGGGT GGATCACGAG GTCAGGAGAT CAAGAATATC     900
CTGGCCAATA TGGTGAAACC CCATCTCTAC TAAAAATACA AAAAAAAAAA AAAAAAAAAA     960
AAGGCCGGGC ATGGTGGCGG GCGCTTGTAG TCCCAGCTAC TCAGGAGGCT GAGGCAGGAG    1020
AATGGTGTGC ACCTGGGAGG CGGAGGTTGC AGTGAGCTGA GATCGCGCCA CTGCACTCCA    1080
GCCTGGGAGA CAGAGCGAGA CTCCGTCTCA ATAAATAAAT AAATAAATAA ATAAAAGGAG    1140
GGCCTGGCAC GAATGACATG CAGGGAAGGC AGTGAGCAGG TGGAGGTCCC TGTACTCGTT    1200
GTGGTGCCTT ATCTACCAGG CGGTTGAGTT GACGTCTTTG TGGACAGAAT TCGAGCTCGG    1260
TACCCGGGGA TCCTCTAGAG TCGACCTGCA GGCATGC                             1297
```

(2) INFORMATION FOR SEQ ID NO:49:

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:49:

```
AATTCCCGCA TGCGGG     16
```

## Claims

**1.** A minigene for expression of an amyloid precursor protein (APP) or derivatives thereof comprising
(a) a regulatory region, said regulatory region capable of directing tissue and cell specific expression,
(b) a gene construct encoding said APP or derivative thereof, and
(c) genetic sequences containing a RNA polyadenylation signal.

**2.** A minigene according to Claim 1 wherein said regulatory region further includes genetic elements conferring a developmental expression pattern of said gene construct of (b) similar to the developmental expression pattern observed in the endogenous APP gene.

**3.** A minigene according to Claim 1 or 2 wherein said minigene further comprises
(d) an intronic sequence containing acceptor and donor sites for splicing.

**4.** A minigene according to Claim 1 wherein said gene construct encodes APP-695, APP-751, APP-770, a mutated APP, a truncated APP or an A4 peptide.

**5.** A minigene according to Claim 1 wherein said gene construct of (b) is replaced by a reporter gene, said reporter gene capable of being monitored to assess the function of said regulatory region, or by a fusion protein containing a reporter gene and a gene encoding said APP or derivative thereof.

**6.** A minigene according to Claim 3 wherein said minigene further comprises

(e) an antigenic tag for the expression of a tagged APP or APP derivative, said tagged APP or APP derivative capable of being detected to assess said expression.

7. A minigene cassette for transfer and expression in transgenic mice of APP or derivatives thereof comprising a NotI fragment containing
(a) a regulatory region, said regulatory region capable of directing tissue and cell specific expression.
(b) a gene construct encoding said APP or derivative thereof,
(c) genetic sequences containing a RNA polyadenylation signal, and
(d) an intronic sequence containing acceptor and donor sites for splicing.

8. A transgenic mouse, including progeny, embryo or cell derived from said transgenic mouse, capable of expressing an amyloid precursor protein (APP) or derivatives thereof in a tissue and cell specific manner.

9. A transgenic mouse, including progeny, embryo or cell derived from said transgenic mouse, comprising a transgene for the expression of an amyloid precursor protein (APP) or derivative thereof.

10. A transgenic mouse according to Claim 9 wherein said transgene comprises a NotI minigene cassette containing
(a) a regulatory region, said regulatory region capable of directing tissue and cell specific expression,
(b) a gene construct encoding said APP or derivative thereof,
(c) genetic sequences containing a RNA polyadenylation signal, and
(d) an intronic sequence containing acceptor and donor sites for splicing.

APP cDNA Sequence

```
                                                            S
                                                            m
                                                            a
                                                            I
      AGTTTCCTCGGCAGCGGTAGGCGAGAGCACGCGGAGGAGCGTGCGCGGGGCCCCGGGAGA
    1 ---------+---------+---------+---------+---------+---------+ 60
      TCAAAGGAGCCGTCGCCATCCGCTCTCGTGCGCCTCCTCGCACGCGCCCCGGGGCCCTCT

c

                                             B
                                             a
                                             m
                                             H
                                             I
      CGGCGGCGGTGGCGGCGCGGGCAGAGCAAGGACGCGGCGGATCCCACTCGCACAGCAGCG
   61 ---------+---------+---------+---------+---------+---------+ 120
      GCCGCCGCCACCGCCGCGCCCGTCTCGTTCCTGCGCCGCCTAGGGTGAGCGTGTCGTCGC

c

                             N
                             r
                             u
                             I
      CACTCGGTGCCCCGCGCAGGGTCGCGATGCTGCCCGGTTTGGCACTGCTCCTGCTGGCCG
  121 ---------+---------+---------+---------+---------+---------+. 180
      GTGAGCCACGGGGCGCGTCCCAGCGCTACGACGGGCCAAACCGTGACGAGGACGACCGGC

c                               M  L  P  G  L  A  L  L  L  L  A  A -

           B                    K
           g                    p
           l                    n
           I                    I
      CCTGGACGGCTCGGGCGCTGGAGGTACCCACTGATGGTAATGCTGGCCTGCTGGCTGAAC
  181 ---------+---------+---------+---------+---------+---------+ 240
      GGACCTGCCGAGCCCGCGACCTCCATGGGTGACTACCATTACGACCGGACGACCGACTTG

c       W  T  A  R  A  L  E  V  P  T  D  G  N  A  G  L  L  A  E  P -

      CCCAGATTGCCATGTTCTGTGGCAGACTGAACATGCACATGAATGTCCAGAATGGGAAGT
  241 ---------+---------+---------+---------+---------+---------+ 300
      GGGTCTAACGGTACAAGACACCGTCTGACTTGTACGTGTACTTACAGGTCTTACCCTTCA

c       Q  I  A  M  F  C  G  R  L  N  M  H  M  N  V  Q  N  G  K  W -

      GGGATTCAGATCCATCAGGGACCAAAACCTGCATTGATACCAAGGAAGGCATCCTGCAGT
  301 ---------+---------+---------+---------+---------+---------+ 360
      CCCTAAGTCTAGGTAGTCCCTGGTTTTGGACGTAACTATGGTTCCTTCCGTAGGACGTCA

c       D  S  D  P  S  G  T  K  T  C  I  D  T  K  E  G  I  L  Q  Y -
```

FIG.1a

```
                A
                c
                c
                I
     ATTGCCAAGAAGTCTACCCTGAACTGCAGATCACCAATGTGGTAGAAGCCAACCAACCAG
 361 ---------+---------+---------+---------+---------+---------+ 420
     TAACGGTTCTTCAGATGGGACTTGACGTCTAGTGGTTACACCATCTTCGGTTGGTTGGTC

c     C  Q  E  V  Y  P  E  L  Q  I  T  N  V  V  E  A  N  Q  P  V -

     TGACCATCCAGAACTGGTGCAAGCGGGGCCGCAAGCAGTGCAAGACCCATCCCCACTTTG
 421 ---------+---------+---------+---------+---------+---------+ 480
     ACTGGTAGGTCTTGACCACGTTCGCCCCGGCGTTCGTCACGTTCTGGGTAGGGGTGAAAC

c     T  I  Q  N  W  C  K  R  G  R  K  Q  C  K  T  H  P  H  F  V -

     TGATTCCCTACCGCTGCTTAGTTGGTGAGTTTGTAAGTGATGCCCTTCTCGTTCCTGACA
 481 ---------+---------+---------+---------+---------+---------+ 540
     ACTAAGGGATGGCGACGAATCAACCACTCAAACATTCACTACGGGAAGAGCAAGGACTGT

c     I  P  Y  R  C  L  V  G  E  F  V  S  D  A  L  L  V  P  D  K -

     AGTGCAAATTCTTACACCAGGAGAGGATGGATGTTTGCGAAACTCATCTTCACTGGCACA
 541 ---------+---------+---------+---------+---------+---------+ 600
     TCACGTTTAAGAATGTGGTCCTCTCCTACCTACAAACGCTTTGAGTAGAAGTGACCGTGT

c     C  K  F  L  H  Q  E  R  M  D  V  C  E  T  H  L  H  W  H  T -

     CCGTCGCCAAAGAGACATGCAGTGAGAAGAGTACCAACTTGCATGACTACGGCATGTTGC
 601 ---------+---------+---------+---------+---------+---------+ 660
     GGCAGCGGTTTCTCTGTACGTCACTCTTCTCATGGTTGAACGTACTGATGCCGTACAACG

c     V  A  K  E  T  C  S  E  K  S  T  N  L  H  D  Y  G  M  L  L -

     TGCCCTGCGGAATTGACAAGTTCCGAGGGGTAGAGTTTGTGTGTTGCCCACTGGCTGAAG
 661 ---------+---------+---------+---------+---------+---------+ 720
     ACGGGACGCCTTAACTGTTCAAGGCTCCCCATCTCAAACACACAACGGGTGACCGACTTC

c     P  C  G  I  D  K  F  R  G  V  E  F  V  C  C  P  L  A  E  E -

     AAAGTGACAATGTGGATTCTGCTGATGCGGAGGAGGATGACTCGGATGTCTGGTGGGGCG
 721 ---------+---------+---------+---------+---------+---------+ 780
     TTTCACTGTTACACCTAAGACGACTACGCCTCCTCCTACTGAGCCTACAGACCACCCCGC

c     S  D  N  V  D  S  A  D  A  E  E  D  D  S  D  V  W  W  G  G -

     GAGCAGACACAGACTATGCAGATGGGAGTGAAGACAAAGTAGTAGAAGTAGCAGAGGAGG
 781 ---------+---------+---------+---------+---------+---------+ 840
     CTCGTCTGTGTCTGATACGTCTACCCTCACTTCTGTTTCATCATCTTCATCGTCTCCTCC

c     A  D  T  D  Y  A  D  G  S  E  D  K  V  V  E  V  A  E  E  E -

     AAGAAGTGGCTGAGGTGGAAGAAGAAGAAGCCGATGATGACGAGGACGATGAGGATGGTG
 841 ---------+---------+---------+---------+---------+---------+ 900
     TTCTTCACCGACTCCACCTTCTTCTTCTTCGGCTACTACTGCTCCTGCTACTCCTACCAC

c     E  V  A  E  V  E  E  E  E  A  D  D  D  E  D  D  E  D  G  D -

     ATGAGGTAGAGGAAGAGGCTGAGGAACCCTACGAAGAAGCCACAGAGAGAACCACCAGCA
 901 ---------+---------+---------+---------+---------+---------+ 960
     TACTCCATCTCCTTCTCCGACTCCTTGGGATGCTTCTTCGGTGTCTCTCTTGGTGGTCGT

c     E  V  E  E  E  A  E  E  P  Y  E  E  A  T  E  R  T  T  S  I -

     TTGCCACCACCACCACCACCACCACAGAGTCTGTGGAAGAGGTGGTTCGAGTTCCTACAA
 961 ---------+---------+---------+---------+---------+---------+ 1020
```

FIG.1b

```
           AACGGTGGTGGTGGTGGTGGTGTCTCAGACACCTTCTCCACCAAGCTCAAGGATGTT

c            A  T  T  T  T  T  T  T  E  S  V  E  E  V  V  R  V  P  T  T -

                                                 X
                                                 h
                                                 o
                                                 I
           CAGCAGCCAGTACCCCTGATGCCGTTGACAAGTATCTCGAGACACCTGGGGATGAGAATG
      1021 ---------+---------+---------+---------+---------+---------+ 1080
           GTCGTCGGTCATGGGGACTACGGCAACTGTTCATAGAGCTCTGTGGACCCCTACTCTTAC

c            A  A  S  T  P  D  A  V  D  K  Y  L  E  T  P  G  D  E  N  E -

           AACATGCCCATTTCCAGAAAGCCAAAGAGAGGCTTGAGGCCAAGCACCGAGAGAGAATGT
      1081 ---------+---------+---------+---------+---------+---------+ 1140
           TTGTACGGGTAAAGGTCTTTCGGTTTCTCTCCGAACTCCGGTTCGTGGCTCTCTCTTACA

c            H  A  H  F  Q  K  A  K  E  R  L  E  A  K  H  R  E  R  M  S -

           CCCAGGTCATGAGAGAATGGGAAGAGGCAGAACGTCAAGCAAAGAACTTGCCTAAAGCTG
      1141 ---------+---------+---------+---------+---------+---------+ 1200
           GGGTCCAGTACTCTCTTACCCTTCTCCGTCTTGCAGTTCGTTTCTTGAACGGATTTCGAC

c            Q  V  M  R  E  W  E  E  A  E  R  Q  A  K  N  L  P  K  A  D -

           ATAAGAAGGCAGTTATCCAGCATTTCCAGGAGAAAGTGGAATCTTTGGAACAGGAAGCAG
      1201 ---------+---------+---------+---------+---------+---------+ 1260
           TATTCTTCCGTCAATAGGTCGTAAAGGTCCTCTTTCACCTTAGAAACCTTGTCCTTCGTC

c            K  K  A  V  I  Q  H  F  Q  E  K  V  E  S  L  E  Q  E  A  A -

           CCAACGAGAGACAGCAGCTGGTGGAGACACACATGGCCAGAGTGGAAGCCATGCTCAATG
      1261 ---------+---------+---------+---------+---------+---------+ 1320
           GGTTGCTCTCTGTCGTCGACCACCTCTGTGTGTACCGGTCTCACCTTCGGTACGAGTTAC

c            N  E  R  Q  Q  L  V  E  T  H  M  A  R  V  E  A  M  L  N  D -

           ACCGCCGCCGCCTGGCCCTGGAGAACTACATCACCGCTCTGCAGGCTGTTCCTCCTCGGC
      1321 ---------+---------+---------+---------+---------+---------+ 1380
           TGGCGGCGGCGGACCGGGACCTCTTGATGTAGTGGCGAGACGTCCGACAAGGAGGAGCCG

c            R  R  R  L  A  L  E  N  Y  I  T  A  L  Q  A  V  P  P  R  P -

           CTCGTCACGTGTTCAATATGCTAAAGAAGTATGTCCGCGCAGAACAGAAGGACAGACAGC
      1381 ---------+---------+---------+---------+---------+---------+ 1440
           GAGCAGTGCACAAGTTATACGATTTCTTCATACAGGCGCGTCTTGTCTTCCTGTCTGTCG

c            R  H  V  F  N  M  L  K  K  Y  V  R  A  E  Q  K  D  R  Q  H -

                                                B
                                                a
                                                m
                                                H
                                                I
           ACACCCTAAAGCATTTCGAGCATGTGCGCATGGTGGATCCCAAGAAAGCCGCTCAGATCC
      1441 ---------+---------+---------+---------+---------+---------+ 1500
           TGTGGGATTTCGTAAAGCTCGTACACGCGTACCACCTAGGGTTCTTTCGGCGAGTCTAGG

c            T  L  K  H  F  E  H  V  R  M  V  D  P  K  K  A  A  Q  I  R -

           GGTCCCAGGTTATGACACACCTCCGTGTGATTTATGAGCGCATGAATCAGTCTCTCTCCC
      1501 ---------+---------+---------+---------+---------+---------+ 1560
           CCAGGGTCCAATACTGTGTGGAGGCACACTAAATACTCGCGTACTTAGTCAGAGAGAGGG

c            S  Q  V  M  T  H  L  R  V  I  Y  E  R  M  N  Q  S  L  S  L -
```

FIG. 1c

```

     TGCTCTACAACGTGCCTGCAGTGGCCGAGGAGATTCAGGATGAAGTTGATGAGCTGCTTC
1561 ---------+---------+---------+---------+---------+---------+ 1620
     ACGAGATGTTGCACGGACGTCACCGGCTCCTCTAAGTCCTACTTCAACTACTCGACGAAG

c      L  Y  N  V  P  A  V  A  E  E  I  Q  D  E  V  D  E  L  L  Q -

                                  A
                                  a
                                  t
                                  I
                                  I
     AGAAAGAGCAAAACTATTCAGATGACGTCTTGGCCAACATGATTAGTGAACCAAGGATCA
1621 ---------+---------+---------+---------+---------+---------+ 1680
     TCTTTCTCGTTTTGATAAGTCTACTGCAGAACCGGTTGTACTAATCACTTGGTTCCTAGT

c      K  E  Q  N  Y  S  D  D  V  L  A  N  M  I  S  E  P  R  I  S -

     GTTACGGAAACGATGCTCTCATGCCATCTTTGACCGAAACGAAAACCACCGTGGAGCTCC
1681 ---------+---------+---------+---------+---------+---------+ 1740
     CAATGCCTTTGCTACGAGAGTACGGTAGAAACTGGCTTTGCTTTTGGTGGCACCTCGAGG

c      Y  G  N  D  A  L  M  P  S  L  T  E  T  K  T  T  V  E  L  L -

     TTCCCGTGAATGGAGAGTTCAGCCTGGACGATCTCCAGCCGTGGCATTCTTTTGGGGCTG
1741 ---------+---------+---------+---------+---------+---------+ 1800
     AAGGGCACTTACCTCTCAAGTCGGACCTGCTAGAGGTCGGCACCGTAAGAAAACCCCGAC

c      P  V  N  G  E  F  S  L  D  D  L  Q  P  W  H  S  F  G  A  D -

     ACTCTGTGCCAGCCAACACAGAAAACGAAGTTGAGCCTGTTGATGCCCGCCCTGCTGCCG
1801 ---------+---------+---------+---------+---------+---------+ 1860
     TGAGACACGGTCGGTTGTGTCTTTTGCTTCAACTCGGACAACTACGGGCGGGACGACGGC

c      S  V  P  A  N  T  E  N  E  V  E  P  V  D  A  R  P  A  A  D -

                                                           B
                                                           g
                                                           l
                                                           I
                                                           I
     ACCGAGGACTGACCACTCGACCAGGTTCTGGGTTGACAAATATCAAGACGGAGGAGATCT
1861 ---------+---------+---------+---------+---------+---------+ 1920
     TGGCTCCTGACTGGTGAGCTGGTCCAAGACCCAACTGTTTATAGTTCTGCCTCCTCTAGA

c      R  G  L  T  T  R  P  G  S  G  L  T  N  I  K  T  E  E  I  S -

                             E
                             c
                             o
                             R
                             I
     CTGAAGTGAAGATGGATGCAGAATTCCGACATGACTCAGGATATGAAGTTCATCATCAAA
1921 ---------+---------+---------+---------+---------+---------+ 1980
     GACTTCACTTCTACCTACGTCTTAAGGCTGTACTGAGTCCTATACTTCAAGTAGTAGTTT

c      E  V  K  M  D  A  E  F  R  H  D  S  G  Y  E  V  H  H  Q  K -

     AATTGGTGTTCTTTGCAGAAGATGTGGGTTCAAACAAAGGTGCAATCATTGGACTCATGG
1981 ---------+---------+---------+---------+---------+---------+ 2040
     TTAACCACAAGAAACGTCTTCTACACCCAAGTTTGTTTCCACGTTAGTAACCTGAGTACC

c      L  V  F  F  A  E  D  V  G  S  N  K  G  A  I  I  G  L  M  V -

     TGGGCGGTGTTGTCATAGCGACAGTGATCGTCATCACCTTGGTGATGCTGAAGAAGAAAC
2041 ---------+---------+---------+---------+---------+---------+ 2100
```

FIG.1d

```
            ACCCGCCACAACAGTATCGCTGTCACTAGCAGTAGTGGAACCACTACGACTTCTTCTTTG

c            G  G  V  V  I  A  T  V  I  V  I  T  L  V  M  L  K  K  K  Q -

            AGTACACATCCATTCATCATGGTGTGGTGGAGGTTGACGCCGCTGTCACCCCAGAGGAGC
      2101  ---------+---------+---------+---------+---------+---------+ 2160
            TCATGTGTAGGTAAGTAGTACCACACCACCTCCAACTGCGGCGACAGTGGGGTCTCCTCG

c            Y  T  S  I  H  H  G  V  V  E  V  D  A  A  V  T  P  E  E  R -

            GCCACCTGTCCAAGATGCAGCAGAACGGCTACGAAAATCCAACCTACAAGTTCTTTGAGC
      2161  ---------+---------+---------+---------+---------+---------+ 2220
            CGGTGGACAGGTTCTACGTCGTCTTGCCGATGCTTTTAGGTTGGATGTTCAAGAAACTCG

c            H  L  S  K  M  Q  Q  N  G  Y  E  N  P  T  Y  K  F  F  E  Q -

            AGATGCAGAACTAGACCCCCGCCACAGCAGCCTCTGAAGTTGGACAGCAAAACCATTGCT
      2221  ---------+---------+---------+---------+---------+---------+ 2280
            TCTACGTCTTGATCTGGGGGCGGTGTCGTCGGAGACTTCAACCTGTCGTTTTGGTAACGA

c            M  Q  N  *

            TCACTACCCATCGGTGTCCATTTATAGAATAATGTGGGAAGAAACAAACCCGTTTTATGA
      2281  ---------+---------+---------+---------+---------+---------+ 2340
            AGTGATGGGTAGCCACAGGTAAATATCTTATTACACCCTTCTTTGTTTGGGCAAAATACT

c

            TTTACTCATTATCGCCTTTTGACAGCTGTGCTGTAACACAAGTAGATGCCTGAACTTGAA
      2341  ---------+---------+---------+---------+---------+---------+ 2400
            AAATGAGTAATAGCGGAAAACTGTCGACACGACATTGTGTTCATCTACGGACTTGAACTT

c

            TTAATCCACACATCAGTAATGTATTCTATCTCTCTTTACATTTTGGTCTCTATACTACAT
      2401  ---------+---------+---------+---------+---------+---------+ 2460
            AATTAGGTGTGTAGTCATTACATAAGATAGAGAGAAATGTAAAACCAGAGATATGATGTA

c

                                                        S
                                                        p
                                                        e
                                                        I
            TATTAATGGGTTTTGTGTACTGTAAAGAATTTAGCTGTATCAAACTAGTGCATGAATAGA
      2461  ---------+---------+---------+---------+---------+---------+ 2520
            ATAATTACCCAAAACACATGACATTTCTTAAATCGACATAGTTTGATCACGTACTTATCT

c

            TTCTCTCCTGATTATTTATCACATAGCCCCTTAGCCAGTTGTATATTATTCTTGTGGTTT
      2521  ---------+---------+---------+---------+---------+---------+ 2580
            AAGAGAGGACTAATAAATAGTGTATCGGGGAATCGGTCAACATATAATAAGAACACCAAA

c

                                    N             C
                                    d             l
                                    e             a
                                    I             I
            GTGACCCAATTAAGTCCTACTTTACATATGCTTTAAGAATCGATGGGGGATGCTTCATGT
      2581  ---------+---------+---------+---------+---------+---------+ 2640
            CACTGGGTTAATTCAGGATGAAATGTATACGAAATTCTTAGCTACCCCCTACGAAGTACA

c
```

FIG. 1e

```
                                                               N
                                                               s
                                                               i
                                                               I
     GAACGTGGGAGTTCAGCTGCTTCTCTTGCCTAAGTATTCCTTTCCTGATCACTATGCATT
2641 ---------+---------+---------+---------+---------+---------+ 2700
     CTTGCACCCTCAAGTCGACGAAGAGAACGGATTCATAAGGAAAGGACTAGTGATACGTAA

c

     TTAAAGTTAAACATTTTTAAGTATTTCAGATGCTTTAGAGAGATTTTTTTCCATGACTG
2701 ---------+---------+---------+---------+---------+---------+ 2760
     AATTTCAATTTGTAAAAATTCATAAAGTCTACGAAATCTCTCTAAAAAAAGGTACTGAC

c

     CATTTTACTGTACAGATTGCTGCTTCTGCTATATTTGTGATATAGGAATTAAGAGGATAC
2761 ---------+---------+---------+---------+---------+---------+ 2820
     GTAAAATGACATGTCTAACGACGAAGACGATATAAACACTATATCCTTAATTCTCCTATG

c

                                                             H
                                                             i
                                                             n
                                                             d
                                                             I
                                                             I
                                                             I
     ACACGTTTGTTTCTTCGTGCCTGTTTTATGTGCACACATTAGGCATTGAGACTTCAAGCT
2821 ---------+---------+---------+---------+---------+---------+ 2880
     TGTGCAAACAAAGAAGCACGGACAAAATACACGTGTGTAATCCGTAACTCTGAAGTTCGA

c

     TTTCTTTTTTTGTCCACGTATCTTTGGGTCTTTGATAAAGAAAAGAATCCCTGTTCATTG
2881 ---------+---------+---------+---------+---------+---------+ 2940
     AAAGAAAAAAACAGGTGCATAGAAACCCAGAAACTATTTCTTTTCTTAGGGACAAGTAAC

c

                                                             E
                                                             c
                                                             o
                                                             R
                                                             I
     TAAGCACTTTTACGGGGCGGGTGGGGAGGGGTGCTCTGCTGGTCTTCAATTACCAAGAAT
2941 ---------+---------+---------+---------+---------+---------+ 3000
     ATTCGTGAAAATGCCCCGCCCACCCCTCCCCACGAGACGACCAGAAGTTAATGGTTCTTA

c

     TCTCCAAAACAATTTTCTGCAGGATGATTGTACAGAATCATTGCTTATGACATGATCGCT
3001 ---------+---------+---------+---------+---------+---------+ 3060
     AGAGGTTTTGTTAAAAGACGTCCTACTAACATGTCTTAGTAACGAATACTGTACTAGCGA

c

                                              S
                                              m
                                              a
                                              I
     TTCTACACTGTATTACATAAATAAATTAAATAAAATAACCCCGGGCAAGACTTTTCTTTG
3061 ---------+---------+---------+---------+---------+---------+ 3120
     AAGATGTGACATAATGTATTTATTTAATTTATTTTATTGGGGCCCGTTCTGAAAAGAAAC
```

FIG. 1f

```
c

     AAGGATGACTACAGACATTAAATAATCGAAGTAATTTTGGGTGGGGAGAAGAGGCAGATT
3121 ---------+---------+---------+---------+---------+---------+ 3180
     TTCCTACTGATGTCTGTAATTTATTAGCTTCATTAAAACCCACCCCTCTTCTCCGTCTAA

c

     CAATTTTCTTTAACCAGTCTGAAGTTTCATTTATGATACAAAAGAAGATGAAAATGGAAG
3181 ---------+---------+---------+---------+---------+---------+ 3240
     GTTAAAAGAAATTGGTCAGACTTCAAAGTAAATACTATGTTTTCTTCTACTTTTACCTTC

c

                                  S
                                  p
                                  h
                                  I
     TGGCAATATAAGGGGATGAGGAAGGCATGCCTGGACAAACCCTTCTTTTAAGATGTGTCT
3241 ---------+---------+---------+---------+---------+---------+ 3300
     ACCGTTATATTCCCCTACTCCTTCCGTACGGACCTGTTTGGGAAGAAAATTCTACACAGA

c

     TCAATTTGTATAAAATGGTGTTTTCATGTAAATAAATACATTCTTGGAGGAGC
3301 ---------+---------+---------+---------+---------+--- 3353
     AGTTAAACATATTTTACCACAAAAGTACATTTATTTATGTAAGAACCTCCTCG
```

FIG.1g

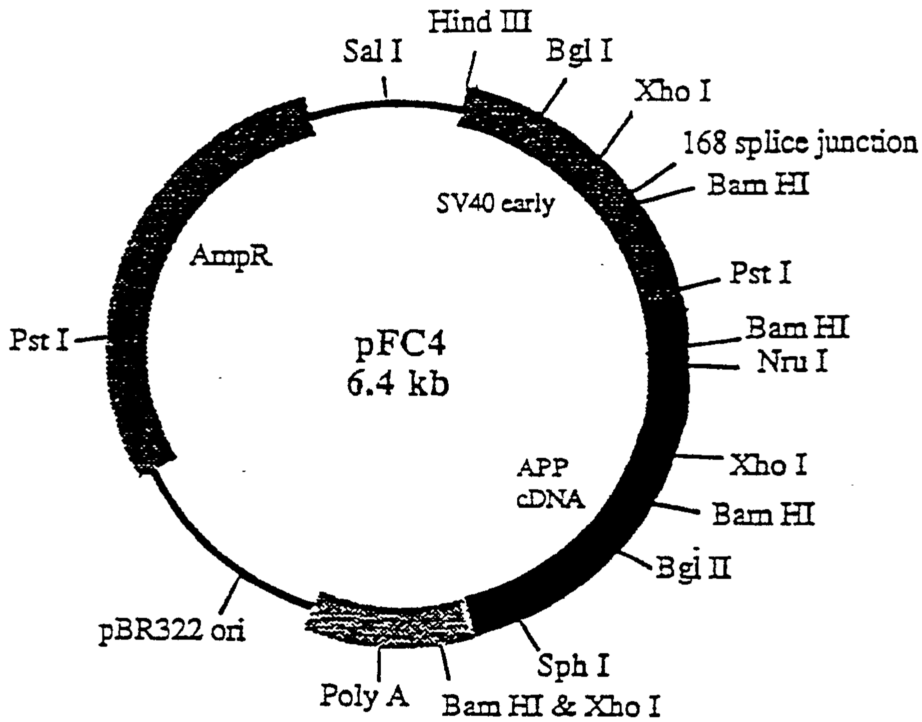
Hind III
Sal I
Bgl I
Xho I
168 splice junction
Bam HI
SV40 early
AmpR
Pst I
Pst I
Bam HI
Nru I
pFC4
6.4 kb
APP
cDNA
Xho I
Bam HI
Bgl II
pBR322 ori
Sph I
Poly A
Bam HI & Xho I

FIG. 2

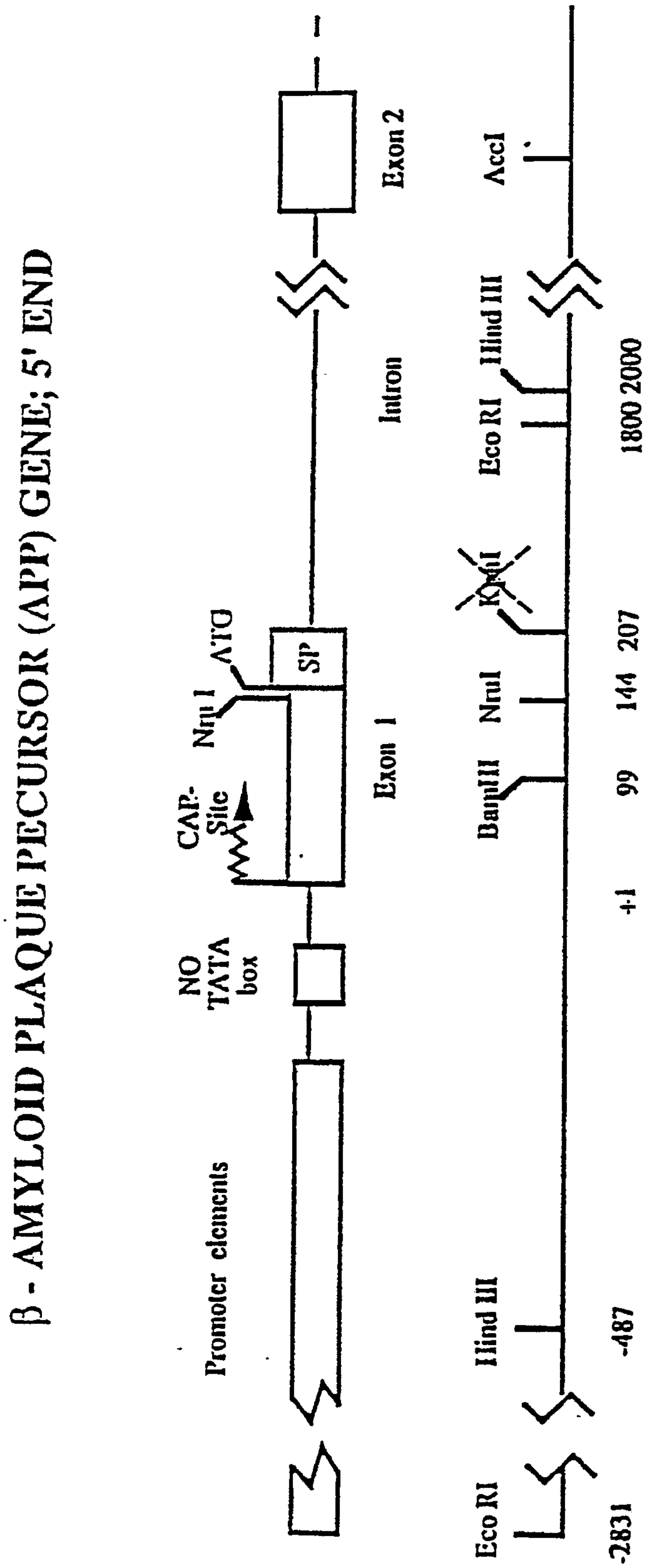
β - AMYLOID PLAQUE PECURSOR (APP) GENE; 5' END
Promoter elements
NO TATA box
CAP-Site
Nru I
ATG
SP
Exon 1
Intron
Exon 2
Eco RI
Hind III
BamHI
NruI
Eco RI
Hind III
AccI
-2831
-487
+1
99
144
207
1800 2000

FIG.3

## APP MINIGENE SERIES

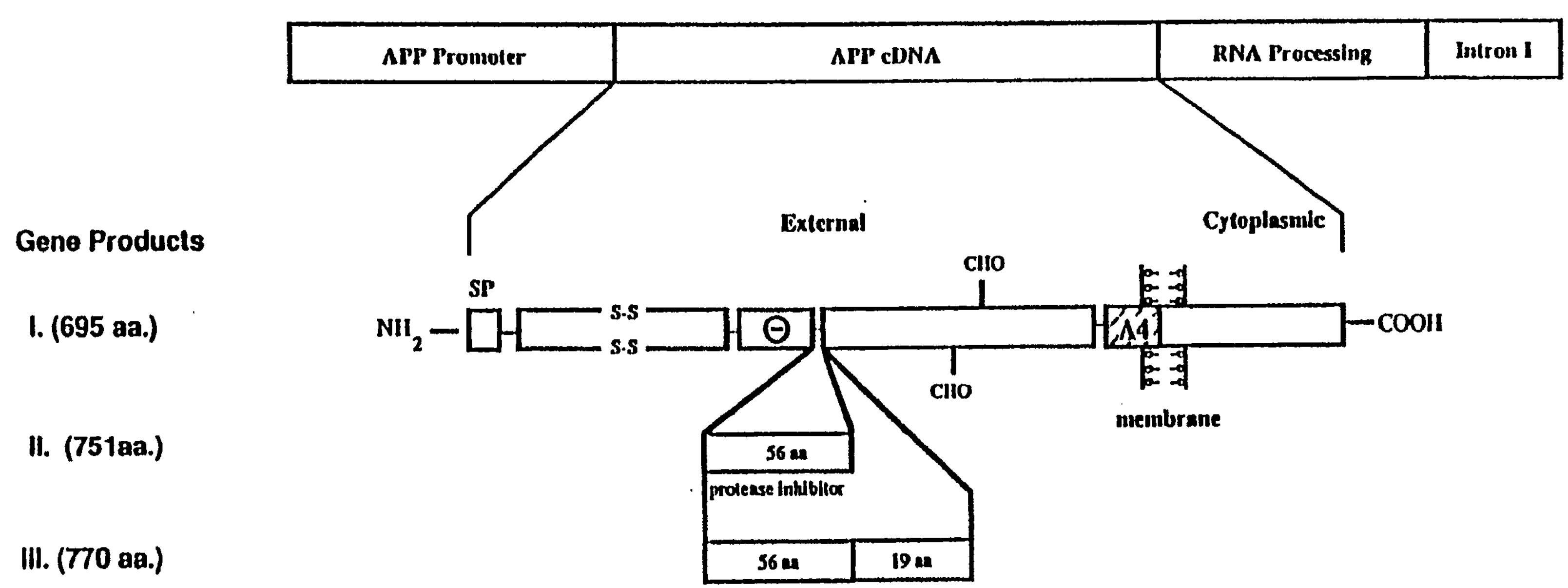


FIG. 4a

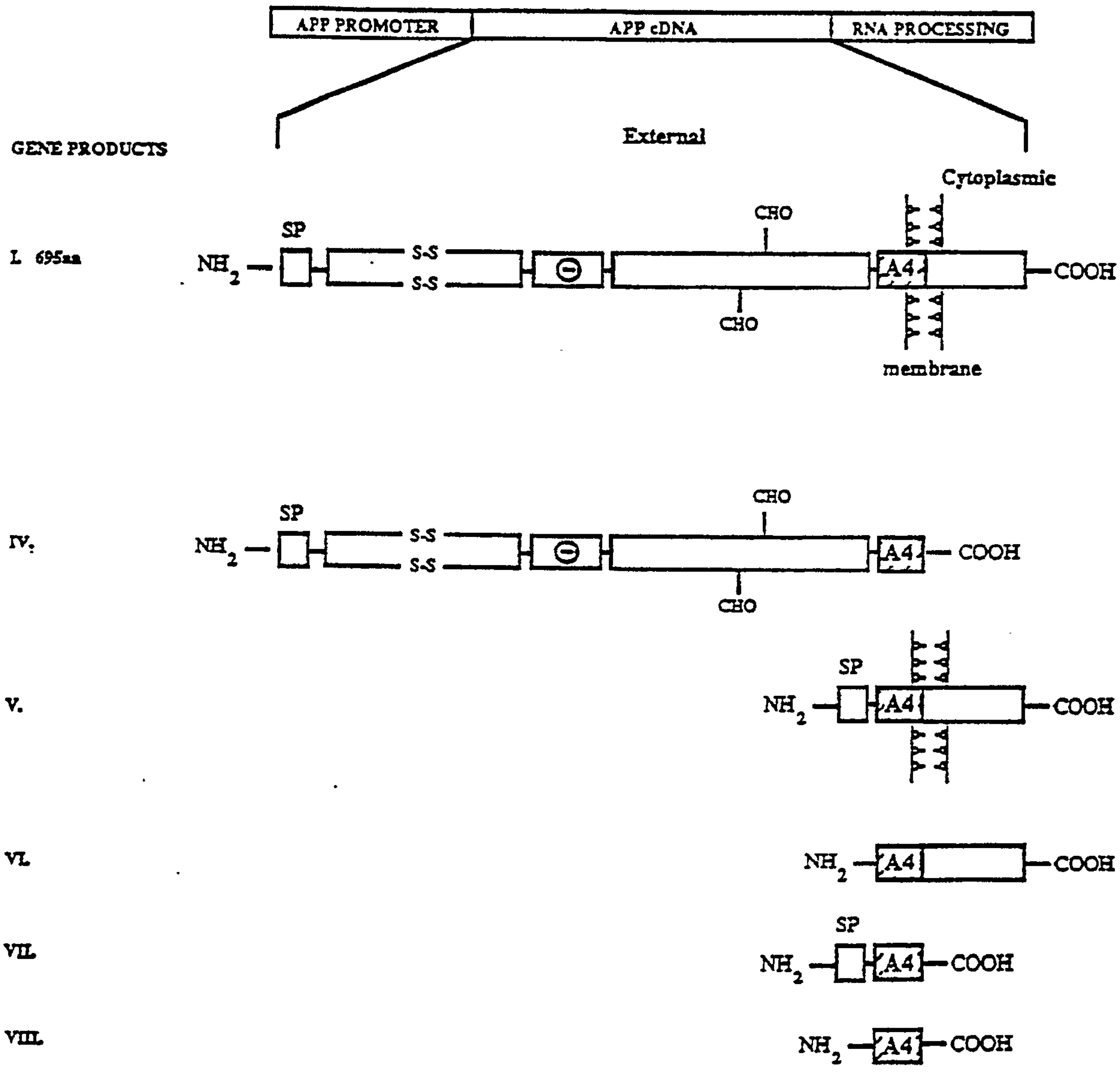
APP PROMOTER
APP cDNA
RNA PROCESSING
GENE PRODUCTS
External
Cytoplasmic
I. 695aa
SP
NH2
S-S
S-S
CHO
CHO
A4
COOH
membrane
IV.
V.
VI.
VII.
VIII.

FIG. 4b

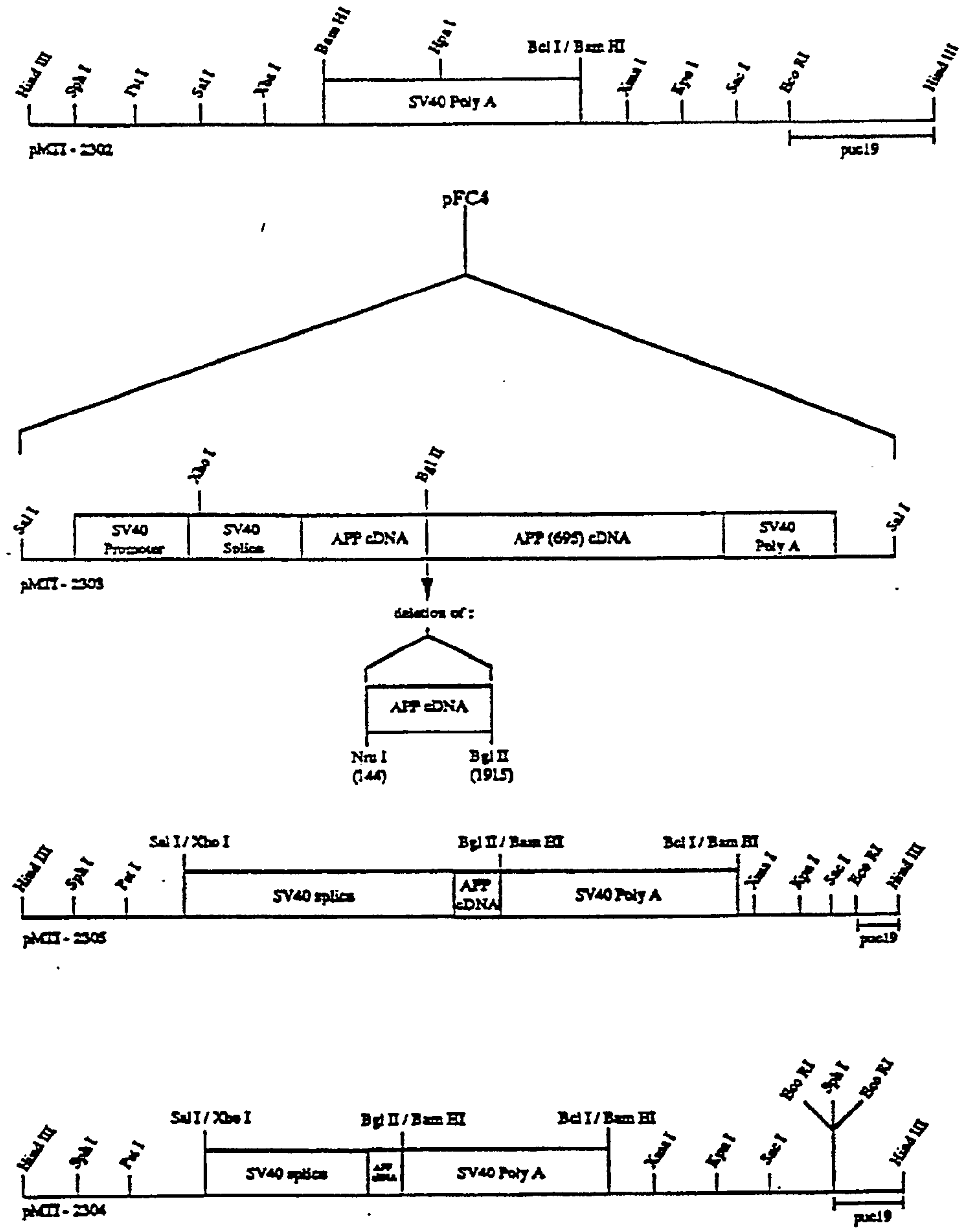
pMTI - 2302
SV40 Poly A
Bcl I / Bam HI
puc19
pFC4
pMTI - 2303
SV40 Promoter
SV40 Splice
APP cDNA
APP (695) cDNA
SV40 Poly A
deletion of :
APP cDNA
Nru I (144)
Bgl II (1915)
pMTI - 2305
Sal I / Xho I
Bgl II / Bam HI
Bcl I / Bam HI
SV40 splice
APP cDNA
SV40 Poly A
puc19
pMTI - 2304
Sal I / Xho I
Bgl II / Bam HI
Bcl I / Bam HI
SV40 splice
SV40 Poly A
puc19

FIG. 5

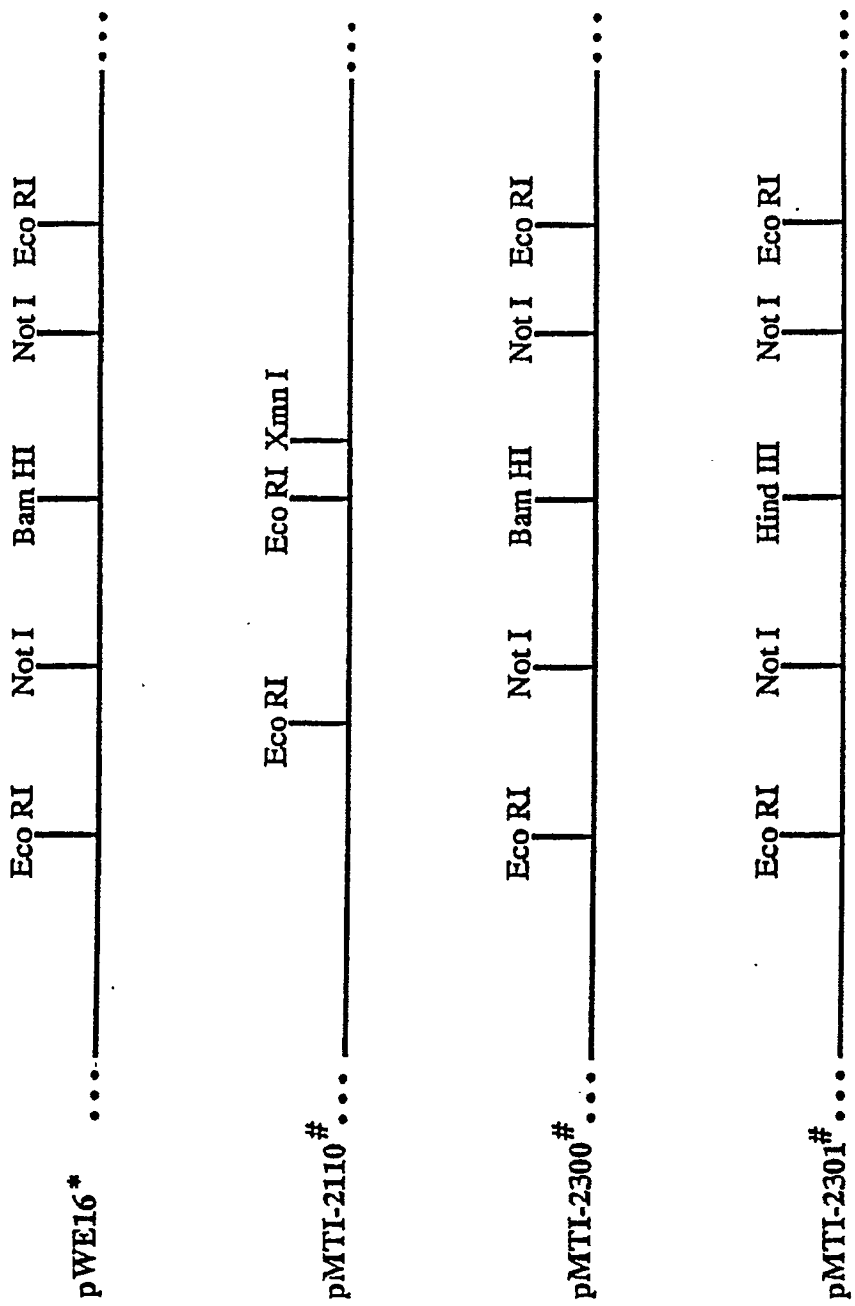


* polylinker region of pWE16

\# polylinker region: puc19 vector sequence not shown

FIG.6a

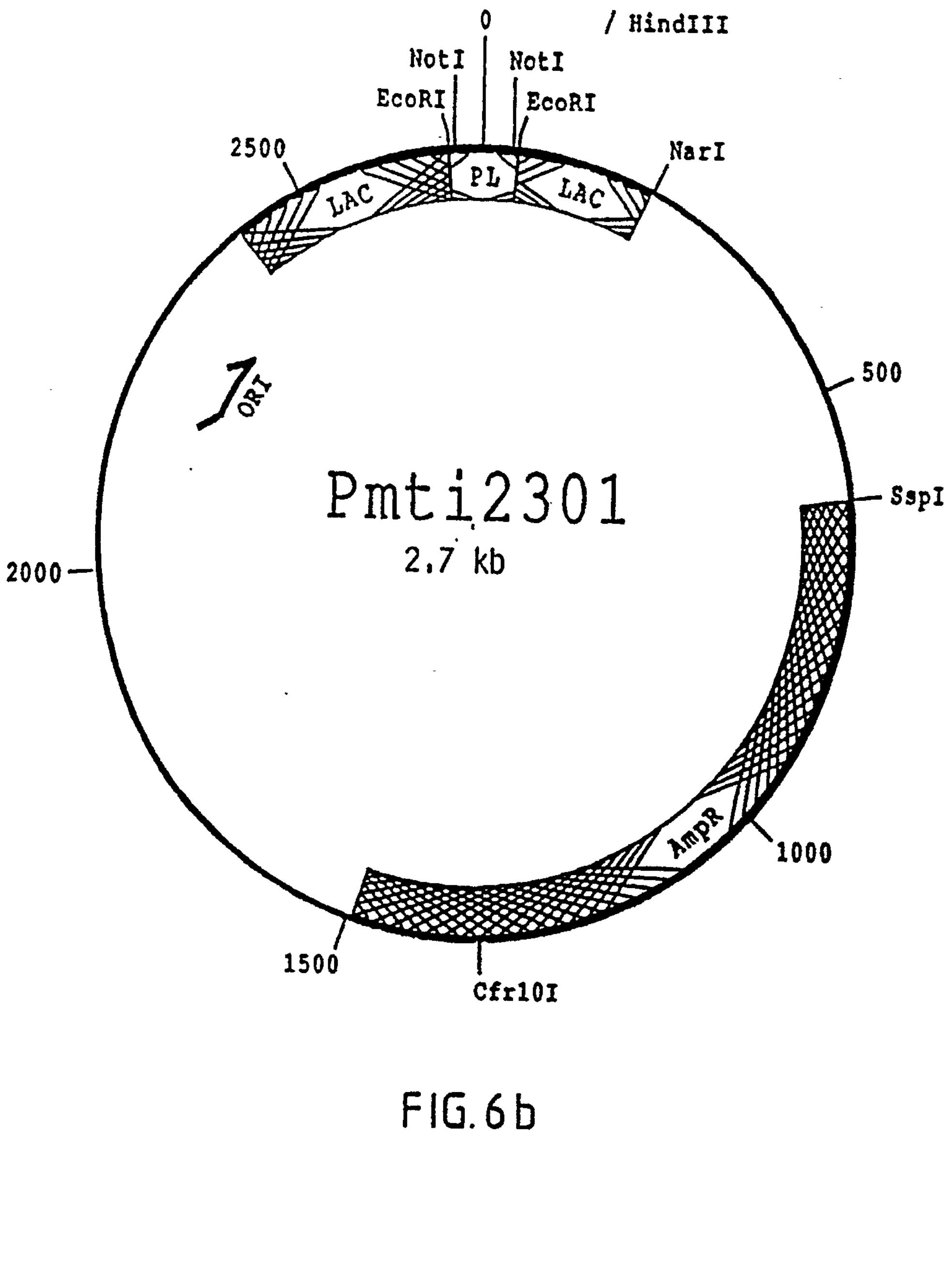
0
/ HindIII
NotI
NotI
EcoRI
EcoRI
2500
NarI
PL
LAC
LAC
ORI
500
Pmti2301
2.7 kb
SspI
2000
AmpR
1000
1500
Cfr10I

FIG.6b

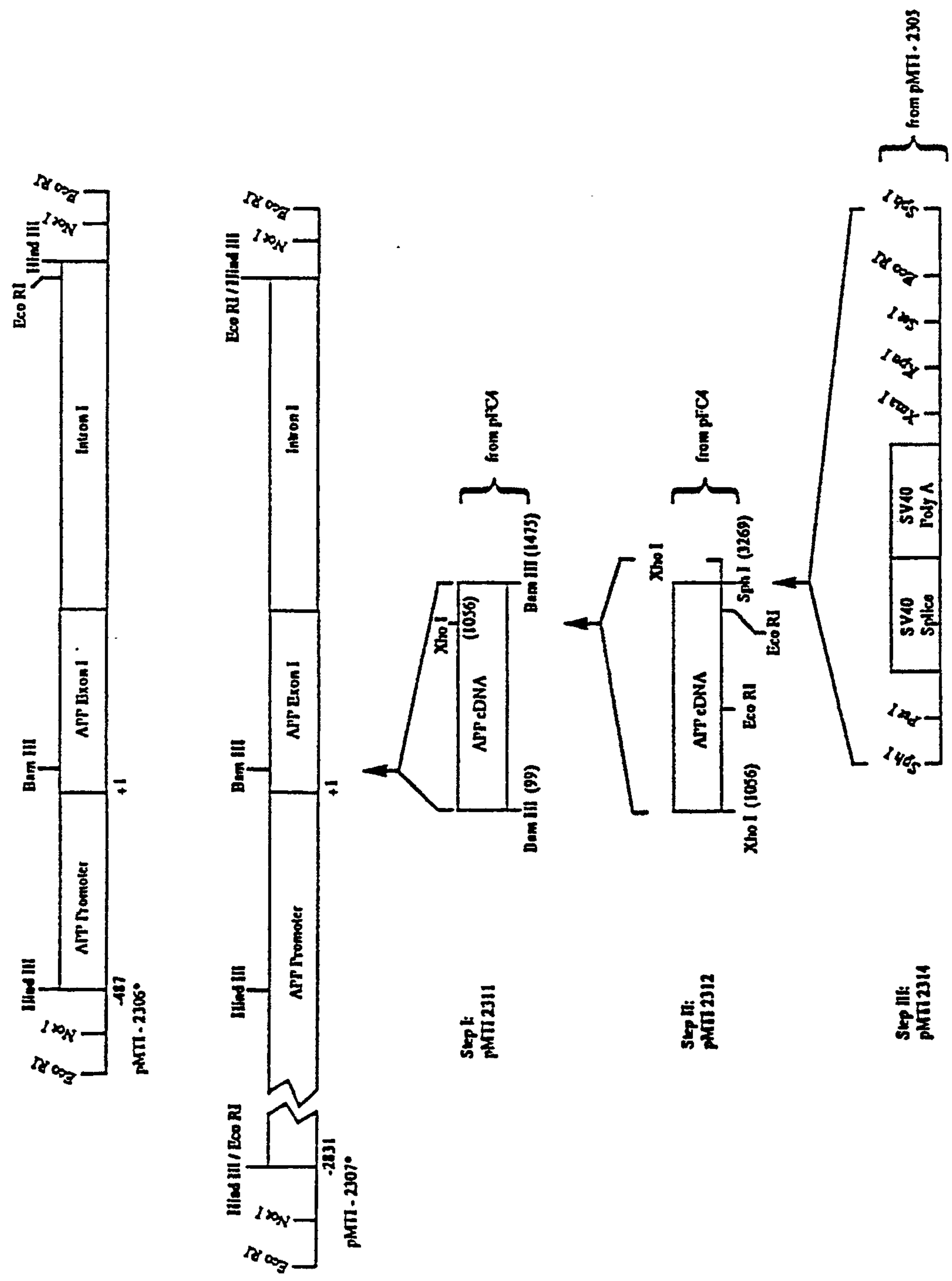


FIG.7a

*vector sequences not shown

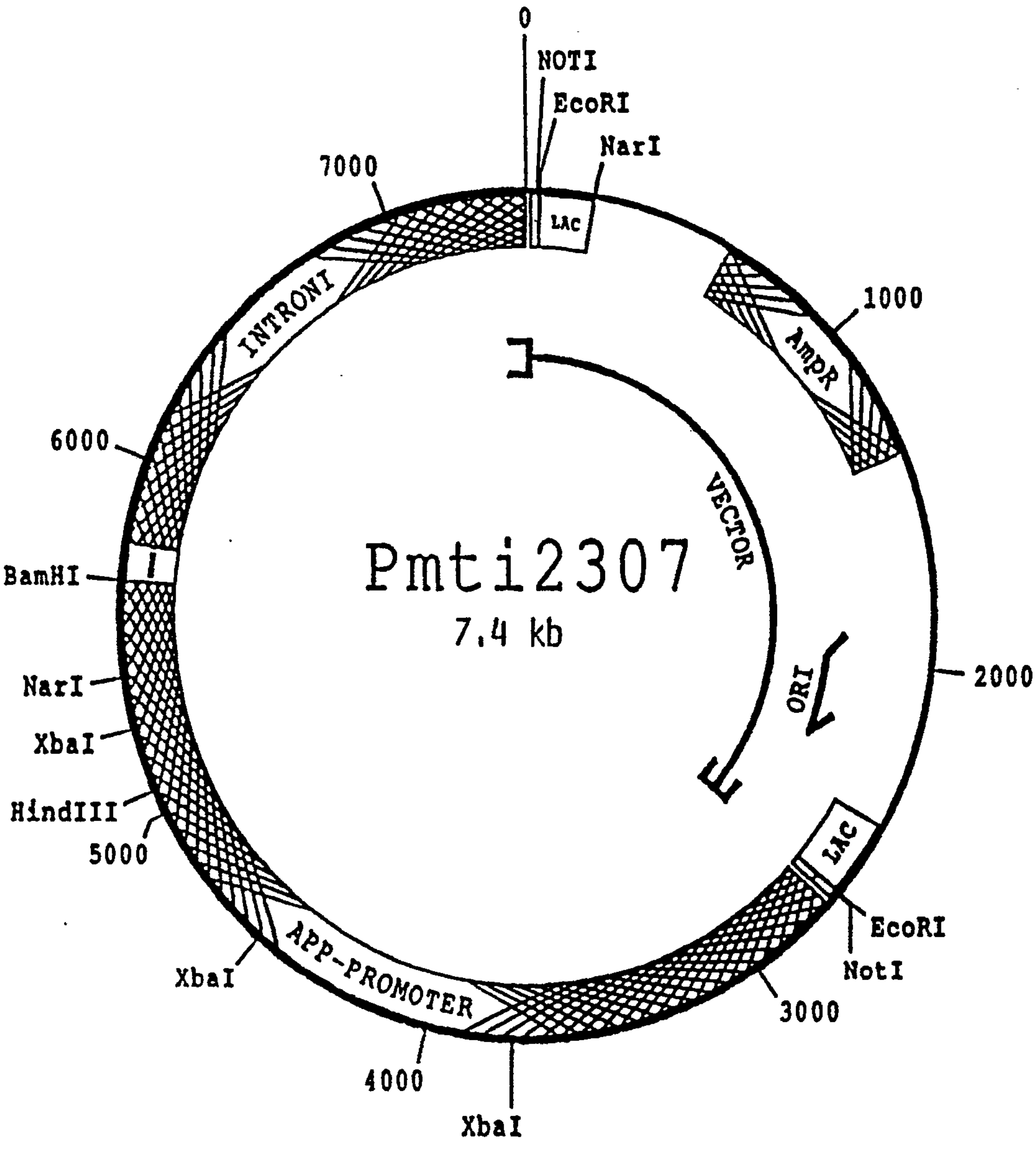
0
NOTI
EcoRI
NarI
LAC
7000
1000
AmpR
INTRONI
VECTOR
6000
Pmti2307
7,4 kb
BamHI
ORI
2000
NarI
XbaI
HindIII
LAC
5000
EcoRI
NotI
XbaI
APP-PROMOTER
3000
4000
XbaI

FIG.7b

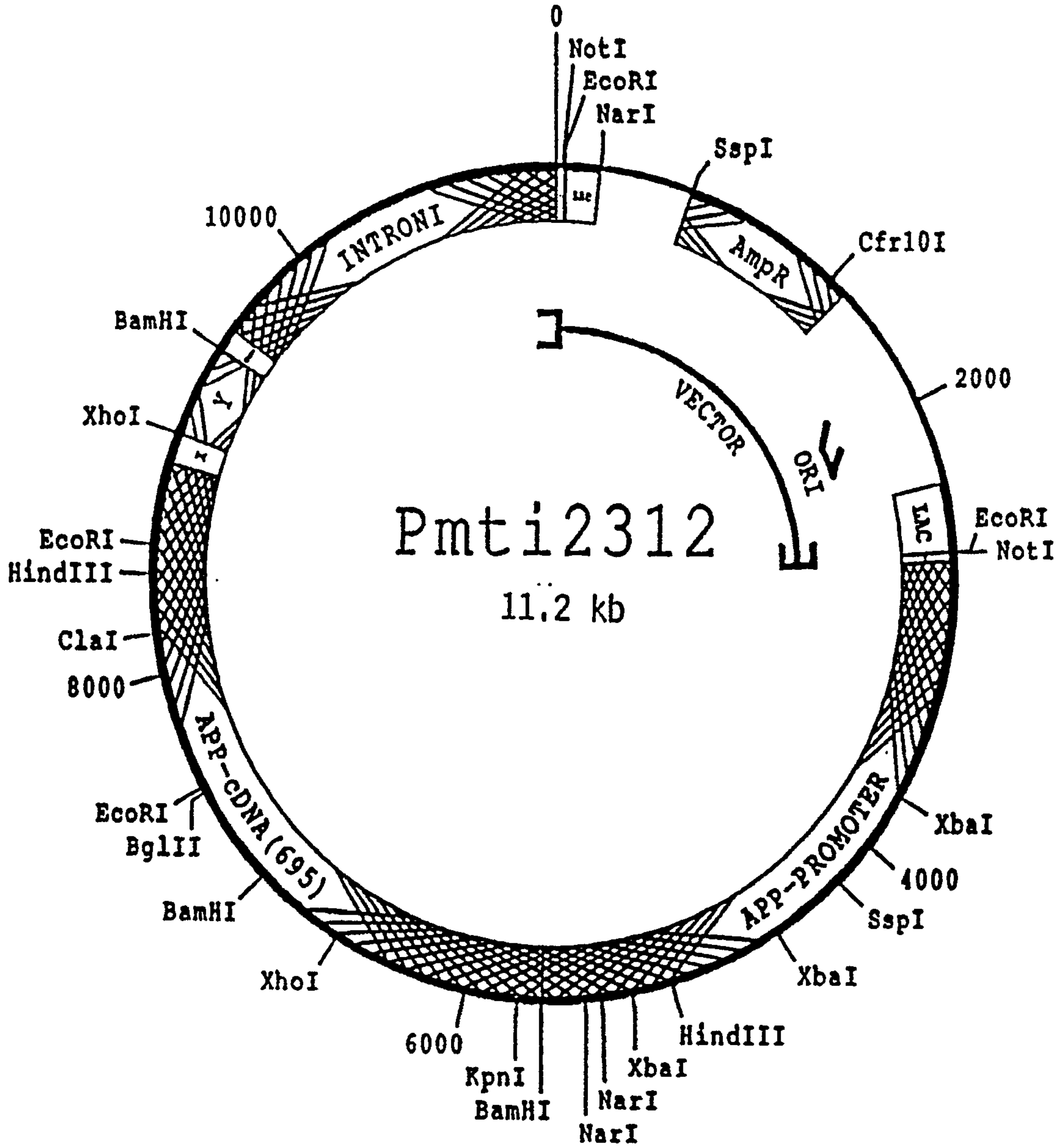
0
NotI
EcoRI
NarI
SspI
Cfr10I
AmpR
2000
VECTOR
ORI
LAC
EcoRI
NotI
Pmti2312
11.2 kb
XbaI
4000
SspI
APP-PROMOTER
XbaI
HindIII
XbaI
NarI
NarI
BamHI
KpnI
6000
XhoI
BamHI
APP-cDNA(695)
EcoRI
BglII
8000
ClaI
HindIII
EcoRI
XhoI
BamHI
10000
INTRONI

FIG.7c

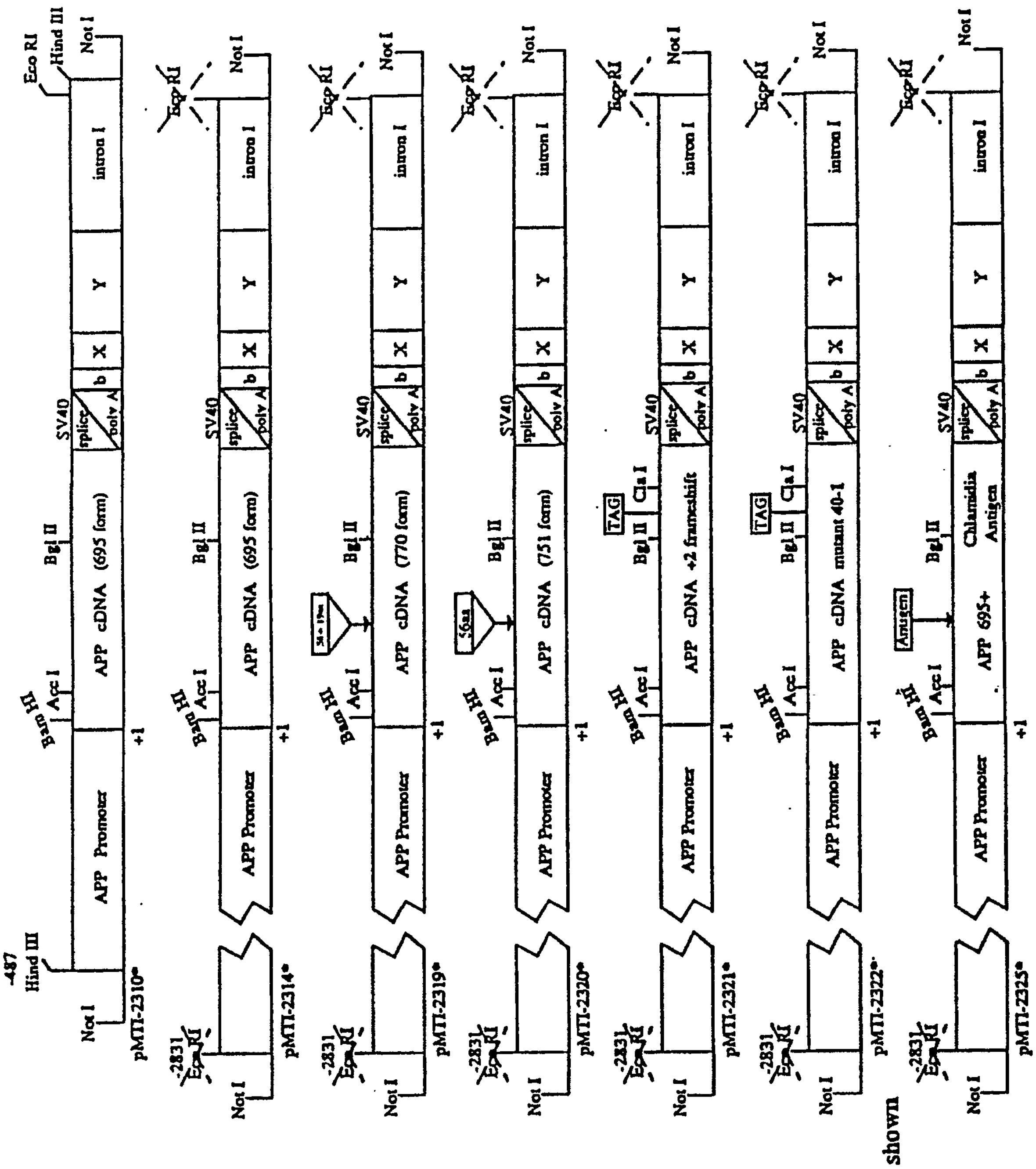


FIG. 8a

* vector sequences not shown

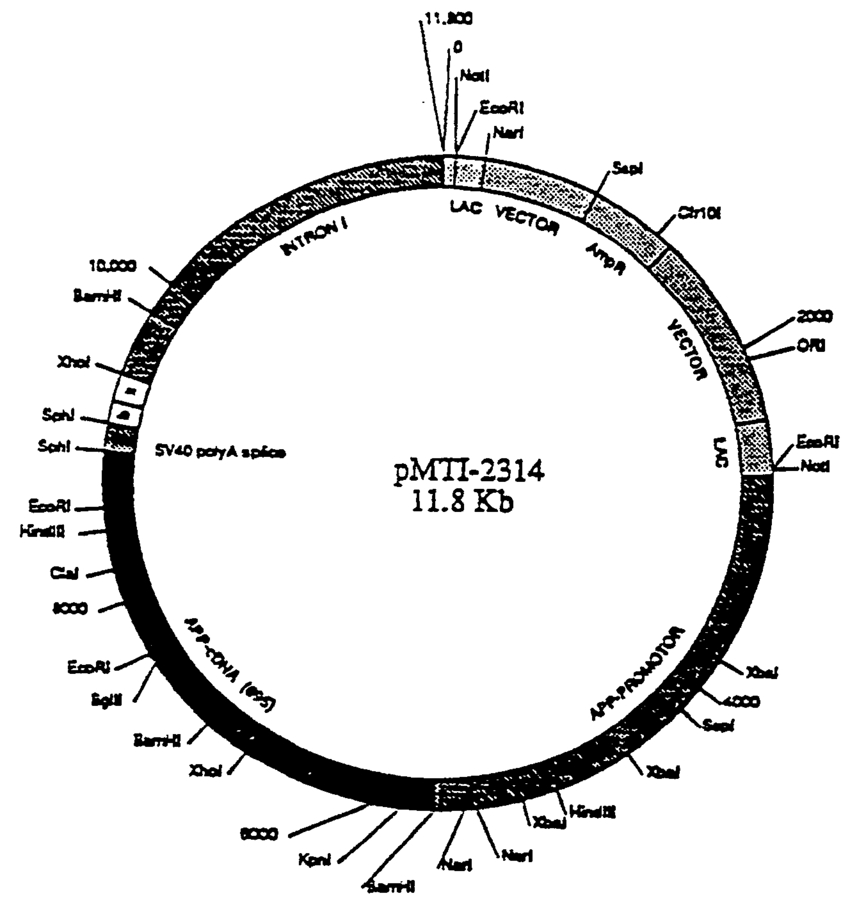
11,800
0
NotI
EcoRI
NarI
SspI
Ctr10I
2000
ORI
EcoRI
NotI
LAC
VECTOR
AmpR
VECTOR
LAC
INTRON I
10,000
BamHI
XhoI
SphI
SphI
SV40 polyA splice
pMTI-2314
11.8 Kb
EcoRI
HindIII
ClaI
8000
EcoRI
BglII
BamHI
XhoI
APP-CDNA (695)
APP-PROMOTOR
XbaI
4000
SspI
XbaI
HindIII
XbaI
NarI
NarI
BamHI
KpnI
6000

FIG. 8b

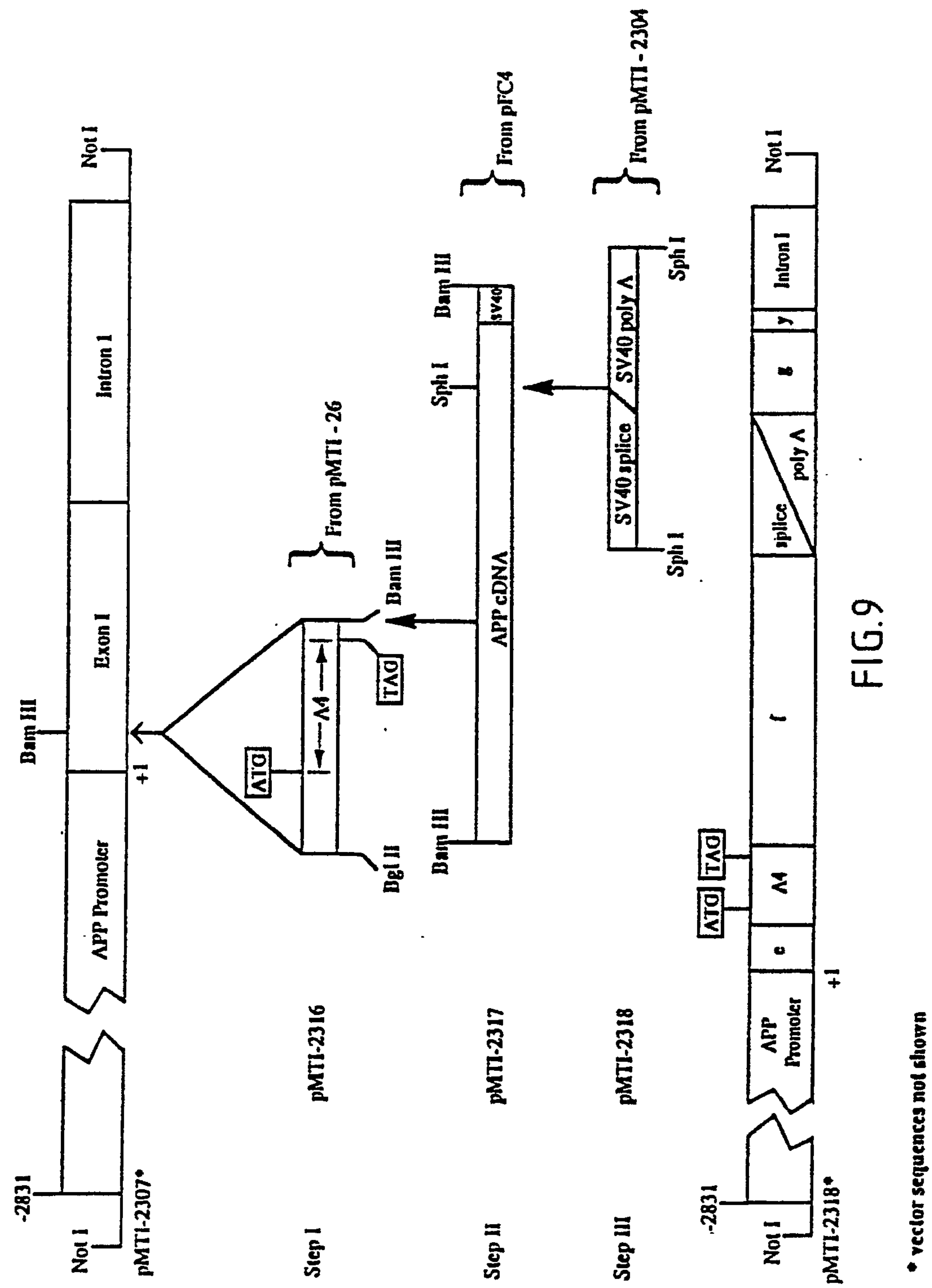
Not I
-2831
pMTI-2307*
APP Promoter
+1
Bam III
Exon I
Intron 1
Not I
Step I
pMTI-2316
Bgl II
ATG
A4
TAG
Bam III
From pMTI - 26
Step II
pMTI-2317
Bam III
APP cDNA
Sph I
sv40
Bam III
From pFC4
Step III
pMTI-2318
Sph I
SV40 splice
SV40 poly A
Sph I
From pMTI - 2304
Not I
-2831
pMTI-2318*
APP Promoter
+1
e
ATG
TAG
A4
f
splice
poly A
g
y
Intron I
Not I
* vector sequences not shown

FIG.9

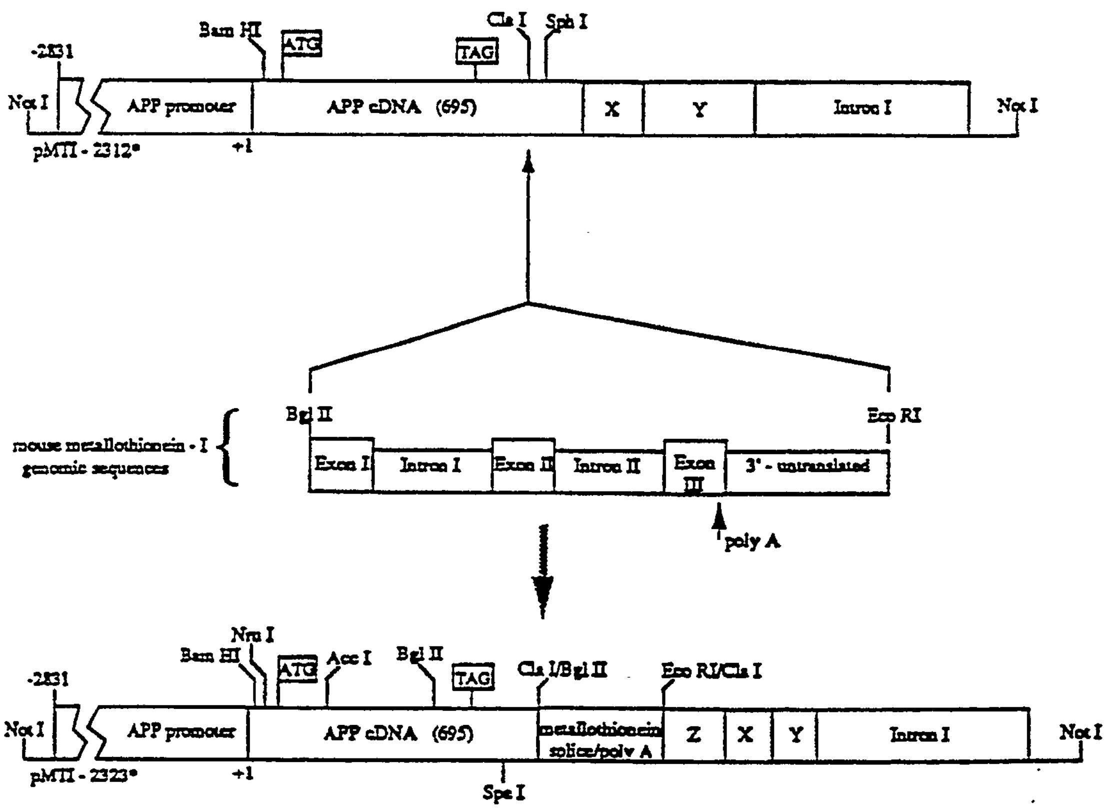


Alternate Forms:

pMTI - 2331: exchange of AccI - BglII fragment from pMTI - 3521 to pMTI - 2323.

pMTI - 2332: exchange of AccI - BglII fragment from pMTI - 3524 to pMTI - 2323.

pMTI - 2324: exchange of AccI - BglII fragment from pMTI - 35 to pMTI - 2323.

pMTI - 2326: exchange of BglII - SpeI fragment from pMTI - 2322 to pMTI - 2323.

*vector sequences not shown

FIG.10a

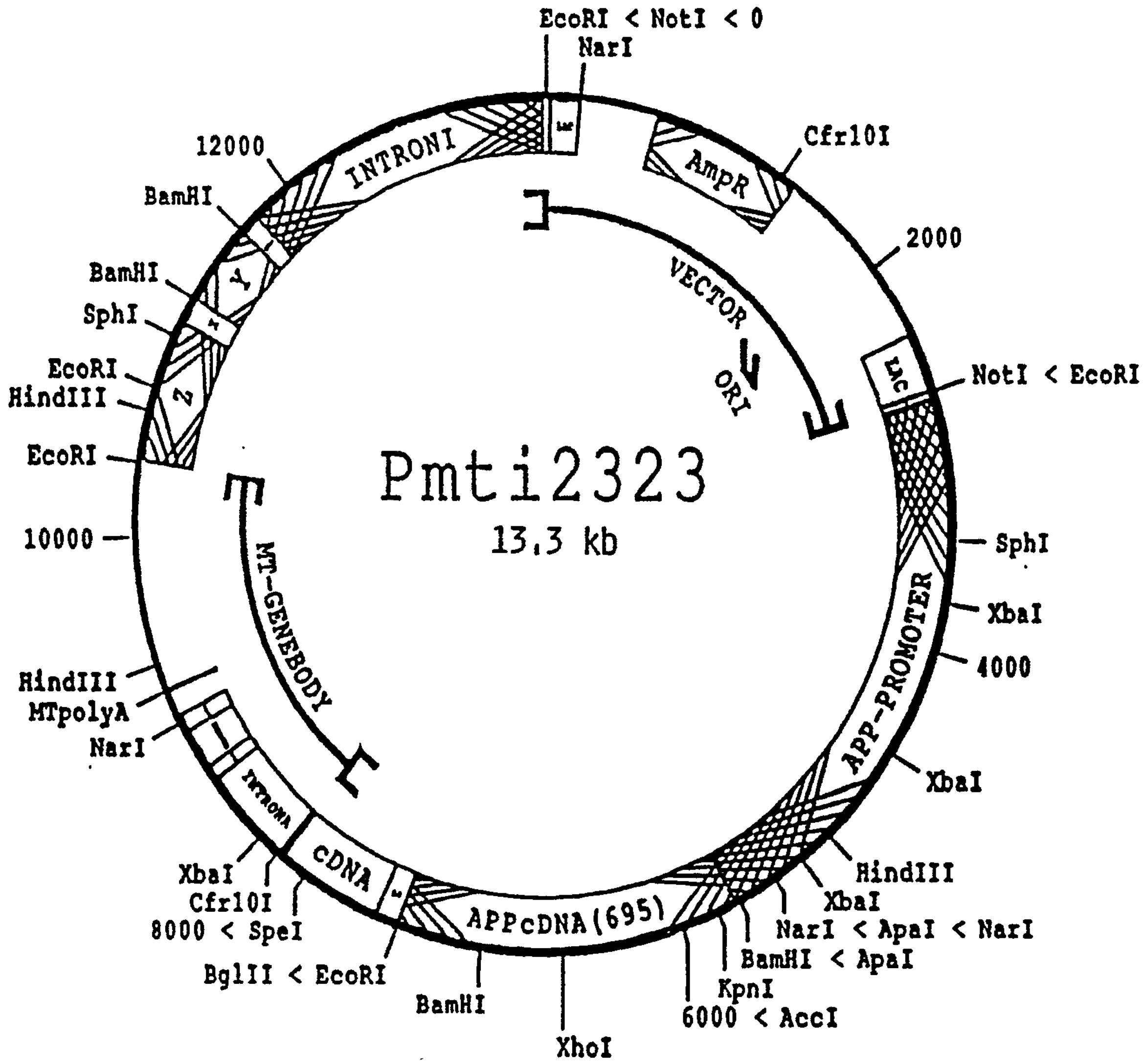
EcoRI < NotI < 0
NarI
Cfr10I
AmpR
2000
VECTOR
ORI
LAC
NotI < EcoRI
SphI
XbaI
4000
APP-PROMOTER
XbaI
HindIII
XbaI
NarI < ApaI < NarI
BamHI < ApaI
KpnI
6000 < AccI
XhoI
APPcDNA(695)
BamHI
BglII < EcoRI
cDNA
INTRONA
XbaI
Cfr10I
8000 < SpeI
NarI
MTpolyA
HindIII
MT-GENEBODY
10000
EcoRI
HindIII
EcoRI
SphI
BamHI
BamHI
12000
INTRONI
Pmti2323
13,3 kb

FIG.10b

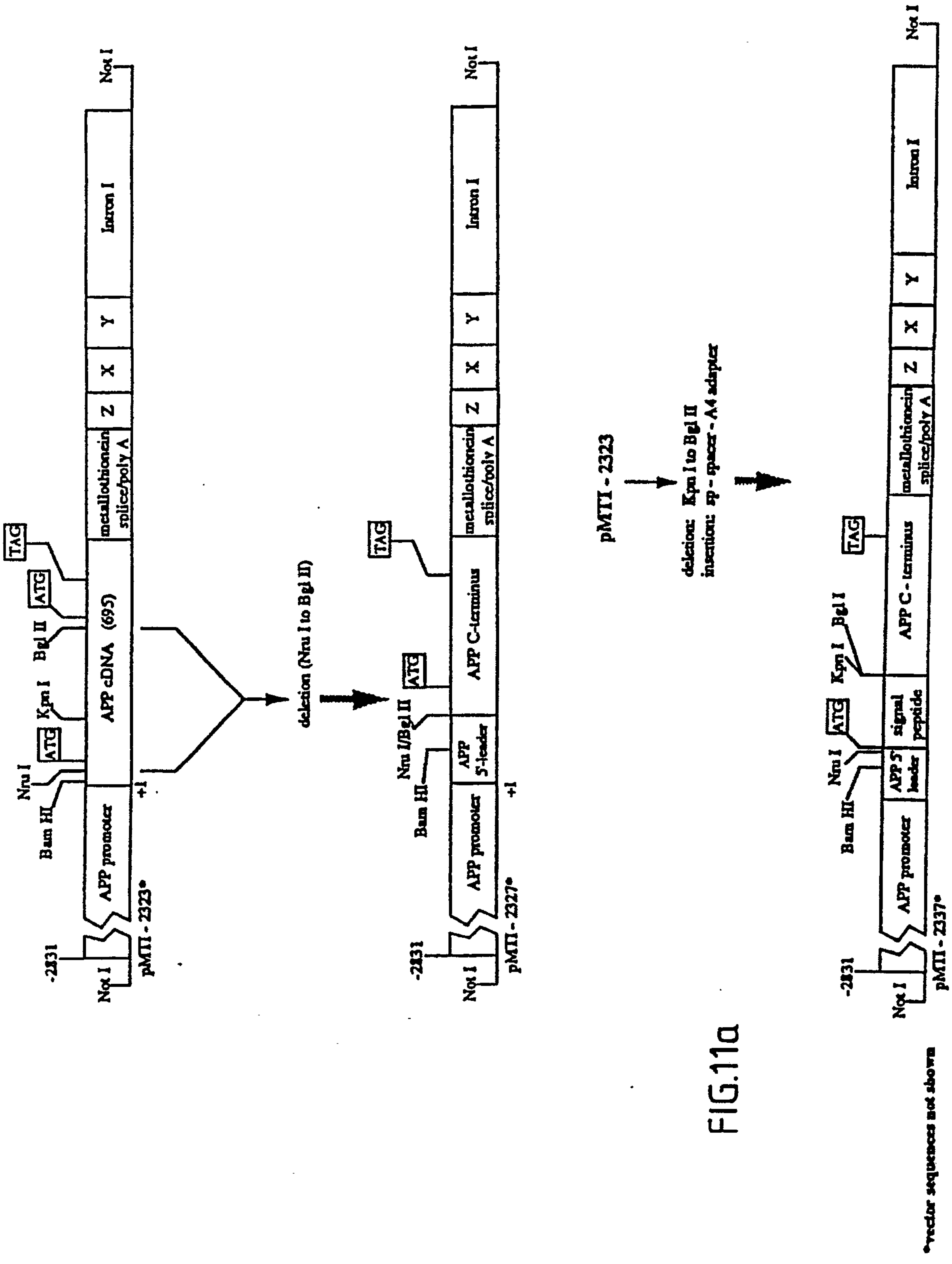

Not I
-2831
pMTI - 2323*
Bam HI
Nru I
ATG
Kpn I
Bgl II
ATG
TAG
APP promoter
+1
APP cDNA (695)
metallothionein splice/poly A
Z
X
Y
Intron I
Not I
deletion (Nru I to Bgl II)
Not I
-2831
pMTI - 2327*
Bam HI
Nru I/Bgl II
APP 5'-leader
+1
ATG
APP C-terminus
TAG
metallothionein splice/poly A
Z
X
Y
Intron I
Not I
pMTI - 2323
deletion: Kpn I to Bgl II
insertion: sp - spacer - A4 adapter
Not I
-2831
pMTI - 2337*
Bam HI
Nru I
ATG
Kpn I
Bgl I
APP promoter
APP 5' leader
signal peptide
APP C - terminus
TAG
metallothionein splice/poly A
Z
X
Y
Intron I
Not I
*vector sequences not shown


FIG.11a

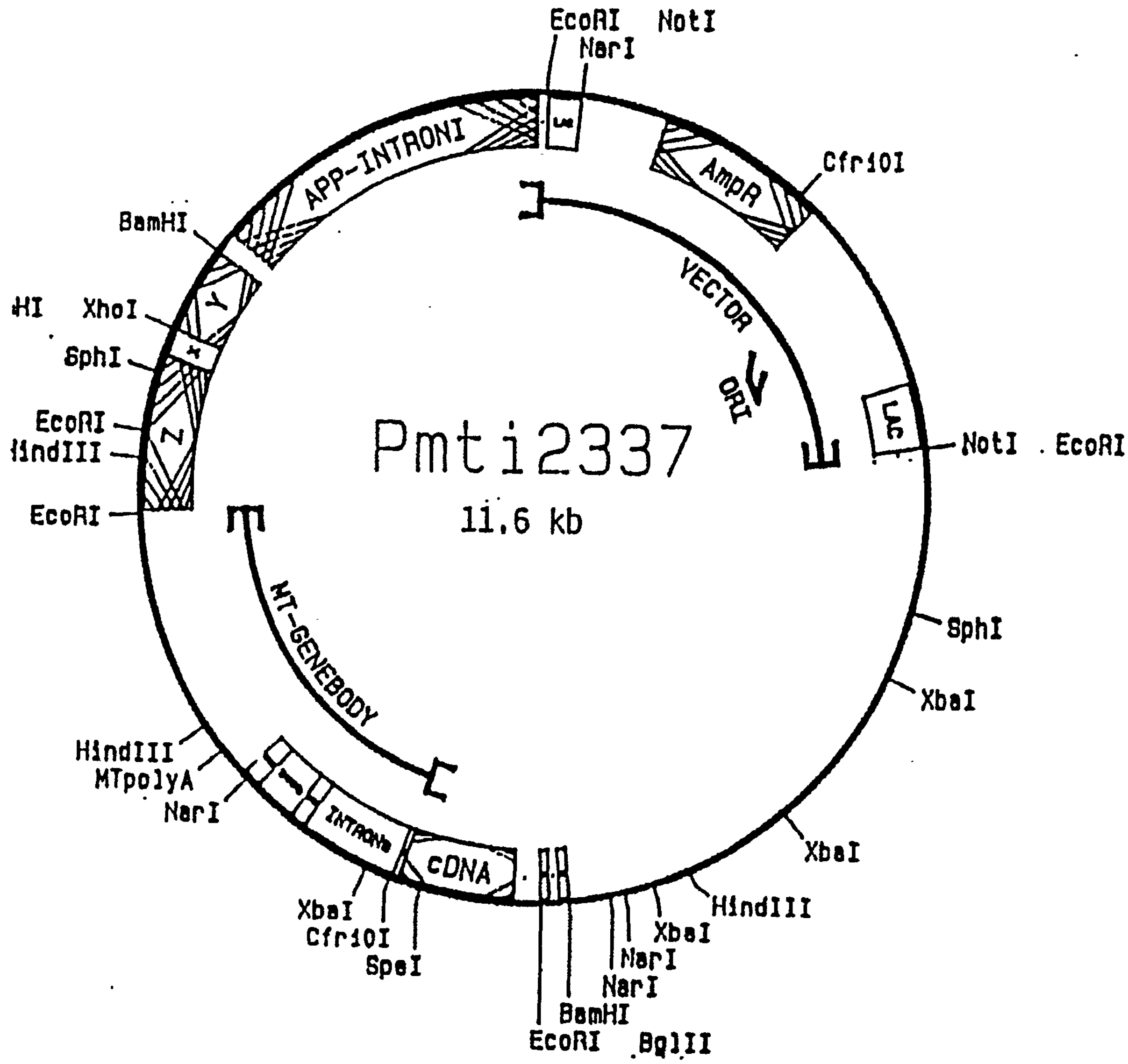
EcoRI
NotI
NarI
APP-INTRONI
AmpR
CfrIOI
BamHI
VECTOR
ORI
XhoI
SphI
EcoRI
HindIII
EcoRI
NotI
EcoRI
Pmti2337
11,6 kb
MT-GENEBODY
SphI
XbaI
HindIII
MTpolyA
NarI
INTRONb
cDNA
XbaI
XbaI
CfrIOI
SpeI
HindIII
XbaI
NarI
NarI
BamHI
EcoRI
BglII

FIG.11b

**A**
**sp - spacer - A4:**

```
KpnI            BglII

 5'- ggacggagga -3'              10mer

3'-catgcctgcctcctctag -5'        18mer
```

**B**
**MC-100:**
**DNA/Amino Acid**
**Sequences**

```
       10                   30                   50
AGTTTCCTCGGCAGCGGTAGGCGAGAGCACGCGGAGGAGCGTGCGCGGGGGCCCCGGGAG

       70                   90                  110
ACGGCGGCGGTGGCGGCGCGGGCAGAGCAAGGACGCGGCGGATCCCACTCGCACAGCAGC

      130                  150                  170
GCACTCGGTGCCCCGCGCAGGGTCGCGATGCTGCCCGGTTTGGCACTGCTCCTGCTGGCC
                          M  L  P  G  L  A  L  L  L  L  A

                  SP          sp-spacer-A4                A4
      190                                       230
GCCTGGACGGCTCGGGCGCTGGAGGTACggacggaggaGATCTCTGAAGTGAAGATGGAT
A  W  T  A  R  A  L  E  V  R  T  E  E  I  S  E  V  K  M  D

      250                  270                  290
GCAGAATTCCGACATGACTCAGGATATGAAGTTCATCATCAAAAATTGGTGTTCTTTGCA
A  E  F  R  H  D  S  G  Y  E  V  H  H  Q  K  L  V  F  F  A

      310                  330                  350
GAAGATGTGGGTTCAAACAAAGGTGCAATCATTGGACTCATGGTGGGCGGTGTTGTCATA
E  D  V  G  S  N  K  G  A  I  I  G  L  M  V  G  G  V  V  I

      370                  390                  410
GCGACAGTGATCGTCATCACCTTGGTGATGCTGAAGAAGAAACAGTACACATCCATTCAT
A  T  V  I  V  I  T  L  V  M  L  K  K  K  Q  Y  T  S  I  H

      430                  450                  470
CATGGTGTGGTGGAGGTTGACGCCGCTGTGACCCCAGAGGAGCGCCACCTGTCCAAGATG
H  G  V  V  E  V  D  A  A  V  T  P  E  E  R  H  L  S  K  M

      490                  510                  530
CAGCAGAACGGCTACGAAAATCCAACCTACAAGTTCTTTGAGCAGATGCAGAACTAGACC
Q  Q  N  G  Y  E  N  P  T  Y  K  F  F  E  Q  M  Q  N  *

      550                             923                1572
CCCGCCACAGCAGC
                                     Cla I               Sph I
                               (end MTI-2337)        (MTI-2343)
```

FIG.12

**A.**
**sp - spacer - A4:**

```
KpnI          BglII
5'-ggacggagga-3'                 10mer
3'-catgcctgcctcctctag-5'         18mer
```

**B.**
**sp-A4:**
**DNA/Amino Acid Sequences**

```
          10                            30                            50
AGTTTCCTCGGCAGCGGTAGGCGAGAGCACGCGGAGGAGCGTGCGCGGGGGCCCCGGGAG

          70                            90                           110
ACGGCGGCGGTGGCGGCGCGGGCAGAGCAAGGACGCGGCGGATCCCACTCGCACAGCAGC

         130                           150                           150
GCACTCGGTGCCCCGCGCAGGGTCGCGATGCTGCCCGGTTTGGCACTGCTCCTGCTGGCC
                           M  L  P  G  L  A  L  L  L  L  A

                      SP                                           A4
         190                 sp-spacer-A4                230
GCCTGGACGGCTCGGGCGCTGGAGGTACggacggaggaGATCTCTGAAGTGAAGATGGAT
A  W  T  A  R  A  L  E  V  R  T  E  E  I  S  E  V  K  M  D

         250                           270                           290
GCAGAATTCCGACATGACTCAGGATATGAAGTTCATCATCAAAAATTGGTGTTCTTTGCA
A  E  F  R  H  D  S  G  Y  E  V  H  H  Q  K  L  V  F  F  A

         310                           330                           350
GAAGATGTGGGTTCAAACAAAGGTGCAATCATTGGACTCATGGTGGGCGGTGTTGTCTAG
E  D  V  G  S  N  K  G  A  I  I  G  L  M  V  G  G  V  V

CGACAGTGATCGTCATCACCTTGGTGATGCTGAAGAAGAAACAGTACACATCCATTCATC
```

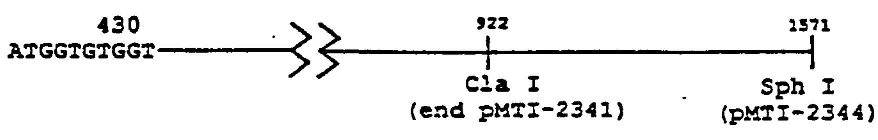


FIG.13

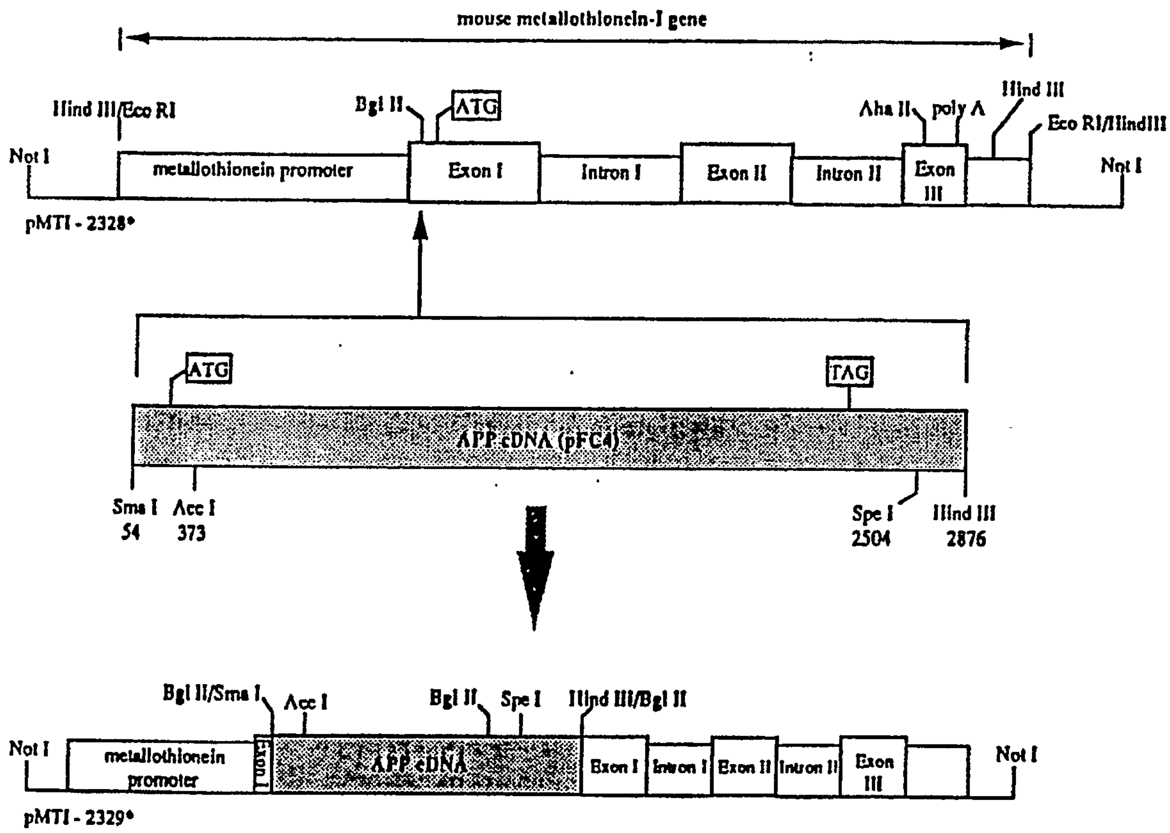


**Alternate Forms:**

pMTI - 2333: exchange of AccI - SpeI fragment from pMTI - 3521 to pMTI - 2329.

pMTI - 2334: exchange of AccI - SpeI fragment from pMTI - 3524 to pMTI - 2329.

pMTI - 2335: exchange of AccI - SpeI fragment from pMTI - 35 to pMTI - 2329.

pMTI - 2336: exchange of BglII - SpeI fragment from pMTI - 2322 to pMTI - 2329.

*vector sequences not shown

FIG. 14

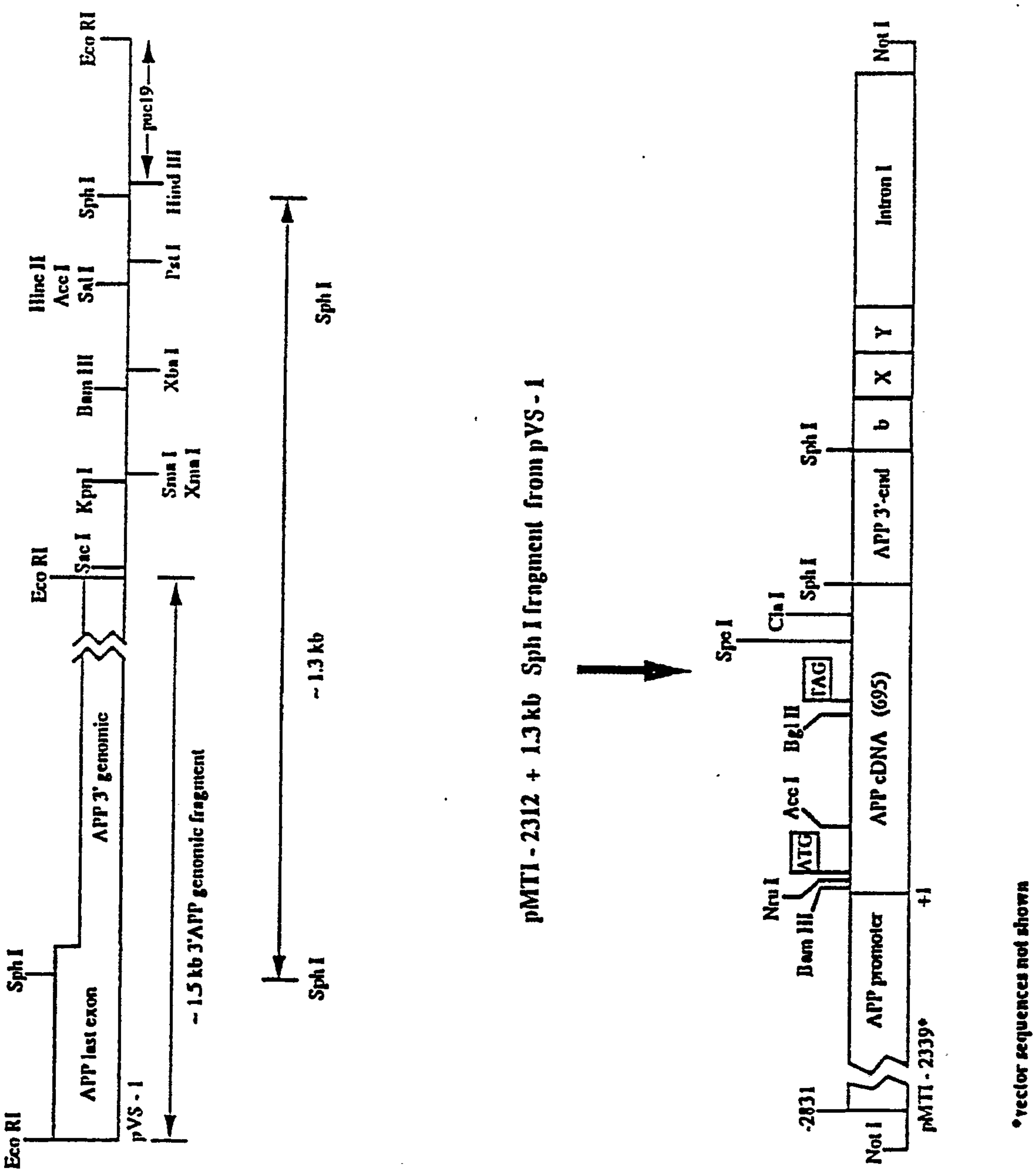
Eco RI
Sph I
APP last exon
APP 3' genomic
Eco RI
Sac I
Kpn I
Bam III
Hinc II
Acc I
Sal I
Sph I
Eco RI
pVS - 1
Sma I
Xmn I
Xba I
Pst I
Hind III
puc19
~ 1.5 kb 3'APP genomic fragment
Sph I
~ 1.3 kb
Sph I
pMTI - 2312 + 1.3 kb Sph I fragment from pVS - 1
Not I
-2831
Bam III
Nru I
ATG
Acc I
Bgl II
TAG
Spe I
Cla I
Sph I
Sph I
APP promoter
APP cDNA (695)
APP 3'-end
b
X
Y
Intron I
Not I
pMTI - 2339*
+1
*vector sequences not shown

FIG. 15a

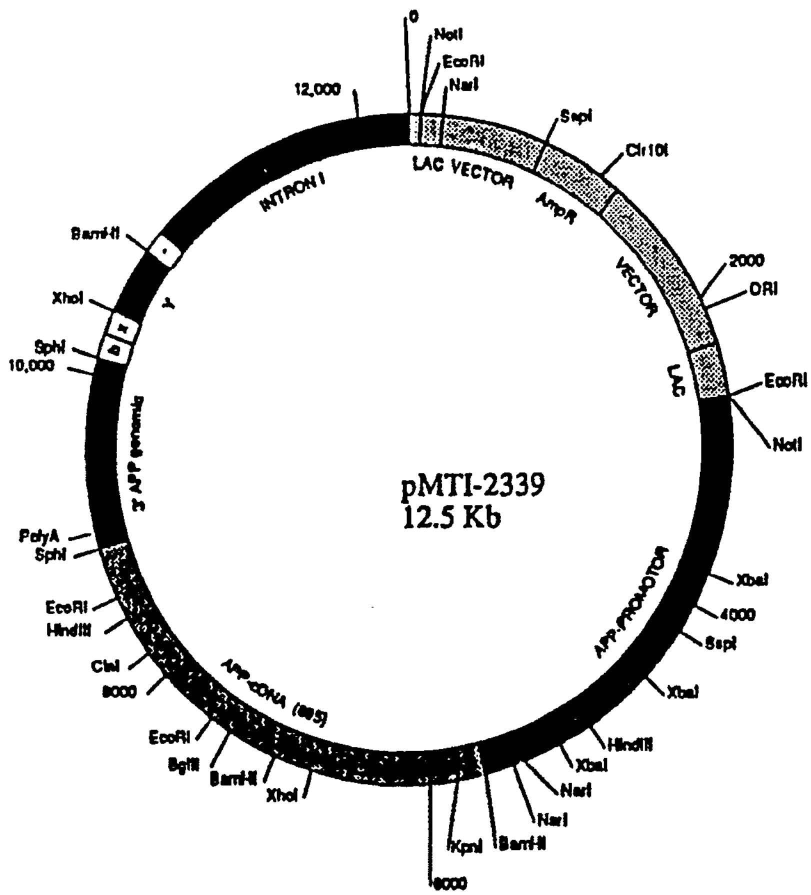
pMTI-2339
12.5 Kb
0
NotI
EcoRI
NarI
SspI
ClI101
LAC VECTOR
AmpR
VECTOR
2000
ORI
LAC
EcoRI
NotI
APP-PROMOTOR
XbaI
4000
SspI
XbaI
HindIII
XbaI
NarI
NarI
BamHI
KpnI
6000
XhoI
BamHI
BglII
EcoRI
8000
ClaI
HindIII
EcoRI
SphI
PolyA
APP-cDNA (695)
3' APP genomic
10,000
SphI
XhoI
BamHI
INTRON I
12,000

FIG. 15 b

3'-End of APP Gene:
DNA Sequence

```
      CATGCCTGG ACAAACCCTT CTTTTAAGAT GTGTCTTCAA TTTGTATAAA
  51  ATGGTGTTTT CATGTAAATA AATACATTCT TGGAGGAGCC ACATTGTGCT
 101  GGTGTGAATG ATTCCATAGT AACAATCTTG ACCATTTACT GACGTACAGA
 151  CCAGTGAGAA GTCTTCGCAT GTTGGGTACC CACACCTGTT GTGTCTTAAT
 201  TGCAAGTCTG AGTAGGAAGT TGGGGCCAAC ATGTGTCTCC CAGTGCTGGG
 251  AAAATATTTC ATAGACCTAA TTTACAGTCT TTACTTGATC TAAAACATTT
 301  TGCTGCCATA TTTTGGCCCT CAAGTTTGTC CCAAATGAGA GACAAAGGGA
 351  AAAGTTCCAG GGAAATAAAA ATTAAGACAG CTGATTATCT GTAAAGCATG
 401  GTTTCTCATC CTGAACGCTA CTAACATTTT GCAGGGAATA ATTCCTTGTT
 451  GAAGGGAGTT GTCCTGACCA GTGTAGGATA TTTATTTATT TTATTTATGT
 501  TTTTTGAGAC GGAGTCTCGC TCTGTCACCC AGGCTGGAGT GCAGTGGCAC
 551  AATCTCGGCT CACTGCAAGC TCCGCCTCCC GGGTTCACGC CATTCTCCTG
 601  CCTCAGCCTC CTGAATAGCT GGGACTCTAG GTGCCCGCCA CCACGCCCGG
 651  CTAATTTTTT GTATTTTTAG TAGAGACGGG GTTTCACCGT GTTAGCCAGG
 701  ACAGTCTTGG TCTCCTGACC TCGTGATCTG CCTGCCTCGG CCTCCCAAAG
 751  TGCTGAGATT ACAGGCGTGC AAGCCGCGCC CAGCCAGTGC TCTCCTTTTA
 801  AAAGTAGCCC ATTGGCTGGG CGCAGTGGCT CACGCCTGTA ATCCCAGCAC
 851  TTTGGGAGGC TGAGGCGGGT GGATCACGAG GTCAGGAGAT CAAGAATATC
 901  CTGGCCAATA TGGTGAAACC CCATCTCTAC TAAAAATACA AAAAAAAAAA
 951  AAAAAAAAAA AAGGCCGGGC ATGGTGGCGG GCGCTTGTAG TCCCAGCTAC
1001  TCAGGAGGCT GAGGCAGGAG AATGGTGTGC ACCTGGGAGG CGGAGGTTGC
1051  AGTGAGCTGA GATCGCGCCA CTGCACTCCA GCCTGGGAGA CAGAGCGAGA
1101  CTCCGTCTCA ATAAATAAAT AAATAAATAA ATAAAAGGAG GGCCTGGCAC
1151  GAATGACATG CAGGGAAGGC AGTGAGCAGG TGGAGGTCCC TGTACTCGTT
1201  GTGGTGCCTT ATCTACCAGG CGGTTGAGTT GACGTCTTTG TGGACAGAAT
1251  TCGAGCTCGG TACCCGGGGA TCCTCTAGAG TCGACCTGCA GGCATG
```

FIG. 16

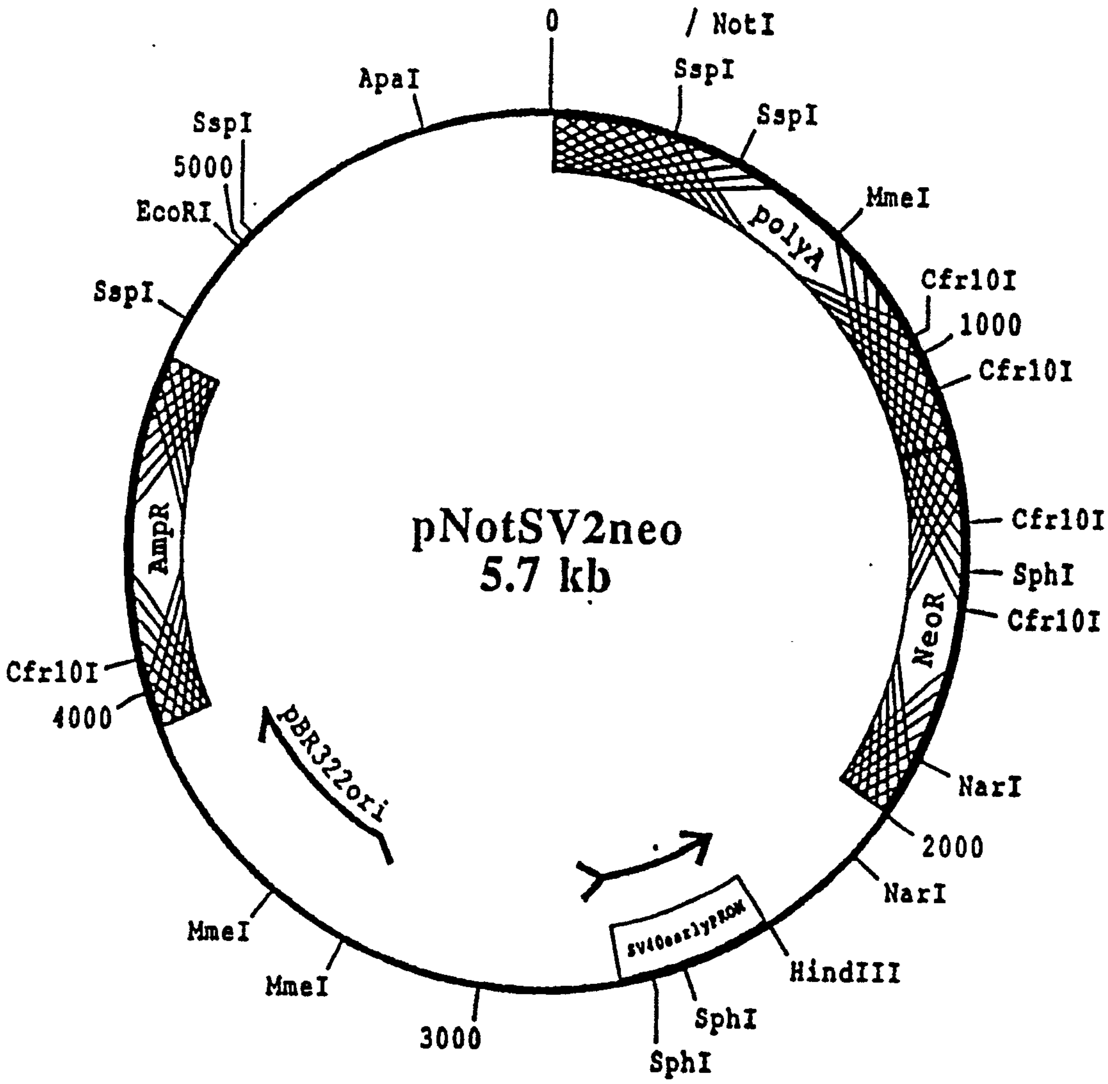
0
NotI
SspI
SspI
MmeI
polyA
Cfr10I
1000
Cfr10I
Cfr10I
SphI
Cfr10I
NeoR
NarI
2000
NarI
HindIII
SV40early PROM
SphI
SphI
3000
MmeI
MmeI
pBR322ori
4000
Cfr10I
AmpR
SspI
EcoRI
5000
SspI
ApaI
pNotSV2neo
5.7 kb

FIG.17

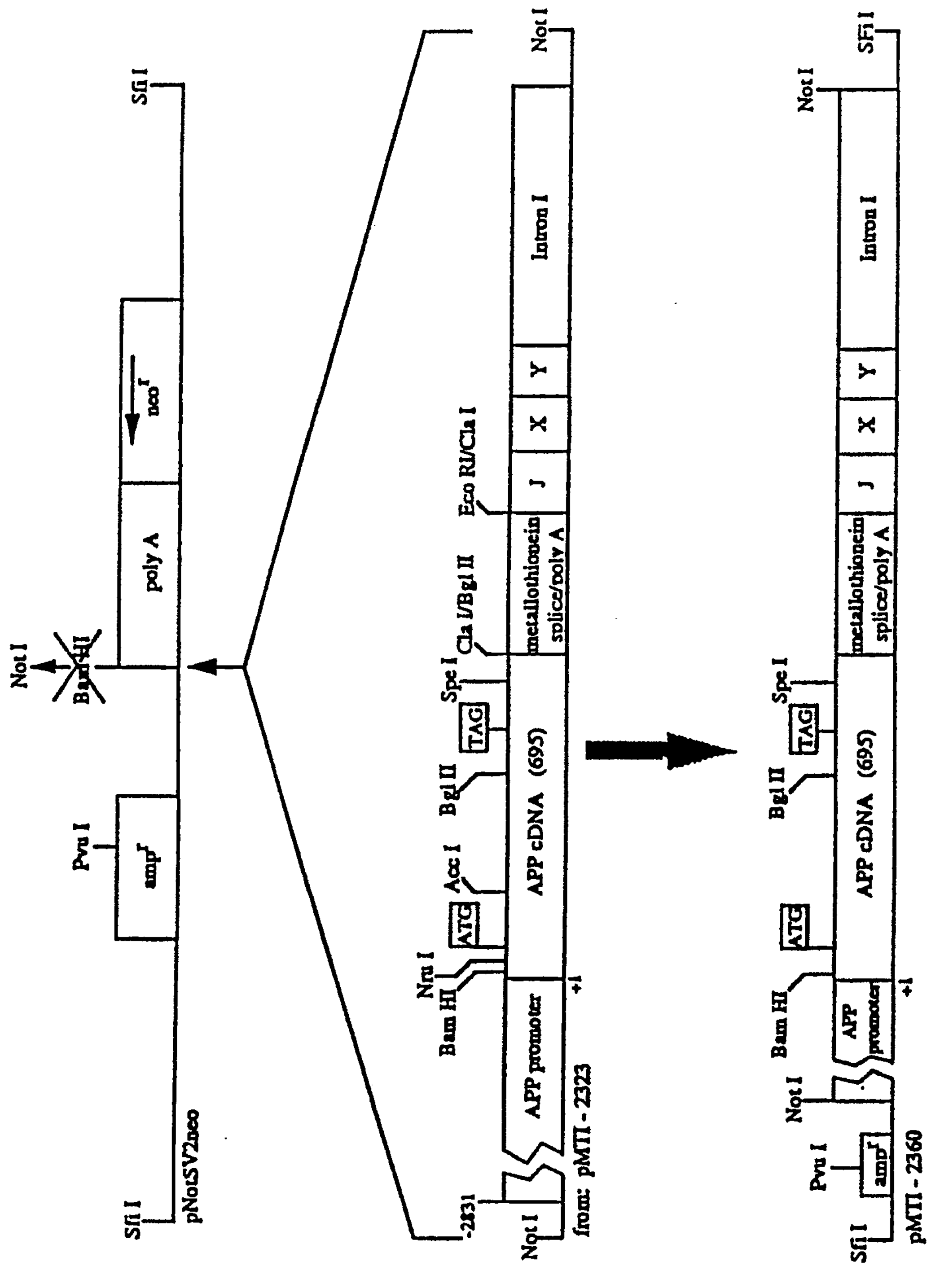
Sfi I
pNotSV2neo
Pvu I
amp^r
Not I
Bam HI
poly A
neo^r
Sfi I
Not I
-2331
from: pMTI - 2323
APP promoter
Bam HI
Nru I
ATG
Acc I
Bgl II
TAG
Spe I
Cla I/Bgl II
Eco RI/Cla I
APP cDNA (695)
+1
metallothionein splice/poly A
J
X
Y
Intron I
Not I
Sfi I
pMTI - 2360
Pvu I
amp^r
Not I
Bam HI
APP promoter
ATG
Bgl II
TAG
Spe I
+1
metallothionein splice/poly A
J
X
Y
Intron I
Not I
Sfi I

FIG.18a1

Other pNotSV2neo subclones:

| | |
|---|---|
| pMTI - 2361 : | Not I fragment of pMTI - 2326 in pNotSV2neo |
| pMTI - 2362 : | Not I fragment of pMTI - 2329 in pNotSV2neo |
| pMTI - 2363 : | Not I fragment of pMTI - 2331 in pNotSV2neo |
| pMTI - 2364 : | Not I fragment of pMTI - 2340 in pNotSV2neo |
| pMTI - 2365 : | Not I fragment of pMTI - 2341 in pNotSV2neo |
| pMTI - 2366 : | Not I fragment of pMTI - 2337 in pNotSV2neo |
| pMTI - 2367 : | Not I fragment of pMTI - 2402 in pNotSV2neo |
| pMTI - 2368 : | Not I fragment of pMTI - 2320 in pNotSV2neo |
| pMTI - 2369 : | Not I fragment of pMTI - 2339 in pNotSV2neo |

FIG.18a2

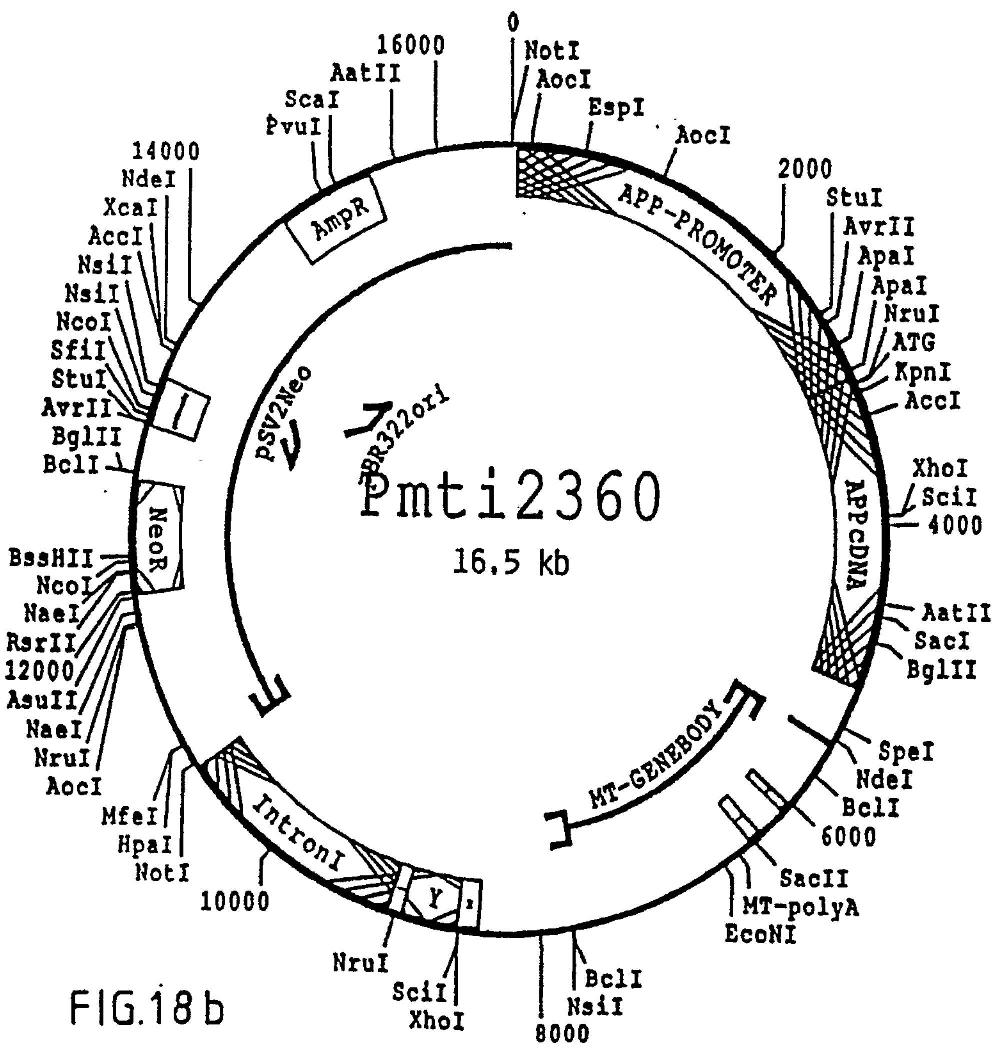


FIG.18b

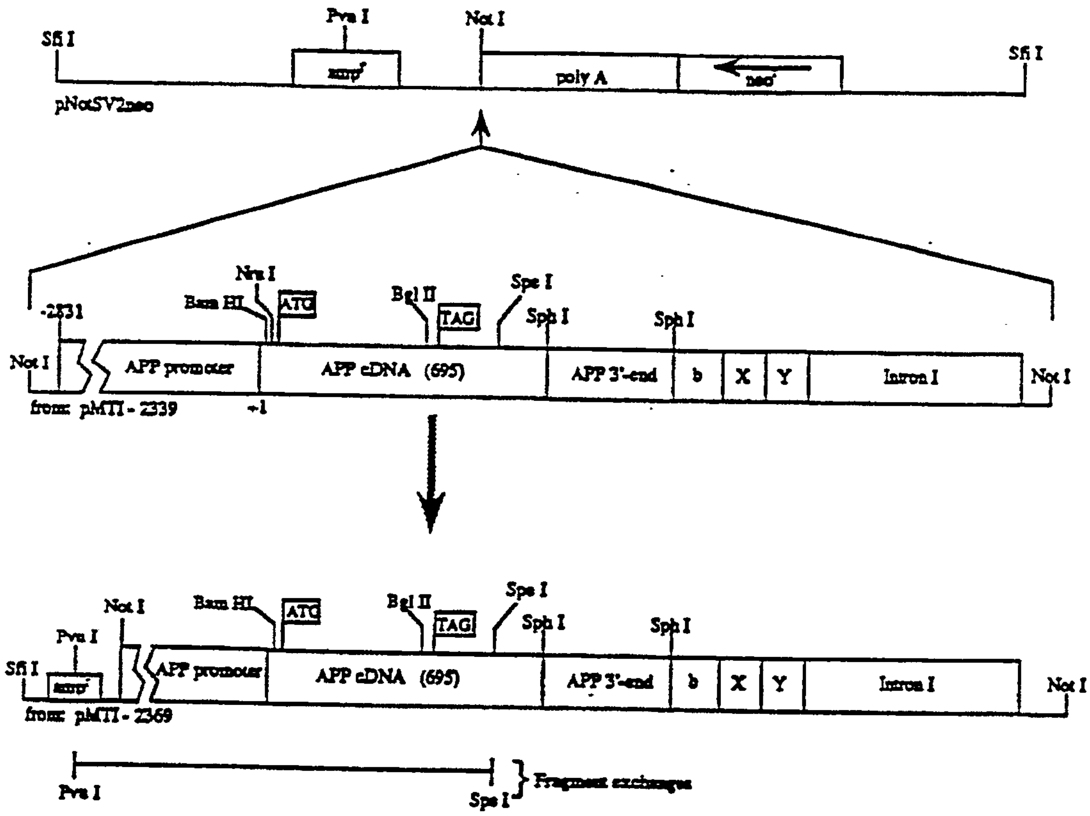


**Alternate Forms:**

pMTI - 2342: exchange of PvuI - SpeI fragment; from pMTI - 2363 to pMTI 2369

pMTI - 2343: exchange of PvuI - SpeI fragment; from pMTI - 2361 to pMTI - 2369

pMTI - 2344: exchange of PvuI-SpeI fragment; from pMTI - 2365 to pMTI - 2369

FIG. 19

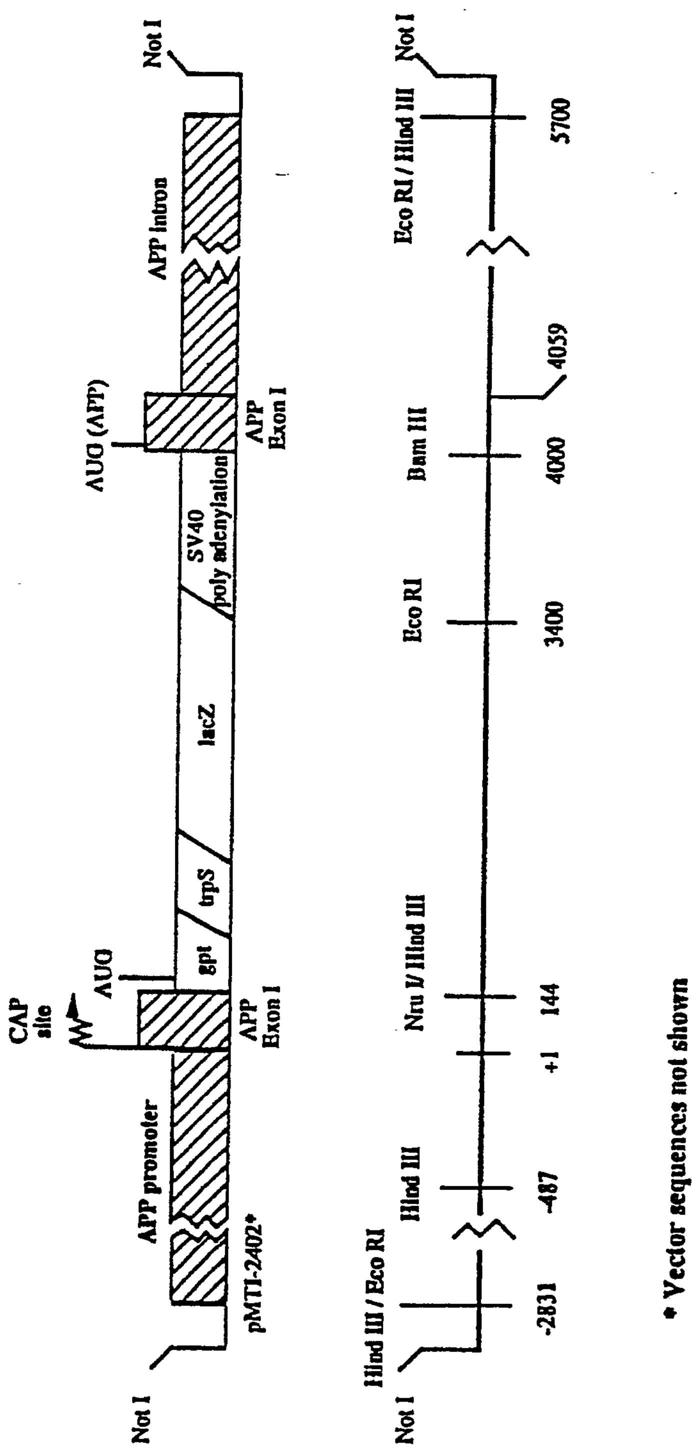
Not I
pMTI-2402*
APP promoter
CAP site
AUG
APP Exon I
gpt
trpS
lacZ
SV40 poly adenylation
AUG (APP)
APP Exon I
APP intron
Not I
Not I
Hind III / Eco RI
Hind III
Nru I/ Hind III
Eco RI
Bam HI
Eco RI / Hind III
Not I
-2831
-487
+1
144
3400
4000
4059
5700
* Vector sequences not shown

FIG.20

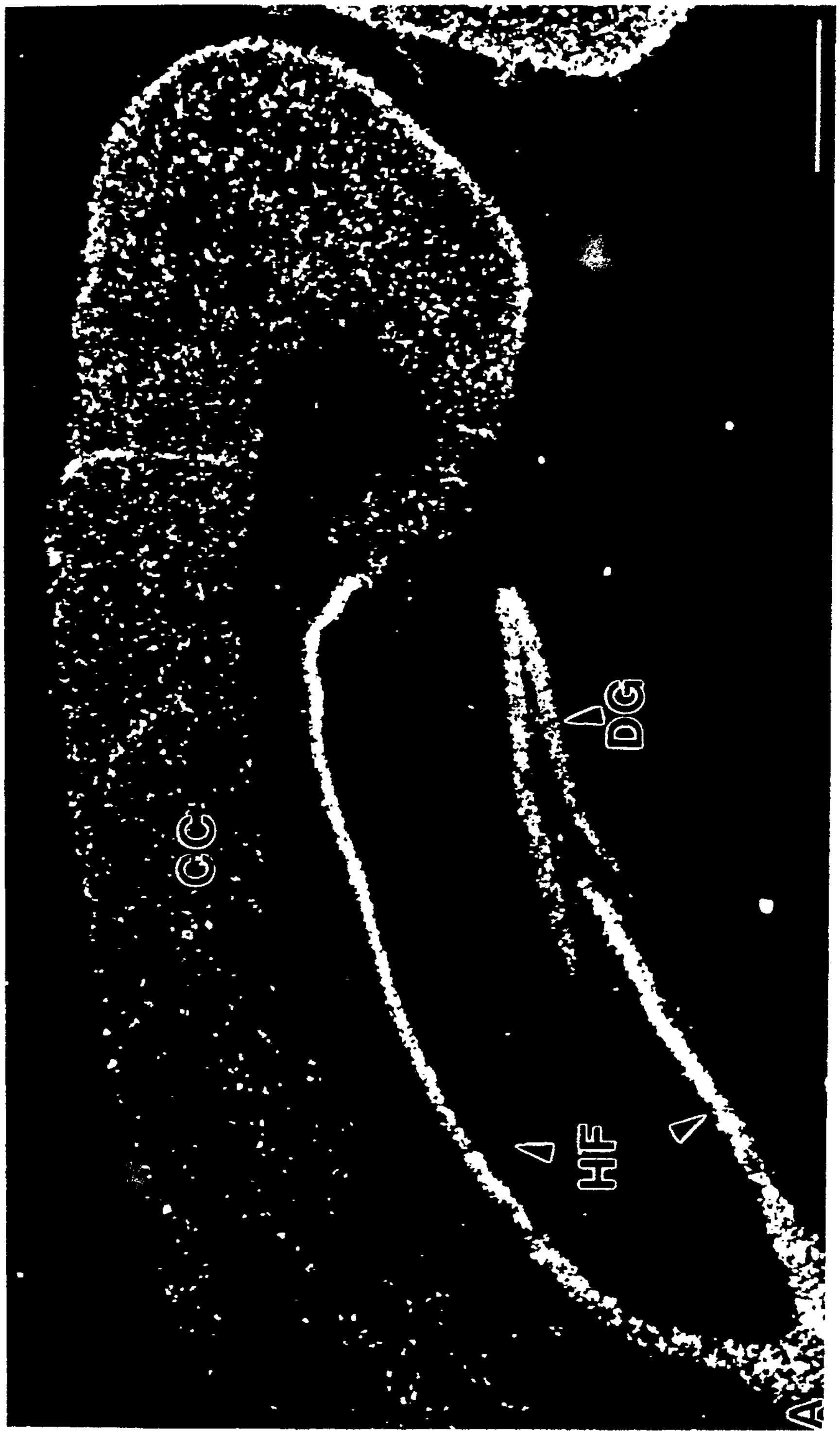


FIG. 21 a

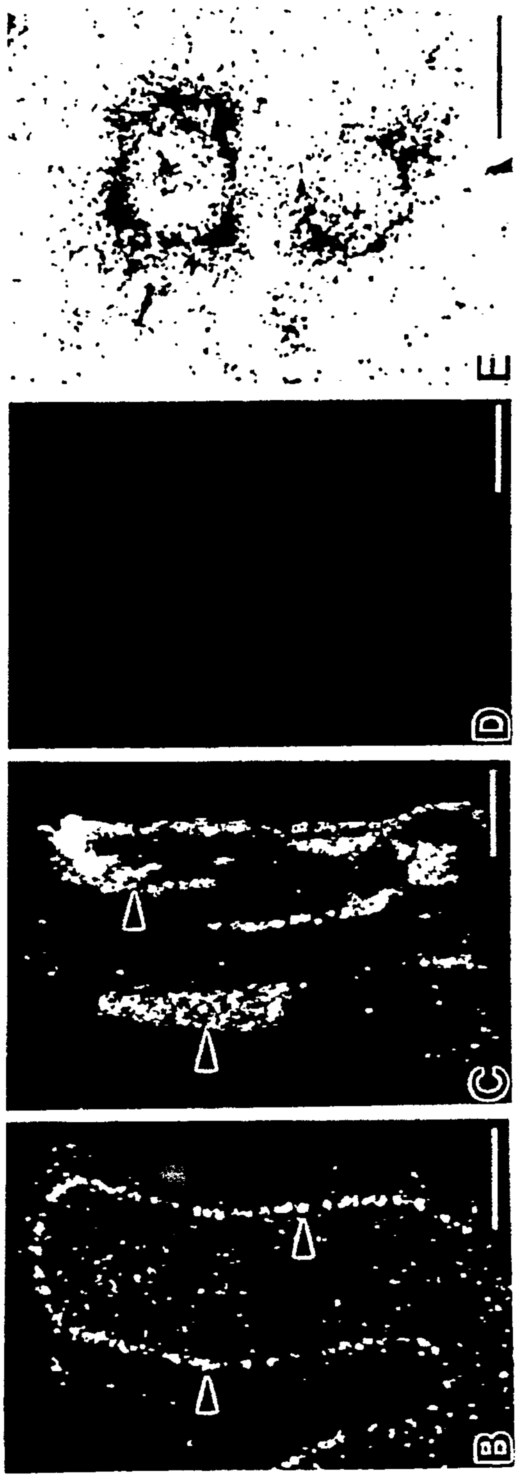


FIG. 21b

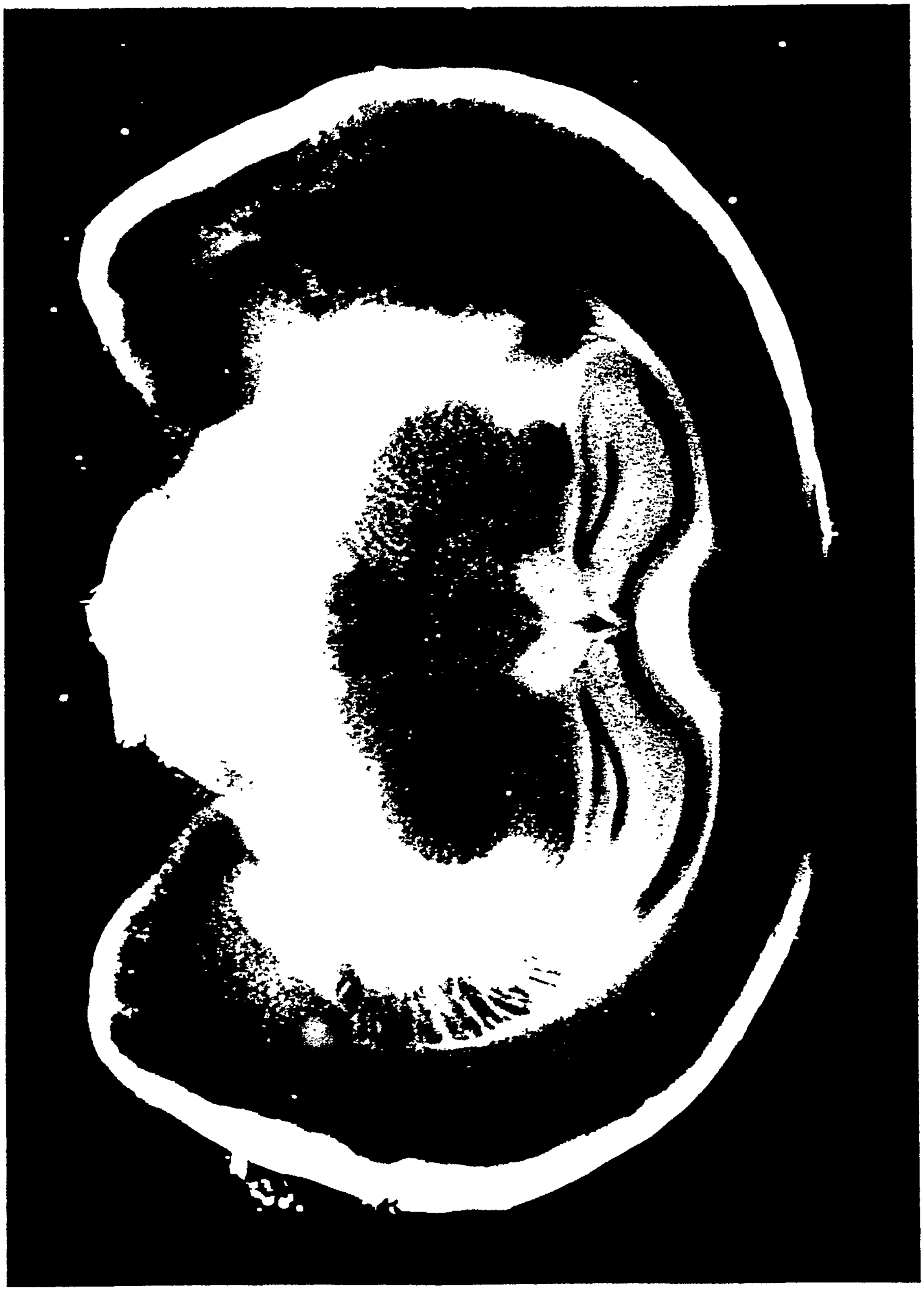

FIG. 22

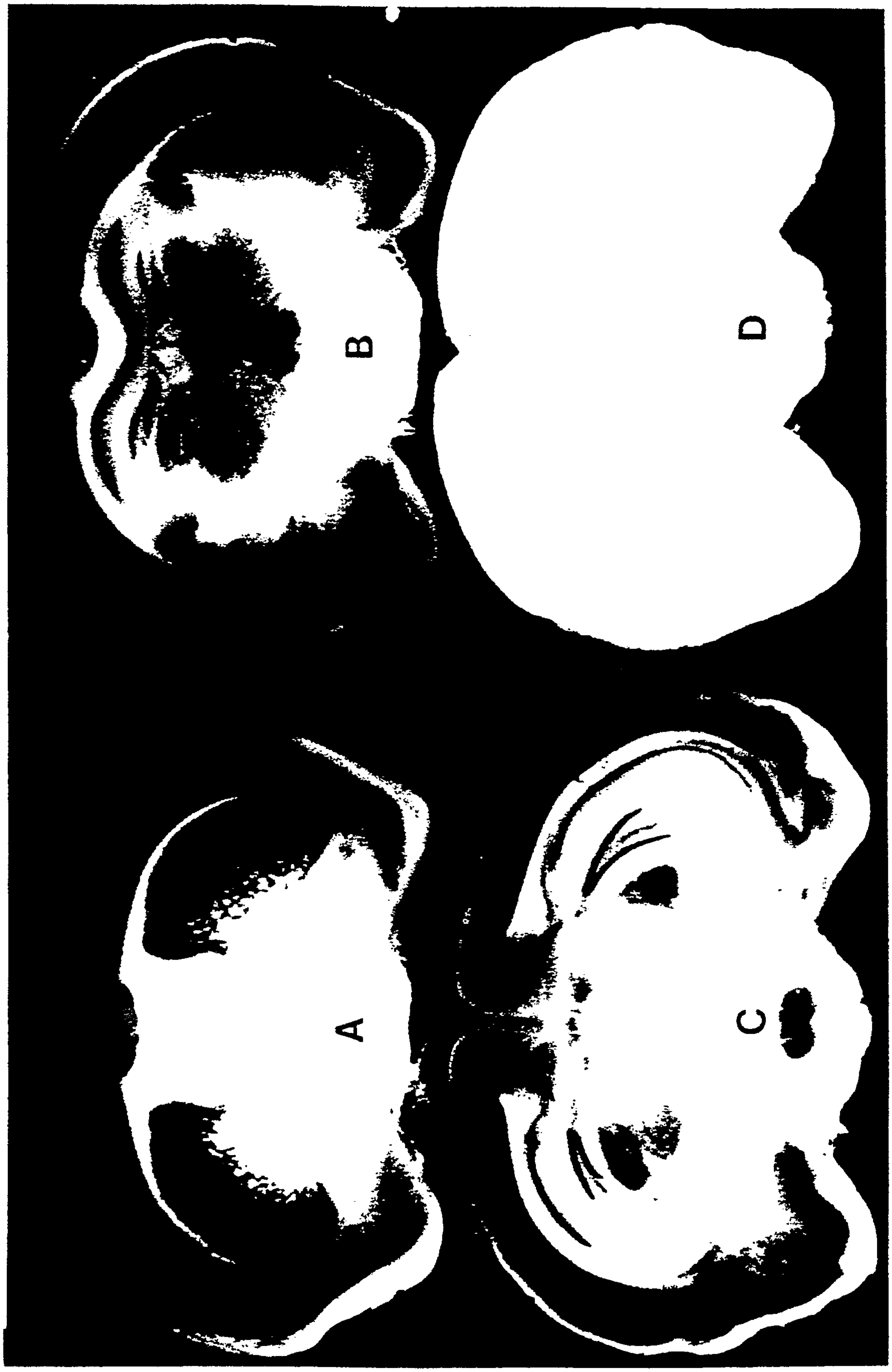


FIG. 23

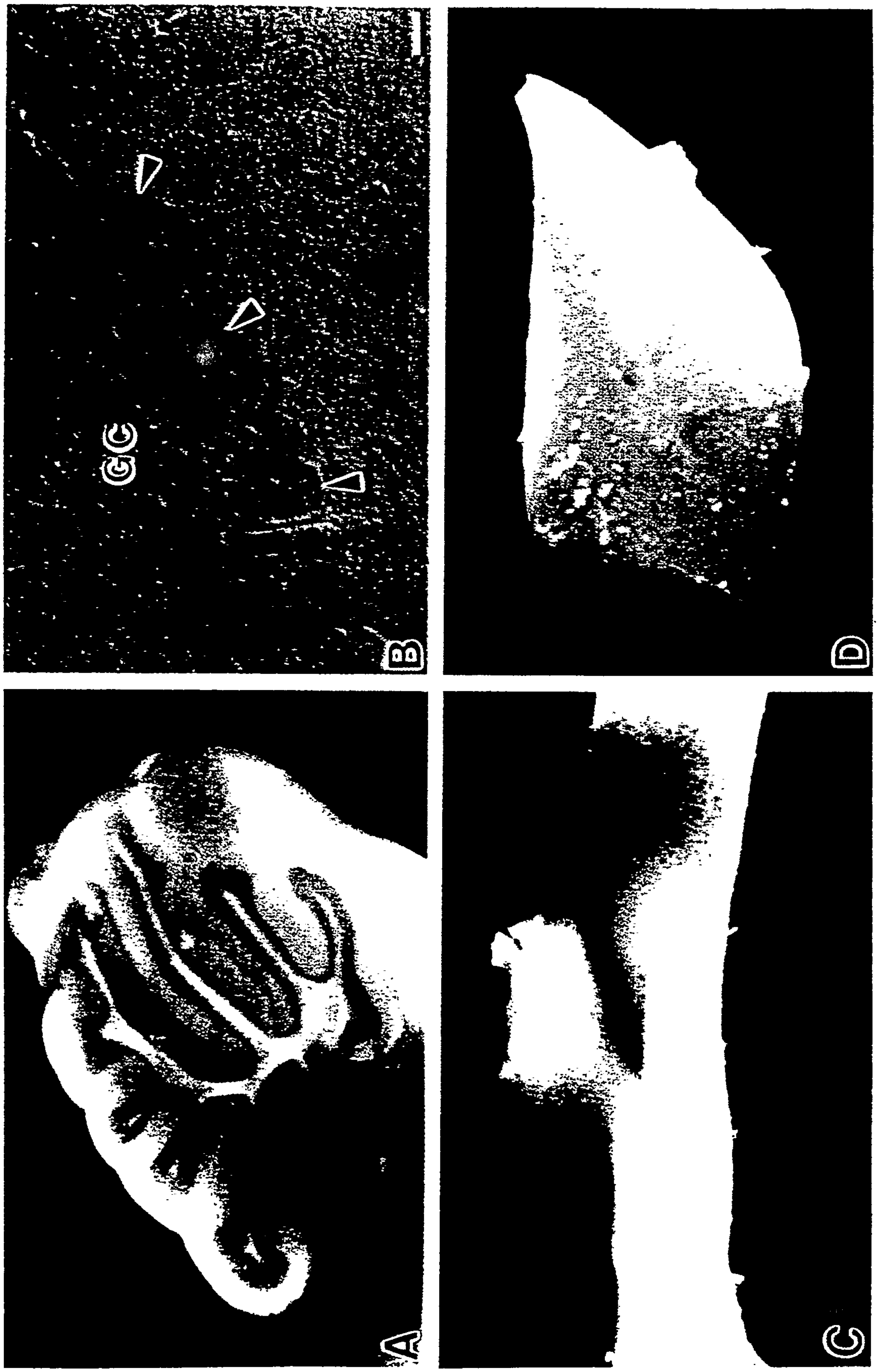


FIG. 24

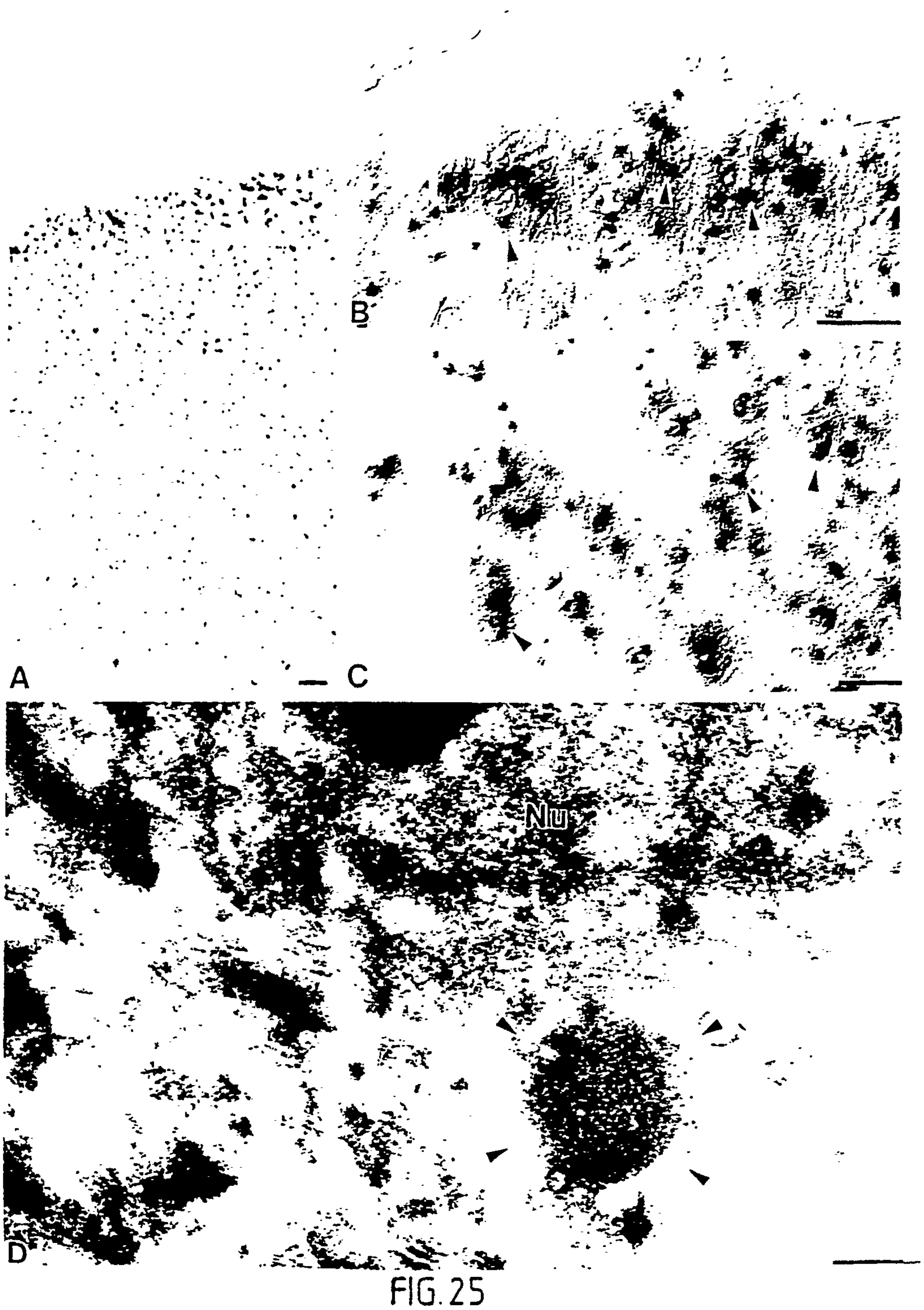


FIG.25

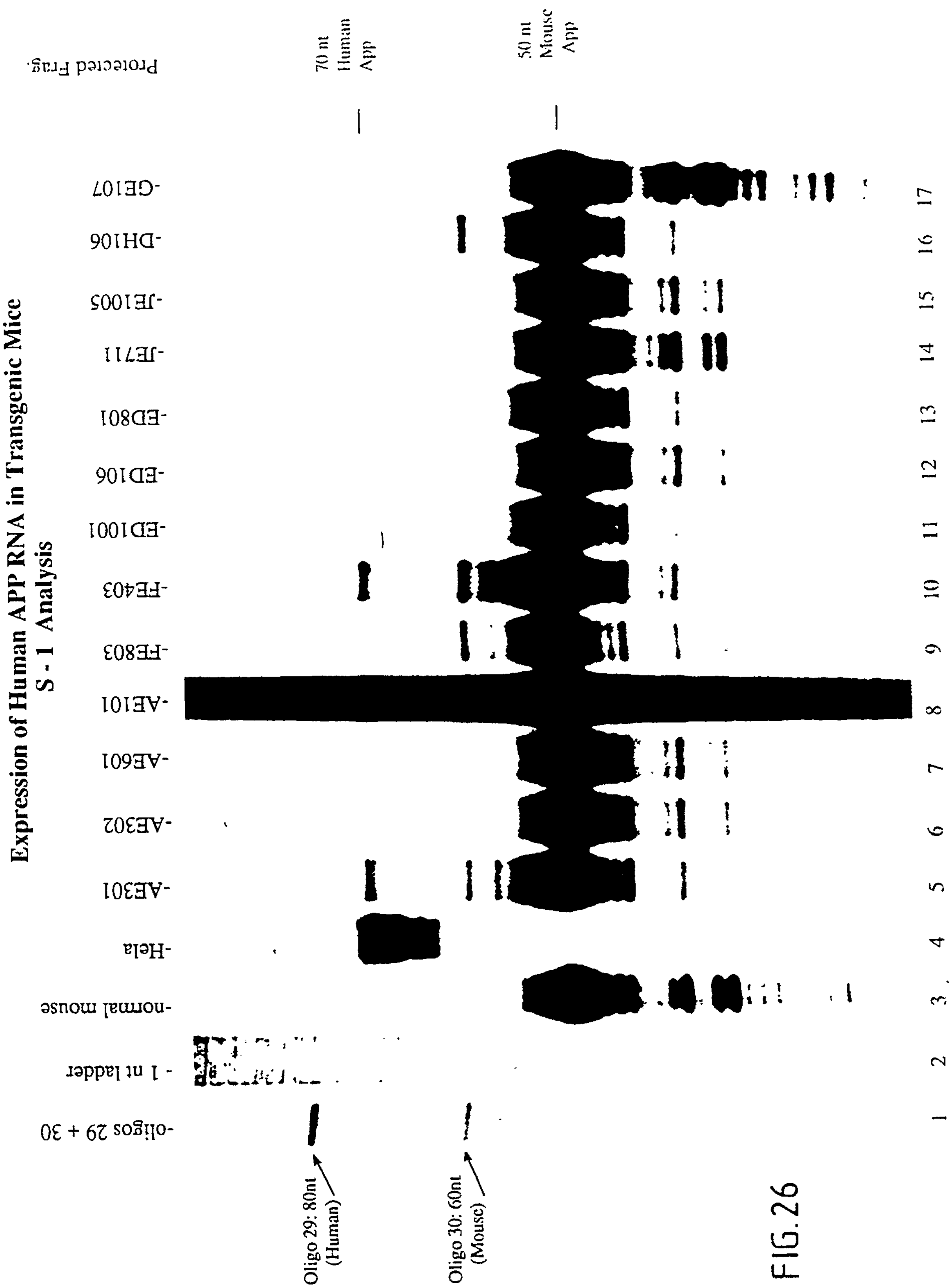
Expression of Human APP RNA in Transgenic Mice
S - 1 Analysis
-oligos 29 + 30
- 1 nt ladder
-normal mouse
-Hela
-AE301
-AE302
-AE601
-AE101
-FE803
-FE403
-ED1001
-ED106
-ED801
-JE711
-JE1005
-DH106
-GE107
Protected Frag.
70 nt Human App
50 nt Mouse App
1
2
3
4
5
6
7
8
9
10
11
12
13
14
15
16
17
Oligo 29: 80nt (Human)
Oligo 30: 60nt (Mouse)

FIG. 26

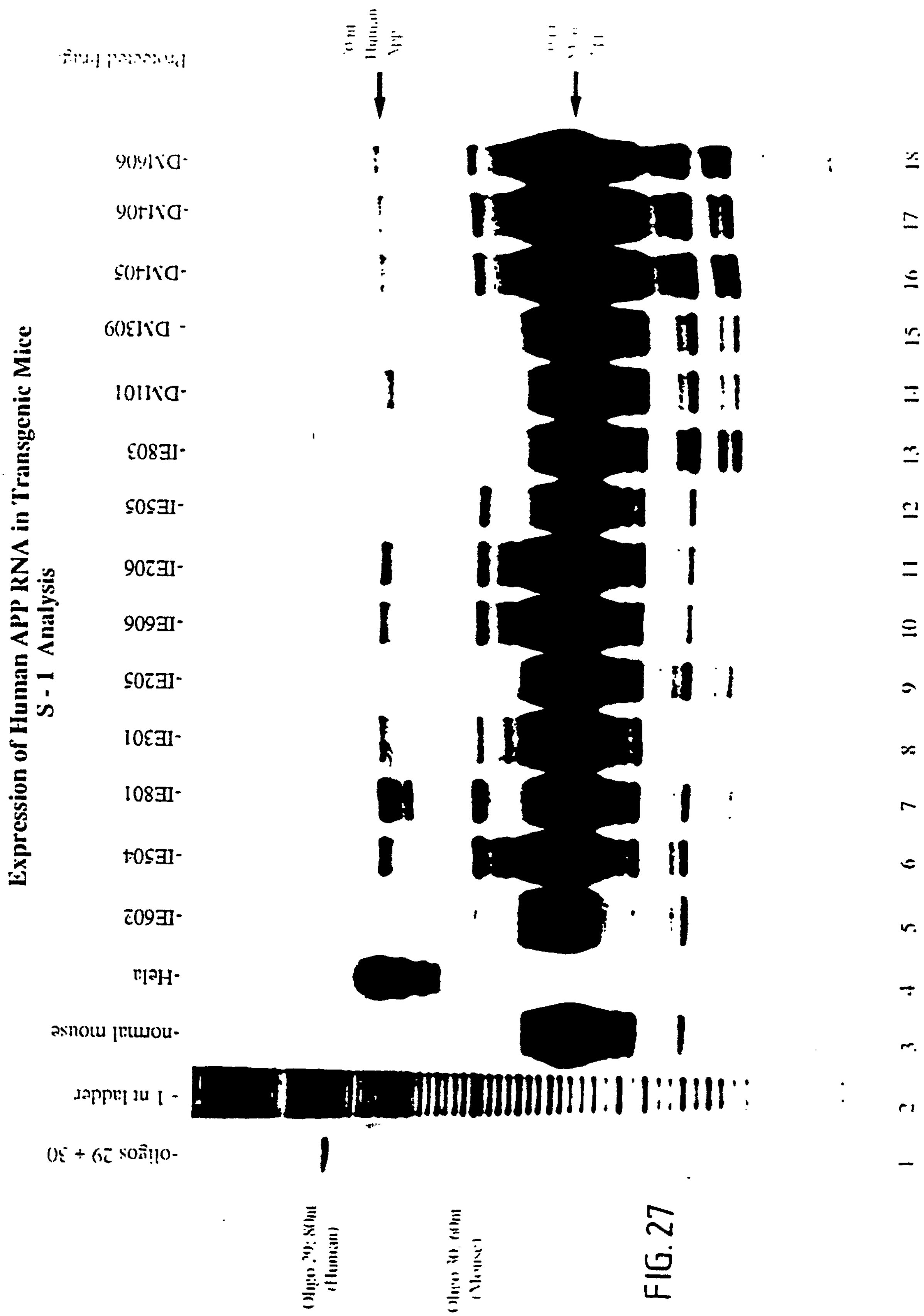
Expression of Human APP RNA in Transgenic Mice
S - 1 Analysis
Protected Frag.
-oligos 29 + 30
- 1 nt ladder
-normal mouse
-Hela
-IE602
-IE504
-IE801
-IE301
-IE205
-IE606
-IE206
-IE505
-IE803
-DM101
- DM309
-DM405
-DM406
-DM606
1
2
3
4
5
6
7
8
9
10
11
12
13
14
15
16
17
18
Oligo 29; 80nt (Human)
Oligo 30; 60nt (Mouse)

FIG. 27

APP 695, 751, and 770 Expression in Normal Mice & Transgenic Mice Carrying Human APP Minigenes
Western - blot : MAb 22C-II

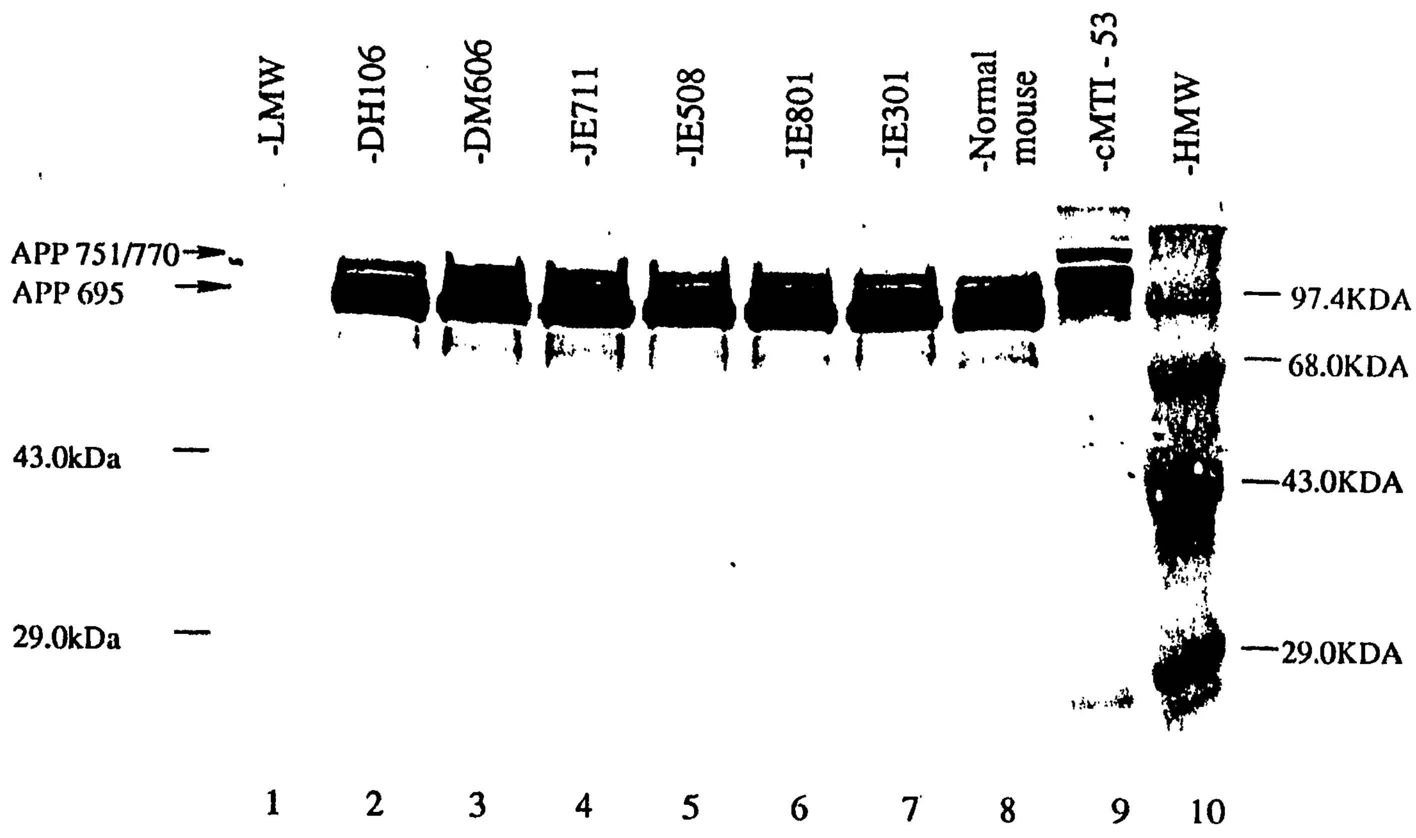


FIG. 28a

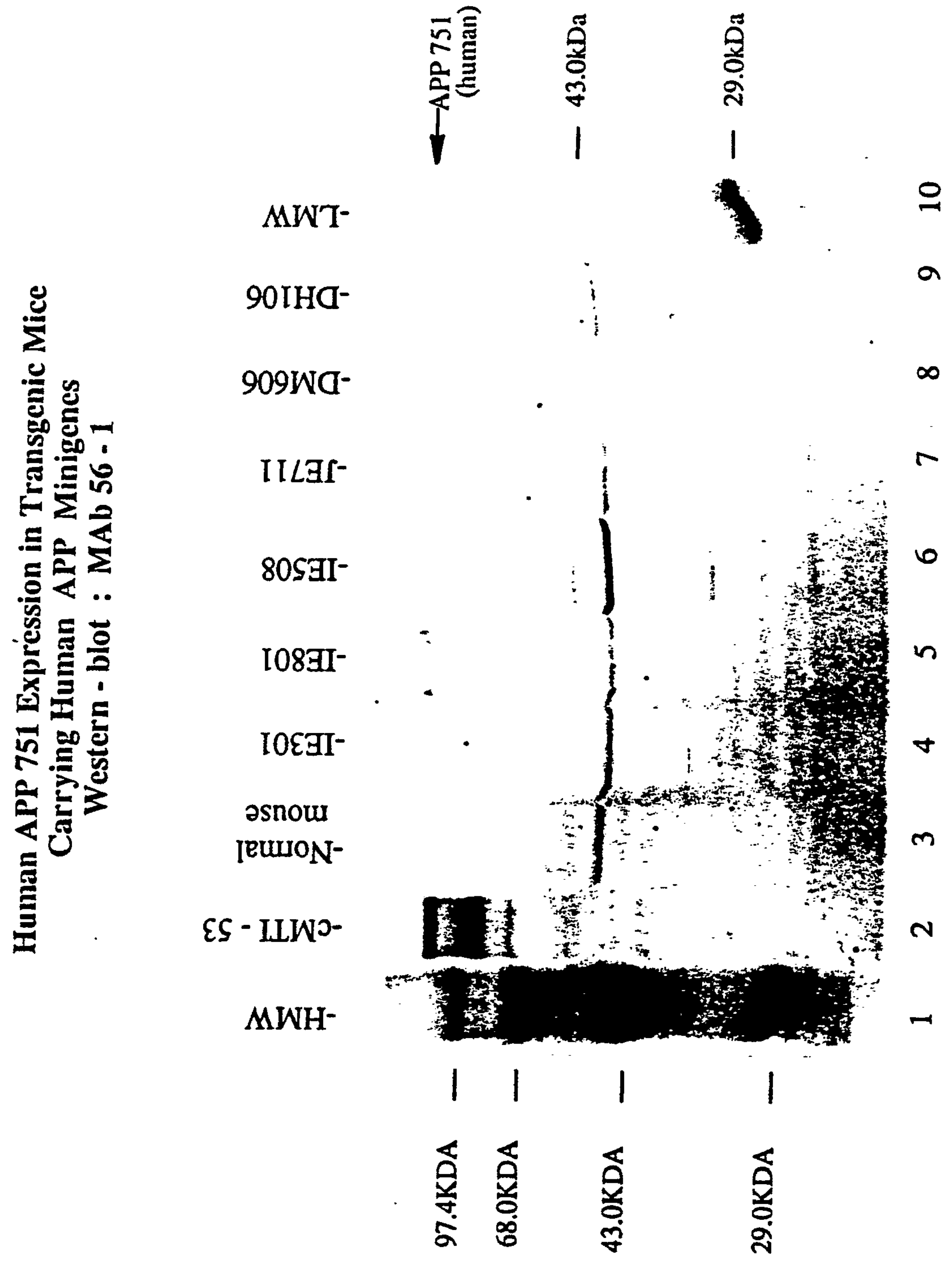
Human APP 751 Expression in Transgenic Mice
Carrying Human APP Minigenes
Western - blot : MAb 56 - 1
-HMW
-cMTI - 53
-Normal mouse
-IE301
-IE801
-IE508
-JE711
-DM606
-DH106
-LMW
APP 751 (human)
43.0kDa
29.0kDa
97.4KDA
68.0KDA
43.0KDA
29.0KDA
1
2
3
4
5
6
7
8
9
10

FIG.28b

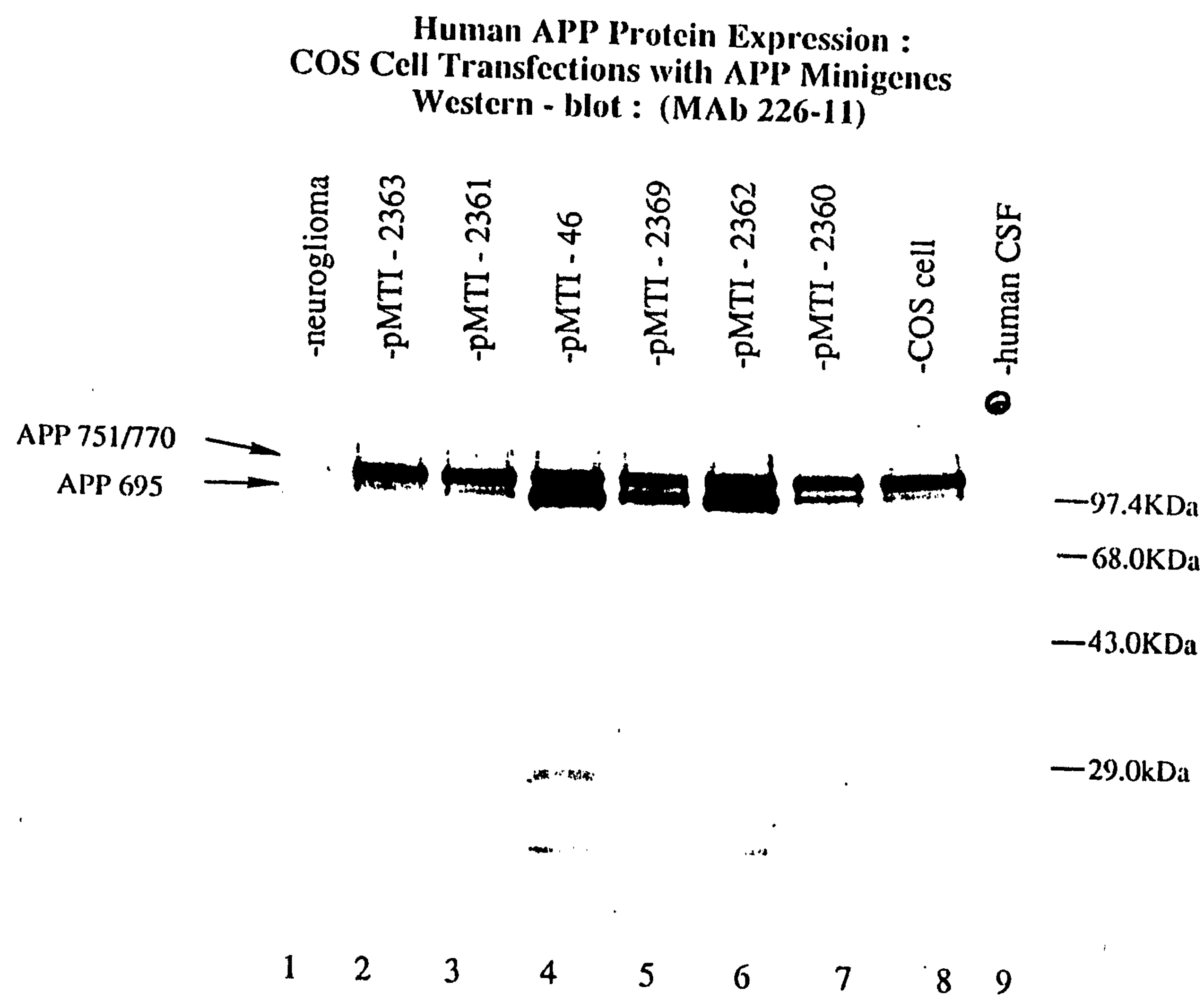
Human APP Protein Expression :
COS Cell Transfections with APP Minigenes
Western - blot : (MAb 226-11)
-neuroglioma
-pMTI - 2363
-pMTI - 2361
-pMTI - 46
-pMTI - 2369
-pMTI - 2362
-pMTI - 2360
-COS cell
-human CSF
APP 751/770
APP 695
97.4KDa
68.0KDa
43.0KDa
29.0kDa
1
2
3
4
5
6
7
8
9

FIG. 29

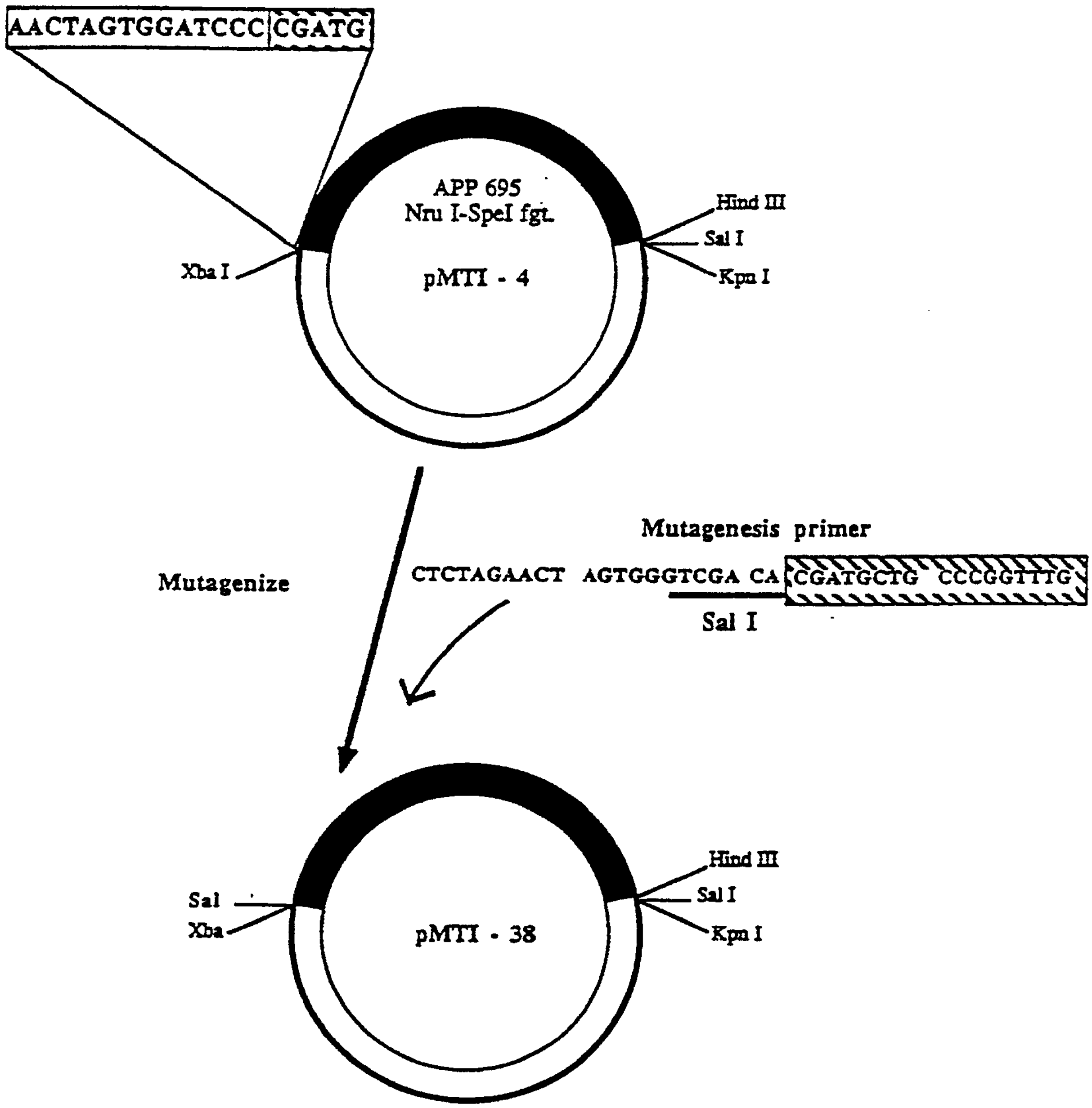
AACTAGTGGATCCC CGATG
APP 695
Nru I-SpeI fgt.
pMTI - 4
Xba I
Hind III
Sal I
Kpn I
Mutagenize
Mutagenesis primer
CTCTAGAACT AGTGGGTCGA CA CGATGCTG CCCGGTTTG
Sal I
Sal
Xba
pMTI - 38
Hind III
Sal I
Kpn I

FIG. 30a

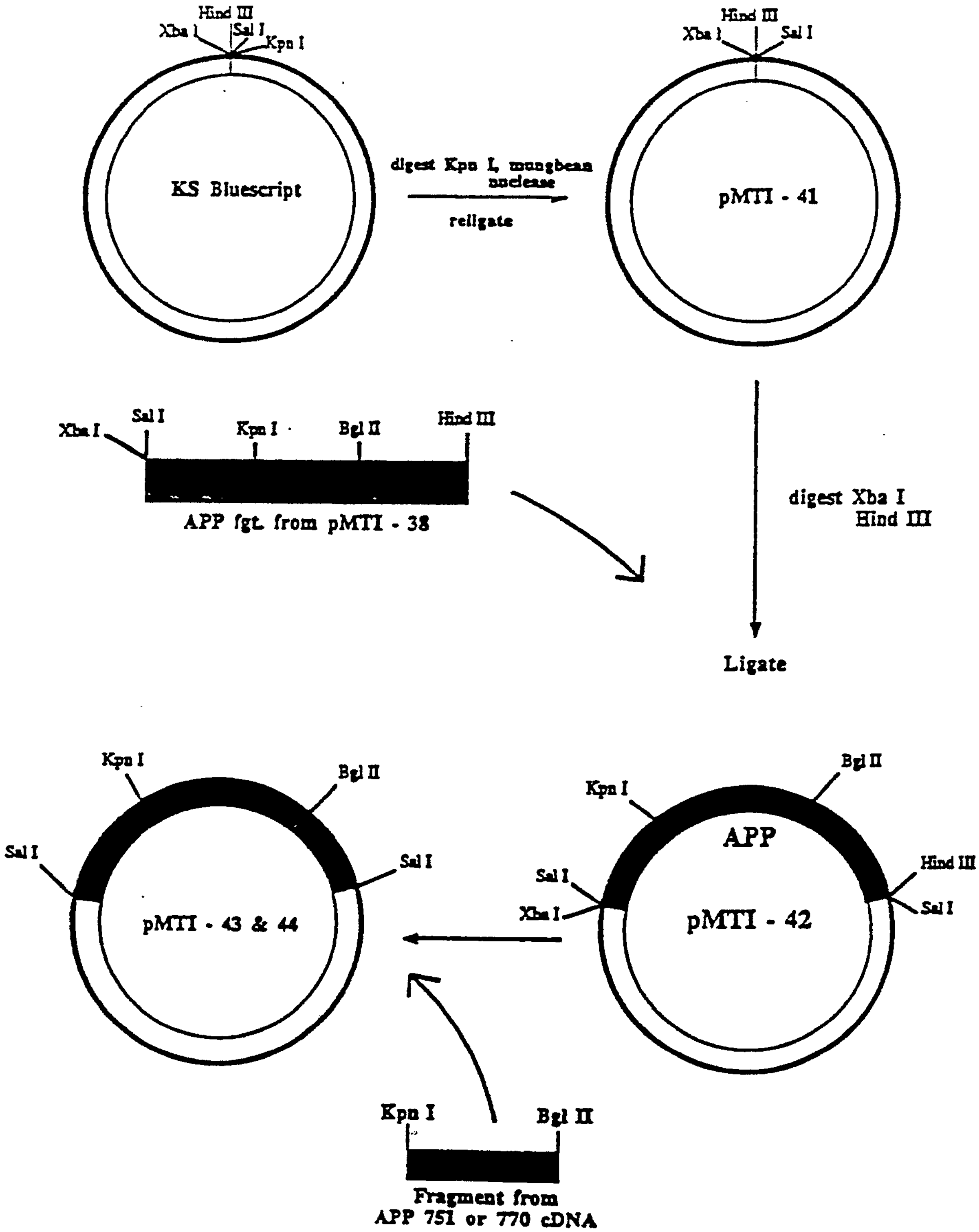
Hind III
Xba I
Sal I
Kpn I
KS Bluescript
digest Kpn I, mungbean nuclease
religate
Hind III
Xba I
Sal I
pMTI - 41
Xba I
Sal I
Kpn I
Bgl II
Hind III
APP fgt. from pMTI - 38
digest Xba I
Hind III
Ligate
Kpn I
Bgl II
Sal I
Sal I
pMTI - 43 & 44
Kpn I
Bgl II
APP
Hind III
Sal I
Xba I
Sal I
pMTI - 42
Kpn I
Bgl II
Fragment from
APP 751 or 770 cDNA

FIG. 30b

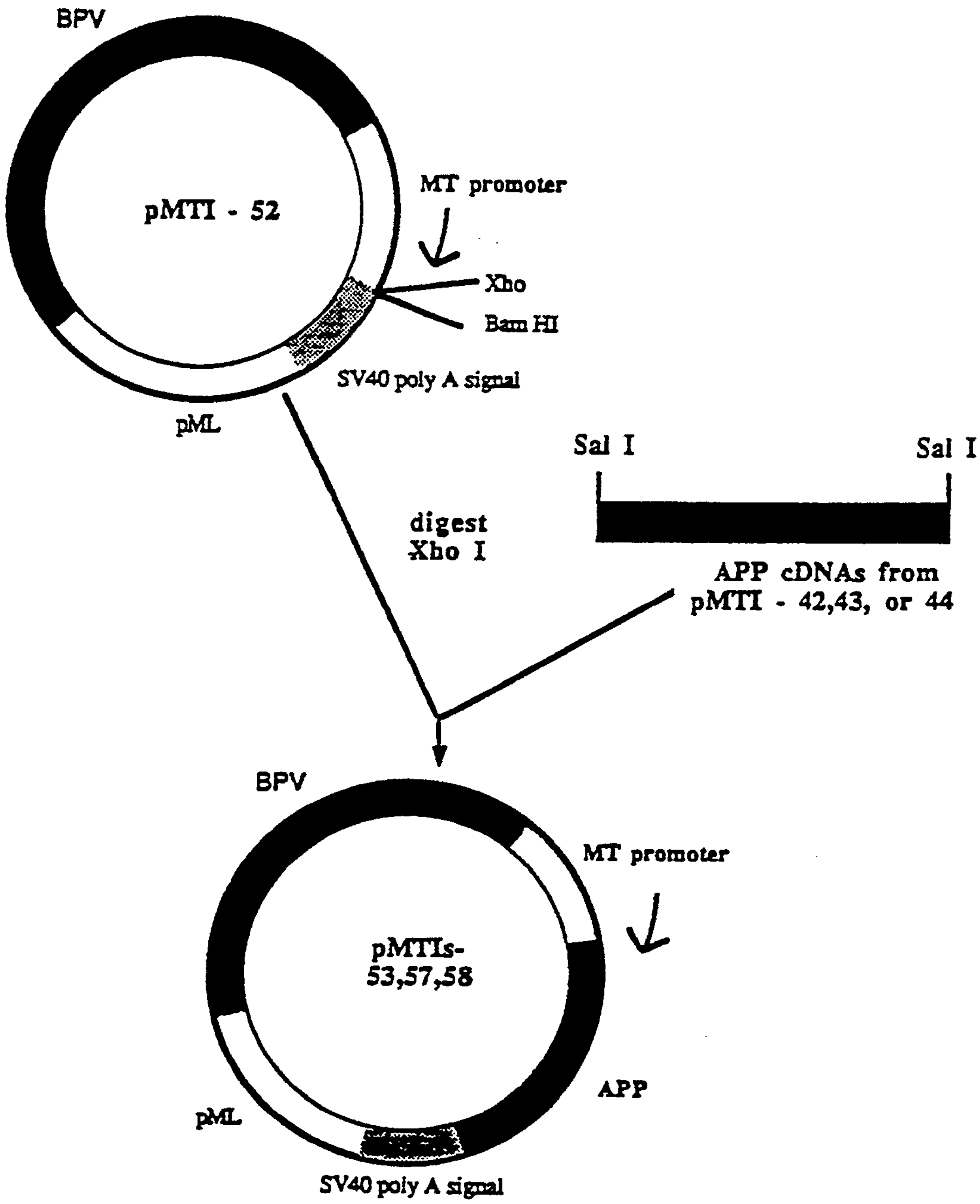
BPV
pMTI - 52
MT promoter
Xho
Bam HI
SV40 poly A signal
pML
digest
Xho I
Sal I
Sal I
APP cDNAs from
pMTI - 42,43, or 44
BPV
MT promoter
pMTIs-
53,57,58
APP
pML
SV40 poly A signal

FIG. 31

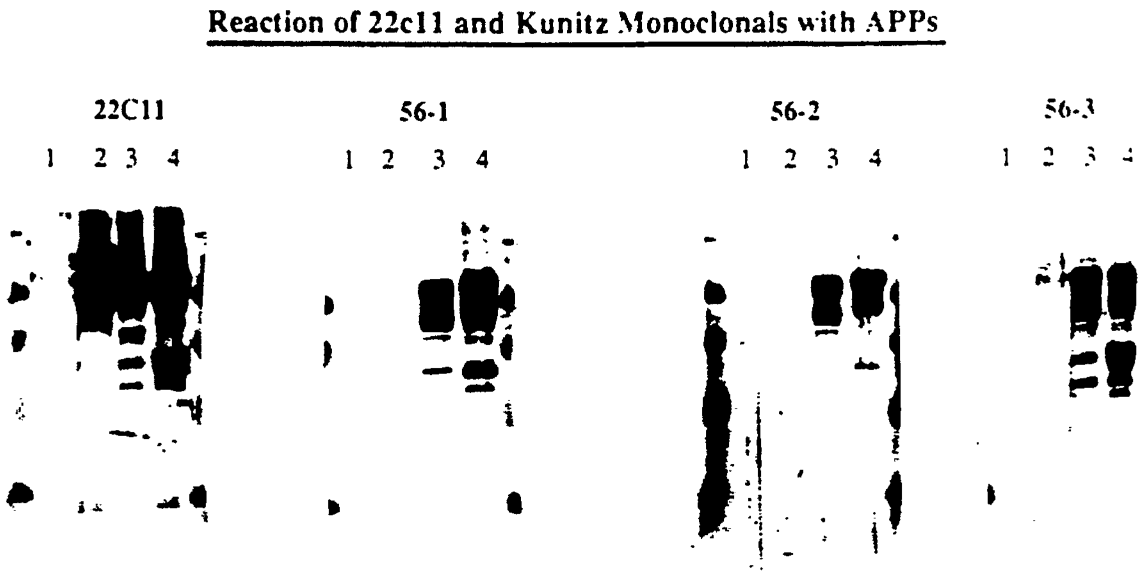


Lane 1 - C127 control; Lane 2, BPV - 695; Lane 3, BPV - 751; Lane 4, BPV - 770.

FIG. 32

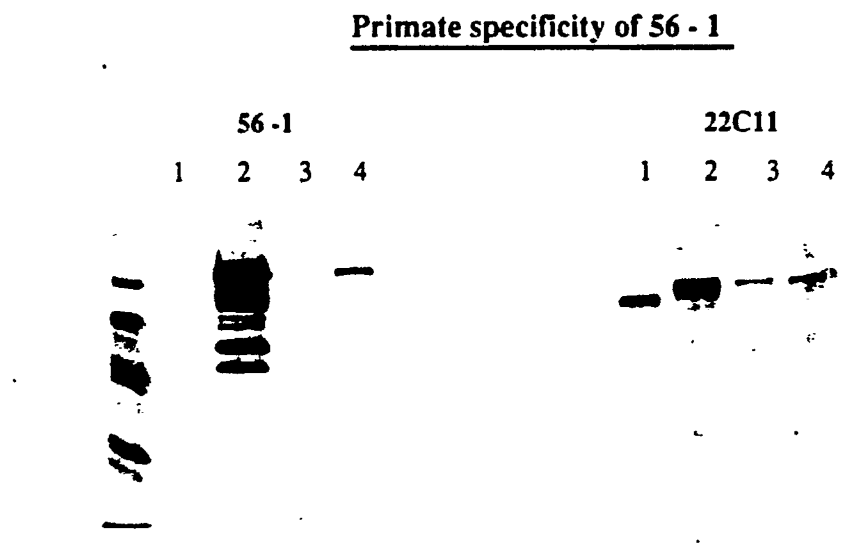


Lane 1 - BPV - 695; Lane 2, BPV - 751; Lane 3, L - cells; Lane 4, cos cells.

FIG. 33

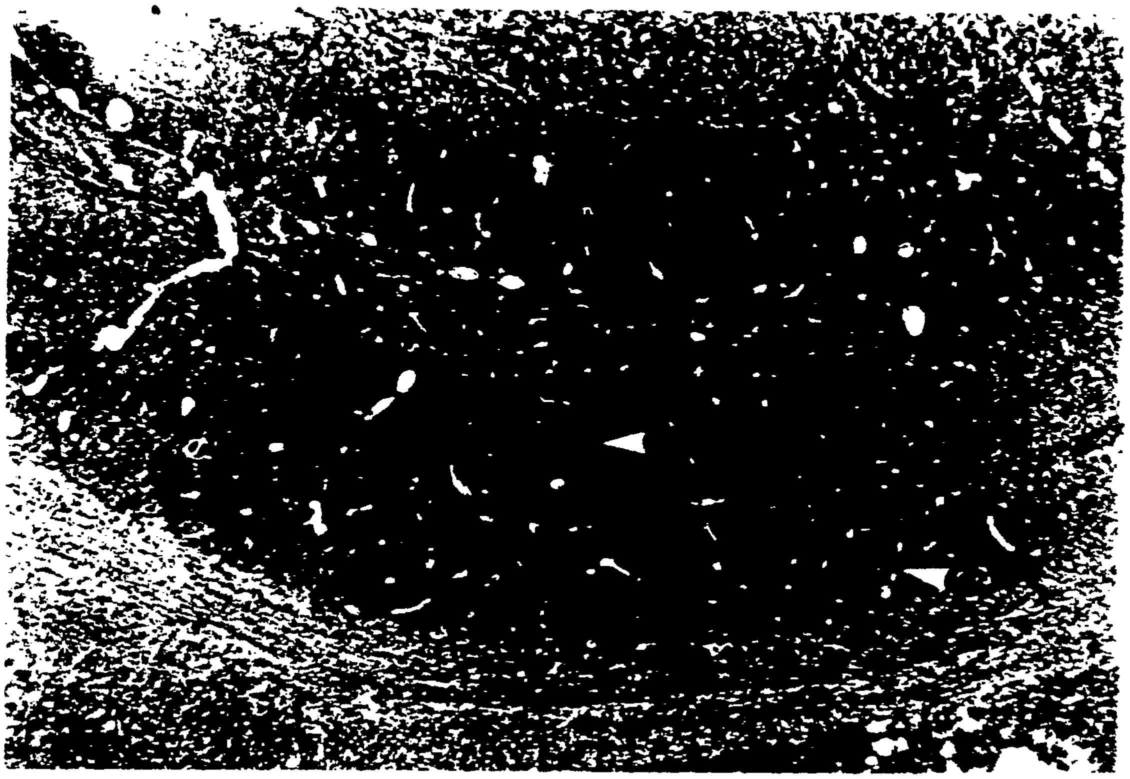

FIG. 34

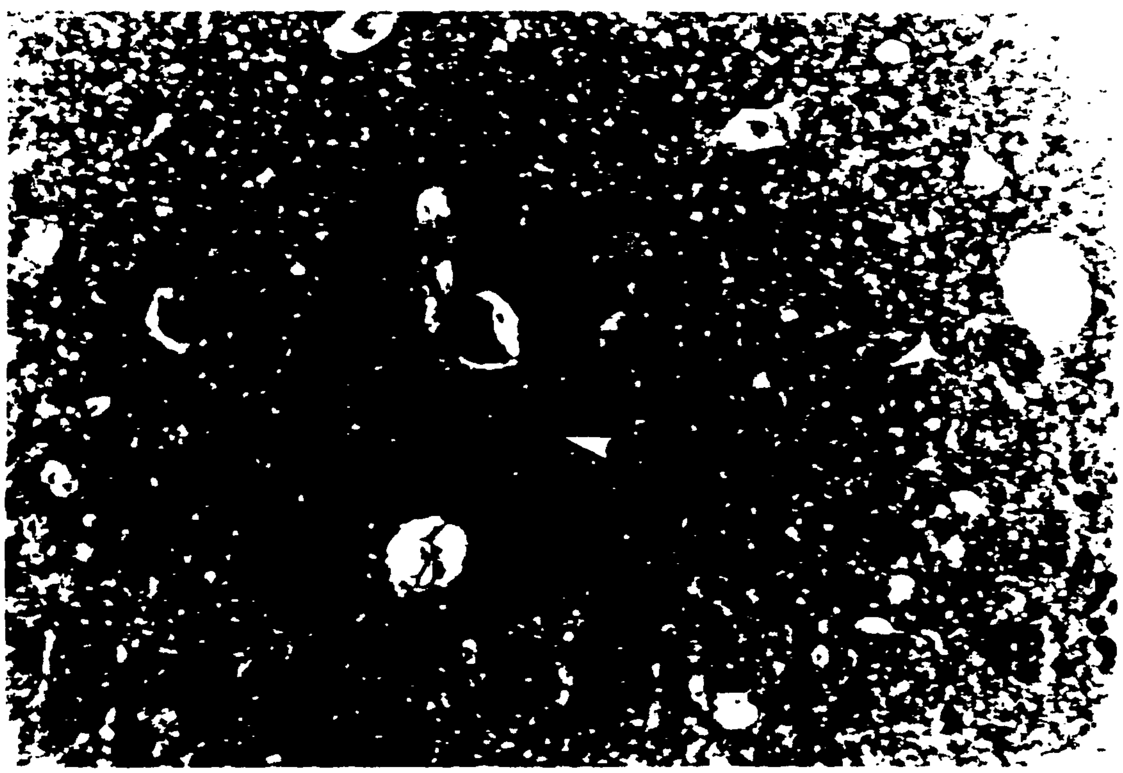

FIG. 35

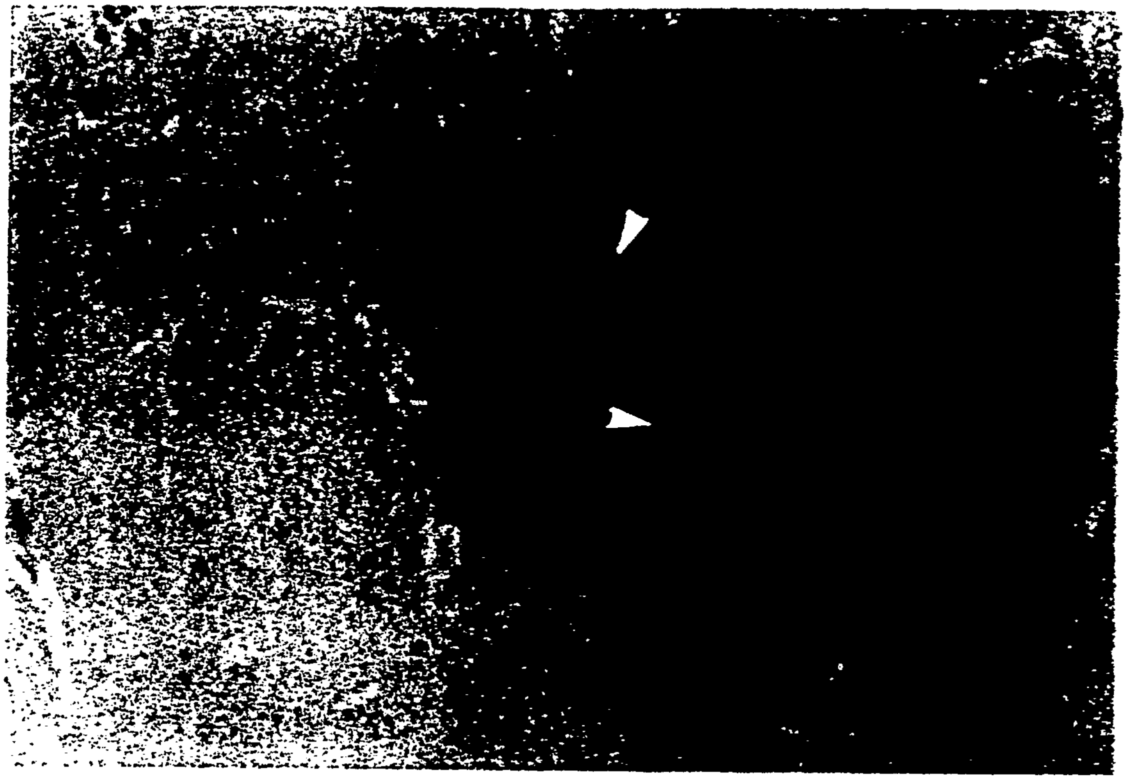

FIG. 36

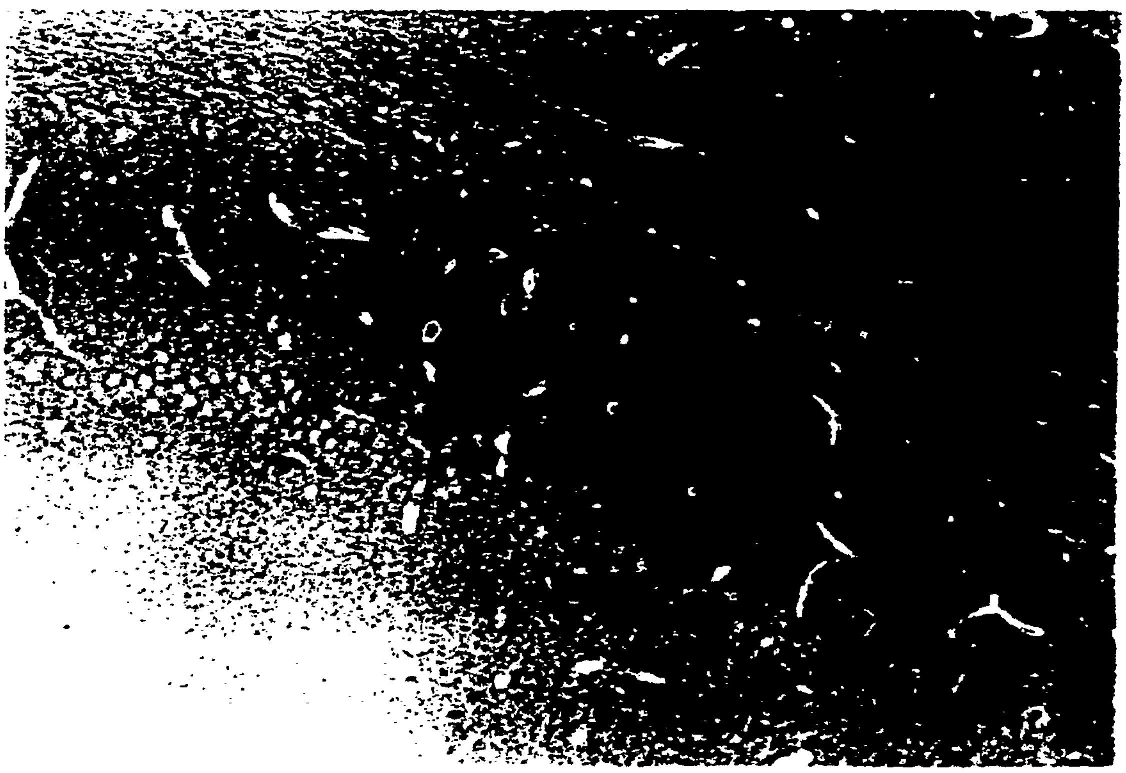

FIG. 37

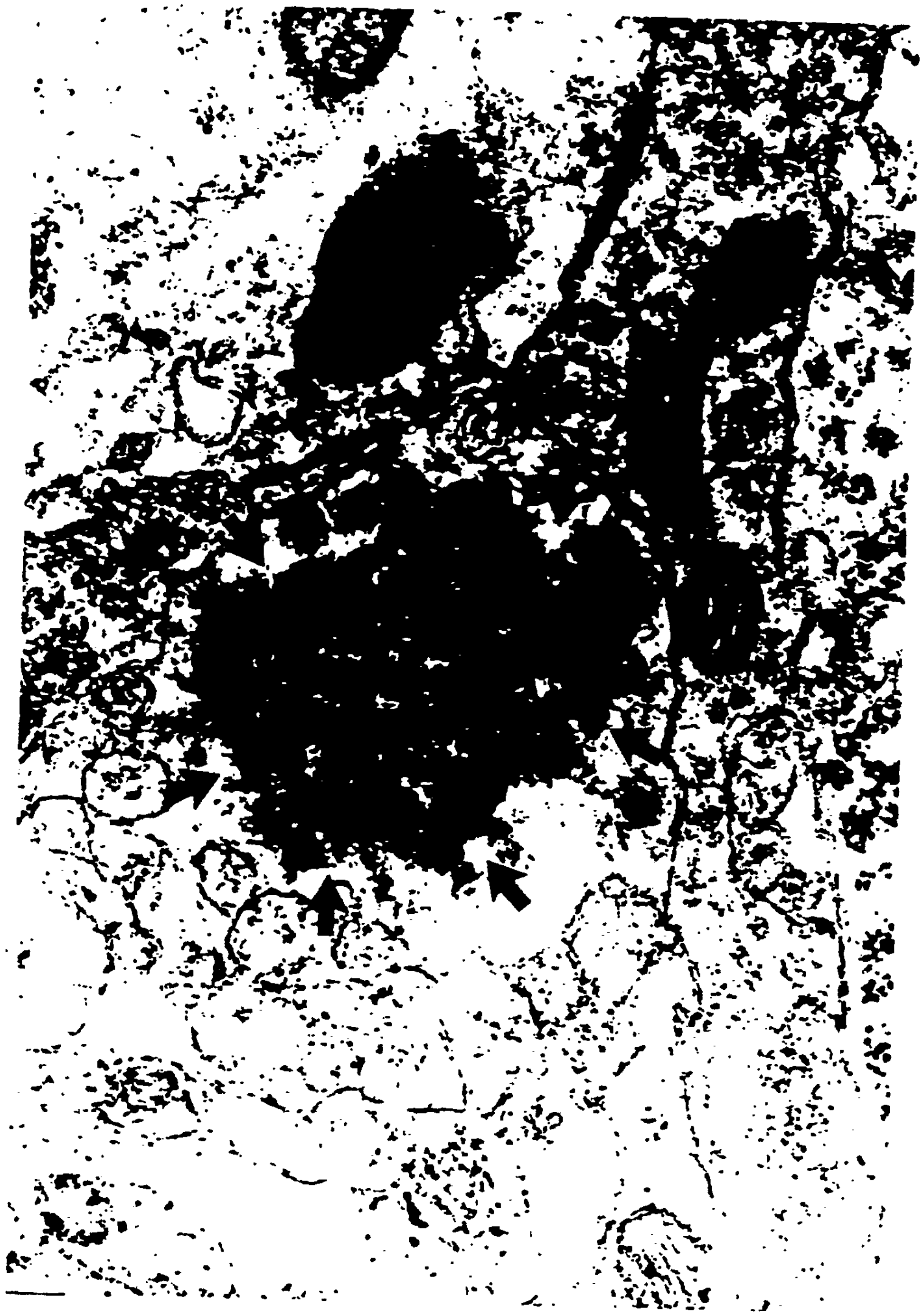

FIG. 38a

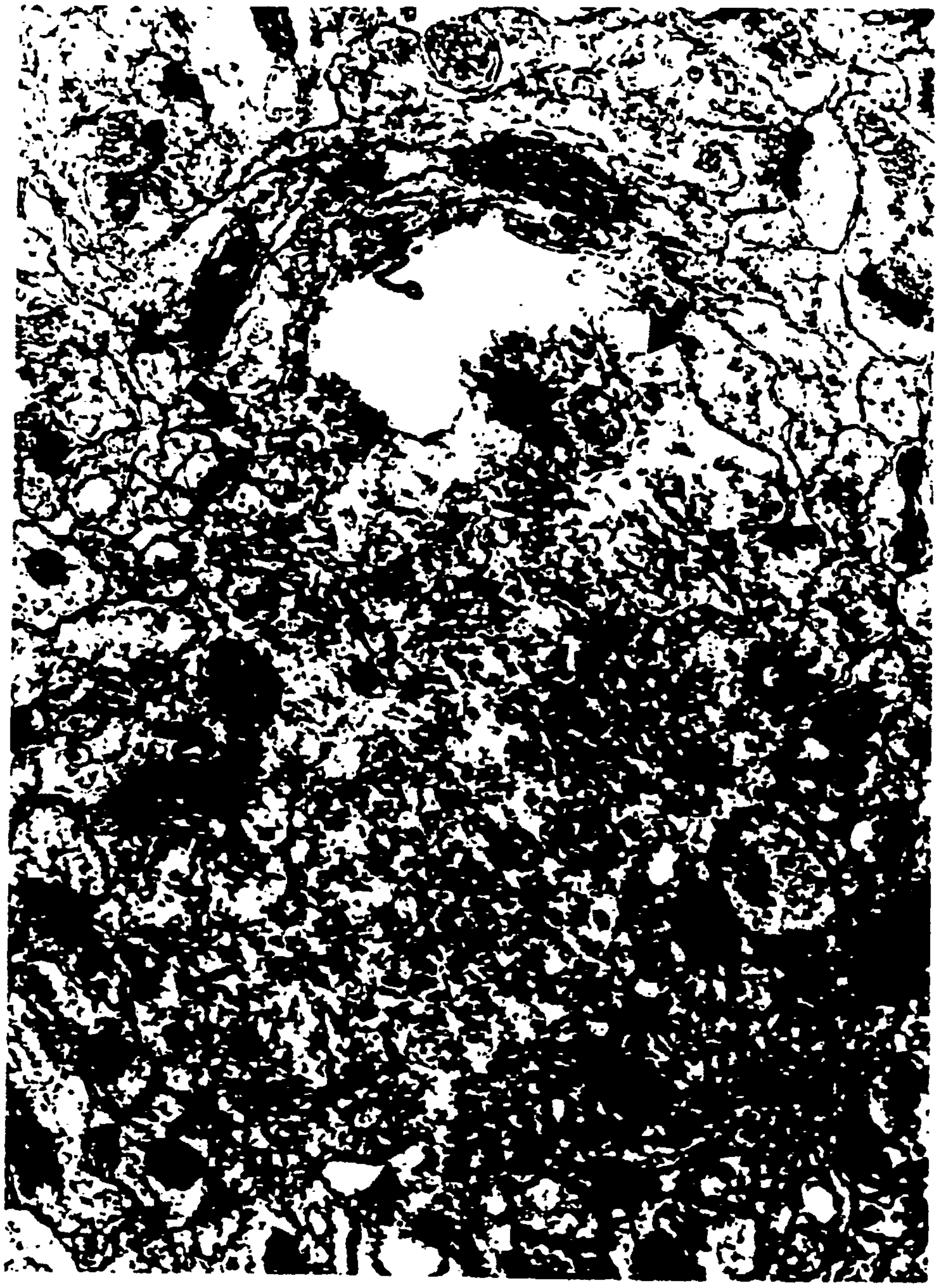

FIG. 38b

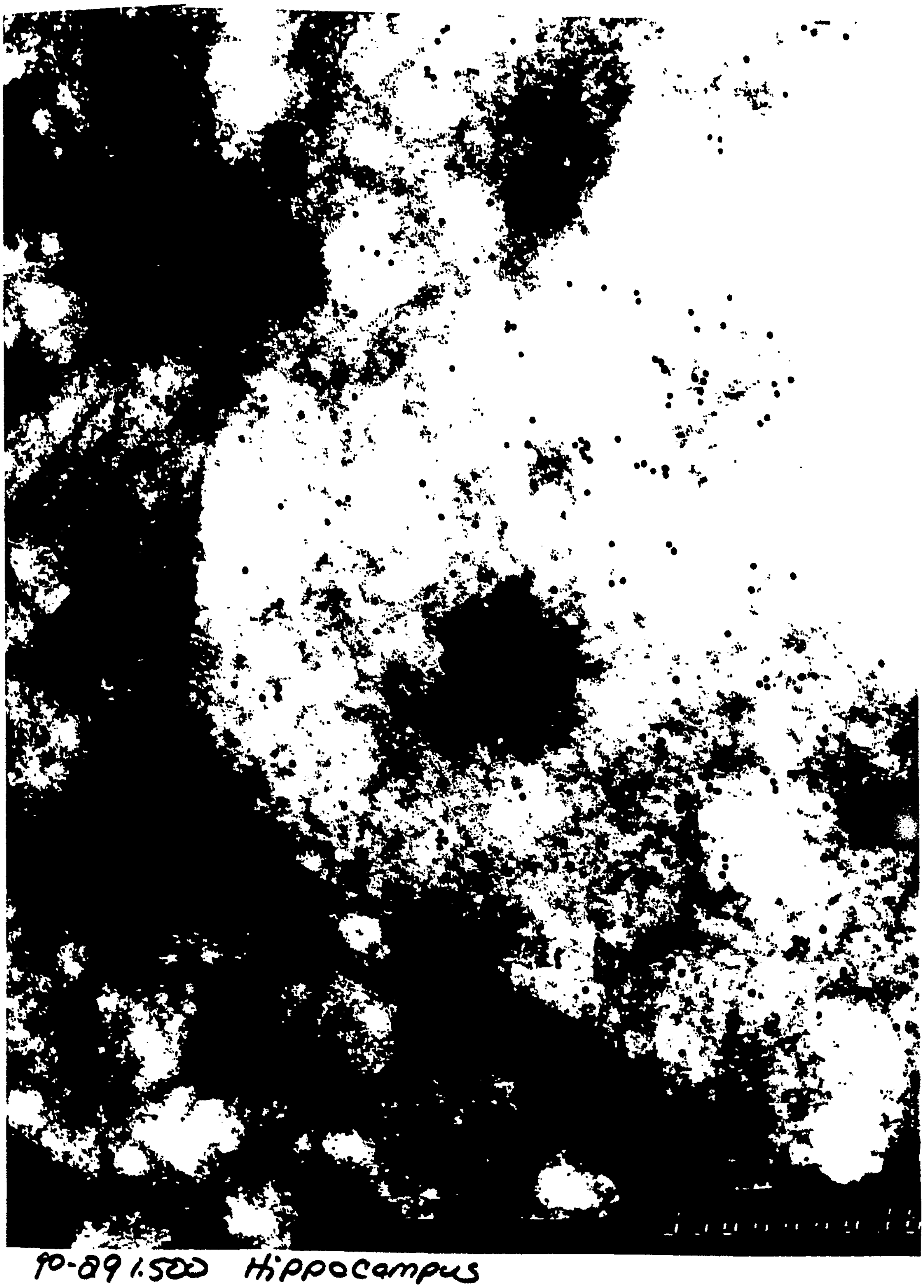

90-89 1.500 Hippocampus

FIG. 39

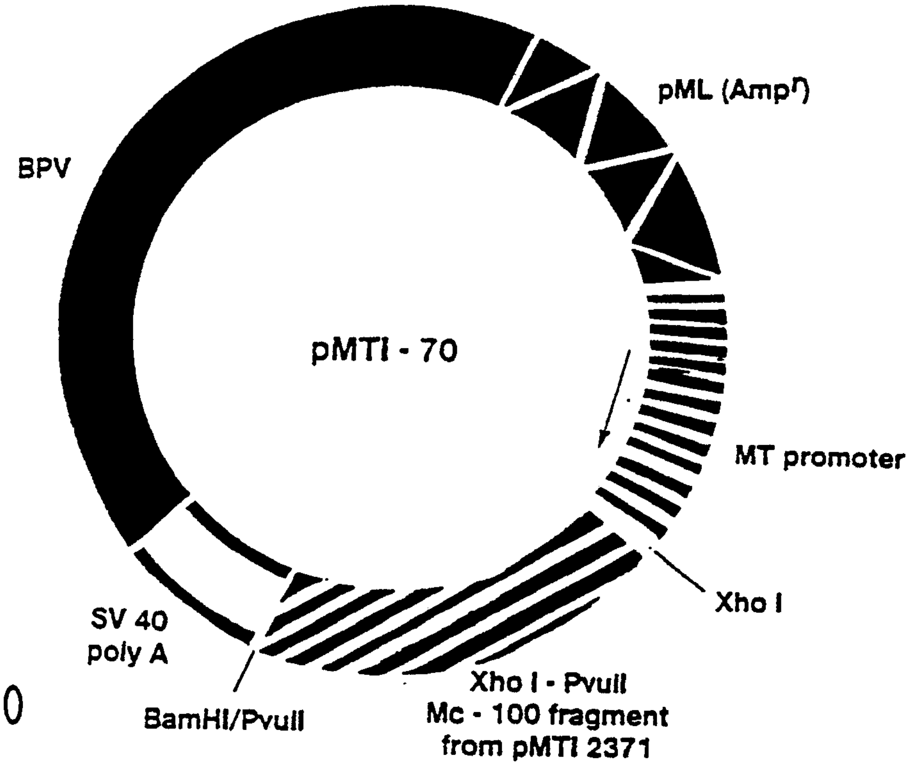
pML (Amp^r)
BPV
pMTI - 70
MT promoter
Xho I
SV 40
poly A
BamHI/PvuII
Xho I - PvuII
Mc - 100 fragment
from pMTI 2371

FIG.40

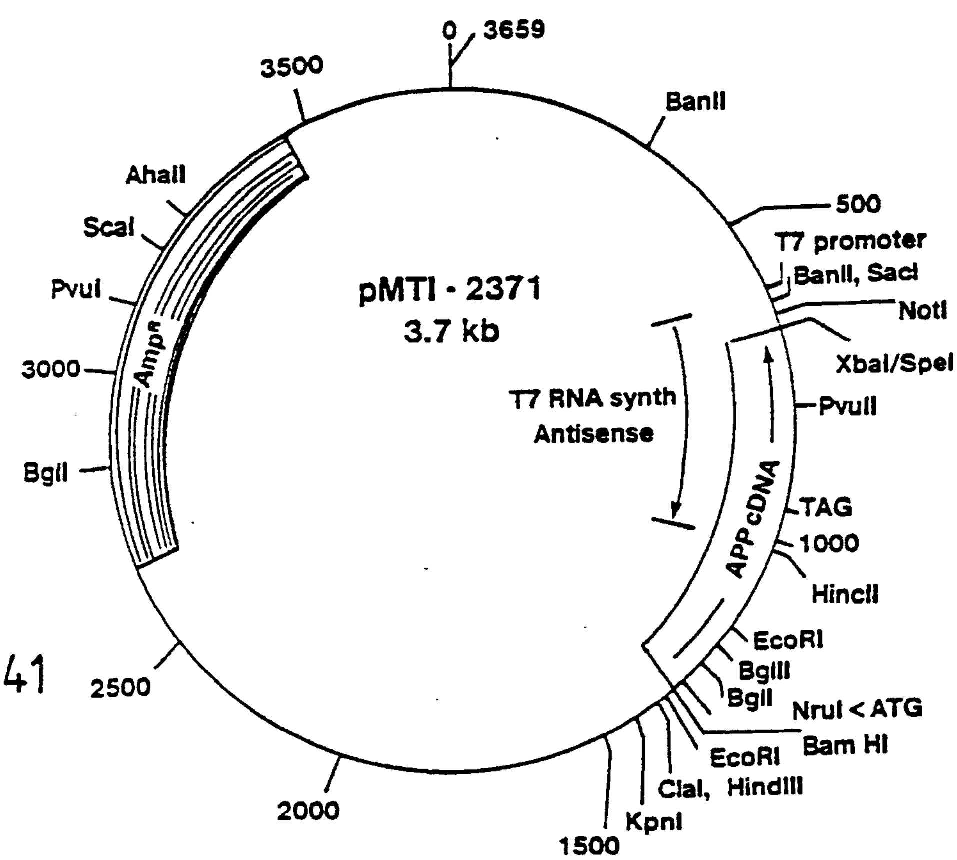
0
3659
3500
BanII
AhaII
500
ScaI
T7 promoter
BanII, SacI
PvuI
pMTI - 2371
3.7 kb
NotI
XbaI/SpeI
3000
Amp^R
T7 RNA synth
Antisense
PvuII
BglI
APP cDNA
TAG
1000
HincII
EcoRI
BglII
BglI
NruI < ATG
2500
Bam HI
EcoRI
ClaI,
HindIII
KpnI
2000
1500

FIG.41

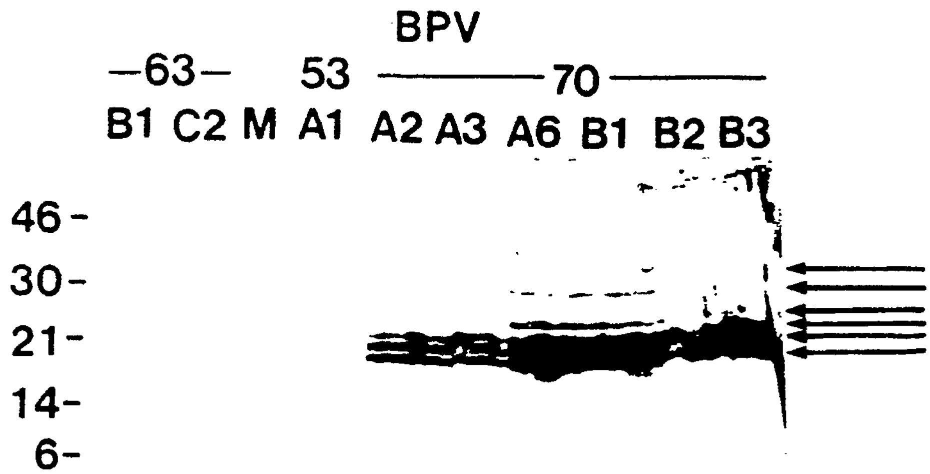
BPV
—63—
53
70
B1
C2
M
A1
A2
A3
A6
B1
B2
B3
46-
30-
21-
14-
6-

FIG.42

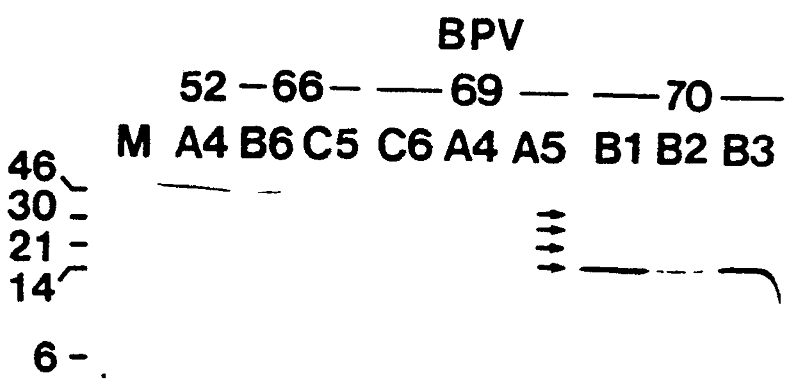
BPV
52
—66—
69
—70—
M
A4
B6
C5
C6
A4
A5
B1
B2
B3
46
30
21
14
6

FIG. 43

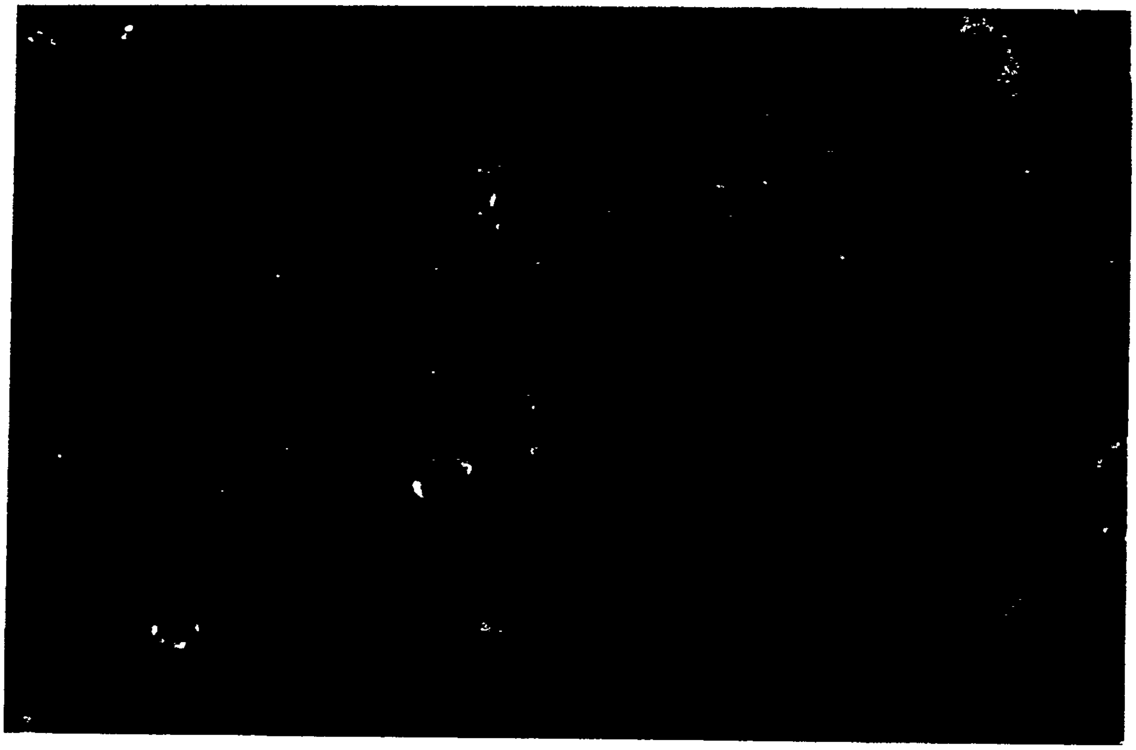

FIG.44a

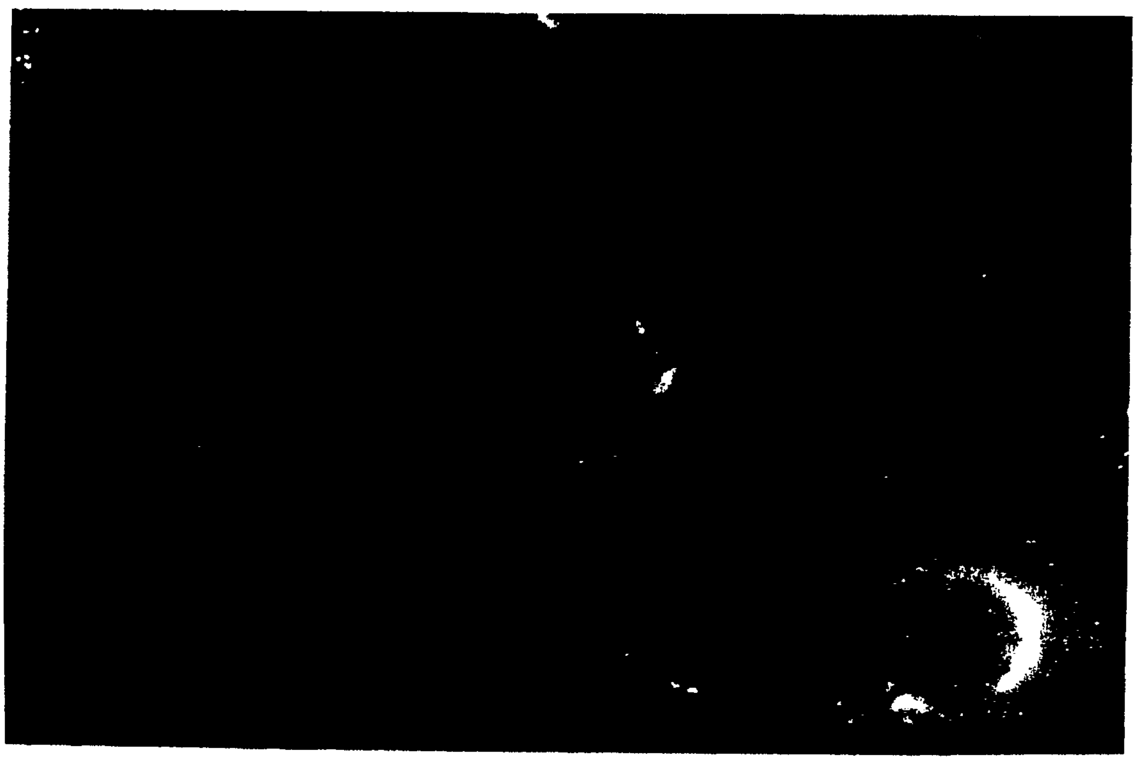

FIG.44b

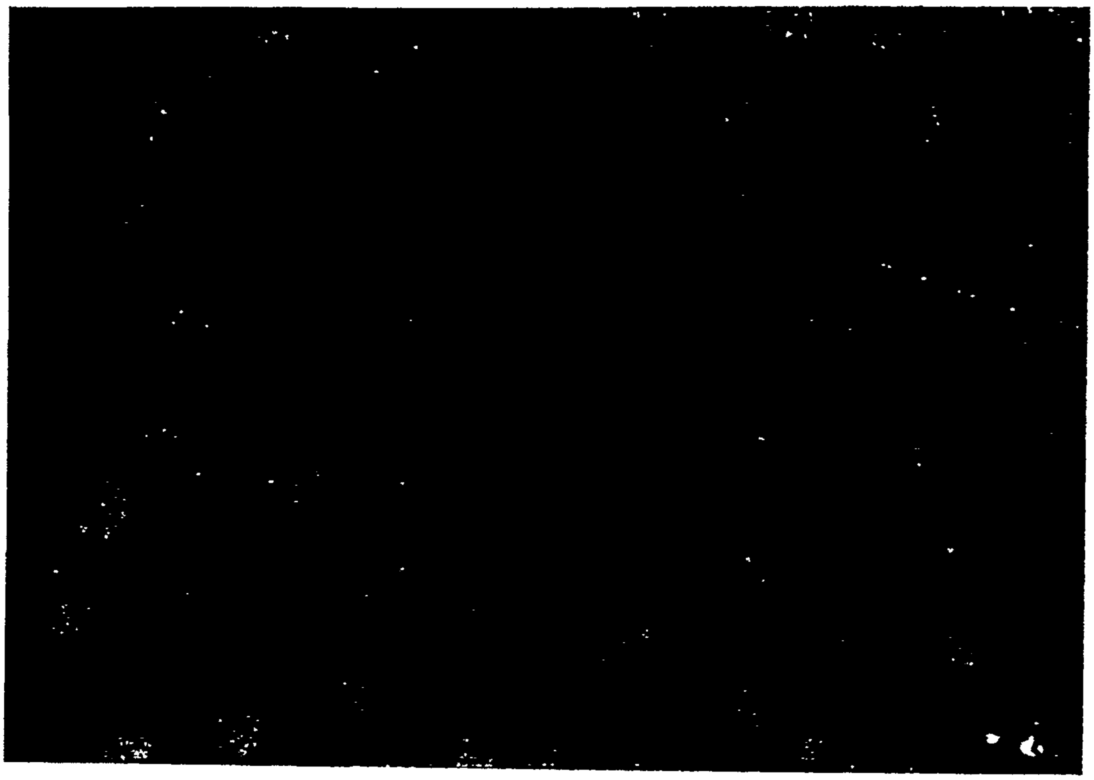

FIG.44c

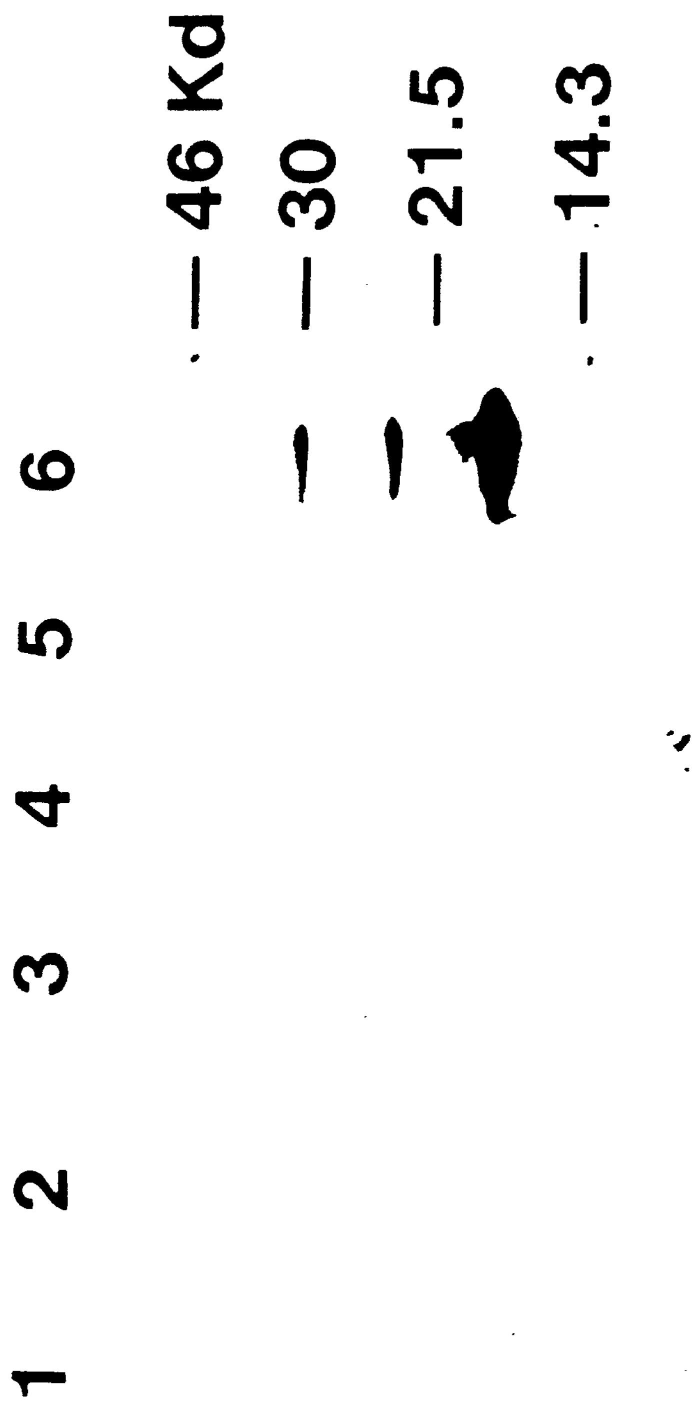


FIG.45

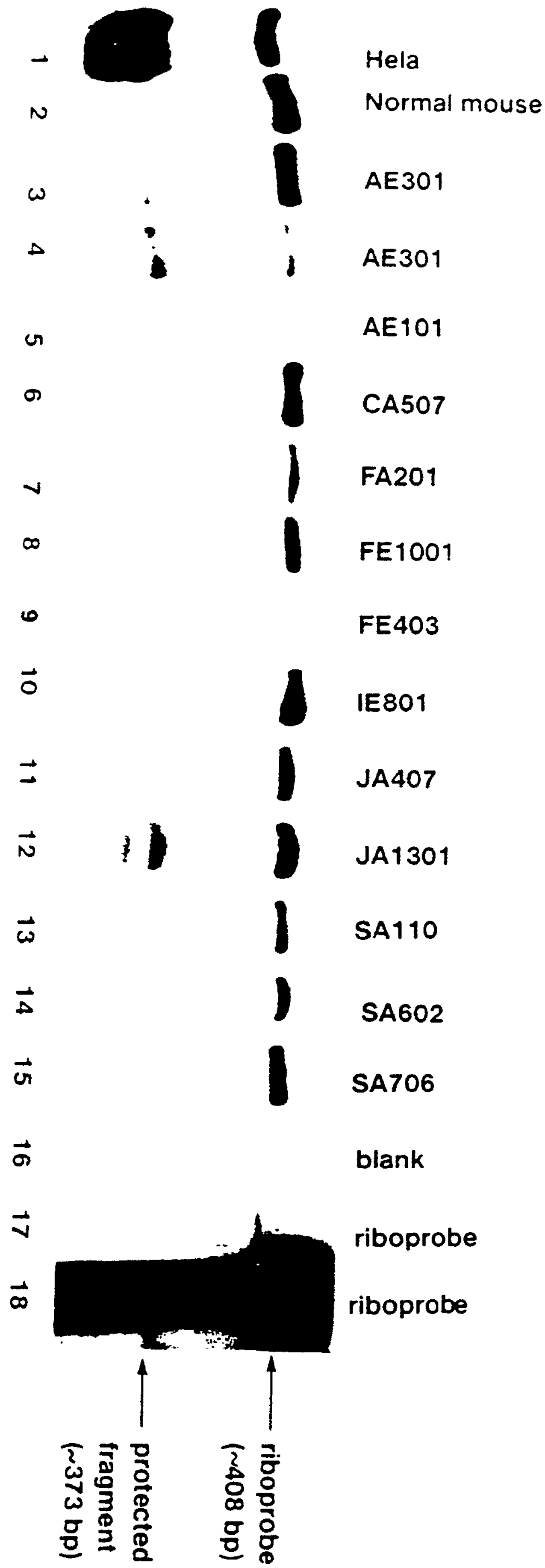

1 Hela
2 Normal mouse
3 AE301
4 AE301
5 AE101
6 CA507
7 FA201
8 FE1001
9 FE403
10 IE801
11 JA407
12 JA1301
13 SA110
14 SA602
15 SA706
16 blank
17 riboprobe
18 riboprobe
protected fragment (~373 bp)
riboprobe (~408 bp)


FIG.46

European Patent Office

# EUROPEAN SEARCH REPORT

Application Number

**EP 91 10 5332**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 304 013 (ASAHI KASEI KOGYO K.K.) * whole document; in particular page 5, line 55 - page 6, line 1; page 15, lines 30-36 * | 1-10 | C 12 N 15/00<br>C 12 N 15/12 |
| X | WO-A-8 906 693 (THE MCLEAN HOSPITAL CORP. et al.) * whole document * | 1-10 | |
| X | WO-A-8 906 689 (THE MCLEAN HOSPITAL CORP. et al.) * whole document * | 1-10 | |
| X | J. NEUROPATHOLOGY AND EXP. NEUROLOGY vol. 48, no. 3, May 1989, page 379; B.D. TRAPP et al.: "Expression of the human amyloid precursor protein gene in the CNS of transgenic mice" * abstract * | 1-10 | |
| X | J. CELLULAR BIOCHEM. SUPPLEMENT vol. 0, no. 13, part B, 1989, page 185; D.O. WIRAK et al.: "Expression of the human beta-amyloid precursor gene in transgenic mice" * abstract * | 1-4,8,9 | |
| X | BEHAVIOR GENETICS vol. 19, no. 6, November 1989, pages 771,772; M.L. OSTER-GRANITE et al.: "Recent studies of mice transgenic for the gene encoding amyloid precursor protein" * abstract * | 1,2,4,8,9 | **TECHNICAL FIELDS SEARCHED (Int. Cl.5)**<br>C 12 N 15/00<br>C 12 N 15/12<br>C 07 K 15/00 |
| X | BIOCHEMICAL AND BIOPHYSICAL RESEARCH COMMUNICATIONS vol. 159, no. 1, 28 February 1989, pages 297-304; G. LA FAUCI et al.: "Characterization of the 5'-end region and the first two exons of the beta-protein precursor gene" * whole article * | 1,2,5 | |

-/-

The present search report has been drawn up for all claims

| Place of search | Date of completion of search | Examiner |
|---|---|---|
| Berlin | 25 June 91 | JULIA P. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document

European
Patent Office

# EUROPEAN SEARCH REPORT

Application Number

**EP 91 10 5332**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | THE EMBO JOURNAL vol. 7, no. 9, 1988, pages 2807-2813, Oxford, GB; J.M. SALBAUM et al.: "The promoter of Alzheimer's disease amyloid A4 precursor gene" * whole article * | 1,2,5 | |
| X | J. CELLULAR BIOCHEM. SUPPLEMENT vol. 0, no. 13, part C, 1989, page 145; A.J. UNTERBECK et al.: "Tissue specificities and developmental patterns of human beta-amyloid gene expression" * abstract * | 1,2,4,8,9 | |
| P,X | DATABASE WPIL/DERWENT abstract no. 91-089452 (13), 1991, Derwent Publications Ltd., London, GB; & JP - A - 3004794 (MOLECULAR THERAPEUT.) 10.01.1991 * abstract * | 1,2,4-6,8,9 | |
| P,X | EP-A-0 375 406 (THE TRUSTEES OF THE UNIVERSITY OF PENNSYLVANIA) * abstract; page 12, lines 25-40; page 15, lines 7-25; claim 29 * | 1-4,7-10 | TECHNICAL FIELDS SEARCHED (Int. Cl.5) |
| P,X | WO-A-9 009 441 (THE GENERAL HOSPITAL CORP.) * whole document; in particular page 5, line 18; page 20, lines 21-23 * | 1,3-7 | |
| A | WO-A-8 703 904 (BOEHRINGER MANNHEIM GMBH) * whole document * | 3,7 | |

-/-

The present search report has been drawn up for all claims

| Place of search | Date of completion of search | Examiner |
|---|---|---|
| Berlin | 25 June 91 | JULIA P. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document

European Patent Office

# EUROPEAN SEARCH REPORT

Application Number

EP 91 10 5332

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | NEUROBIOLOGY OF AGING vol. 11, no. 3, 1990, page 326; J. BEER et al.: "Transgenic mice and Alzheimer's disease" * abstract * | 1,4,8,9 | |
| P,X | J. CELLULAR BIOCHEM. SUPPLEMENT vol. 0, no. 14, part F, 1990, page 66; N. FOX et al.: "Differential expression of a human amyloid beta protein-cat fusion gene in transgenic mice" * abstract * | 1,5 | |
| | | | TECHNICAL FIELDS SEARCHED (Int. Cl.5) |

The present search report has been drawn up for all claims

| Place of search | Date of completion of search | Examiner |
|---|---|---|
| Berlin | 25 June 91 | JULIA P. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document